# EUROPEAN PATENT APPLICATION

(11) **EP 4 685 141 A1**
(43) Date of publication of application: **28.01.2026**
(21) Application number: 24774205.9
(22) Date of filing: 21.03.2024
(51) Int. Cl.: C07D 401/14, C07D 417/14, C07D 413/14, C07D 471/08, A61K 31/506, A61K 31/4545, A61K 35/00, A61P 35/02, A61P 37/00, A61P 29/00

(54) **ISOINDOLINE DERIVATIVE, BENZOINDOLE DERIVATIVE AND USE THEREOF**

(30) Priority: 21.03.2023 CN 202310278026; 14.12.2023 CN 202311716807
(71) Applicant: Shanghai Helioson Pharmaceutical Co., Ltd., Shanghai 201114 (CN)
(72) Inventor: ZHAO, Liwen, Shanghai 201114 (CN); WU, Jinhua, Shanghai 201114 (CN); ZHOU, Gang, Shanghai 201114 (CN)
(74) Representative: Cabinet Beau de Loménie
(86) International application number: PCT/CN2024/082990
(87) International publication number: WO 2024/193641

(57) **Abstract**

The present application belongs to the technical field of biomedicine, and specifically relates to a compound as shown in formula (I), a pharmaceutically acceptable salt thereof, a deuterated compound thereof, a stereoisomer thereof, a tautomer thereof, or a mixture thereof, and the use of the compound as an immunomodulator for IKZF1 and IKZF3 double targets.

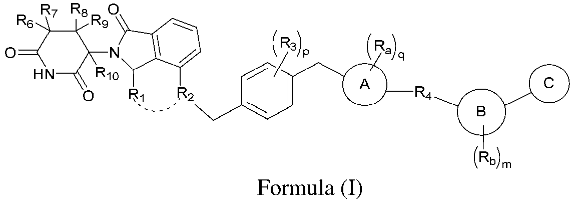

## Description

### FIELD OF TECHNOLOGY

The present application belongs to the field of pharmaceutical chemistry, and relates to an immunomodulator for dual targets of IKZF1 and IKZF3 and use thereof.

### BACKGROUND

Multiple myeloma (MM) is a hematological malignancy caused by abnormal proliferation of plasmocytes (Terpos E, Politou M, Rahemtulla. The role of markers of bone remodeling in multiple myeloma. Blood Reviews 2005;19: 125-142). It is mainly characterized by significantly active proliferation of the plasmocytes in the bone marrow, accompanied by excessive secretion of monoclonal immunoglobulins. Only a few patients have non-secretory MM that does not produce M protein. Currently, significant breakthroughs have been made in treatment of the MM, and several drugs such as proteasome inhibitors (bortezomib), immunomodulatory drugs (lenalidomide and pomalidomide), CD38 monoclonal antibodies (daratumumab and elotuzumab), and histone deacetylase (HDAC) inhibitors (panobinostat) have been approved by FDA for treatment of the MM (Taylor D. Multiple Myeloma Therapy in 2015: "An Extraordinary Moment in Oncology". Am Health Drug Benefits 2016; 9: 1-12). Among them, molecular glue-type immunomodulators are currently a focus of drug development.

Molecular glue degraders are a class of small molecules that can induce and stabilize binding of E3 ubiquitin ligase to target proteins to lead to ubiquitination and proteasomal degradation of the target proteins. Compared with bifunctional molecular degraders (protac), molecular glues have lower binding affinity to the target proteins, but can bind to the E3 ubiquitin ligase and mediate its connection with the target proteins through the same. Therefore, the molecular glues can degrade target proteins without certain binding pockets. In addition, target proteins of the molecular glues themselves need to have non-functional weak affinity to the E3 ligase. The molecular glue can bind to the gap between the interfaces to enhance the connection of these two, resulting in strong functional interactions. Currently, the molecular glues for the MM, such as lenalidomide and pomalidomide, all act by recruiting CRBN ubiquitin ligase to promote the binding of CRBN to zinc finger protein transcription factor IKZF1/3, thereby leading to ubiquitination and degradation of IKZF1/3 (Gao SB. Novel immunomodulatory drugs and neo-substrates. Biomarker Res 2020; 8:2). Since IKZF1/3 can promote proliferation of MM cells by regulating expression of proto-oncogenes IRF4 and c-myc, molecular-glue-induced degradation of IKZF1/3 can significantly inhibit growth of the MM cells. Although lenalidomide has good efficacy for MM, Drug resistance usually emerges after 2 years of medication use on most patients. Pomalidomide is a preferred drug for MM patients with drug resistance to lenalidomide. Studies have shown the combination of pomalidomide with dexamethasone can inhibit growth of lenalidomide-resistant tumor cells and induce apoptosis. Although pomalidomide showed improved efficacy in patients with drug resistance to lenalidomide, about 1/3 of the patients might have point mutations of a CRBN gene after continuous administration, resulting in the drug resistance to pomalidomide (Gooding et al. Multiple cereblon genetic changes are associated with acquired resistance to lenalidomide or pomalidomide in multiple myeloma. Blood 2021; 14: 232-237). Moreover, although lenalidomide and pomalidomide can inhibit proliferation of MM, incidence rates of G3/G4 neutropenia can reach up to 35% and approximately 50%, respectively (Ghobrial et al. J Support Oncol. 2003; 1: 194-205; Thakurta et al. Oncotarget 2021; 12: 1555-63). A molecular glue CC-92480 developed by Bristol-Myers Squibb Company in a clinical phase I trial has an objective response rate (ORR) of 54.4%, but has a neutropenia incidence rate of up to 53% (Richardson PG. First-in-human phase I study of the novel CELMoD agent CC-92480 combined with dexamethasone (DEX) in patients (pts) with relapsed/refractory multiple myeloma (RRMM). J Clinical Oncol 2020; 38: 8500). Therefore, development of molecular glues that are effective for the patients with drug resistance to lenalidomide and pomalidomide, and simultaneously with lower hematotoxicity, especially neutrophil toxicity, is of great significance for treatment of MM.

### SUMMARY OF THE INVENTION

One objective of the present application is to provide a compound of formula (I), a pharmaceutically acceptable salt thereof, a deuterated compound thereof, a stereoisomer thereof, a tautomer thereof, or a mixture thereof: 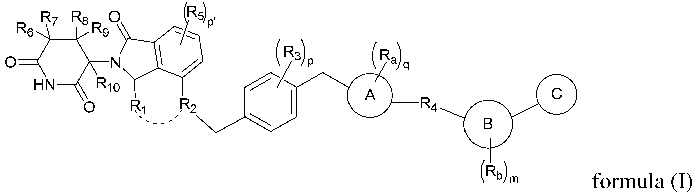 wherein,
ring A is selected from 3-10 membered monocyclic heterocyclyl, 6-13 membered bridged heterocyclyl, or 6-13 membered fused heterocyclyl;
ring B is selected from 5-6 membered heteroaryl or 5-6 membered heterocyclyl;
ring C is selected from optionally substituted 5-10 membered heteroaryl or optionally substituted 5-10 membered heterocyclyl;
a dashed line between R₁ and R₂ represents connection or disconnection;
when R₁ and R₂ are disconnected, R₁ is selected from H or D, and R₂ is selected from O, NH, or S;
when R₁ and R₂ are connected, R₁ and R₂, together with the carbon atoms to which they are attached, form an optionally substituted 5-6 membered aromatic ring;
R₃ is selected from H, D, or halogen;
Rₐ is each independently selected from H, C₁-C₆ alkyl, cyano, C₁-C₆ haloalkyl, or C₃-C₁₀ cycloalkyl, wherein the C₁-C₆ alkyl and C₃-C₁₀ cycloalkyl are each optionally substituted with one or more of H, halogen, hydroxy, or cyano; or two Rₐ attached to the same carbon atom, together with the carbon atom to which they are attached, form C₃-C₆ cycloalkyl or 4-6 membered heterocyclyl;
R₄ is selected from a single bond, -C(O)-, -NR₁₁C(O)-, -NR₁₁-, -CR₁₁R₁₂, -CR₁₁R₁₂-(4-6 membered heterocyclyl)-, 4-6 membered heterocyclyl, -(C₃-C₆ cycloalkyl)-NR₁₁C(O)-, or -O-(C₅-C₆ cycloalkyl)-NR₁₁C(O)-, wherein the heterocyclyl and cycloalkyl are each optionally substituted with one or more selected from H, halogen, C₁-C₆ alkyl, or C₁-C₆ haloalkyl; or R₄ and any one Rₐ, together with the atoms to which they are attached, form C₃-C₈ cycloalkyl or 4-6 membered heterocyclyl;
R₅ is selected from H, C₁-C₆ alkyl, C₁-C₆ haloalkyl, or halogen;
R₆, R₇, R₈, R₉, and R₁₀ are each independently selected from H or D;
R₁₁ and R₁₂ are each independently selected from H or C₁-C₆ alkyl; or R₁₁ and R₁₂, together with the carbon atoms to which they are attached, form C₃-C₈ cycloalkyl or 4-6 membered heterocyclyl;
R_{b} is selected from H, halogen, C₁-C₆ alkyl, C₁-C₃ haloalkyl, C₃-C₆ cycloalkyl, C₁-C₆ alkoxy, optionally substituted amino, or cyano; or two adjacent R_{b}, together with the carbon atoms to which they are attached respectively, form C₃-C₆ cycloalkyl, 4-6 membered heterocyclyl, 5-6 membered heteroaryl, or 5-6 membered aryl;
m is selected from 0, 1, 2, or 3;
p is selected from 0, 1, 2, 3, or 4;
p' is selected from 0, 1, 2, or 3; and
q is selected from 0, 1, 2, 3, or 4.

In some embodiments, R₁ and R₂ are disconnected, R₁ is selected from H or D, and R₂ is selected from O or S. In some embodiments, R₁ and R₂ are disconnected, R₁ is selected from H or D, and R₂ is selected from O; and preferably, R₁ is selected from H, and R₂ is selected from O.

In some embodiments, R₁ and R₂ are connected, and R₁ and R₂, together with the carbon atoms to which they are attached, form a benzene ring.

In some embodiments, the ring A is selected from 4-8 membered monocyclic heterocyclyl or 6-10 membered bridged heterocyclyl containing 1-3 heteroatoms selected from N, O, or S (preferably N or O, and more preferably N). In some embodiments, the ring A is selected from 4-8 membered monocyclic heterocycloalkyl or 6-10 membered bridged heterocycloalkyl containing 1-3 (e.g., 1-2) heteroatoms selected from N, O, or S (preferably N or O, and more preferably N).

In some embodiments, the ring A is selected from 4-6 membered monocyclic heterocyclyl or 6-8 membered bridged heterocyclyl. In some embodiments, the ring A is selected from 4-6 membered monocyclic heterocycloalkyl or 6-8 membered bridged heterocycloalkyl containing 1-2 heteroatoms selected from N, O, or S (preferably N or O, and more preferably N).

In some preferred embodiments, the ring A is selected from 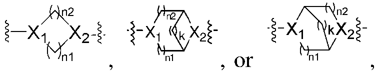 wherein at least one of X₁ and X₂ is N, and the other one is optionally C or N; and n1, n2, and k are each independently selected from 1 or 2; and more preferably, the ring A is selected from 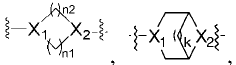 or

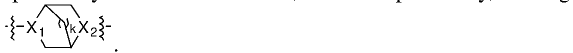

In some preferred embodiments, the ring A is selected from

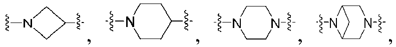

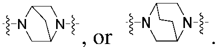

In some more preferred embodiments, the ring A is selected from 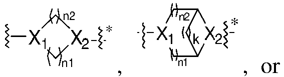 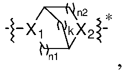 wherein at least one of X₁ and X₂ is N, and the other one is optionally C or N; n1, n2, and k are each independently selected from 1 or 2; and * represents a connection site with R₄; and more preferably, the ring A is selected from

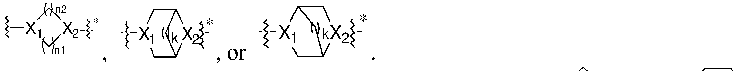

In some more preferred embodiments, the ring A is selected from 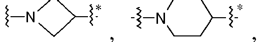 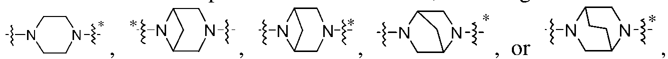 wherein * represents a connection site with R₄.

In some embodiments, Rₐ is each independently selected from H, C₁-C₆ alkyl, C₁-C₆ haloalkyl, or C₃-C₆ cycloalkyl, wherein the C₁-C₆ alkyl is optionally substituted with one or more of H, halogen, hydroxy, or cyano; or two Rₐ attached to the same carbon atom, together with the carbon atom to which they are attached, form C₃-C₆ cycloalkyl.

In some preferred embodiments, Rₐ is each independently selected from H, C₁-C₃ alkyl, or C₁-C₃ haloalkyl, wherein the C₁-C₃ alkyl or C₁-C₃ haloalkyl is each optionally substituted with one or more of H, hydroxy, or cyano; and preferably, Rₐ is selected from H, -CH₂OH, -CH₂CH₂OH, -CN, or -CH₂CN.

In some embodiments, Rₐ is each independently selected from H or C₁-C₆ alkyl (preferably C₁-C₅ alkyl, C₁-C₄ alkyl, or C₁-C₃ alkyl), wherein the C₁-C₆ alkyl is optionally substituted with one or more of hydroxy or cyano; or two Rₐ attached to the same carbon atom, together with the carbon atom to which they are attached, form C₃-C₆ cycloalkyl (preferably C₃-C₅ cycloalkyl or C₃-C₄ cycloalkyl).

In some embodiments, q is selected from 0, 1, or 2.

In some preferred embodiments, two Rₐ attached to the same carbon atom, together with the atom to which they are attached, form C₃-C₆ cycloalkyl; and preferably, two Rₐ attached to the same carbon atom, together with the atom to which they are attached, form cyclopropyl.

In some preferred embodiments, structural unit 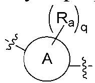 is selected from 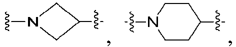 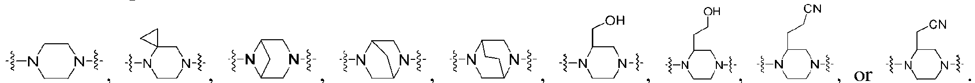 In some preferred embodiments, structural unit 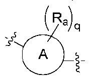 is selected from

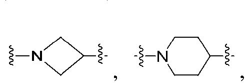

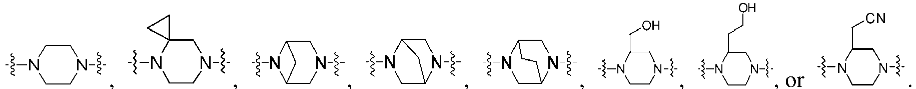

Preferably, 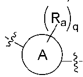 is selected from 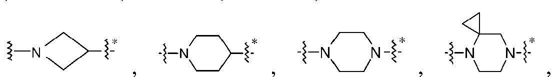 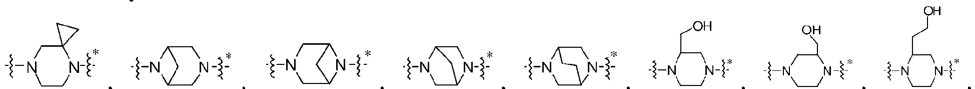 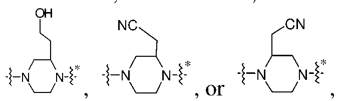 wherein * represents a connection site with R₄.

In some embodiments, the ring B is selected from 5-6 membered heteroaryl containing 1-3 heteroatoms selected from N, O, or S (preferably N or O, and more preferably N). In some embodiments, the ring B is selected from 6-membered heteroaryl containing 1-3 (preferably 1-2) heteroatoms selected from N or O (preferably N). In some embodiments, the ring B is selected from 6-membered heteroaryl, for example, pyridinyl, pyrimidinyl, pyrazinyl, and pyridazinyl.

In some preferred embodiments, the ring B is selected from 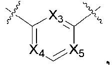 wherein X₃, X₄, and X₅ are each independently selected from C or N; and at least one of X₃, X₄, and X₅ is N; and preferably, 1-2 of X₃, X₄, and X₅ are N.

In some preferred embodiments, the ring B is selected from

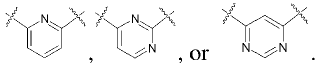

In some more preferred embodiments, the ring B is selected from 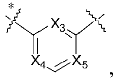 wherein * represents a connection site with R₄; X₃, X₄, and X₅ are each independently selected from C or N; and at least one of X₃, X₄, and X₅ is N; preferably, 1-2 of X₃, X₄, and X₅ are N; and further preferably, the ring B is selected from 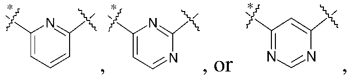 wherein * represents a connection site with R₄.

In some embodiments, R_{b} is each independently selected from H, halogen, C₁-C₆ alkyl, C₁-C₃ haloalkyl, C₃-C₆ cycloalkyl, C₁-C₆ alkoxy, optionally substituted amino, or cyano; and m is selected from 0, 1, 2, or 3. In some embodiments, R_{b} is each independently selected from H, halogen, C₁-C₆ alkyl (preferably C₁-C₅ alkyl, C₁-C₄ alkyl, or C₁-C₃ alkyl), C₁-C₃ haloalkyl, C₃-C₆ cycloalkyl, amino, or cyano; and m is selected from 0, 1, or 2 (preferably, m is 0 or 1). In some embodiments, R_{b} is each independently selected from H, halogen, C₁-C₆ alkyl (preferably C₁-C₅ alkyl, C₁-C₄ alkyl, or C₁-C₃ alkyl), or C₁-C₃ haloalkyl; and m is selected from 0, 1, or 2 (preferably, m is 0 or 1).

In some preferred embodiments, R_{b} is each independently selected from H, F, Cl, Br, methyl, ethyl, n-propyl, isopropyl, cyclopropyl, fluoromethyl, fluoroethyl, fluoropropyl, chloromethyl, chloroethyl, chloropropyl, bromomethyl, bromoethyl, or bromopropyl (e.g., trifluoromethyl, trichloromethyl, or tribromomethyl); and m is selected from 0 or 1. In some preferred embodiments, R_{b} is each independently selected from H, F, Cl, methyl, ethyl, isopropyl, cyclopropyl, or trifluoromethyl; and m is selected from 0 or 1. In some preferred embodiments, R_{b} is each independently selected from H, F, Cl, methyl, ethyl, propyl, or trifluoromethyl; and m is selected from 0 or 1.

In some embodiments, the ring C is selected from optionally substituted 5-6 membered heteroaryl or optionally substituted 5-6 membered heterocyclyl. In some embodiments, the ring C is selected from 5-6 membered heteroaryl or 5-6 membered heterocyclyl containing 1-3 heteroatoms selected from N, O, or S, wherein the 5-6 membered heteroaryl or 5-6 membered heterocyclyl is optionally substituted with one or more selected from H, C₁-C₆ alkyl, C₃-C₆ cycloalkyl, C₁-C₃ haloalkyl, halogen, -C(O)R₁₃, or cyano, wherein R₁₃ is selected from H, NH₂, C₁-C₃ alkyl, C₃-C₆ cycloalkyl, or C₁-C₃ haloalkyl.

In some embodiments, the ring C is selected from 5-6 membered heteroaryl or 5-6 membered heterocyclyl containing 1-3 heteroatoms selected from N, O, or S, wherein the 5-6 membered heteroaryl or 5-6 membered heterocyclyl is optionally substituted with one or more (e.g., 1-3, 1-2, or 1) of C₁-C₆ alkyl (preferably C₁-C₅ alkyl, C₁-C₄ alkyl, or C₁-C₃ alkyl), C₃-C₆ cycloalkyl (preferably C₃-C₅ cycloalkyl or C₃-C₄ cycloalkyl), C₁-C₃ haloalkyl (e.g., C₁-C₃ fluoroalkyl, C₁-C₃ chloroalkyl, or C₁-C₃ bromoalkyl), halogen (preferably F, Cl, or Br), -C(O)R₁₃, or cyano, wherein R₁₃ is selected from H, NH₂, or C₁-C₃ alkyl (preferably, R₁₃ is selected from NH₂).

In some preferred embodiments, the ring C is selected from 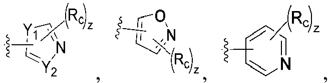 or 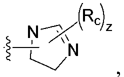 wherein R_{c} is independently selected from H, C₁-C₆ alkyl, C₃-C₆ cycloalkyl, C₁-C₃ haloalkyl, halogen, -C(O)R₁₃, or cyano; z is selected from 0, 1, 2, 3, or 4; Y₁ is selected from S or N; Y₂ is selected from C or N; and R₁₃ is selected from H, NH₂, C₁-C₃ alkyl, C₃-C₆ cycloalkyl, or C₁-C₃ haloalkyl;
preferably, R_{c} is independently selected from H, methyl, ethyl, propyl, cyclopropyl, cyclobutyl, fluoromethyl, fluoroethyl, fluoropropyl, chloromethyl, chloroethyl, chloropropyl, bromomethyl, bromoethyl, bromopropyl, F, Cl, Br, -C(O)NH₂, or cyano; and further preferably, R_{c} is independently selected from H, methyl, ethyl, isopropyl, cyclopropyl, trifluoromethyl, F, Cl, -C(O)NH₂, or cyano; and z is selected from 0, 1, or 2.

In some preferred embodiments, the ring C is selected from 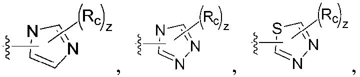 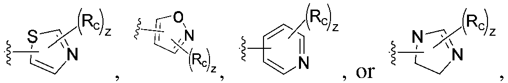 wherein R_{c} is independently selected from H, C₁-C₆ alkyl, C₃-C₆ cycloalkyl, C₁-C₃ haloalkyl, halogen, -C(O)R₁₃, or cyano; z is selected from 0, 1, 2, 3, or 4; and R₁₃ is selected from H, NH₂, C₁-C₃ alkyl, C₃-C₆ cycloalkyl, or C₁-C₃ haloalkyl;
preferably, R_{c} is independently selected from H, methyl, ethyl, propyl, cyclopropyl, cyclobutyl, fluoromethyl, fluoroethyl, fluoropropyl, chloromethyl, chloroethyl, chloropropyl, bromomethyl, bromoethyl, bromopropyl, F, Cl, Br, -C(O)NH₂, or cyano; and further preferably, R_{c} is independently selected from H, methyl, ethyl, isopropyl, cyclopropyl, trifluoromethyl, F, Cl, -C(O)NH₂, or cyano; and z is selected from 0, 1, or 2.

In some preferred embodiments, the ring C is selected from 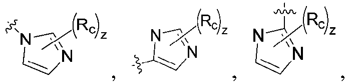 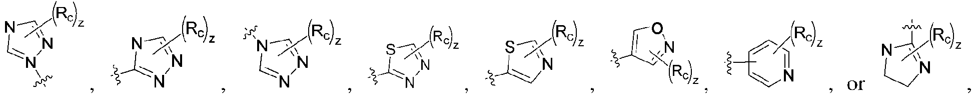 wherein R_{c} is independently selected from H, C₁-C₆ alkyl, C₃-C₆ cycloalkyl, C₁-C₃ haloalkyl, halogen, -C(O)R₁₃, or cyano; z is selected from 0, 1, 2, 3, or 4; and R₁₃ is selected from H, NH₂, C₁-C₃ alkyl, C₃-C₆ cycloalkyl, or C₁-C₃ haloalkyl;
preferably, R_{c} is independently selected from H, methyl, ethyl, propyl, cyclopropyl, cyclobutyl, fluoromethyl, fluoroethyl, fluoropropyl, chloromethyl, chloroethyl, chloropropyl, bromomethyl, bromoethyl, bromopropyl, F, Cl, Br, -C(O)NH₂, or cyano; and further preferably, R_{c} is independently selected from H, methyl, ethyl, isopropyl, cyclopropyl, trifluoromethyl, F, Cl, -C(O)NH₂, or cyano; and z is selected from 0, 1, or 2.

In some embodiments, R₄ is selected from a single bond, -C(O)-, -NR₁₁C(O)-, -NR₁₁-, -CR₁₁R₁₂-, -CR₁₁R₁₂-(4-6 membered heterocycloalkyl)-, 4-6 membered heterocycloalkyl, -(C₃-C₆ cycloalkyl)-NR₁₁C(O)-, or -O-(C₅-C₆ cycloalkyl)-NR₁₁C(O)-, wherein the heterocycloalkyl and cycloalkyl are optionally substituted with one or more selected from H or C₁-C₃ alkyl; the heterocycloalkyl contains 1-3 heteroatoms selected from N, O, or S; and R₁₁ and R₁₂ are each independently selected from H or C₁-C₃ alkyl.

In some embodiments, R₄ is selected from a single bond, -C(O)-, -NHC(O)-, -NH-, -CH₂-(5-6 membered heterocyclyl)-, 5-6 membered heterocyclyl, -(C₅-C₆ cycloalkyl)-NHC(O)-, or -O-(C₅-C₆ cycloalkyl)-NHC(O)-.

In some embodiments, R₄ is selected from a single bond, -C(O)-, -NHC(O)-, -NH-, -CH₂-(5-6 membered heterocycloalkyl)-, 5-6 membered heterocycloalkyl, -(C₅-C₆ cycloalkyl)-NHC(O)-, or -O-(C₅-C₆ cycloalkyl)-NR₁₁C(O)-, wherein the heterocycloalkyl contains 1-3 heteroatoms selected from N, O, or S (preferably, 1-2 heteroatoms selected from N or O (e.g., N)).

In some preferred embodiments, R₄ is selected from a single bond, -C(O)-, -NHC(O)-, -NH-,

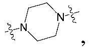

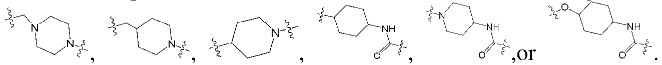

In some preferred embodiments, R₄ is selected from a single bond.

In some embodiments, R₃ is selected from H or D; and preferably, R₃ is H. In some embodiments, p is selected from 0, 1, or 2, and preferably, p is 0.

In some embodiments, R₅ is H. In some embodiments, p' is selected from 0, 1, or 2, and preferably, p' is 0.

In some embodiments, R₆, R₇, R₈, R₉, and R₁₀ are each independently H.

In some embodiments, in the compound of formula (I), the ring A is selected from 4-8 membered monocyclic heterocycloalkyl or 6-10 membered bridged heterocycloalkyl containing 1-3 (e.g., 1-2) heteroatoms selected from N, O, or S (preferably N or O, and more preferably N); and preferably, the ring A is selected from 4-6 membered monocyclic heterocycloalkyl or 6-8 membered bridged heterocycloalkyl containing 1-2 heteroatoms selected from N, O, or S (preferably N or O, and more preferably N);
the ring B is selected from 5-6 membered heteroaryl containing 1-3 heteroatoms selected from N, O, or S (preferably N or O, and more preferably N); and preferably, the ring B is selected from 6-membered heteroaryl containing 1-3 (preferably 1-2) heteroatoms selected from N or O (preferably N);
the ring C is selected from 5-6 membered heteroaryl or 5-6 membered heterocyclyl containing 1-3 heteroatoms selected from N, O, or S, wherein the 5-6 membered heteroaryl or 5-6 membered heterocyclyl is optionally substituted with one or more selected from H, C₁-C₆ alkyl, C₃-C₆ cycloalkyl, C₁-C₃ haloalkyl, halogen, -C(O)R₁₃, or cyano, wherein R₁₃ is selected from H, NH₂, C₁-C₃ alkyl, C₃-C₆ cycloalkyl, or C₁-C₃ haloalkyl; and preferably, the ring C is selected from 5-6 membered heteroaryl or 5-6 membered heterocyclyl containing 1-3 heteroatoms selected from N, O, or S, wherein the 5-6 membered heteroaryl or 5-6 membered heterocyclyl is optionally substituted with one or more selected from C₁-C₆ alkyl, C₃-C₆ cycloalkyl, C₁-C₃ haloalkyl, halogen, -C(O)R₁₃, or cyano, wherein R₁₃ is selected from H, NH₂, or C₁-C₃ alkyl;
the dashed line between R₁ and R₂ represents connection or disconnection;
when R₁ and R₂ are disconnected, R₁ is selected from H or D, and R₂ is selected from O or S; and preferably, R₁ is selected from H, and R₂ is selected from O;
when R₁ and R₂ are connected, R₁ and R₂, together with the carbon atoms to which they are attached, form a benzene ring;
R₃ is selected from H or D; and preferably, R₃ is H;
Rₐ is each independently selected from H, C₁-C₆ alkyl, C₁-C₆ haloalkyl, or C₃-C₆ cycloalkyl, wherein the C₁-C₆ alkyl is optionally substituted with one or more of H, halogen, hydroxy, or cyano; or two Rₐ attached to the same carbon atom, together with the carbon atom to which they are attached, form C₃-C₆ cycloalkyl; and preferably, Rₐ is each independently selected from H, C₁-C₆ alkyl, or cyano, wherein the C₁-C₆ alkyl is optionally substituted with one or more of hydroxy or cyano; or two Rₐ attached to the same carbon atom, together with the carbon atom to which they are attached, form C₃-C₆ cycloalkyl (preferably C₃-C₅ cycloalkyl or C₃-C₄ cycloalkyl);
R₄ is selected from a single bond, -C(O)-, -NR₁₁C(O)-, -NR₁₁-, -CR₁₁R₁₂-, -CR₁₁R₁₂-(4-6 membered heterocycloalkyl)-, 4-6 membered heterocycloalkyl, -(C₃-C₆ cycloalkyl)-NR₁₁C(O)-, or -O-(C₅-C₆ cycloalkyl)-NR₁₁C(O)-, wherein the heterocycloalkyl and cycloalkyl are each optionally substituted with one or more selected from H or C₁-C₃ alkyl; the heterocycloalkyl contains 1-3 heteroatoms selected from N, O, or S; and R₁₁ and R₁₂ are each independently selected from H or C₁-C₃ alkyl;
R₅ is H;
R₆, R₇, R₈, R₉, and R₁₀ are each independently H;
R_{b} is each independently selected from H, halogen, C₁-C₆ alkyl (preferably C₁-C₅ alkyl, C₁-C₄ alkyl, or C₁-C₃ alkyl), C₁-C₃ haloalkyl, C₃-C₆ cycloalkyl, amino, or cyano; and preferably, R_{b} is each independently selected from H, halogen, C₁-C₆ alkyl (preferably C₁-C₅ alkyl, C₁-C₄ alkyl, or C₁-C₃ alkyl), or C₁-C₃ haloalkyl;
m is selected from 0, 1, or 2, and preferably, m is 0 or 1;
p is selected from 0, 1, or 2, and preferably, p is 0;
p' is selected from 0, 1, or 2; and
q is selected from 0, 1, or 2.

In some embodiments, in the compound of formula (I), the ring A is selected from 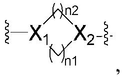 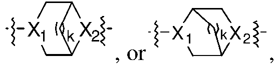 wherein at least one of X₁ and X₂ is N, and the other one is optionally C or N; and n1, n2, and k are each independently selected from 1 or 2; and preferably, the ring A is selected from 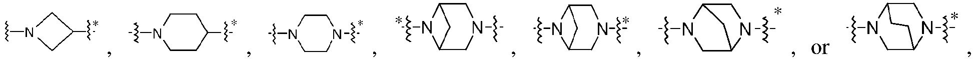 wherein * represents a connection site with R₄;
the ring B is selected from 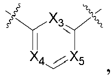 wherein X₃, X₄, and X₅ are each independently selected from C or N; and at least one of X₃, X₄, and X₅ is N; and preferably, the ring B is selected from 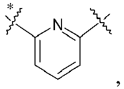 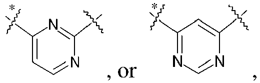 wherein * represents a connection site with R₄;
the ring C is selected from 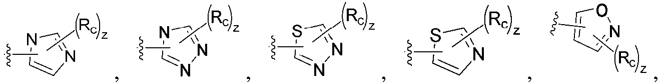 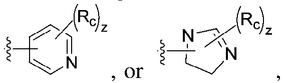 wherein R_{c} is independently selected from H, C₁-C₆ alkyl, C₃-C₆ cycloalkyl, C₁-C₃ haloalkyl, halogen, -C(O)R₁₃, or cyano; z is selected from 0, 1, 2, 3, or 4; and R₁₃ is selected from H, NH₂, C₁-C₃ alkyl, C₃-C₆ cycloalkyl, or C₁-C₃ haloalkyl; and preferably, the ring C is selected from 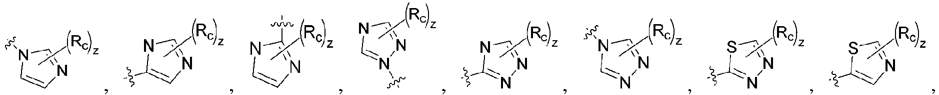 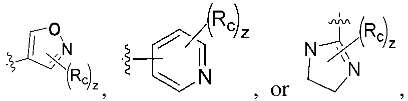 wherein R_{c} is independently selected from H, methyl, ethyl, propyl, cyclopropyl, cyclobutyl, fluoromethyl, fluoroethyl, fluoropropyl, chloromethyl, chloroethyl, chloropropyl, bromomethyl, bromoethyl, bromopropyl, F, Cl, Br, -C(O)NH₂, or cyano, and z is selected from 0, 1, or 2;
the dashed line between R₁ and R₂ represents connection or disconnection;
when R₁ and R₂ are disconnected, R₁ is selected from H or D, and R₂ is selected from O or S; and preferably, R₁ is selected from H, and R₂ is selected from O;
when R₁ and R₂ are connected, R₁ and R₂, together with the carbon atoms to which they are attached, form a benzene ring;
R₃ is selected from H or D; and preferably, R₃ is H;
Rₐ is each independently selected from H or C₁-C₆ alkyl (preferably C₁-C₅ alkyl, C₁-C₄ alkyl, or C₁-C₃ alkyl), wherein the C₁-C₆ alkyl is optionally substituted with one or more of hydroxy or cyano; or two Rₐ attached to the same carbon atom, together with the carbon atom to which they are attached, form C₃-C₆ cycloalkyl (preferably C₃-C₅ cycloalkyl or C₃-C₄ cycloalkyl);
R₄ is selected from a single bond, -C(O)-, -NHC(O)-, -NH-, -CH₂-(5-6 membered heterocycloalkyl)-, 5-6 membered heterocycloalkyl, -(C₅-C₆ cycloalkyl)-NHC(O)-, or -O-(C₅-C₆ cycloalkyl)-NR₁₁C(O)-, wherein the heterocycloalkyl contains 1-3 heteroatoms selected from N, O, or S (preferably, 1-2 heteroatoms selected from N or O (e.g., N)), and R₁₁ is selected from H or C₁-C₃ alkyl; and preferably, R₄ is selected from a single bond, -C(O)-, -NHC(O)-, -NH-, 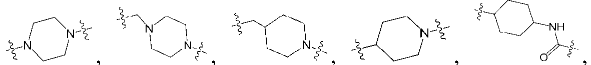 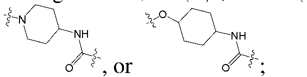
R₅ is H;
R₆, R₇, R₈, R₉, and R₁₀ are each independently H;
R_{b} is each independently selected from H, F, Cl, Br, methyl, ethyl, n-propyl, isopropyl, cyclopropyl, fluoromethyl, fluoroethyl, fluoropropyl, chloromethyl, chloroethyl, chloropropyl, bromomethyl, bromoethyl, or bromopropyl (e.g., trifluoromethyl, trichloromethyl, or tribromomethyl); and preferably, R_{b} is each independently selected from H, F, Cl, methyl, ethyl, propyl, or trifluoromethyl;
m is selected from 0 or 1;
p is selected from 0, 1, or 2, and preferably, p is 0;
p' is selected from 0, 1, or 2, preferably, p' is 0; and
q is selected from 0, 1, or 2.

In some embodiments, the above compound of formula (I) may be one of compounds of the following general formulas: 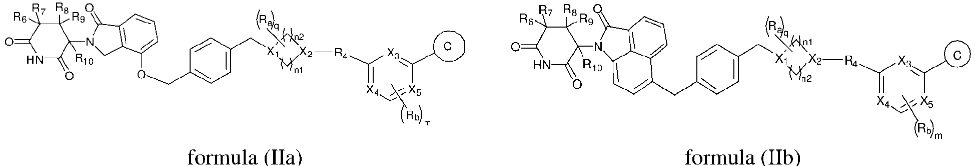 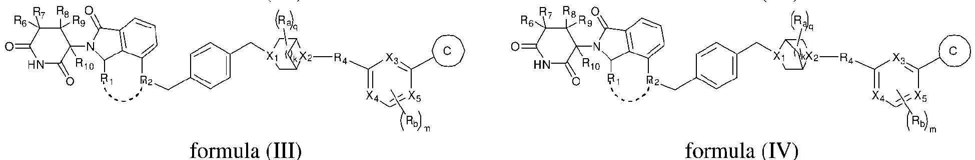 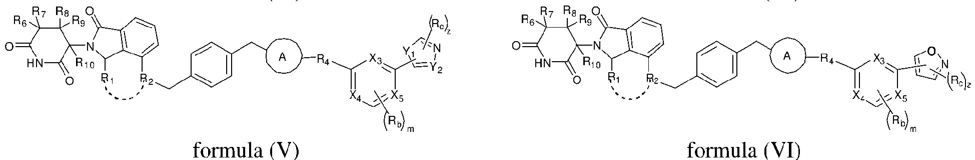 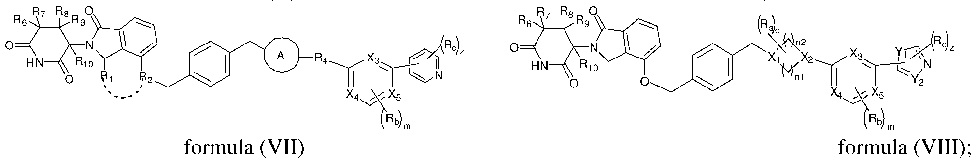
wherein R₁, R₂, R₄, R₆, R₇, R₈, R₉, R₁₀, Rₐ, R_{b}, R_{c}, z, m, q, k, n1, n2, X₁, X₂, X₃, X₄, X₅, Y₁, Y₂, the ring A, and ring C are as defined in any one of the foregoing definitions; and
preferably, R₆, R₇, R₈, R₉, and R₁₀ are each independently H.

In some embodiments, the above compound of formula (I) may be a compound of the following general formula: 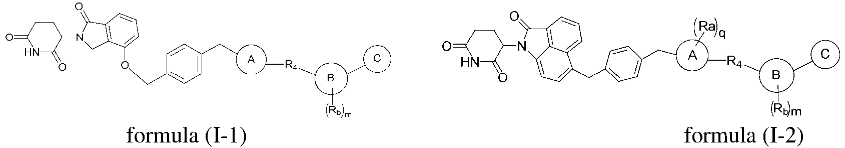 wherein the ring A, ring B, ring C, Rₐ, R_{b}, R₄, m, and q are as defined above.

In some embodiments, the above compound of formula (I-1) may be a compound of formula (I-1'): 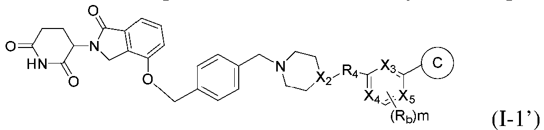 wherein X₂, X₃, X₄, and X₅ are each independently selected from C or N, and at least one of X₃, X₄, and X₅ is N (preferably, 1-2 of X₃, X₄, and X₅ are N); R₄ is selected from a single bond, -C(O)-, -NHC(O)-, -NH-, -CH₂-(5-6 membered heterocycloalkyl)-, 5-6 membered heterocycloalkyl, -(C₅-C₆ cycloalkyl)-NHC(O)-, or -O-(C₅-C₆ cycloalkyl)-NR₁₁C(O)-, wherein the heterocycloalkyl contains 1-3 heteroatoms selected from N, O, or S (preferably, 1-2 heteroatoms selected from N or O (e.g., N)), and R₁₁ is selected from H or C₁-C₃ alkyl; R_{b} is each independently selected from H or C₁-C₅ alkyl (preferably C₁-C₅ alkyl, C₁-C₄ alkyl, or C₁-C₃ alkyl); m is selected from 0, 1, or 2 (preferably, m is 0 or 1); and the ring C is selected from 5-membered heteroaryl or 5-membered heterocyclyl containing 1-3 heteroatoms selected from N, O, or S, wherein the 5-membered heteroaryl or 5-membered heterocyclyl is optionally substituted with one or more (e.g., 1-3, 1-2, or 1) selected from C₁-C₆ alkyl (preferably C₁-C₅ alkyl, C₁-C₄ alkyl, or C₁-C₃ alkyl), C₁-C₃ haloalkyl (e.g., C₁-C₃ fluoroalkyl, C₁-C₃ chloroalkyl, or C₁-C₃ bromoalkyl), halogen (preferably F, Cl, or Br), or -C(O)NH₂.

In some embodiments, in the compound of formula (I-1'), X₂ and X₃ are N, X₄ is C or N, X₅ is, m is 0, R₄ is a single bond, 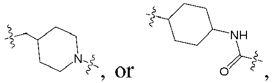 and the ring C is selected from 5-membered heteroaryl containing 1-2 heteroatoms selected from N, O, or S (preferably N or S) (preferably, the ring C is selected from

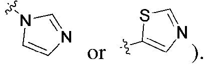

In some embodiments, in the compound of formula (I-2), the ring A is selected from 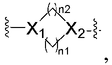 wherein at least one of X₁ and X₂ is N, and the other one is optionally C or N; and n1 and n2 are each independently selected from 1 or 2; and preferably, the ring A is selected from 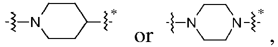 wherein * represents a connection site with R₄;
q is 0;
R₄ is selected from a single bond, -C(O)-, -NHC(O)-, -NH-, -CH₂-(5-6 membered heterocycloalkyl)-, or -O-(C₅-C₆ cycloalkyl)-NR₁₁C(O)-, wherein the heterocycloalkyl contains 1-2 heteroatoms selected from N, O, or S (preferably, 1-2 N atoms), and R₁₁ is H; and preferably, R₄ is selected from a single bond, -C(O)-, -NHC(O)-, -NH-, 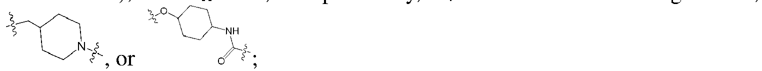
the ring B is selected from 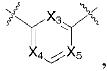 wherein X₃, X₄, and X₅ are each independently selected from C or N; and at least one of X₃, X₄, and X₅ is N; and preferably, the ring B is selected from 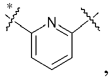 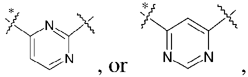 wherein * represents a connection site with R₄;
R_{b} is selected from halogen, C₁-C₆ alkyl (preferably C₁-C₅ alkyl, C₁-C₄ alkyl, or C₁-C₃ alkyl), or C₁-C₃ haloalkyl (fluoromethyl, fluoroethyl, fluoropropyl, chloromethyl, chloroethyl, chloropropyl, bromomethyl, bromoethyl, or bromopropyl); and preferably, R_{b} is selected from methyl, ethyl, propyl, or trifluoromethyl;
m is selected from 0 or 1; and
the ring C is selected from 5-6 membered heteroaryl containing 1-3 heteroatoms selected from N, O, or S (preferably N or S), wherein the 5-6 membered heteroaryl is optionally substituted with one or more selected from C₁-C₆ alkyl (e.g., C₁-C₅ alkyl, C₁-C₄ alkyl, or C₁-C₃ alkyl), C₃-C₆ cycloalkyl (e.g., C₃-C₅ cycloalkyl or C₃-C₄ cycloalkyl), C₁-C₃ haloalkyl, or halogen; and preferably, the ring C is selected from 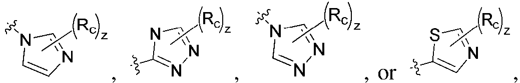 wherein R_{c} is independently selected from methyl, ethyl, propyl, cyclopropyl, cyclobutyl, fluoromethyl, fluoroethyl, fluoropropyl, chloromethyl, chloroethyl, chloropropyl, bromomethyl, bromoethyl, or bromopropyl, and z is selected from 0 or 1.

In some embodiments, the compound, the pharmaceutically acceptable salt thereof, the deuterated compound thereof, the stereoisomer thereof, the tautomer thereof, or the mixture thereof as described in any one of the foregoing includes, but is not limited to, the following compounds:

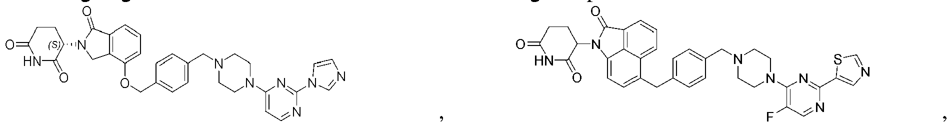

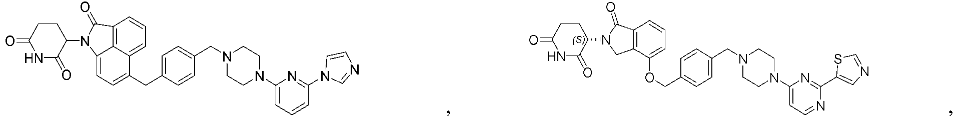

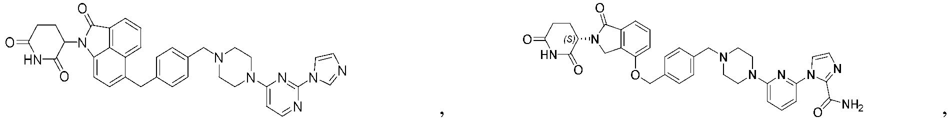

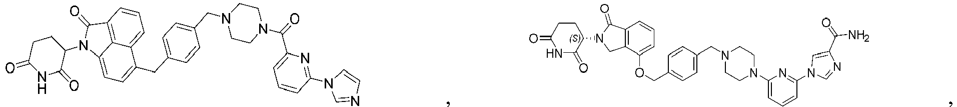

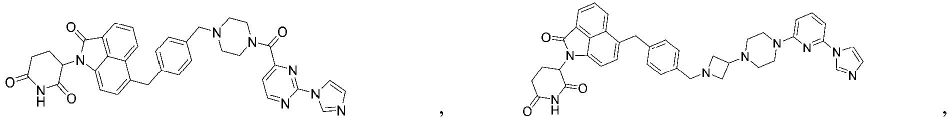

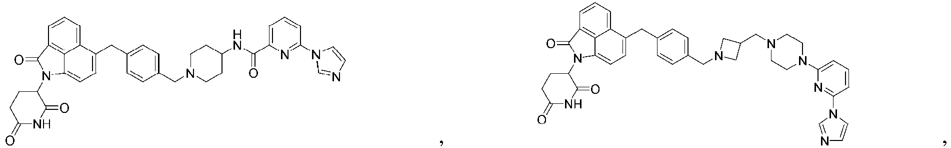

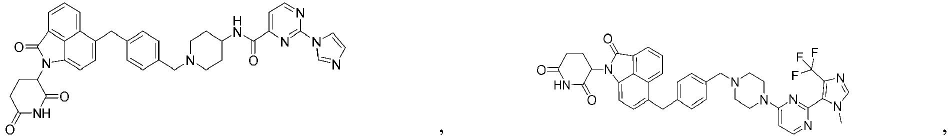

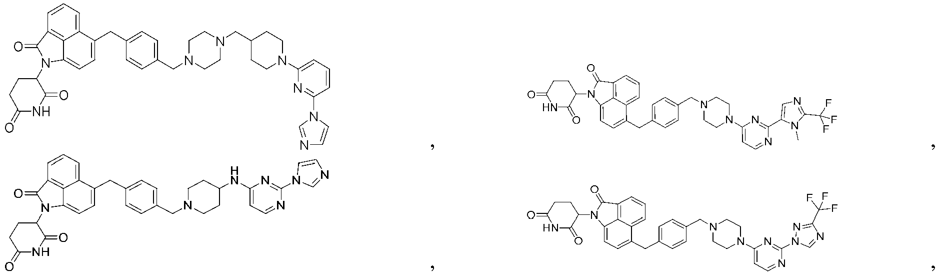

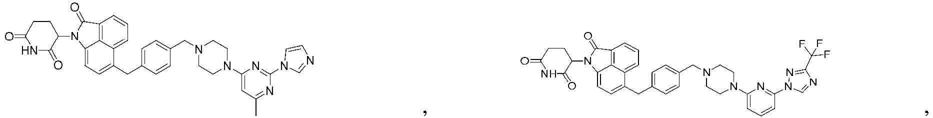

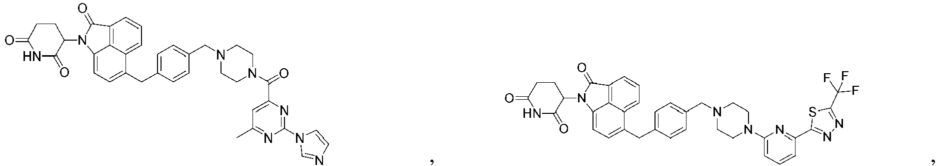

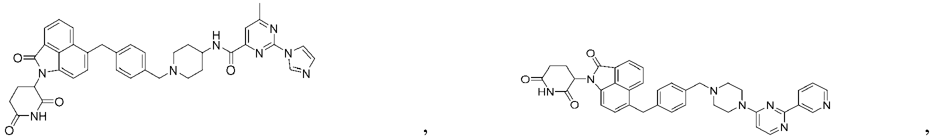

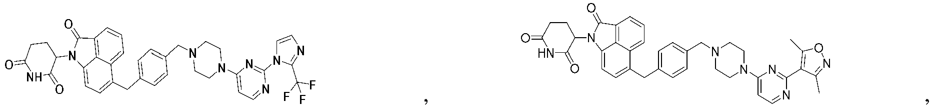

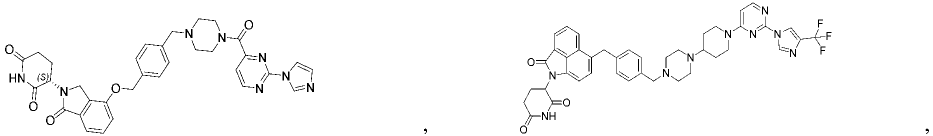

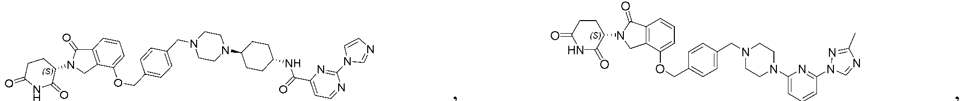

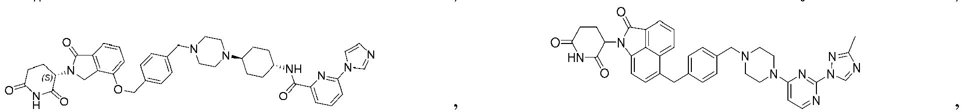

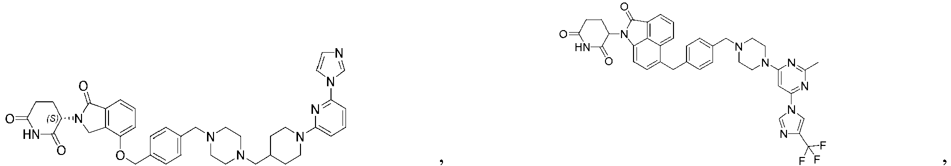

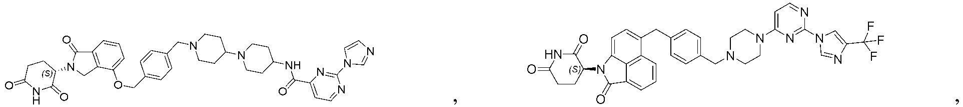

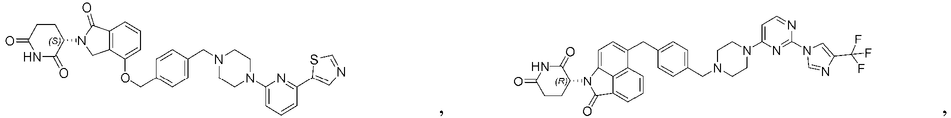

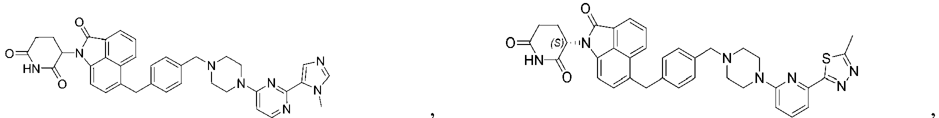

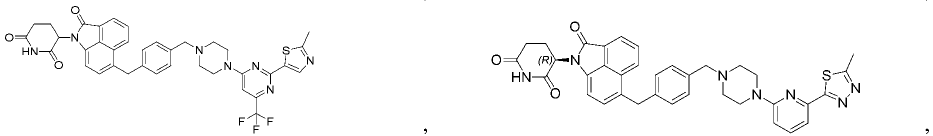

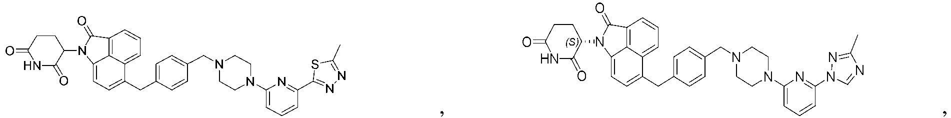

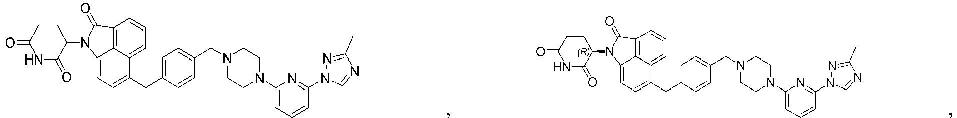

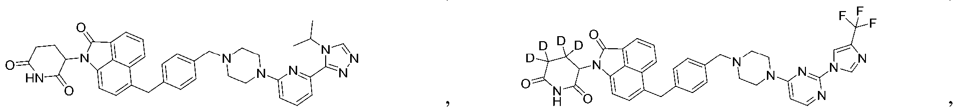

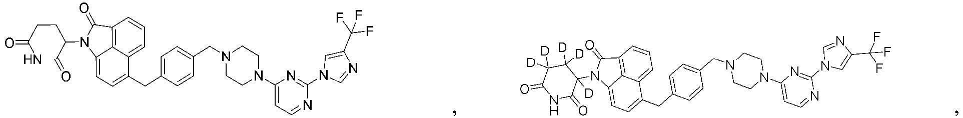

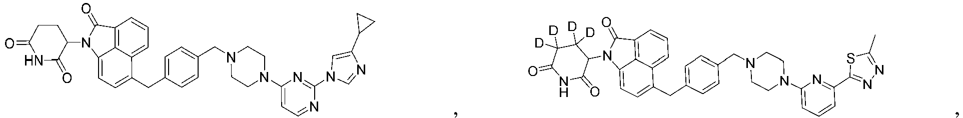

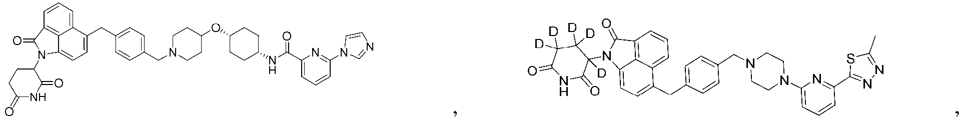

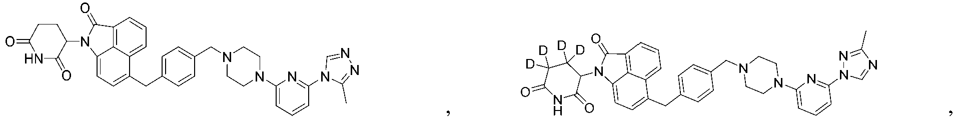

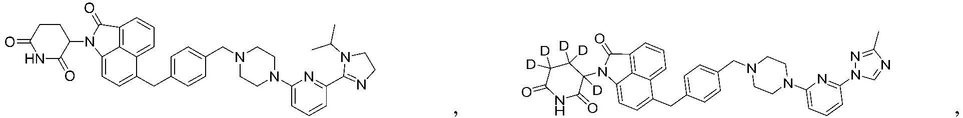

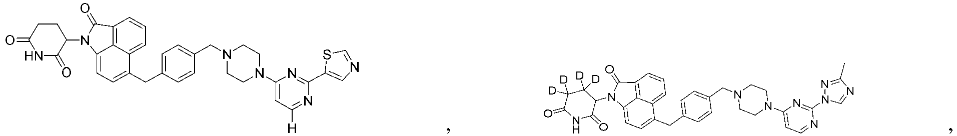

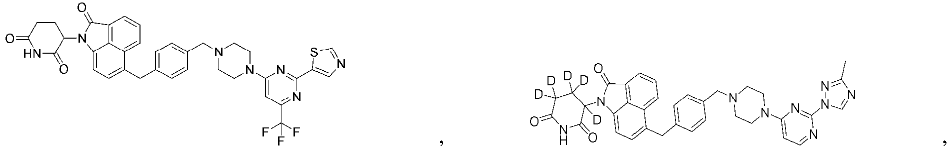

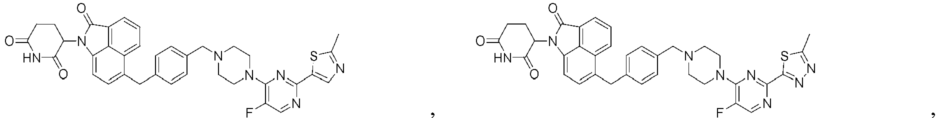

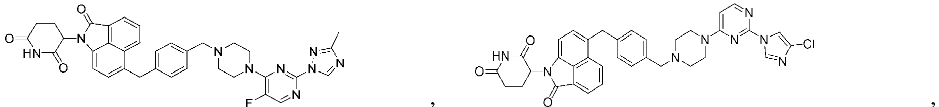

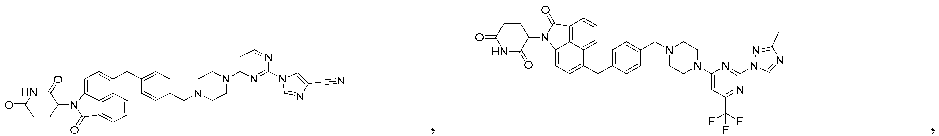

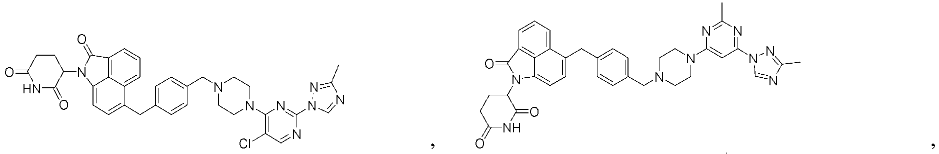

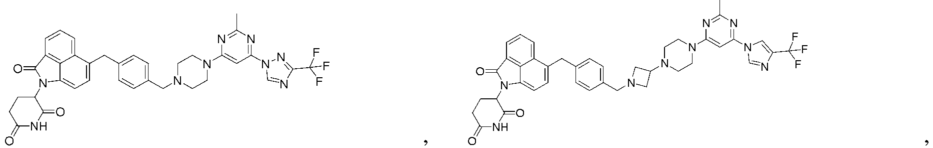

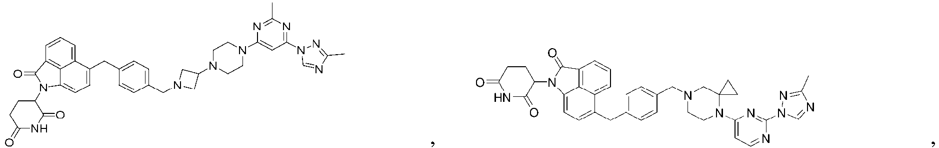

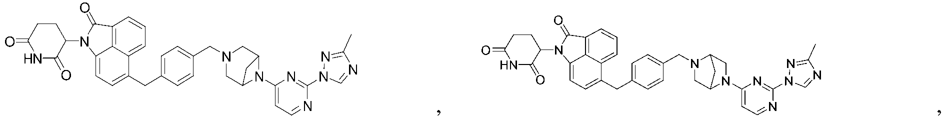

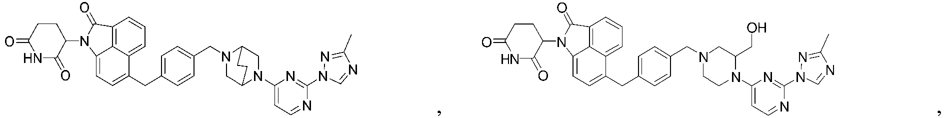

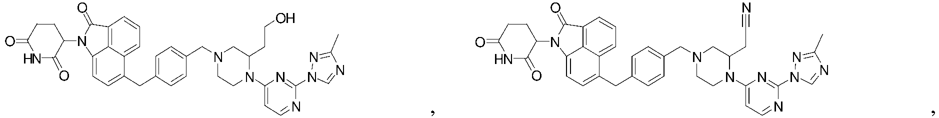

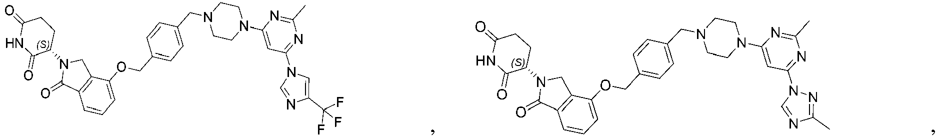

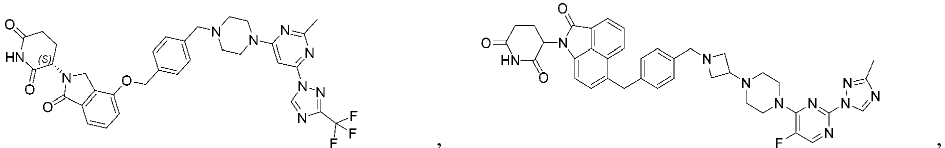

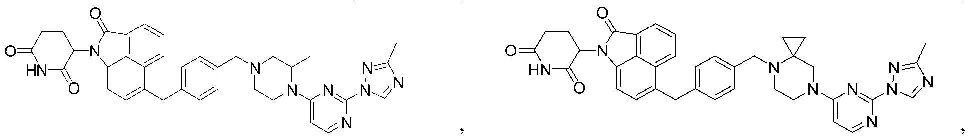

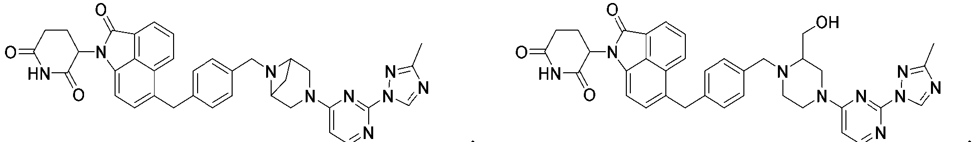

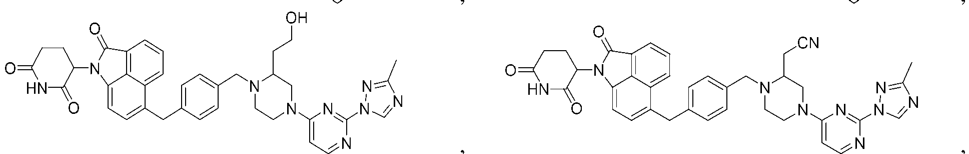

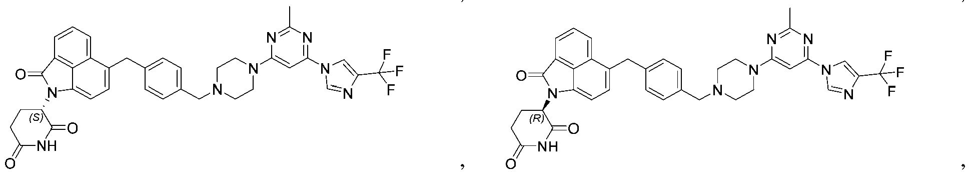

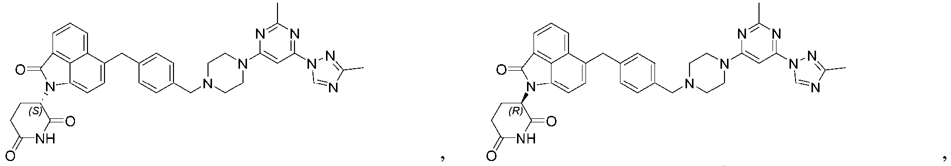

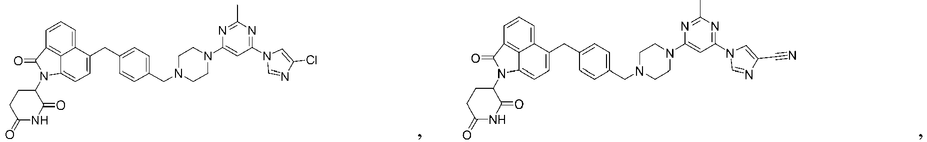

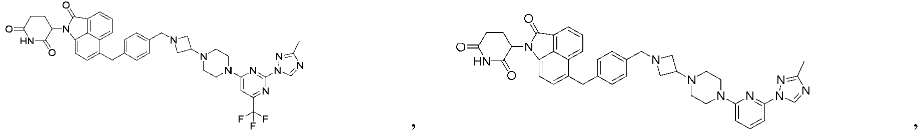

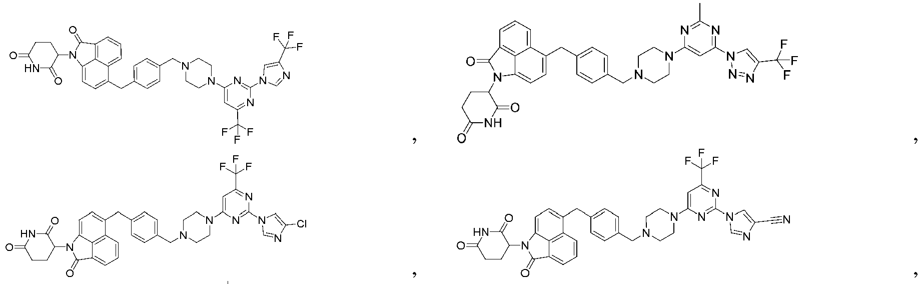

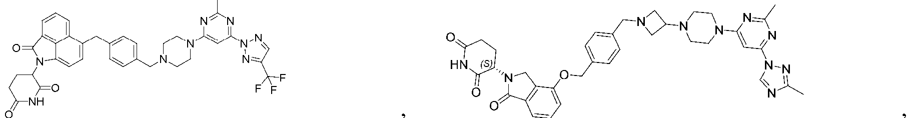

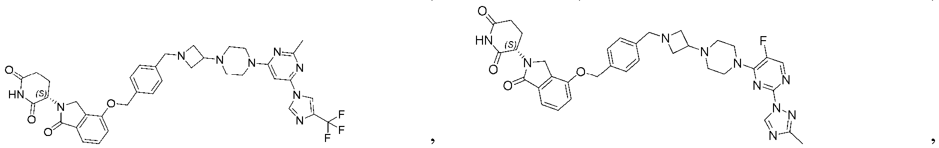

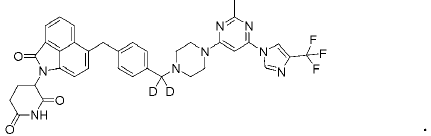

The present application further encompasses embodiments obtained by any combination, deletion, or substitution of the above embodiments and preferred embodiments.

In another aspect, the present application provides use of the compound, the pharmaceutically acceptable salt thereof, the deuterated compound thereof, the stereoisomer thereof, the tautomer thereof, or the mixture thereof as described in any one of the foregoing in preparation of a medicament for preventing and/or treating a cereblon-mediated disease. Alternatively, the present application provides the compound, the pharmaceutically acceptable salt thereof, the deuterated compound thereof, the stereoisomer thereof, the tautomer thereof, or the mixture thereof as described in any one of the foregoing for use in preventing and/or treating a cereblon-mediated disease. Alternatively, the present application provides a method for preventing and/or treating a cereblon-mediated disease, comprising administering the compound, the pharmaceutically acceptable salt thereof, the deuterated compound thereof, the stereoisomer thereof, the tautomer thereof, or the mixture thereof as described in any one of the foregoing to a subject in need thereof.

In some preferred embodiments, the above cereblon-mediated disease is mediated by IKZF1 (Ikaros) and/or IKZF3 (Aiolos).

In some preferred embodiments, the above cereblon-mediated disease is cancer, a tumor, an immune disease, or an inflammatory disease.

In some preferred embodiments, the above cereblon-mediated disease is an autoimmune disease.

In some preferred embodiments, the above cereblon-mediated disease is a hematological malignancy.

In some preferred embodiments, the above cereblon-mediated disease is multiple myeloma, leukemia, lymphocytic leukemia, chronic lymphocytic leukemia, Hodgkin's lymphoma, or non-Hodgkin's lymphoma.

In another aspect, the present application provides use of the compound, the pharmaceutically acceptable salt thereof, the deuterated compound thereof, the stereoisomer thereof, the tautomer thereof, or the mixture thereof as described in any one of the foregoing in the preparation of an immunomodulator for dual targets of IKZF1 and IKZF3. Alternatively, the present application provides the compound, the pharmaceutically acceptable salt thereof, the deuterated compound thereof, the stereoisomer thereof, the tautomer thereof, or the mixture thereof as described in any one of the foregoing for use in the immunomodulation of dual targets of IKZF1 and IKZF3. Alternatively, the present application provides a method for immunomodulating dual targets of IKZF1 and IKZF3, comprising administering the compound, the pharmaceutically acceptable salt thereof, the deuterated compound thereof, the stereoisomer thereof, the tautomer thereof, or the mixture thereof as described in any one of the foregoing to a subject in need thereof.

### DETAILED DESCRIPTION OF THE EMBODIMENTS

In order to make the objectives and technical solutions of the present application clearer, the present application is further elaborated below in conjunction with specific examples. It should be understood that these examples are used only to illustrate the present application and are not used to limit the scope of the present application. Furthermore, specific experimental methods not mentioned in the following examples are all carried out according to conventional experimental methods.

In the present application, when there is a discrepancy between a chemical name and a structural formula, the structural formula shall prevail unless it can be inferred from the context that the chemical name, rather than the structural formula, is correct.

Structures of compounds are determined by mass spectrometry (MS) or nuclear magnetic resonance (¹H NMR).

A chemical shift (δ) of proton nuclear magnetic resonance (¹H NMR) is given in the unit of parts per million (ppm). Determination by the nuclear magnetic resonance (¹H NMR) is performed using a Bruker AVANCE-400 nuclear magnetic resonance apparatus with deuterated dimethyl sulfoxide (DMSO-*d*₆) as a solvent and tetramethylsilane (TMS) as an internal standard, and the chemical shift is given in the unit of 10⁻⁶ (ppm).

Determination by the mass spectrometry (MS) is performed using a FINNIGAN LCQAd (ESI) mass spectrometer (manufacturer: Therm, model: Finnigan LCQ advantage MAX).

A Yantai Huanghai HSGF254 or Qingdao GF254 silica gel plate is used as a silica gel for thin-layer chromatography.

A Yantai Huanghai silica gel, 200-300 mesh, is generally used as a carrier for column chromatography.

### Definitions and general terms:

Unless otherwise defined, all technical and scientific terms used herein have the meanings commonly understood by those skilled in the art to which the present application belongs. As used herein, unless otherwise indicated, the following terms have the meanings assigned to them below.

The term "substitution" in the present application means that one or more (e.g., one, two, three, or four) hydrogens on a specified atom are replaced by a selection from specified groups, provided that a normal valence of the specified atom is not exceeded in a current instance and the substitution forms a stable compound. Combinations of substituents and/or variables are permitted only when such combinations form stable compounds. It should be understood that, as required, other atoms, such as hydrogen atoms or substituents as described herein, may be present to satisfy valencies of the atoms.

When the present application refers to a number of substituents, it means that the number of substituents on a group may be in a range from 0 to a maximum possible number of substituents.

When a connection site is not specifically defined in the present application, it means that it may be attached to any site on a ring. For example, 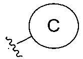 represents that it may be attached to any site on ring C. For groups with two connection sites, for example, 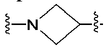 represents that connection modes of two connection sites may be interchanged, that is, sites on two sides may be optionally attached to corresponding other groups, and preferably, a site on a left side represents attachment to a group at a connection site on the left side in a compound of a corresponding general formula. As used herein, 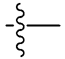 represents a connection site of a group.

When a substitution site is not specifically indicated in the present application, it represents that any substitutable site is substituted. For example, R_{c} in 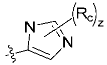 represents that substitution may occur at any site on a ring, including: 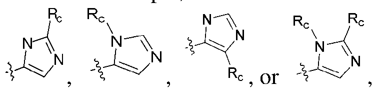 etc.

The term "independently" means that when more than one substituent is selected from many possible substituents, those substituents may be the same or different; or when one substituent occurs more than once, each occurrence may be selected to be the same or different.

The term "optional" or "optionally" means that an event or circumstance subsequently described may or may not occur, and the description includes both an instance where the event or circumstance occurs and an instance where the event or circumstance does not occur. Whenever a group is described as "optionally substituted", the group may be unsubstituted or substituted with one or more indicated substituents. When no substituent is specified, it means that the group may be optionally substituted with one or more groups selected from, but not limited to, alkyl, haloalkyl, cycloalkyl, heterocycloalkyl, alkenyl, alkynyl, aryl, heteroaryl, halogen, hydroxy, alkoxy, cyano, nitro, amino, or aminohydroxy. Preferably, the "optionally substituted" refers to being substituted with substituents independently selected from C₁-C₆ alkyl, haloalkyl, C₃-C₆ cycloalkyl, F, Cl, Br, hydroxy, alkoxy, cyano, amino, aryl, or heteroaryl, with specific illustrative examples including but not limited to methyl, ethyl, propyl, trifluoromethyl, cyclopropyl, fluoro, chloro, methoxy, phenyl, naphthyl, pyridyl, pyrazinyl, etc. When a composition "optionally" includes a certain component, the composition may or may not include the component.

The term "Cₘ-Cₙ" in the present application means that the number of carbon atoms is at least m and at most n, including both endpoints m and n; and m and n are integers not equal to 0, wherein m < n.

The term "heterocyclyl" in the present application refers to a monocyclic, bicyclic, or tricyclic group composed of carbon atoms and those optionally selected from one or more heteroatoms, including a bridged ring, fused ring, or spiro ring structure, wherein the cyclic group may be saturated or unsaturated (including aromatic), and at least one ring has at least one heteroatom (O, S, or N). Thus, the term "heterocyclyl" encompasses heterocycloalkyl, heterocycloalkenyl, heterocycloalkynyl, and heteroaryl. For example, "3-10 membered heterocyclyl" refers to a 3-10 membered ring composed of carbon atoms and heteroatoms, with specific non-limiting examples including but not limited to aziridine, ethylene oxide, azetidine, oxetane, tetrahydrofuran, tetrahydrothiophene, tetrahydropyrrole, piperidine, pyrroline, oxazolidine, piperazine, dioxolane, dioxane, morpholine, thiomorpholine, thiomorpholine-1,1-dioxide, tetrahydropyran, 1,2-dihydroazete, 1,2-dihydrooxete, 2,5-dihydro-1H-pyrrole, 2,5-dihydrofuran, 2,3-dihydrofuran, 2,3-dihydro-1H-pyrrole, 3,4-dihydro-2H-pyran, 1,2,3,4-tetrahydropyridine, 3,6-dihydro-2H-pyran, 1,2,3,6-tetrahydropyridine, etc. The term "heterocyclyl" in the present application is intended to include "heteroaryl".

The term "bridged heterocyclyl" in the present application refers to a 5-14 membered polycyclic heterocyclic group, in which every two rings in the system share two indirectly attached atoms, wherein the rings may have one or more double bonds, but none of the rings has a fully conjugated π-electron system, the rings have one or more heteroatoms as ring atoms selected from N, O, and S(O)ₘ (wherein m is an integer selected from 0 to 2), and the remaining ring atoms are carbon atoms; and according to the number of rings, the bridged heterocyclyl may be bicyclic, tricyclic, tetracyclic, or polycyclic bridged heterocyclyl, preferably bicyclic heterocyclyl.

The term "fused heterocyclyl" in the present application refers to a 5-20 membered polycyclic heterocyclic group, in which each ring in the system shares a pair of adjacent atoms with another ring, wherein one or more rings may contain one or more double bonds, but none of the rings has a fully conjugated π-electron system, and the rings have one or more heteroatoms as ring atoms selected from N, O, and S(O)ₘ (wherein m is an integer selected from 0 to 2), and the remaining ring atoms are carbon atoms; and according to the number of rings, the fused heterocyclyl may be bicyclic, tricyclic, tetracyclic, or polycyclic fused heterocyclyl, preferably bicyclic or tricyclic fused heterocyclyl.

The term "spiro heterocyclyl" in the present application refers to a 5-20 membered polycyclic heterocyclic group in which rings are connected through a common carbon atom (referred to as a spiro atom), wherein the rings have one or more heteroatoms as ring atoms selected from N, O, and S(O)ₘ (wherein m is an integer selected from 0 to 2), and the remaining ring atoms are carbon atoms, wherein one or more rings may contain one or more double bonds, but none of the rings has a fully conjugated π-electron system; and according to the number of common spiro atoms, the spiro heterocyclyl may be monospiro heterocyclyl, dispiro heterocyclyl, or polyspiro heterocyclyl, preferably monospiro heterocyclyl.

The term "heteroaryl" in the present application refers to a monocyclic, bicyclic, or tricyclic system including spiro ring and fused ring structures, containing, for example, 5-14 ring atoms, wherein at least one ring system is aromatic, and at least one ring system contains one or more heteroatoms selected from N, O, and S (preferably contains 1-3 heteroatoms), wherein each ring system contains a ring composed of 5-7 atoms, and one or more connection sites are connected to other parts of a molecule. For example, "5-10 membered heteroaryl" refers to 5-10 membered heteroaryl composed of carbon atoms and 1 or 3 heteroatoms independently selected from N, O, or S, with non-limiting examples including but not limited to furanyl, imidazolyl, pyridyl, pyrrolyl, thiazolyl, purinyl, quinolinyl, thienyl, isoxazolyl, oxazolyl, pyrazinyl, benzimidazolyl, benzofuranyl, benzothienyl, furanopyridinyl, etc.

The term "alkyl" appearing alone or in combination in the present application refers to a linear or branched saturated hydrocarbon group composed only of carbon atoms and hydrogen atoms, including but not limited to C₁-C₆ alkyl, C₁-C₅ alkyl, C₁-C₄ alkyl, C₁-C₃ alkyl, C₁-C₂ alkyl, and C₁ alkyl. As non-limiting examples of the alkyl, the following linear or branched saturated hydrocarbon groups may be listed: methyl, ethyl, n-propyl, isopropyl, n-butyl, isobutyl, sec-butyl, tert-butyl, n-pentyl and other seven isomers thereof, and n-hexyl and other sixteen isomers thereof. For example, "C₁-C₆ alkyl" includes methyl, ethyl, n-propyl, isopropyl, n-butyl, isobutyl, sec-butyl, tert-butyl, pentyl, hexyl, and all isomers thereof.

The term "cycloalkyl" appearing alone or in combination in the present application refers to saturated monocyclic, bicyclic, or tricyclic alkyl, including a monocyclic ring, fused ring, or spiro ring structure, wherein each ring portion contains 3 to 12 carbon atoms as ring members. As non-limiting examples of the cycloalkyl, the following may be listed: cyclopropyl, cyclobutyl, cyclopentyl, cyclohexyl, cycloheptyl, tetrahydronaphthyl, indanyl, octahydronaphthyl, 2,3-dihydro-1H-indenyl, adamantyl, etc. For example, non-limiting examples of "C₃-C₈ cycloalkyl" include, but are not limited to, cyclopropyl, cyclobutyl, cyclopentyl, cyclohexyl, cycloheptyl, etc., wherein the cycloalkyl is optionally substituted.

The term "haloalkyl" in the present application refers to alkyl in which hydrogen atoms are substituted with halogen, for example, substituted with F or Cl. For example, non-limiting examples of "C₁-C₆ haloalkyl" include, but are not limited to, -CF₃, -CHCF₂, -CH₂CH₂F, or -CH₂CF₃.

The term "aryl" or "aromatic ring" in the present application refers to an aromatic monocarbocyclic or polycarbocyclic (e.g., fused ring or spiro ring) group having at least one aromatic ring and containing 6 to 12 carbon atoms, with specific non-limiting examples including phenyl, naphthyl or tetrahydronaphthyl, 1,2-dihydronaphthyl, indanyl, and 1H-indenyl, etc.

The term "alkoxy" in the present application refers to an "-O-alkyl" group. For example, the term "C₁-C₃ alkoxy" refers to a saturated linear or branched hydrocarbon moiety having at least 1 and at most 3 carbon atoms, bonded by connecting oxygen to atoms. Specific exemplary examples include, but are not limited to, methoxy, ethoxy, n-propoxy, isopropoxy, n-butoxy, sec-butoxy, and tert-butoxy, etc.

The term "amino" in the present application refers to an -NH₂ group, which may be substituted or unsubstituted. When referred to as optionally substituted amino, the amino is -NR'R", wherein R' and R" are independently optional suitable substituents. For example, in specific examples of the present application, "optionally substituted amino" refers to that the amino may be substituted with one or more substituents independently selected from H, C=O, C₁-C₆ alkyl, C₁-C₆ haloalkyl, C₃-C₆ cycloalkyl, F, Cl, Br, hydroxy, C₁-C₆ alkoxy, cyano, 5-6 membered aryl, or 5-6 membered heteroaryl.

Herein, unless otherwise specifically defined, a heteroatom may be any atom other than a C atom and an H atom commonly used in the art, such as N, O, S, P, Si, etc.

The term "halogen" in the present application refers to F, Cl, Br, or I.

The term "cyano" in the present application refers to a -CN group.

The above alkyl, cycloalkyl, heterocyclyl, aryl, and heteroaryl and the like may be unsubstituted or optionally substituted with one or more substituents, for example, substituted with the following substituents: alkyl, haloalkyl, cycloalkyl, heterocycloalkyl, alkenyl, alkynyl, aryl, heteroaryl, halogen, hydroxy, alkoxy, cyano, nitro, amino, or aminohydroxy groups. Preferably, they may be optionally substituted with substituents independently selected from C₁-C₆ alkyl, haloalkyl, C₃-C₆ cycloalkyl, halogen, hydroxy, hydroxy, alkoxy, cyano, amino, aryl, or heteroaryl, with specific illustrative examples including but are not limited to methyl, ethyl, propyl, F, Cl, trifluoromethyl, cyclopropyl, methoxy, phenyl, naphthyl, pyridyl, pyrazinyl, etc.

The term "cancer" in the present application refers to any malignant and/or invasive growth or tumor caused by abnormal cell growth. The cancer includes solid tumors named for the types of cells forming the solid tumors, hematologic cancer, bone marrow cancer, or lymphatic system cancer. Examples of the solid tumors include sarcomas and carcinomas. The hematologic cancer includes, but is not limited to, leukemia, lymphoma, and myeloma. The "cancer" may include a "tumor".

The term "tumor" in the present application refers to any tissue mass caused by excessive cell growth or proliferation, which may be benign or malignant, including a precancerous lesion. For example, the tumor may be breast cancer, gastric cancer, bladder cancer, brain cancer, cervical cancer, colorectal cancer, esophageal cancer, hepatocellular carcinoma, melanoma, oral cancer, ovarian cancer, non-small cell lung cancer, prostate cancer, small cell lung cancer, or spleen cancer.

Compounds of the present application each contain one or more asymmetric centers (also referred to as chiral centers), both the compounds and intermediates of the present application may exist in different tautomeric forms, and all the above forms are encompassed within the protection scope of the present application. The present application includes all isomeric forms (e.g., enantiomers, diastereomers, and geometric isomers (or conformational isomers)) of such structures.

Among them, the term "stereoisomer" (also referred to as "optical isomer") refers to stable isomers having vertical unsymmetrical planes due to at least one chiral factor (including a chiral center, a chiral axis, a chiral plane, etc.), which may rotate plane-polarized light. A single stereoisomer in the present application may be synthesized and prepared from an optically rotating starting material containing a desired chiral center, or may be prepared and obtained by preparing a mixture of enantiomer products and then performing separation or resolution by a method well known in the art, for example, performing separation or recrystallization, chromatographic treatment, use of a chiral resolving agent, or direct separation of enantiomers on a chiral chromatographic column after conversion into a mixture of diastereomers. Starting compounds having specific stereochemistry may be commercially available or may be obtained by preparation according to a method described below and then resolution by a method well known in the art. All stereoisomeric forms of the compounds of the present application are within the scope of the present application.

The term "tautomer" (also referred to as "tautomeric form") refers to structural isomers with different energies that may interconvert via a low energy barrier. For example, proton tautomers (also referred to as proton transfer tautomers) include (but are not limited to) interconversions via proton migration, such as keto-enol isomerization, imine-enamine isomerization, amide-iminol isomerization, etc.

In addition, the compounds of the present application each may contain one or more isotopic forms, that is, isotopic forms of hydrogen in D or T, or isotopic forms of any atom, such as all isotopic forms of C or N. All isotopic forms of any C, N, or H in the optionally structured compounds of the present application are encompassed within the protection scope of the present application. In the present application, the term "deuterated compound" refers to the compound of formula (I) of the present application in which a hydrogen atom at any position is substituted with deuterium (that is, D), and the amount of deuterium at the position is far greater than (e.g., at least greater than 1,000 times) the abundance of naturally occurring deuterium.

The terms "include", "comprise", "comprising", and their equivalents should be understood to have open and non-exclusive meanings, and that is, "including but not limited to" means that in addition to listed elements, components, and steps, other unspecified elements, components, and steps may also be encompassed.

The term "subject" encompasses mammals, such as humans, non-human primates (e.g., Rhesus macaque), pigs, cattle, horses, sheep, dogs, rabbits, mice, etc., preferably humans.

Herein, unless otherwise stated expressly in the context, singular terms encompass plural referents, and vice versa. Similarly, unless otherwise specified clearly in the context, the word "or" is intended to include "and". Unless otherwise indicated, herein, parameter values representing amounts, physicochemical properties, or reaction conditions, etc., of components should be understood to be modified by the term "approximately" in all cases. When the description in the present application involves the term "approximately", the term "approximately" indicates the presence of an error value, for example, a variation within a range of ±5%, such as ±1% or ±0.1%, of a particular value.

Unless otherwise specified in the present application, solutions referred to in reactions of the present application are aqueous solutions.

The term "room temperature" in the present application refers to a temperature between 10°C and 25°C.

For the purposes of describing and disclosing, all patents, patent applications, and other publications are expressly incorporated herein by reference. These publications are provided solely for their disclosure prior to the application date of the present application. All statements regarding the dates of these documents or the representation of the contents of these documents are based on information available to applicants and do not constitute any admission to the correctness of the dates of these documents or the contents of these documents.

### Example

### Intermediate 1: Tert-butyl (S)-5-amino-4-(4-hydroxy-1-oxoisoindolin-2-yl)-5-oxopentanoate

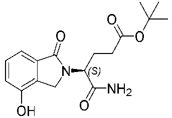

Intermediate 1 was synthesized according to a method provided in a document (Journal of Medicinal Chemistry, 2020, vol. 63, #13, p. 6648 - 6676).

### Intermediate 2: 4-(4-(4-(chloromethyl)benzyl)piperazin-1-yl)-2-(1H-imidazol-1-yl)pyrimidine

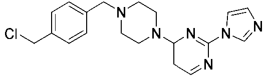

### Tert-butyl 4-(2-chloropyrimidin-4-yl)piperazin-1-formate

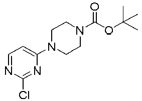

2,4-dichloropyrimidine (8.4 g, 30 mmol, CAS: 3934-20-1) was dissolved in anhydrous N,N-dimethylformamide (50 mL), and triethylamine (6.3 mL, 45 mmol, d = 0.728 g/mL) was added at room temperature and stirred for 30 minutes. Subsequently, a solution of 1-(tert-butoxycarbonyl)piperazine (6.14 g, 33 mmol, CAS: 57260-71-6) in N,N-dimethylformamide (50 mL) was slowly added dropwise into the above solution. After the addition was completed, the reaction mixture was stirred at room temperature for 12 hours. After TLC (petroleum ether/ethyl acetate = 1:1) determined the completion, the mixture was diluted with brine (200 mL), extracted with ethyl acetate (50 mL) for 3 times, concentrated under reduced pressure, and purified by silica gel column chromatography (petroleum ether/ethyl acetate = 5:1) to obtain the title compound as a white solid (8.23 g, yield: 92%). LC-MS: m/z [M+H]⁺ = 299.

### Tert-butyl 4-(2-(1H-imidazol-1-yl)pyrimidin-4-yl)piperazin-1-carboxylate

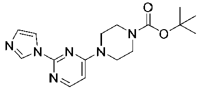

The tert-butyl 4-(2-chloropyrimidin-4-yl)piperazin-1-formate (6 g, 20 mmol), imidazole (1.91 g, 28 mmol), cuprous iodide (200 mg, 1 mmol), and cesium carbonate (7.82 g, 24 mmol) were added into a reaction tube, N,N-dimethylformamide (20 mL) was added in a nitrogen atmosphere, and the reaction mixture was stirred at room temperature for half an hour. The reaction solution was then heated to 100°C and stirred for 12 hours. After TLC (petroleum ether/ethyl acetate = 1:1) determined the completion, the reaction solution was diluted with brine (100 mL), extracted with ethyl acetate (50 mL) for 3 times, concentrated under reduced pressure, and purified by silica gel column chromatography (petroleum ether/ethyl acetate = 5:1) to obtain the title compound as a white solid (5.0 g, yield: 75%). LC-MS: m/z [M+H]⁺ = 331.

### 2-(1H-imidazolyl)-4-(piperazin-1-yl)pyrimidine

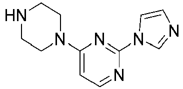

Tert-butyl 4-(2-(1H-imidazol-1-yl)pyrimidin-4-yl)piperazin-1-carboxylate (5.0 g, 15 mmol) was dissolved in dichloromethane (20 mL), and trifluoroacetic acid (10 mL) was added dropwise at 0°C. The reaction mixture was stirred at 0°C for 10 minutes, and then slowly heated to room temperature and stirred for 1 hour. The reaction solution was directly concentrated to obtain the title compound as a gray oil (6.1 g of a crude product, TFA salt), which was used directly in the next step without further purification. LC-MS: m/z [M+H]⁺ = 231.

### 4-(4-(4-(chloromethyl)benzyl)piperazin-1-yl)-2-(1H-imidazol-1-yl)pyrimidine

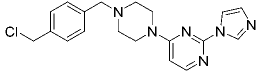

1,4-bis(chloromethyl)benzene (7.9 g, 45 mmol, CAS: 623-25-6) was added into a 150 mL reaction flask, and anhydrous acetonitrile (50 mL) and N,N-dimethylformamide (50 mL) were added into the reaction flask through a syringe. The mixture was stirred at room temperature until 1,4-bis(chloromethyl)benzene was completely dissolved. N,N-diisopropylethylamine (6.0 mL, 45 mmol, d = 0.782 g/mL) was slowly added dropwise into the reaction flask through a syringe. After the addition was completed, the reaction solution was stirred at room temperature for 30 minutes. Then, 2-(1H-imidazolyl)-4-(piperazin-1-yl)pyrimidine (6.1 g of a crude product, TFA salt) was dissolved in a mixed solvent of acetonitrile (10 mL) and N,N-dimethylformamide (10 mL), and added dropwise into the above reaction solution at room temperature. The reaction mixture was stirred at 60°C for 2 hours. After TLC (petroleum ether/ethyl acetate = 1:1) determined the completion, brine (100 mL) was added into the reaction solution, and the reaction solution was extracted with ethyl acetate (50 mL) for 2 times. Organic phases were dried over anhydrous sodium sulfate, concentrated under reduced pressure, and purified by silica gel column chromatography (petroleum ether/ethyl acetate = 5:1) to obtain the title compound as a white solid (2.0 g, yield: 36%). LC-MS: m/z [M+H]⁺ = 369.

### Intermediate 3:

### 3-(2-oxo-6-(4-(piperazin-1-ylmethyl)benzyl)benzo[cd]indol-1(2H)-yl)piperidin-2,6-dione

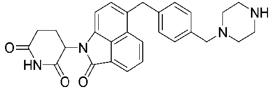

Intermediate 3 was synthesized according to a method provided in Patent Application No. WO2020210630A1.

### Intermediate 4: 2-fluoro-6-(1H-imidazol-1-yl)pyridine

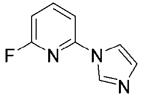

2,6-difluoropyridine (345 mg, 3 mmol) and imidazole (136 mg, 2 mmol) were added into a reaction flask, then potassium carbonate (552 mg, 4 mmol) and dimethyl sulfoxide (10 mL) were added, and the mixture was stirred at 90°C for 6 hours. Brine (50 mL) was added into the reaction solution, and the reaction solution was extracted with ethyl acetate (20 mL) for 3 times. Organic phases were combined, dried over anhydrous sodium sulfate, concentrated under reduced pressure, and purified by silica gel column chromatography (petroleum ether/ethyl acetate = 1:1) to obtain the title compound as a white solid (228 mg, yield: 70%). LC-MS: m/z [M+H]⁺ = 164.

Referring to the table below, except that a raw material listed in the "Raw material" column was used to replace the corresponding raw material, the following intermediate 64 was prepared according to a preparation method of the intermediate 4.

| Number | Name | Structure | LC-MS (m/z [M+H] ⁺) | Raw material |
|---|---|---|---|---|
| Intermedia te 64 | 2-fluoro-6-(3-(trifluoromethyl)-1H -1,2,4 -triazol-1-yl)pyridine | 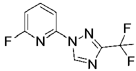 | 233 | 3-(trifluoromethyl)-1H-1,2,4-tri azole (CAS: 60406-75-9) |

### Intermediate 5: 2-(1H-imidazol-1-yl)-4-(methylsulfonyl)pyrimidine

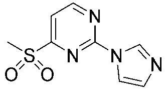

### 2-(1H-imidazol-1-yl)-4-(methylthio)pyrimidine

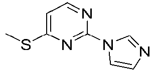

2-chloro-4-methylthiopyrimidine (805 mg, 5 mmol, CAS: 49844-93-1), imidazole (510 mg, 7.5 mmol), cuprous iodide (181 mg, 1.0 mmol), and cesium carbonate (3.26 g, 10 mmol) were added into a 50 mL two-necked flask, then anhydrous N,N-dimethylacetamide (20 mL) was added in a nitrogen atmosphere, and the mixture was stirred at 80°C overnight. Brine (100 mL) was added into the reaction solution, and the reaction solution was extracted with ethyl acetate (30 mL) for 3 times. Organic phases were combined, dried over anhydrous sodium sulfate, concentrated under reduced pressure, and purified by silica gel column chromatography (petroleum ether/ethyl acetate = 1:1) to obtain the title compound as a white solid (682 mg, yield: 71%). LC-MS: m/z [M+H]⁺ = 193.

### 2-(1H-imidazol-1-yl)-4-(methylsulfonyl)pyrimidine

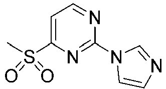

2-(1H-imidazol-1-yl)-4-(methylthio)pyrimidine (682 mg, 3.55 mmol) was dissolved in dichloromethane (20 mL), and m-chloroperoxybenzoic acid (1.85 g, 10.65 mmol) was added in portions at 0°C. The mixture was stirred at the same temperature for 10 minutes, and then heated to room temperature and stirred overnight. A saturated sodium thiosulfate solution (50 mL) was added for quenching, and the mixture was extracted with dichloromethane (30 mL) for 3 times. Organic phases were combined, washed with a saturated sodium bicarbonate solution (30 mL) for 3 times, dried over anhydrous sodium sulfate, concentrated under reduced pressure, and purified by silica gel column chromatography (petroleum ether/ethyl acetate = 1:1) to obtain the title compound as a white solid (604 mg, yield: 76%). LC-MS: m/z [M+H]⁺ = 225.

Referring to the table below, except that raw materials listed in the "Raw material" column were used to replace the corresponding raw material, the following intermediates 13, 15, 30, 32, 36, 40, 43, 45, 46, 48, 51, 52, 57, 71, 74, 75 76 and 77 were prepared according to a preparation method of the intermediate 5.

| Number | Name | Structure | LC-MS (m/z [M+H]⁺ ) | Raw material |
|---|---|---|---|---|
| Intermediat e 13 | 2-(1H-imidazol-1-yl)-4-methyl-6-(methylsulfony 1) pyrimidine | 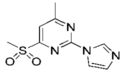 | 239 | 2-chloro-4-methyl-6-methylthiopyr imidine (CAS: 89466-59-1) and imidazole |
| Intermediat e 15 | 4-(methylsulfonyl)-2-(2-(trifluoromethyl)-1H -imidazol-1-yl)pyrimidine | 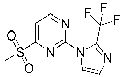 | 293 | 2-chloro-4-methylthiopyrimidine (CAS: 49844-93-1) and 2-(trifluoromethyl)-1H-imidazole (CAS: 66675-22-7) |
| Intermediat e 30 | 4-(methylsulfonyl)-2-(4-(trifluoromethyl)-1H -imidazol-1-yl)pyrimidine | 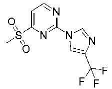 | 293 | 2-chloro-4-methylthiopyrimidine (CAS: 49844-93-1) and 4-(trifluoromethyl)-1H-imidazole (CAS: 33468-69-8) |
| Intermediat e 32 | 2-(4-cyclopropyl-1H-imidazol-1-yl)-4-(methylsulfonyl)pyrimidine | 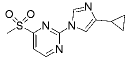 | 265 | 2-chloro-4-methylthiopyrimidine (CAS: 49844-93-1) and 4-cyclopropyl-1H-imidazole (intermediate 31) |
| Intermediat e 36 | 2-methyl-4-(methylsulfonyl) -6-(4-(trifluoromethyl) -1H-imidazol-1-yl)pyrimidine | 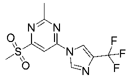 | 307 | 4-chloro-2-methyl-6-(methylthio) pyrimidine (CAS: 867131-59-7) and 4-(trifluoromethyl)-1H-imidazole (CAS: 33468-69-8) |
| Intermediat e 40 | 4-(methylsulfonyl)-2-(3-(trifluoromethyl)-1H-1,2,4-triazol-1-yl)pyrimidi ne | 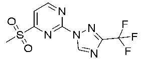 | 294 | 2-chloro-4-methylthiopyrimidine (CAS: 49844-93-1) and 3-(trifluoromethyl)-1H-1,2,4-triazo le (CAS: 60406-75-9) |
| Intermediat e 43 | 2-(3-methyl-1H-1,2,4-triazol -1-yl)-4-(methylsulfonyl) pyrimidine | 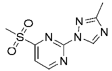 | 240 | 2-chloro-4-methylthiopyrimidine (CAS: 49844-93-1) and 3-methyl-1H-1,2,4-triazole (CAS: 7170-01-6) |
| Intermediat e 45 | 2-(4-chloro-1H-imidazol-1-yl) -4 -(methylsulfonyl)pyrimidine | 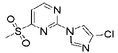 | 259 | 2-chloro-4-methylthiopyrimidine (CAS: 49844-93-1) and 4-chloroimidazole (CAS: 15965-31-8) |
| Intermediat e 46 | 2-(4-cyano-1H-imidazol-1-yl)-4 -(methylsulfonyl)pyrimidine | 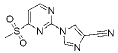 | 250 | 2-chloro-4-methylthiopyrimidine (CAS: 49844-93-1) and 1H-imidazol-4-carbonitrile (CAS: 57090-88-7) |
| Intermediat e 48 | 2-methyl-4-(3-methyl-1H-1,2, 4 -triazol-1-yl)-6-(methylsulfonyl)pyrimidine | 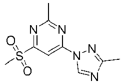 | 254 | 4-chloro-2-methyl-6-(methylthio) pyrimidine (CAS: 867131-59-7) and 3-methyl-1H-1,2,4-triazole (CAS: 7170-01-6) |
| Intermediat e 51 | 4-(4-chloro-1H-imidazol-1-yl) -2-methyl-6-(methylsulfonyl)pyrimidine | 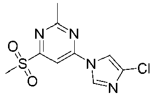 | 273 | 4-chloro-2-methyl-6-(methylthio) pyrimidine (CAS: 867131-59-7) and 4-chloroimidazole (CAS: 15965-31-8) |
| Intermediat e 52 | 4-(4-cyano-1H-imidazol -1-yl)-2-methyl-6-(methylsulfonyl)pyrimidine | 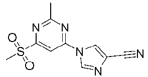 | 264 | 4-chloro-2-methyl-6-(methylthio) pyrimidine (CAS: 867131-59-7) and 1H-imidazol-4-carbonitrile (CAS: 57090-88-7) |
| Intermediat e 57 | 4-(methylsulfonyl)-6-(trifluoro methyl) -2-(4-(trifluoromethyl)-1H -imidazol-1-yl)pyrimidine | 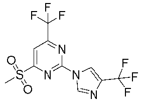 | 361 | Intermediate 56 and 4-(trifluoromethyl)-1H-imidazole (CAS: 33468-69-8) |
| Intermediat e 71 | 2-methyl-4-(methylsulfonyl)-6 (3-(trifluoromethyl)-1H -1,2,4-triazol-1-yl)pyrimidine | 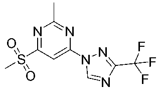 | 308 | 4-chloro-2-methyl-6-(methylthio) pyrimidine (CAS: 867131-59-7) and 3-(trifluoromethyl)-1H-1,2,4-triazo le (CAS: 60406-75-9) |
| Intermediat e 74 | 2-methyl-4-(methylsulfonyl) -6-(4-(trifluoromethyl)-1H -1,2,3-triazol-1-yl)pyrimidine | 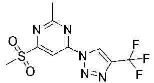 | 308 | 4-chloro-2-methyl-6-(methylthio) pyrimidine (CAS: 867131-59-7) and 5-(trifluoromethyl)-1H-1,2,3-triazo le (CAS: 40964-54-3) |
| Intermediat e 75 | 2-(4-chloro-1H-imidazol-1-yl) -4-(methylsulfonyl) -6-(trifluoromethyl)pyrimidine | 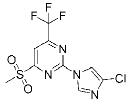 | 327 | Intermediate 56 and 1H-imidazol-4-carbonitrile (CAS: 57090-88-7) |
| Intermediat e 76 | 2-(4-cyano-1H-imidazol-1-yl) -4-(methylsulfonyl) -6-(trifluoromethyl)pyrimidine | 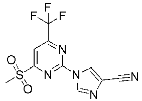 | 318 | Intermediate 56 and 4-chloroimidazole (CAS: 15965-31-8) |
| Intermediat e 77 | 2-methyl-4-(methylsulfonyl) -6-(4-(trifluoromethyl) -2H-1,2,3-triazol-2-yl)pyrimidi ne | 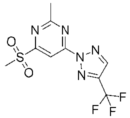 | 308 | 4-chloro-2-methyl-6-(methylthio) pyrimidine (CAS: 867131-59-7) and 5-(trifluoromethyl)-1H-1,2,3-triazo le (CAS: 40964-54-3) |

### Intermediate 6: 6-(1H-imidazol-1-yl)-2-picolinic acid

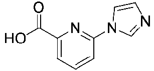

### Methyl 6-(1H-imidazol-1-yl)-2-picolinate

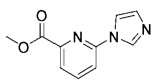

Methyl 6-bromo-2-picolinate (6.48 g, 30 mmol, CAS: 26218-75-7), imidazole (6.13 g, 90 mmol), cuprous iodide (0.6 g, 3 mmol), and potassium carbonate (10.37 g, 75 mmol) were added into a reaction flask, nitrogen replacement was performed for 3 times, N,N-dimethylformamide (100 mL) was added into the reaction flask through a syringe, and the reaction mixture was stirred at 130°C overnight. The reaction solution was diluted with water (300 mL), and extracted with ethyl acetate (50 mL) for 3 times. Organic phases were combined, washed with brine (50 mL) for 3 times, dried over anhydrous sodium sulfate, and concentrated under reduced pressure to obtain the title compound as a white solid (1.5 g, yield: 25%). LC-MS: m/z [M+H]⁺ = 204.

### 6-(1H-imidazol-1-yl)-2-picolinic acid

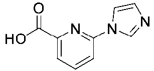

Methyl 6-(1H-imidazol-1-yl)-2-picolinate (1.8 g, 8.9 mmol) was dissolved in a mixed solvent of tetrahydrofuran/methanol (20 mL/20 mL), into which a 2M lithium hydroxide aqueous solution (8.9 mL, 17.8 mmol) was slowly added dropwise. After the addition was completed, the reaction mixture was stirred for a reaction at 50°C for 2 hours. The reaction solution was directly concentrated. The residue was adjusted to pH~2 with 2 N hydrochloric acid, concentrated, and dried to obtain the title compound as a white solid (1.7 g of a crude product). LC-MS: m/z [M+H]⁺ = 190.

Referring to the table below, except that a raw material listed in the "Raw material" column was used to replace the corresponding raw material, the following intermediate 14 was prepared according to a preparation method of the intermediate 6.

| Number | Name | Structure | LC-M S (m/z [M+H] ⁺) | Raw material |
|---|---|---|---|---|
| Intermediat e 14 | 2-(1H-imidazol-1-yl)-6-methylpyr imidin -4-carboxylic acid | 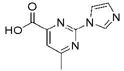 | 205 | Methyl 2-chloro-6-methylpyrimidin -4-carboxylate (CAS: 89793-11-3) |

### Intermediate 7: 2-(1H-imidazol-1-yl)pyrimidin-4-carboxylic acid

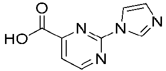

Intermediate 7 was synthesized according to a method provided in Patent Application No. WO2021207186A1.

### Intermediate 8: 3-bromopiperidin-2,6-dione

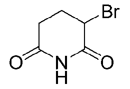

Piperidin-2,6-dione (5 g, 44 mmol, CAS: 1121-89-7) was dissolved in acetic acid (10 mL), and liquid bromine (2.3 mL, 45 mmol, d = 3.119 g/mL) was slowly added dropwise. The reaction solution was refluxed at 130°C for 12 hours, and then concentrated. The residue was neutralized with saturated sodium bicarbonate solution, and extracted with dichloromethane (50 mL) for 3 times. Organic phases were combined, washed with brine (50 mL), dried over anhydrous sodium sulfate, concentrated under reduced pressure, and purified by silica gel column chromatography (petroleum ether/ethyl acetate = 5:1) to obtain the title compound as a grayish-white solid (5.50 g, yield: 65%). LC-MS: m/z [M+H]⁺ = 192/194.

### Intermediate 9: 1-(4-(chloromethyl)benzyl)-4-((di-tert-butoxycarbonyl)amino)piperidine

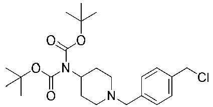

### Benzyl 4-((tert-butoxycarbonyl)amino)piperidin-1-formate

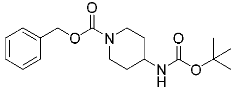

4-tert-butoxycarbonylaminopiperidine (3.72 g, 20 mmol, CAS: 73874-95-0) was added into a reaction flask, and anhydrous tetrahydrofuran (20 mL) was added into the reaction flask through a syringe. Under ice bath conditions, benzyl chloroformate (4.1 g, 24 mmol) was added in portions. The mixture was stirred at room temperature for 2 hours. The reaction solution was diluted with water (100 mL), and extracted with ethyl acetate (30 mL) for 3 times. Organic phases were combined, washed with brine (30 mL) for 3 times, dried over anhydrous sodium sulfate, concentrated under reduced pressure, and purified by silica gel column chromatography (petroleum ether/ethyl acetate = 5:1) to obtain the title compound as a white solid (5.9 g, yield: 88%). LC-MS: m/z [M+H]⁺ = 335.

### Benzyl 4-((di-tert-butoxycarbonyl)amino)piperidin-1-formate

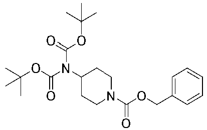

Benzyl 4-((tert-butoxycarbonyl)amino)piperidin-1-formate (5.9 g, 17.6 mmol) was added into a reaction flask, and anhydrous tetrahydrofuran (20 mL) was added into the reaction flask through a syringe in a nitrogen atmosphere atmosphere. The temperature was lowered to -78°C, a 2 M lithium bis(trimethylsilyl)amide tetrahydrofuran solution (11 mL, 22 mmol) was added dropwise into the reaction solution, and the mixture was stirred at -78°C for 0.5 hour. Di-tert-butyl dicarbonate (7.68 g, 35.2 mmol, CAS: 24424-99-5) was slowly added dropwise into the reaction solution, and the mixture was stirred at room temperature for 18 hours. A saturated ammonium chloride solution (50 mL) was added dropwise into the reaction solution to quench the reaction, and the reaction solution was extracted with ethyl acetate (40 mL) for 3 times. Organic phases were combined, washed with brine (30 mL) for 3 times, dried over anhydrous sodium sulfate, concentrated under reduced pressure, and subjected to silica gel column chromatography (petroleum ether/ethyl acetate = 5:1) to obtain the title compound as a white solid (5.7 g, yield: 75%). LC-MS: m/z [M+H]⁺ = 435.

### 4-((di-tert-butoxycarbonyl)amino)piperidine

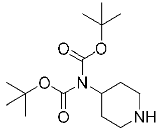

Benzyl 4-((di-tert-butoxycarbonyl)amino)piperidin-1-formate (5.7 g, 13.2 mmol) was added into a reaction flask. Methanol (15 mL), tetrahydrofuran (5 mL), and a palladium hydroxide on carbon catalyst (1.85 g) were added into the reaction flask through a syringe. Hydrogen replacement was performed for 3 times, and the mixture was stirred at room temperature for 12 hours. The reaction solution was filtered through celite, and The filtrate was concentrated under reduced pressure to obtain the title compound as a colorless liquid (3.96 g, yield: 100%). LC-MS: m/z [M+H]⁺ = 301.

### 1-(4-(chloromethyl)benzyl)-4-((di-tert-butoxycarbonyl)amino)piperidine

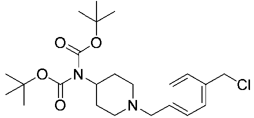

4-((di-tert-butoxycarbonyl)amino)piperidine (3.96 g, 13.2 mmol) was added into a reaction flask. N,N-dimethylformamide (15 mL), acetonitrile (15 mL), and N,N-diisopropylethylamine (4.4 mL, 26.4 mmol, d = 0.782 g/mL) were added into the reaction flask through a syringe. In during stirring, 1,4-bis(chloromethyl)benzene (5.18 g, 29.6 mmol) was added in portions. The mixture was stirred at room temperature for 30 minutes, and then heated to 60°C for 1.5 hours. The reaction solution was diluted with brine (75 mL), and extracted with ethyl acetate (30 mL) for 3 times. Organic phases were combined, washed with brine (30 mL) for 3 times, dried over anhydrous sodium sulfate, concentrated under reduced pressure, and purified by silica gel column chromatography (petroleum ether/ethyl acetate = 5:1) to obtain the title compound as a white solid (3.8 g, yield: 65%). LC-MS: m/z [M+H]⁺ = 439.

### Intermediate 10: Tert-butyl 4-((4-(4-(chloromethyl)benzyl)piperazin-1-yl)methyl)piperidin-1-formate

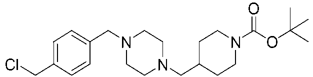

### Benzyl 4-((1-(tert-butoxycarbonyl)piperidin-4-yl)methyl)piperazin-1-carboxylate

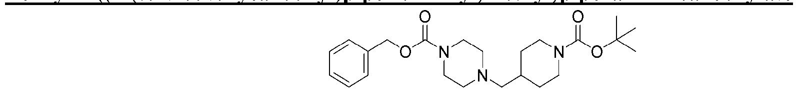

Benzyl piperazin-1-carboxylate (4.4 g, 20 mmol, CAS: 31166-44-6) and 1-tert-butoxycarbonylpiperidin-4-carboxaldehyde (4.3 g, 20 mmol, CAS: 137076-22-3) were added into a round-bottomed flask, anhydrous dichloroethane (50 mL) was added in a nitrogen atmosphere, and then acetic acid (0.9 mL, 40 mmol, d = 1.05 g/mL) was added. The mixture was stirred at room temperature for 2 hours, then sodium triacetoxyborohydride (9 g, 40 mmol, CAS: 56553-60-7) was slowly added in a nitrogen atmosphere, and the reaction mixture was stirred at room temperature for 2 hours. A saturated sodium carbonate solution (50 mL) was added into the reaction solution to quench the reaction, and the reaction solution was extracted with dichloromethane (40 mL) for 3 times. Organic phases were combined, concentrated under reduced pressure, and separated by silica gel column chromatography (dichloromethane/methanol = 30:1) to obtain the title compound as a colorless oil (7.1 g, yield: 85%). LC-MS: m/z [M+H]⁺ = 418.

### Tert-butyl 4-(piperazin-1-ylmethyl)piperidin-1-formate

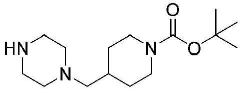

Benzyl 4-((1-(tert-butoxycarbonyl)piperidin-4-yl)methyl)piperazin-1-carboxylate (7.1 g, 17 mmol) and palladium on carbon (1.1 g, 1.7 mmol) were added into tetrahydrofuran (5 mL) and methanol (10 mL) in a hydrogen atmosphere, and the mixture was stirred at room temperature in a hydrogen atmosphere for 3 hours. The reaction solution was filtered through celite, and The filtrate was concentrated under reduced pressure to obtain the title compound as a colorless oil (5.1 g, yield: 106%). LC-MS: m/z [M+H]⁺ = 284.

### Tert-butyl 4-((4-(4-(chloromethyl)benzyl)piperazin-1-yl)methyl)piperidin-1-formate

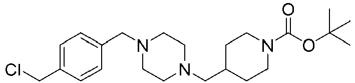

1,4-bis(chloromethyl)benzene (9.5 g, 51 mmol) was added into a reaction flask, anhydrous acetonitrile (20 mL) and N,N-dimethylformamide (20 mL) were added into the reaction flask through a syringe, and the mixture was stirred at room temperature until 1,4-bis(chloromethyl)benzene was completely dissolved. N,N-diisopropylethylamine (4.8 mL, 34 mmol, d = 0.742 g/mL) was slowly added dropwise into the reaction flask through a syringe, and after the addition was completed, the reaction solution was stirred at room temperature for 30 minutes. Then, tert-butyl 4-(piperazin-1-ylmethyl)piperidin-1-formate (5.1 g, 17 mmol) was dissolved in a mixed solvent of acetonitrile (30 mL) and N,N-dimethylformamide (30 mL), and then added dropwise into the above reaction solution. The reaction solution was stirred at room temperature for 2 hours. Brine (200 mL) was added into the reaction solution, and the reaction solution was extracted with ethyl acetate (50 mL) for 3 times. Organic phases were combined, dried over anhydrous sodium sulfate, concentrated under reduced pressure, and purified by silica gel column chromatography (dichloromethane/methanol = 30:1) to obtain the title compound as a white solid (3.08 g, yield: 43%). LC-MS: m/z [M+H]⁺ = 422.

Referring to the table below, except that a raw material listed in the "Raw material" column was used to replace the corresponding raw material, the following intermediate 41 was prepared according to a preparation method of the intermediate 10.

| Number | Name | Structure | LC-MS (m/z [M+H]⁺ ) | Raw material |
|---|---|---|---|---|
| Intermedia te 41 | Tert-butyl 4-(4-(4-(chloromethyl)benzyl ) piperazin-1-yl)piperidin-1-fo rmate | 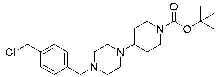 | 408 | N-tert-butoxycarbonyl-4-piperidone (CAS: 79099-07-3) |

### Intermediate 11:

### 3-(6-(4-((4-aminopiperidin-1-yl)methyl)benzyl)-2-oxobenzo[cd]indol-1(2H)-yl)piperidin-2,6-dione

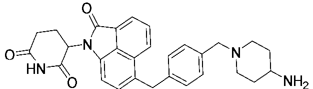

### Tert-butyl (1-(4-((2-oxo-1,2-dihydrobenzo[cd]indol-6-yl)methyl)benzyl)piperidin-4-yl)amino dicarboxylate

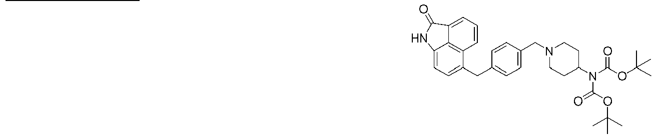

1-(4-(chloromethyl)benzyl)-4-((di-tert-butoxycarbonyl)amino)piperidine (3.8 g, 8.58 mmol, intermediate 9), 6-(4,4,5,5-tetramethyl-1,3,2-dioxaborolan-2-yl)benzo[cd]indol-2(1H)-one (2.1 g, 7.15 mmol, synthesized according to a method provided in Patent Application No. WO2020210630A1), potassium phosphate (4.5 g, 21.5 mmol), tris(dibenzylideneacetone)dipalladium (363 mg, 0.36 mmol, CAS: 51364-51-3), and tri(2-methylphenyl)phosphine (218 mg, 0.715 mmol, CAS: 6163-58-2) were added into a reaction flask, toluene (20 mL) and ethanol (10 mL) were added in a nitrogen atmosphere, and the mixture was heated at 100°C for 3 hours. The reaction solution was filtered through celite, and The filtrate was concentrated under reduced pressure, and purified by silica gel column chromatography (petroleum ether/ethyl acetate = 1:1) to obtain the title compound as a yellow solid (2.7 g, yield: 66%). LC-MS: m/z [M+H]⁺ = 572.

### Tert-butyl (1-(4-((1-(2.6-dioxopiperidin-3-yl)-2-oxo-1,2-dihydrobenzo[cd]indol-6-yl)methyl)benzyl) piperidin-4-yl)aminodicarboxylate

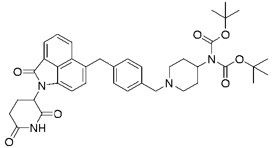

Tert-butyl (1-(4-((2-oxo-1,2-dihydrobenzo[cd]indol-6-yl)methyl)benzyl)piperidin-4-yl)aminodicarboxylate (2.7 g, 4.6 mmol) and anhydrous tetrahydrofuran (30 mL) were added into a round-bottomed flask, sodium hydride (1.84 g, 46 mmol, 60% stored in mineral oil) was added in portions at 0°C, and the mixture was stirred at 0°C for 20 minutes. 3-bromopiperidin-2,6-dione (7.0 g, 36.8 mmol, intermediate 8) was added at 0°C, and the reaction mixture was heated at 70°C overnight. Brine (50 mL) was added into the reaction solution to quench the reaction, and ethyl acetate (30 mL) was added for extraction for 3 times. Organic phases were combined, dried over anhydrous sodium sulfate, concentrated under reduced pressure, and subjected to silica gel column chromatography (petroleum ether/ethyl acetate = 1:3) to obtain the title compound as a yellow solid (1.4 g, yield: 45%). LC-MS: m/z [M+H]⁺ = 683.

### 3-(6-(4-((4-aminopiperidin-1-yl)methyl)benzyl)-2-oxobenzo[cd]indol-1(2H)-yl)piperidin-2.6-dione

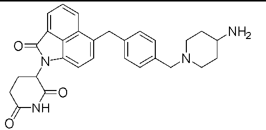

Tert-butyl (1-(4-((1-(2,6-dioxopiperidin-3-yl)-2-oxo-1,2-dihydrobenzo[cd]indol-6-yl)methyl)benzyl)piperidin-4-yl)ami nodicarboxylate (683 mg, 1.0 mmol) and dichloromethane (8 mL) were added into a reaction flask, and trifluoroacetic acid (2 mL) was slowly added dropwise at 0°C. The mixture was stirred at 0°C for 10 minutes, and then warmed to room temperature and stirred overnight. The reaction solution was directly concentrated under reduced pressure to obtain the title compound as a yellow solid (483 mg, yield: 81%, TFA salt). LC-MS: m/z [M+H]⁺ = 483.

Referring to the table below, except that raw materials listed in the "Raw material" column were used to replace the corresponding raw material, the following intermediates 12, 34, and 42 were prepared according to a preparation method of the intermediate 11.

| Number | Name | Structure | LC-M S (m/z [M+H ]⁺) | Raw material |
|---|---|---|---|---|
| Intermedi ate 12 | 3-(2-oxo-6-(4-((4-(piperidin-4-ylmethyl)piperazin-1 -yl) methyl)benzyl)benzo[cd]indol-1(2H)-yl)piperidin-2 ,6-dione | 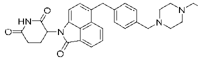 | 566 | Intermedi ate 10 |
| Intermedi ate 34 | 3-(6-(4-(4-((((1s,4s)-4-aminocyclohexyl)oxy)piperi din-1-yl) methyl)benzyl)-2-oxobenzo[cd]indol-1(2H)-yl)pipe ridin-2,6-dione | 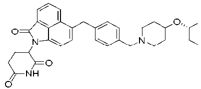 | 581 | Intermedi ate 33 |
| Intermedi ate 42 | 3-(2-oxo-6-(4-((4-(piperidin-4-yl)piperazin-1-yl) methyl)benzyl)benzo[cd]indol-1(2H)-yl)piperidin-2 ,6-dione | 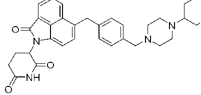 | 552 | Intermedi ate 41 |

### Intermediate 16: (S)-3-(4-((4-(chloromethyl)benzyl)oxy)-1-oxoisoindolin-2-yl)piperidin-2,6-dione

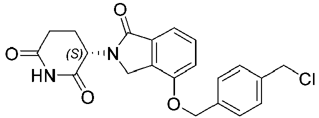

### Tert-butyl (S)-5-amino-4-(4-((4-(chloromethyl)benzyl)oxy)-1-oxoisoindolin-2-yl)-5-oxopentanoate

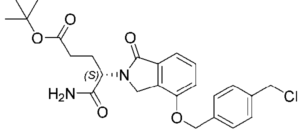

Tert-butyl (S)-5-amino-4-(4-hydroxy-1-oxoisoindolin-2-yl)-5-oxopentanoate (3.6 g, 10.8 mmol, intermediate 1), 1,4-bis(chloromethyl)benzene (5.67 g, 32.4 mmol, CAS: 623-25-6), and potassium carbonate (4.48 g, 32.4 mmol) were added into a reaction flask, acetonitrile (90 mL) was added into the reaction flask through a syringe, and the reaction solution was stirred at 45°C for 13.5 hours. The reaction solution was filtered. The filter cake was washed with ethyl acetate (30 mL). The filtrate was concentrated under reduced pressure, and purified by silica gel column chromatography (petroleum ether/ethyl acetate = 1:1) to obtain the title compound as a white solid (4.1 g, yield: 80%). ¹H NMR (400 MHz, CDCl₃) δ 7.45 - 7.39 (m, 6H), 7.05 (d, J = 7.7 Hz, 1H), 6.39 (s, 1H), 5.46 (s, 1H), 5.15 (s, 2H), 4.92 - 4.88 (m, 1H), 4.61 (s, 2H), 4.50 (d, J = 17.6 Hz, 1H), 4.41 (d, J = 17.6 Hz, 1H), 2.43 - 2.10 (m, 4H), 1.41 (s, 9H).

### (S)-3-(4-((4-(chloromethyl)benzyl)oxy)-1-oxoisoindolin-2-yl)piperidin-2,6-dione

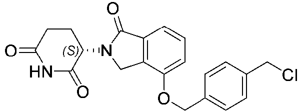

Tert-butyl (S)-5-amino-4-(4-((4-(chloromethyl)benzyl)oxy)-1-oxoisoindolin-2-yl)-5-oxopentanoate (3.8 g, 8.03 mmol) and benzenesulfonic acid (1.65 g, 10.4 mmol, CAS: 98-11-3) were added into a reaction flask., acetonitrile (80 mL) was added into the reaction flask through a syringe, and the reaction solution was stirred at 85°C for 23 hours. At 0°C, a saturated sodium bicarbonate solution was added dropwise into the reaction solution until a pH was 8, and the reaction solution was extracted with ethyl acetate (40 mL) for 2 times. Organic phases were combined, dried over anhydrous sodium sulfate, concentrated under reduced pressure, and purified by silica gel column chromatography (petroleum ether/ethyl acetate = 1:1) to obtain the title compound as a white solid (2.2 g, yield: 69%). ¹H NMR (400 MHz, CDCl₃) δ 8.18 (s, 1H), 7.51 (d, J = 7.3 Hz, 1H), 7.45 - 7.39 (m, 5H), 7.08 (d, J = 7.9 Hz, 1H), 5.23 (dd, J = 13.3, 5.1 Hz, 1H), 5.15 (s, 2H), 4.60 (s, 2H), 4.45 (d, J = 16.6 Hz, 1H), 4.31 (d, J = 16.6 Hz, 1H), 2.94 - 2.78 (m, 2H), 2.41 - 2.29 (m, 1H), 2.24 - 2.17 (m, 1H).

### Intermediate 17: (2-(1H-imidazol-1-yl)pyrimidin-4-yl)(piperazin-1-yl)methanone hydrochloride

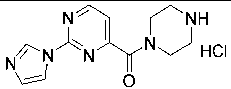

### Tert-butyl 4-(2-(1H-imidazol-1-yl)pyrimidin-4-carbonyl)piperazin-1-carboxylate

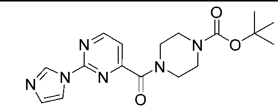

2-(1H-imidazol-1-yl)pyrimidin-4-carboxylic acid (209.2 mg, 1.1 mmol, intermediate 7), tert-butyl piperazine-1-carboxylate acetate (204.9 mg, 1.1 mmol, CAS: 143238-38-4), and 2-(7-azabenzotriazol)-N,N,N',N'-tetramethyluronium hexafluorophosphate (627.3 mg, 1.65 mmol, CAS: 148893-10-1) were added into a reaction flask, anhydrous N,N-dimethylformamide (5 mL) was added into the reaction flask through a syringe in a nitrogen atmosphere, N,N-diisopropylethylamine (355.0 mg, 2.75 mmol, CAS: 7087-68-5) was added in during stirring, and the reaction solution was stirred at room temperature for 5 hours. The reaction solution was diluted with brine (30 mL), and extracted with ethyl acetate (15 mL) for 2 times. Organic phases were combined, dried over anhydrous sodium sulfate, concentrated under reduced pressure, and purified by silica gel column chromatography (dichloromethane/methanol = 30:1) to obtain the title compound as a yellow solid (230 mg, yield: 58%). ¹H NMR (400 MHz, CDCl₃) δ 8.83 (d, J = 5.0 Hz, 1H), 8.58 (s, 1H), 7.84 (s, 1H), 7.48 (d, J = 4.8 Hz, 1H), 7.17 (s, 1H), 3.79 = 3.76 (m, 2H), 3.57 - 3.56 (m, 4H), 3.52 - 3.50 (m, 2H), 1.46 (s, 9H).

### (2-(1H-imidazol-1-yl)pyrimidin-4-yl)(piperazin-1-yl)methanone hydrochloride

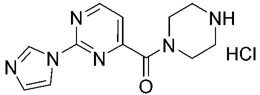

Tert-butyl 4-(2-(1H-imidazol-1-yl)pyrimidin-4-carbonyl)piperazin-1-carboxylate (200.0 mg, 0.56 mmol) was added into a reaction flask, ethyl acetate (1 mL) and dichloromethane (1 mL) were added into the reaction flask through a syringe, a saturated solution of hydrogen chloride in ethyl acetate (5 mL) was added dropwise at 0°C, and the reaction solution was stirred at room temperature for 16 hours. The reaction solution was filtered. The filter cake was washed with ethyl acetate (10 mL), collected, and dried to obtain the title compound as a white solid (140 mg, yield: 85%). LC-MS: m/z [M+H]⁺ = 259.

### Intermediate 18: Tert-butyl 4-((1R,4R)-4-aminocyclohexyl)piperazin-1-carboxylate

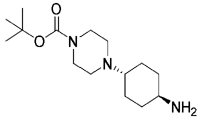

### Tert-butyl 4-((1r,4r)-4-(((benzyloxy)carbonyl)amino)cyclohexyl)piperazin-1-carboxylate

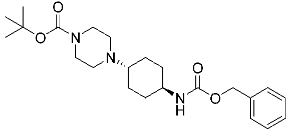

Tert-butyl piperazine-1-carboxylate acetate (1.86 g, 10 mmol, CAS: 143238-38-4) and 4-N-benzyloxycarbonylaminocyclohexanone (2.47 g, 10 mmol, CAS: 16801-63-1) were added into a reaction flask, dichloromethane (35 mL) was added into the reaction flask through a syringe in a nitrogen atmosphere, and acetic acid (240 mg, 4 mmol) was added into the reaction flask through a syringe in during stirring. Then, sodium triacetoxyborohydride (6.4 g, 30 mmol, CAS: 56553-60-7) was added in 3 portions at 0°C, and the reaction mixture was stirred at room temperature for 16 hours. At 0°C, a saturated sodium bicarbonate solution was added dropwise into the reaction solution until pH ~8, and the reaction solution was extracted with dichloromethane (20 mL) for 3 times. Organic phases were combined, dried over anhydrous sodium sulfate, concentrated under reduced pressure, and purified by silica gel column chromatography (dichloromethane/methanol = 40:1) to obtain the title compound as a white solid (2.1 g, yield: 50%). ¹H NMR (400 MHz, CDCl₃) δ 7.38 - 7.30 (m, 5H), 5.07 (s, 2H), 4.61 (d, J = 7.7 Hz, 1H), 3.48 - 3.40 (m, 5H), 2.48 (s, 4H), 2.27 (t, J = 11.8 Hz, 1H), 2.08 (d, J = 12.2 Hz, 2H), 1.88 (d, J = 12.5 Hz, 2H), 1.45 (s, 9H), 1.40 - 1.30 (m, 2H), 1.18 - 1.08 (m, 2H).

### Tert-butyl 4-((1R,4R)-4-aminocyclohexyl)piperazin-1-carboxylate

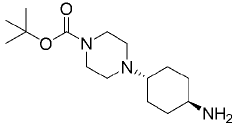

Tert-butyl 4-((1R,4R)-4-(((benzyloxy)carbonyl)amino)cyclohexyl)piperazin-1-carboxylate (300 mg, 0.7 mmol) was added into a reaction flask, methanol (2 mL) was added into the reaction flask through a syringe in a hydrogen atmosphere, and palladium on carbon (30 mg, CAS: 7440-05-3) was added. The reaction mixture was stirred at room temperature in a hydrogen atmosphere for 16 hours. The reaction solution was filtered through celite. The filter cake was washed with ethyl acetate (10 mL). The filtrate was concentrated under reduced pressure to obtain the title compound as a white solid (198 mg, yield: 100%). ¹H NMR (400 MHz, CDCl₃) δ 3.41 - 3.39 (m, 4H), 2.69 - 2.62 (m, 1H), 2.47 (t, J = 4.9 Hz, 4H), 2.29 - 2.22 (m, 1H), 1.93 (d, J = 12.0 Hz, 2H), 1.86 (d, J = 12.2 Hz, 2H), 1.44 (s, 9H), 1.35 - 1.07 (m, 4H).

### Intermediate 19:

### 2-(1H-imidazol-1-yl)-N-((1R,4R)-4-(piperazin-1-yl)cyclohexyl)pyrimidin-4-carboxamide hydrochloride

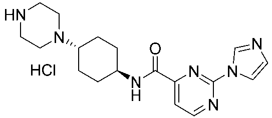

### Tert-butyl 4-((1R,4R)-4-(2-(1H-imidazol-1-yl)pyrimidin-4-carboxamido)cyclohexyl)piperazin-1-carboxylate

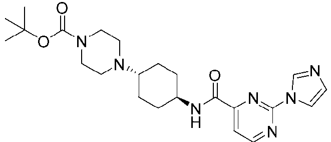

2-(1H-imidazol-1-yl)pyrimidin-4-carboxylic acid (88 mg, 0.46 mmol, intermediate 7), tert-butyl 4-((1R,4R)-4-aminocyclohexyl)piperazin-1-carboxylate (130 mg, 0.46 mmol, intermediate 18), and 2-(7-azabenzotriazol)-N,N,N',N'-tetramethyluronium hexafluorophosphate (262 mg, 0.69 mmol, CAS: 148893-10-1) were added into a reaction flask, anhydrous N,N-dimethylformamide (5 mL) was added into the reaction flask through a syringe in a nitrogen atmosphere, and N,N-diisopropylethylamine (149 mg, 1.15 mmol, CAS: 7087-68-5) was added in during stirring. The reaction solution was stirred at room temperature for 11 hours. The reaction solution was diluted with brine (10 mL), and extracted with ethyl acetate (5 mL) for 2 times. Organic phases were combined, dried over anhydrous sodium sulfate, concentrated under reduced pressure, and purified by silica gel column chromatography (dichloromethane/methanol = 30:1) to obtain the title compound as a yellow solid (146 mg, yield: 70%). LC-MS: m/z [M+H]⁺ = 456.

### 2-(1H-imidazol-1-yl)-N-((1R,4R)-4-(piperazin-1-yl)cyclohexyl)pyrimidin-4-carboxamide hydrochloride

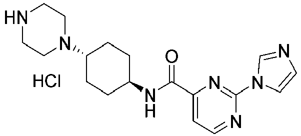

Tert-butyl 4-((1R,4R)-4-(2-(1H-imidazol-1-yl)pyrimidin-4-carboxamido)cyclohexyl)piperazin-1-carboxylate (220 mg, 0.48 mmol) was added into a reaction flask, ethyl acetate (3 mL) was added into the reaction flask through a syringe, and a saturated solution of hydrogen chloride in ethyl acetate (10 mL) was added dropwise at 0°C. The reaction solution was stirred at room temperature for 7 hours. The reaction solution was filtered. The filter cake was washed with ethyl acetate (10 mL), collected, and dried to obtain the title compound as a white solid (150 mg, yield: 80%). LC-MS: m/z [M+H]⁺ = 356.

Referring to the table below, except that raw materials listed in the "Raw material" column were used to replace the corresponding raw material, the following intermediates 20 and 22 were prepared according to a preparation method of the intermediate 19.

| Number | Name | Structure | LC-MS (m/z [M+H]⁺ ) | Raw material |
|---|---|---|---|---|
| Intermediat e 20 | 6-(1H-imidazol-1-yl)-N-((1R,4 R) -4-(piperazin-1-yl) cyclohexyl)pyridin-2-carboxam ide hydrochloride | 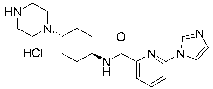 | 355 | Intermediate 6 and intermediate 18 |
| Intermediat e 22 | N-([1,4'-bipiperidin]-4-yl) -2-(1H-imidazol-1-yl) pyrimidin-4-carboxamide hydrochloride | 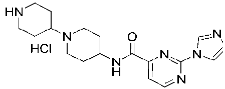 | 356 | Intermediate 7 and tert-butyl 4-amino-[1,4'-bipiperidi n] -1'-carboxylate (CAS: 959237-16-2) |

### Intermediate 21: 1-((1-(6-(1H-imidazol-1-yl)pyridin-2-yl)piperidin-4-yl)methyl)piperazine hydrochloride

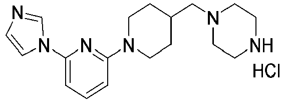

### (1-(6-bromopyridin-2-yl)piperidin-4-yl)methanol

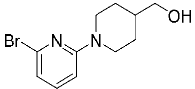

2-bromo-6-fluoropyridine (1.5 g, 8.5 mmol, CAS: 144100-07-2) and 4-hydroxymethylpiperidine (982 mg, 8.5 mmol, CAS: 6457-49-4) were added into a reaction flask, 1,4-dioxane (10 mL) was added into the reaction flask through a syringe, and triethylamine (946 mg, 9.4 mmol) was added in during stirring. The reaction solution was heated to 110°C, and refluxed under stirring for 8 hours. The reaction solution was cooled to room temperature, concentrated under reduced pressure, and purified by silica gel column chromatography (petroleum ether/ethyl acetate = 4:1) to obtain the title compound as a white solid (1.62 g, yield: 70%). ¹H NMR (400 MHz, CDCl₃) δ 7.27 - 7.23 (m, 1H), 6.69 (d, J = 7.4 Hz, 1H), 6.53 (d, J = 8.4 Hz, 1H), 4.29 (d, J = 13.3 Hz, 2H), 3.53 (d, J = 6.2 Hz, 2H), 2.83 (td, J = 12.7, 2.2 Hz, 2H), 1.84 - 1.71 (m, 3H), 1.26 (qd, J = 12.6, 4.2 Hz, 2H).

### 2-bromo-6-(4-(((tert-butyldimethylsilyl)oxy)methyl)piperidin-1-yl)pyridine

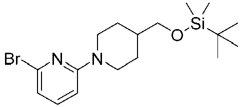

(1-(6-bromopyridin-2-yl)piperidin-4-yl)methanol (1.2 g, 4.4 mmol) and imidazole (452 mg, 6.6 mmol) were added into a reaction flask, dichloromethane (10 mL) was added into the reaction flask through a syringe in a nitrogen atmosphere, and a solution of tert-butyldimethylsilyl chloride (995 mg, 6.6 mmol, CAS: 18162-48-6) in dichloromethane (15 mL) was added dropwise into the reaction flask at 0°C. The reaction mixture was stirred at room temperature for 1 hour. The reaction solution was filtered. The filter cake was washed with ethyl acetate (15 mL). The filtrate was concentrated under reduced pressure, and purified by silica gel column chromatography (petroleum ether/ethyl acetate = 3:1) to obtain the title compound as a white solid (1.57 g, yield: 93%). LC-MS: m/z [M+H]⁺ = 385.

### 2-(4-(((tert-butyldimethylsilyl)oxy)methyl)piperidin-1-yl)-6-(1H-imidazol-1-yl)pyridine

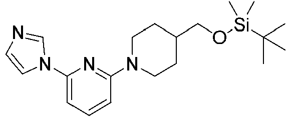

2-bromo-6-(4-(((tert-butyldimethylsilyl)oxy)methyl)piperidin-1-yl)pyridine (1.1 g, 2.85 mmol), imidazole (972 mg, 14.3 mmol), a tris(dibenzylideneacetone)dipalladium-chloroform adduct (148 mg, 0.14 mmol, CAS: 52522-40-4), 2-di-tert-butylphosphino-2',4',6'-triisopropylbiphenyl (109 mg, 0.26 mmol, CAS: 564483-19-8), and potassium phosphate (1.2 g, 5.7 mmol, CAS: 7778-53-2) were added into a reaction flask, and toluene (20 mL) was added into the reaction flask through a syringe in a nitrogen atmosphere. The reaction solution was stirred at 110°C for 8 hours. The reaction solution was cooled to room temperature, and filtered through celite. The filter cake was washed with ethyl acetate (10 mL). The filtrate was concentrated under reduced pressure, and purified by silica gel column chromatography (petroleum ether/ethyl acetate = 8:1) to obtain the title compound as a colorless oily liquid (977 mg, yield: 92%). ¹H NMR (400 MHz, CDCl₃) δ 8.64 (s, 1H), 7.92 (s, 1H), 7.87 (t, J = 8.0 Hz, 1H), 7.48 (s, 1H), 6.89 (t, J = 8.3 Hz, 2H), 4.71 (d, J = 13.2 Hz, 2H), 3.81 (d, J = 6.2 Hz, 2H), 3.24 - 3.18 (m, 2H), 2.18 - 2.05 (m, 3H), 1.58 (qd, J = 12.4, 4.4 Hz, 2H), 1.23 (s, 9H), 0.38 (s, 6H).

### (1-(6-(1H-imidazol-1-yl)pyridin-2-yl)piperidin-4-yl)methanol

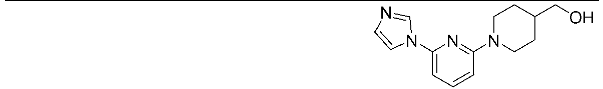

2-(4-(((tert-butyldimethylsilyl)oxy)methyl)piperidin-1-yl)-6-(1H-imidazol-1-yl)pyridine (1.0 g, 2.68 mmol) was added into a reaction flask, anhydrous tetrahydrofuran (15 mL) was added into the reaction flask through a syringe in a nitrogen atmosphere, and a 1 M solution of tetrabutylammonium fluoride in tetrahydrofuran (4 mL, 4 mmol, CAS: 429-41-4) was added dropwise at 0°C. The reaction mixture was stirred at room temperature for 2 hours. Brine (50 mL) was added into the reaction solution, and the reaction solution was extracted with ethyl acetate (30 mL) for 2 times. Organic phases were combined, dried over anhydrous sodium sulfate, concentrated under reduced pressure, and purified by silica gel column chromatography (petroleum ether/ethyl acetate = 1:1) to obtain the title compound as a white solid (597 mg, yield: 86%). ¹H NMR (400 MHz, CDCl₃) δ 8.30 (s, 1H), 7.56 - 7.51 (m, 2H), 7.13 (s, 1H), 6.57 - 6.53 (m, 2H), 4.38 (d, J = 13.2 Hz, 2H), 3.53 (d, J = 6.2 Hz, 2H), 2.89 (td, J = 12.9, 2.2 Hz, 2H), 2.44 (s, 1H), 1.87 - 1.75 (m, 3H), 1.28 (qd, J = 12.6, 4.3 Hz, 2H).

### (1-(6-(1H-imidazol-1-yl)pyridin-2-yl)piperidin-4-yl)methyl-4-methylbenzenesulfonate

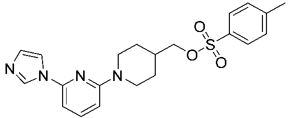

(1-(6-(1H-imidazol-1-yl)pyridin-2-yl)piperidin-4-yl)methanol (450 mg, 1.74 mmol) was added into a reaction flask, anhydrous dichloromethane (6 mL) was added into the reaction flask through a syringe in a nitrogen atmosphere, and a solution of p-toluenesulfonyl chloride (498 mg, 2.61 mmol, CAS: 98-59-9) in anhydrous dichloromethane (8 mL) was added dropwise into the reaction flask at 0°C. The reaction solution was stirred at room temperature for 2 hours. The reaction solution was filtered. The filter cake was washed with ethyl acetate (30 mL). The filtrate was concentrated under reduced pressure, and purified by silica gel column chromatography (petroleum ether/ethyl acetate = 1:1) to obtain the title compound as a yellow oil (413 mg, yield: 58%). ¹H NMR (400 MHz, CDCl₃) δ 8.27 (s, 1H), 7.77 (d, J = 8.1 Hz, 2H), 7.54 - 7.50 (m, 2H), 7.34 (d, J = 8.1 Hz, 2H), 7.12 (s, 1H), 6.56 (d, J = 7.6 Hz, 1H), 6.50 (d, J = 8.5 Hz, 1H), 4.33 (d, J = 13.2 Hz, 2H), 3.87 (d, J = 6.5 Hz, 2H), 2.83 (t, J = 12.7 Hz, 2H), 2.43 (s, 3H), 2.01 - 1.91 (m, 1H), 1.77 (d, J = 12.7 Hz, 2H), 1.28 - 1.18 (m, 2H).

### 2-(4-(bromomethyl)piperidin-1-yl)-6-(1H-imidazol-1-yl)pyridine

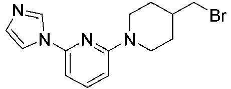

(1-(6-(1H-imidazol-1-yl)pyridin-2-yl)piperidin-4-yl)methyl-4-methylbenzenesulfonate (300 mg, 0.72 mmol) and lithium bromide (126 mg, 1.45 mmol, CAS: 7550-35-8) were added into a reaction flask, and acetone (8 mL) was added into the reaction flask through a syringe. The reaction solution was stirred at 60°C for 17 hours. The reaction solution was concentrated under reduced pressure, and purified by silica gel column chromatography (petroleum ether/ethyl acetate = 2:1) to obtain the title compound as a yellow oil (202 mg, yield: 87%). LC-MS: m/z [M+H]⁺ = 321.

### Tert-butyl 4-((1-(6-(1H-imidazol-1-yl)pyridin-2-yl)piperidin-4-yl)methyl)piperazin-1-carboxylate

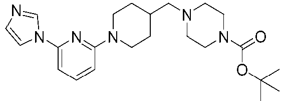

2-(4-(bromomethyl)piperidin-1-yl)-6-(1H-imidazol-1-yl)pyridine (450 mg, 1.44 mmol), tert-butyl piperazine-1-carboxylate acetate (322 mg, 1.73 mmol, CAS: 143238-38-4), and potassium carbonate (299 mg, 2.16 mmol) were added into a reaction flask, and acetonitrile (12 mL) was added into the reaction flask through a syringe. The reaction solution was stirred at 85°C for 17 hours. The reaction solution was filtered. The filter cake was washed with ethyl acetate (10 mL). The filtrate was concentrated under reduced pressure, and purified by silica gel column chromatography (dichloromethane/methanol = 30:1) to obtain the title compound as a colorless oil (473 mg, yield: 77%). ¹H NMR (400 MHz, CDCl₃) δ 8.31 (s, 1H), 7.58 (s, 1H), 7.49 (t, J = 8.0 Hz, 1H), 7.13 (s, 1H), 6.52 (d, J = 7.6 Hz, 1H), 6.22 (d, J = 8.3 Hz, 1H), 3.71 (t, J = 8.7 Hz, 1H), 3.63 - 3.59 (m, 1H), 3.47 - 3.37 (m, 5H), 3.05 (t, J = 9.3 Hz, 1H), 2.45 - 2.27 (m, 7H), 2.21 - 2.14 (m, 1H), 1.72 - 1.61 (m, 3H), 1.45 (s, 9H).

### 1-((1-(6-(1H-imidazol-1-yl)pyridin-2-yl)piperidin-4-yl)methyl)piperazine hydrochloride

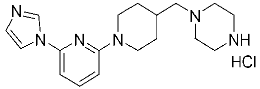

Tert-butyl 4-((1-(6-(1H-imidazol-1-yl)pyridin-2-yl)piperidin-4-yl)methyl)piperazin-1-carboxylate (300 mg, 0.7 mmol) was added into a reaction flask, ethyl acetate (1 mL) and dichloromethane (1 mL) were added into the reaction flask through a syringe, and a saturated solution of hydrogen chloride in ethyl acetate (5 mL) was added dropwise at 0°C. The reaction solution was stirred at room temperature for 22 hours. The reaction solution was filtered. The filter cake was washed with ethyl acetate (10 mL), collected, and dried to obtain the title compound as a yellow solid (241 mg, yield: 95%). LC-MS: m/z [M+H]⁺ = 327.

### Intermediate 23: Tert-butyl 4-(6-bromopyridin-2-yl)piperazin-1-carboxylate

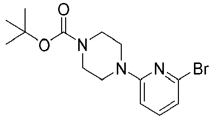

2-bromo-6-fluoropyridine (528 mg, 3 mmol, CAS: 144100-07-2) and tert-butyl piperazine-1-carboxylate acetate (559 mg, 3 mmol, CAS: 143238-38-4) were added into a reaction flask, 1,4-dioxane (12 mL) was added into the reaction flask through a syringe, and triethylamine (334 mg, 3.3 mmol) was added in during stirring. The reaction solution was stirred at 105°C for 12 hours. The reaction solution was cooled to room temperature, concentrated under reduced pressure, and purified by silica gel column chromatography (petroleum ether/ethyl acetate = 20:1) to obtain the title compound as a white solid (860 mg, yield: 84%). ¹H NMR (400 MHz, CDCl₃) δ 7.29 (t, J = 7.9 Hz, 1H), 6.77 (d, J = 7.5 Hz, 1H), 6.51 (d, J = 8.3 Hz, 1H), 3.52 (s, 8H), 1.47 (s, 9H).

### Intermediate 24: 5-(6-(piperazin-1-yl)pyridin-2-yl)thiazole hydrochloride

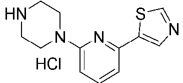

### Tert-butyl 4-(6-(thiazol-5-yl)pyridin-2-yl)piperazin-1-carboxylate

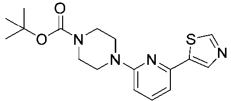

Tert-butyl 4-(6-bromopyridin-2-yl)piperazin-1-carboxylate (550 mg, 1.6 mmol, intermediate 23), 5-(4,4,5,5-tetramethyl-1,3,2-dioxaborolan-2-yl)thiazole (407 mg, 1.9 mmol, CAS: 1086111-09-2), [1,1'-bis(diphenylphosphino)ferrocene]dichloropalladium (141 mg, 0.19 mmol, CAS: 72287-26-4), and potassium carbonate (442 mg, 3.2 mmol) were added into a reaction flask, and 1,4-dioxane/water (16 mL, v/v, 4:1) was added into the reaction flask through a syringe in a nitrogen atmosphere. The reaction solution was stirred at 95°C for 12 hours. Brine (20 mL) was added into the reaction solution, and the reaction solution was extracted with ethyl acetate (10 mL) for 2 times. Organic phases were combined, dried over anhydrous sodium sulfate, concentrated under reduced pressure, and purified by silica gel column chromatography (dichloromethane/methanol = 30:1) to obtain the title compound as a white solid (70 mg, yield: 70%). ¹H NMR (400 MHz, CDCl₃) δ 7.75 (d, J = 7.5 Hz, 1H), 7.60 (t, J = 7.8 Hz, 1H), 7.25 (s, 2H), 6.66 (d, J = 8.4 Hz, 1H), 3.62 - 3.57 (m, 8H), 1.48 (s, 9H).

### 5-(6-(piperazin-1-yl)pyridin-2-yl)thiazole hydrochloride

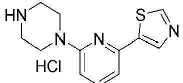

Tert-butyl 4-(6-(thiazol-5-yl)pyridin-2-yl)piperazin-1-carboxylate (230 mg, 0.66 mmol) was added into a reaction flask, ethyl acetate (1 mL) was added into the reaction flask through a syringe, and a saturated solution of hydrogen chloride in ethyl acetate (7 mL) was added dropwise at 0°C. The reaction solution was stirred at room temperature for 22 hours. The reaction solution was filtered. The filter cake was washed with ethyl acetate (10 mL), collected, and dried to obtain the title compound as a yellow solid (180 mg, yield: 96%). LC-MS: m/z [M+H]⁺ = 247.

Referring to the table below, except that a raw material listed in the "Raw material" column was used to replace the corresponding raw material, the following intermediate 83 was prepared according to a preparation method of the intermediate 24.

| Number | Name | Structure | LC-MS (m/z [M+H]⁺) | Raw material |
|---|---|---|---|---|
| Intermediate 83 | 5-(4-(piperazin-1-yl)pyrimidin-2-yl) thiazole hydrochloride | 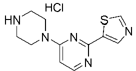 | 248 | Tert-butyl 4-(2-chloropyrimidin-4-yl) piperazin-1-formate |

### Intermediate 25:

### 3-(6-(4-((4-(2-chloropyrimidin-4-yl)piperazin-1-yl)methyl)benzyl)-2-oxobenzo[cd]indol-1(2H)-yl)p iperidin-2.6-dione

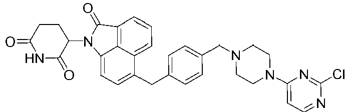

To a mixed solution of 3-(2-oxo-6-(4-(piperazin-1-ylmethyl)benzyl)benzo[cd]indol-1(2H)-yl)piperidin-2,6-dione (240 mg, 0.5 mmol, intermediate 3), triethylamine (0.18 mL, 1.28 mmol, d = 0.728 g/mL), and N,N-dimethylformamide (4 mL), 2,4-dichloropyrimidine (95 mg, 0.64 mmol) was added. The reaction solution was stirred at 20°C for 3 hours. The reaction solution was slowly added into water (20 mL) until precipitation formed, and then filtered. The filter cake was washed once with water (5 mL), collected, and dried to obtain the title compound as a yellow solid (180 mg, yield: 62%). LC-MS: m/z [M+H]⁺ = 581.

Referring to the table below, except that a raw material listed in the "Raw material" column was used to replace the corresponding raw material, the following intermediate 26 was prepared according to a preparation method of the intermediate 25.

| Number | Name | Structure | LC-MS (m/z [M+H]⁺) | Raw material |
|---|---|---|---|---|
| Intermediat e 26 | 3-(6-(4-((4-(2-chloro-6-(trifluoromethyl)pyrimidin-4-yl) piperazin-1-yl)methyl)benz yl)-2-oxobenzo[cd]indol-1(2H)-y 1) piperidin-2,6-dione | 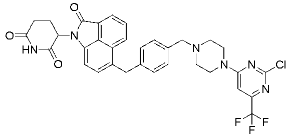 | 649 | 2,4-dichloro-6-trifl uoromethylpyrimid ine (CAS: 16097-64-6) |

### Intermediate 27: 2-(6-chloropyridin-2-yl)-5-methyl-1,3,4-thiadiazole

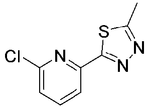

### N'-acetyl-6-chloropyridinhydrazide

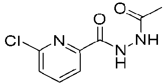

At room temperature, 6-chloronicotinic acid (1 g, 6.35 mmol, CAS: 5326-23-8) was added into thionyl chloride (2.30 mL, 31.74 mmol, d = 1.638 g/mL), and refluxed under nitrogen atmosphere for 2 hours. The reaction solution was concentrated, and then dissolved in dichloromethane (20 mL). 4-dimethylaminopyridine (77.58 mg, 0.64 mmol, CAS: 1122-58-3), triethylamine (2.64 mL, 19.05 mmol, d = 0.728 g/mL), and acethydrazide (0.51 mL, 7.62 mmol, CAS: 1068-57-1) were sequentially added into the reaction solution. Then, the reaction solution was stirred at room temperature overnight under nitrogen atmosphere. Water (20 mL) was slowly added into the reaction solution, and stirred at room temperature for 2 hours. A solid was precipitated, and filtration was performed. The filter cake was collected and dried to obtain the title compound as a light yellow solid (650 mg, yield: 48%). ¹H NMR (400 MHz, CD₃OD) δ 7.99 (dd, J = 7.7, 0.9 Hz, 1H), 7.86 (t, J = 7.8 Hz, 1H), 7.44 (dd, J = 8.0, 0.9 Hz, 1H), 2.02 (s, 3H).

### 2-(6-chloropyridin-2-yl)-5-methyl-1,3,4-thiadiazole

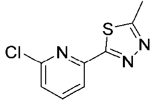

At room temperature, Lawesson's reagent (1703.98 mg, 4.21 mmol, CAS: 19172-47-5) was added in portions into a solution of the N'-acetyl-6-chloropyridinhydrazide (600 mg, 2.81 mmol) in toluene (10 mL). Then, the reaction solution was heated to 90°C, and stirred overnight under nitrogen atmosphere. The reaction solution was filtered. The filtrate was concentrated under reduced pressure, and purified by silica gel column chromatography (petroleum ether/ethyl acetate = 5:1 - 3:1) to obtain the title compound as a yellow solid (250 mg, yield: 42%). ¹H NMR (400 MHz, DMSO-d6) δ 8.23 (dd, J = 7.7, 0.9 Hz, 1H), 8.09 (t, J = 7.8 Hz, 1H), 7.70 (dd, J = 8.0, 0.9 Hz, 1H), 2.80 (s, 3H).

### Intermediate 28: 2-chloro-6-(3-methyl-1H-1,2,4-triazol-1-yl)pyridine

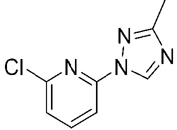

In a nitrogen atmosphere, cuprous oxide (167.67 mg, 1.16 mmol, CAS: 1317-39-1) and N1,N2-bis(furan-2-ylmethyl)oxalamide (287 mg, 1.28 mmol, CAS: 69010-90-8) were added into a mixed solution of 2,6-dichloropyridine (570 mg, 3.85 mmol, CAS: 2402-78-0), 3-methyl-1H-1,2,4-triazole (320 mg, 3.85 mmol, CAS: 7170-01-6), tripotassium phosphate (1460 mg, 6.88 mmol), and dimethyl sulfoxide (10 mL). The reaction solution reacted at 145°C for 18 hours. The reaction solution was poured into water (50 mL), and extracted with ethyl acetate (30 mL) for 2 times. Organic phases were combined, washed once with brine (20 mL), dried over anhydrous sodium sulfate, concentrated under reduced pressure, and purified by silica gel column chromatography (dichloromethane/ethyl acetate = 3:1) to obtain the title compound as a white solid (130 mg, yield: 17%). ¹H NMR (400 MHz, DMSO-d6) δ 9.19 (s, 1H), 8.09 (t, J = 7.9 Hz, 1H), 7.80 (d, J = 8.0 Hz, 1H), 7.56 (d, J = 7.9 Hz, 1H), 2.39 (s, 3H).

### Intermediate 29: 2-chloro-6-(4-isopropyl-4H-1,2,4-triazol-3-yl)pyridine

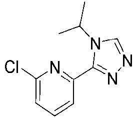

6-(4-isopropyl-4H-1,2,4-triazol-3-yl)pyridin-2-amine (1 g, 4.9 mmol, CAS: 1448427-99-3) and benzyltriethylammonium chloride (216 mg, 9.8 mmol, CAS: 56-37-1) were dissolved in dichloromethane (20 mL), and tert-butyl nitrite (2.5 g, 24.6 mmol, CAS: 540-80-7) was slowly added at 0°C. After the addition was completed, the reaction solution was heated to 30°C, and stirred overnight. The reaction solution was diluted with water (100 mL), and extracted with dichloromethane (60 mL) for 2 times. Organic phases were combined, dried over anhydrous sodium sulfate, concentrated under reduced pressure, and purified by silica gel column chromatography (dichloromethane/methanol = 30:1) to obtain the title compound as a yellow oil (700 mg, yield: 64%).LC-MS: m/z [M+H]⁺ = 223.

### Intermediate 31: 4-cyclopropyl-1H-imidazole

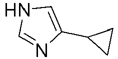

Formamidine acetate (3.12 g, 30 mmol, CAS: 3473-63-0) and sodium carbonate (3.18 g, 30 mmol) were added into a reaction flask, nitrogen replacement was performed for 3 times, and anhydrous tetrahydrofuran (30 mL) was added into the reaction flask through a syringe. The reaction mixture was stirred at 100°C for 3 hours, and then filtered. 1-cyclopropyl-2-bromoethanone (4.89 g, 30 mmol, CAS: 69267-75-0) was slowly added dropwise into The filtrate. The reaction solution was stirred at room temperature overnight. A saturated sodium bicarbonate solution (100 mL) was added into the reaction solution to quench a reaction, and the reaction solution was extracted with ethyl acetate (40 mL) for 3 times. Organic phases were combined, dried over anhydrous sodium sulfate, and concentrated under reduced pressure to obtain the title compound as a white solid (1.62 g, yield: 50%). LC-MS: m/z [M+H]⁺ = 109.

### Intermediate 33:

### Tert-butyl ((1s,4s)-4-((1-(4-(chloromethyl)benzyl)piperidin-4-yl)oxy)cyclohexyl)aminodicarboxylate

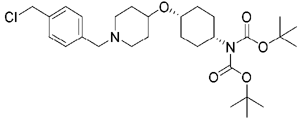

### Tert-butyl ((1R,4R)-4-((tert-butyldimethylsilyl)oxy)cyclohexyl)carbamate

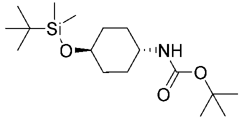

Trans-4-(tert-butoxycarbonylamino)cyclohexanol (6.45 g, 30 mmol, CAS: 111300-06-2) and imidazole (4.08 g, 60 mmol) were placed in a reaction flask, into which N,N-dimethylformamide (100 mL) was added through a syringe. Tert-butyldimethylsilyl chloride (9.04 g, 60 mmol, CAS: 18162-48-6) was added at 0°C. Then, the mixture was heated to room temperature, and stirred for a reaction for 2 hours. A saturated ammonium chloride solution (200 mL) was added into the reaction solution, and the reaction solution was extracted with ethyl acetate (50 mL) for 3 times. Organic phases were combined, washed with a saturated ammonium chloride solution (50 mL) for 2 times and with brine (50 mL) for 3 times, dried over anhydrous sodium sulfate, and concentrated under reduced pressure to obtain the title compound as a white solid (9.9 g, yield: 100%). LC-MS: m/z [M+H]⁺ = 330.

### Tert-butyl ((1R,4R)-4-((tert-butyldimethylsilyl)oxy)cyclohexyl)dicarbamate

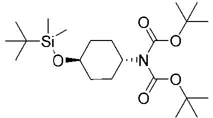

The tert-butyl ((1R,4R)-4-((tert-butyldimethylsilyl)oxy)cyclohexyl)carbamate (9.9 g, 30 mmol) was added into a reaction flask, and anhydrous tetrahydrofuran (100 mL) was added into the reaction flask through a syringe in a nitrogen atmosphere atmosphere. The temperature was lowered to -78°C, a 1.6 M solution of n-butyllithium in hexane (30 mL, 48 mmol) was added dropwise into the reaction solution, and the mixture was stirred at -78°C for 0.5 hour. Di-tert-butyl dicarbonate (9.8 g, 45 mmol, CAS: 24424-99-5) was slowly added dropwise into the reaction solution. The mixture was stirred at -78°C for 3 hours, and then heated to room temperature for a reaction overnight. A saturated ammonium chloride solution (50 mL) was added dropwise into the reaction solution to quench the reaction, and the reaction solution was extracted with ethyl acetate (40 mL) for 3 times. Organic phases were combined, washed with brine (30 mL) for 3 times, dried over anhydrous sodium sulfate, concentrated under reduced pressure, and subjected to silica gel column chromatography (petroleum ether/ethyl acetate = 50:1) to obtain the title compound as a white solid (9.3 g, yield: 72%). LC-MS: m/z [M+H]⁺ = 430.

### Tert-butyl ((1R,4R)-4-hydroxycyclohexyl)aminodicarboxylate

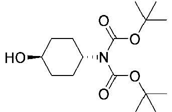

Tert-butyl ((1R,4R)-4-((tert-butyldimethylsilyl)oxy)cyclohexyl)aminodicarboxylate (6.45 g, 20 mmol) was placed in a reaction flask, into which tetrahydrofuran (50 mL) was added through a syringe. Tetrabutylammonium fluoride (15.7 g, 60 mmol, CAS: 429-41-4) was added at 0°C. Then, the mixture was heated to room temperature, and stirred for a reaction for 2 hours. A saturated ammonium chloride solution (50 mL) was added into the reaction solution, and the reaction solution was extracted with ethyl acetate (40 mL) for 3 times. Organic phases were combined, washed with a saturated ammonium chloride solution (50 mL) for 2 times and with brine (50 mL) for 1 time, dried over anhydrous sodium sulfate, concentrated under reduced pressure, and purified by silica gel column chromatography (petroleum ether/ethyl acetate = 1:1) to obtain the title compound as a white solid (5.7 g, yield: 90%). LC-MS: m/z [M+H]⁺ = 316.

### Tert-butyl ((1s,4s)-4-(pyridin-4-yloxy)cyclohexyl)aminodicarboxylate

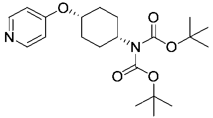

4-hydroxypyridine (6.48 g, 30 mmol, CAS: 626-64-2), tert-butyl ((1R,4R)-4-hydroxycyclohexyl)aminodicarboxylate (6.13 g, 30 mmol), and triphenylphosphine (11.8 g, 45 mmol, CAS: 603-35-0) were placed in a reaction flask, nitrogen replacement was performed for 3 times, and tetrahydrofuran (60 mL) was added into the reaction flask through a syringe. At 0°C, diisopropyl azodicarboxylate (6.13 g, 45 mmol, CAS: 2446-83-5) was slowly added dropwise, and after the addition was completed, the reaction mixture was stirred at room temperature overnight. The reaction solution was concentrated under reduced pressure, and purified by silica gel column chromatography (petroleum ether/ethyl acetate = 3:1) to obtain the title compound as a white solid (8.2 g, yield: 70%). LC-MS: m/z [M+H]⁺ = 393.

### 1-benzyl-4-(((1s,4s)-4-((di-tert-butoxycarbonyl)amino)cyclohexyl)oxy)pyridin-1-ium bromide

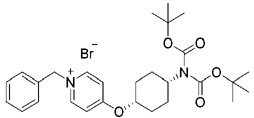

Tert-butyl ((1s,4s)-4-(pyridin-4-yloxy)cyclohexyl)aminodicarboxylate (6.45 g, 20 mmol) was placed in a reaction flask, into which dichloromethane (20 mL) was added through a syringe, and benzyl bromide (10 g, 60 mmol) was added. The reaction solution was stirred at room temperature overnight. The reaction solution was concentrated under reduced pressure to obtain the title compound as a white solid (16.9 g of a crude product).

### Tert-butyl ((1s,4s)-4-((1-benzyl-1,2,3,6-tetrahydropyridin-4-yl)oxy)cyclohexyl)aminodicarboxylate

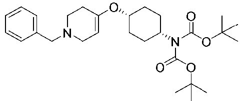

1-benzyl-4-(((1s,4s)-4-((di-tert-butoxycarbonyl)amino)cyclohexyl)oxy)pyridin-1-ium bromide (16.9 g, 20 mmol) was placed in a reaction flask, into which methanol (20 mL) was added through a syringe, and sodium borohydride (2.3 g, 60 mmol) was added. The reaction solution was stirred at room temperature for 3 hours. Brine (100 mL) was added into the reaction solution to quench a reaction, and the reaction solution was extracted with dichloromethane (40 mL) for 5 times. Organic phases were combined, dried over anhydrous sodium sulfate, concentrated under reduced pressure, and purified by silica gel column chromatography (petroleum ether/ethyl acetate = 1:1) to obtain the title compound as a white solid (4.4 g, two-step yield: 45%). LC-MS: m/z [M+H]⁺ = 487.

### Tert-butyl ((1s,4s)-4-(piperidin-4-yloxy)cyclohexyl)aminodicarboxylate

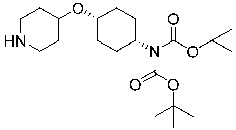

Tert-butyl ((1s,4s)-4-((1-benzyl-1,2,3,6-tetrahydropyridin-4-yl)oxy)cyclohexyl)aminodicarboxylate (4.4 g, 9 mmol) and a palladium hydroxide on carbon catalyst (0.7 g) were added into a reaction flask, methanol (15 mL) and tetrahydrofuran (5 mL) were added into the reaction flask through a syringe, and hydrogen replacement was performed for 3 times. The mixture was stirred at 60°C for 24 hours. The reaction solution was filtered through celite. The filtrate was concentrated under reduced pressure, and purified by silica gel column chromatography (dichloromethane/methanol = 20:1) to obtain the title compound as a colorless liquid (3.2 g, yield: 89%). LC-MS: m/z [M+H]⁺ = 399.

### Tert-butyl ((1s,4s)-4-((1-(4-(chloromethyl)benzyl)piperidin-4-yl)oxy)cyclohexyl)aminodicarboxylate

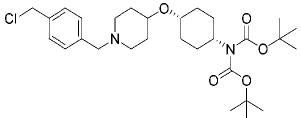

Tert-butyl ((1s,4s)-4-(piperidin-4-yloxy)cyclohexyl)aminodicarboxylate (3.2 g, 8.1 mmol) was added into a reaction flask, N,N-dimethylformamide (15 mL), acetonitrile (15 mL), and N,N-diisopropylethylamine (4 mL, 16.2 mmol, d = 0.782 g/mL) were added into the reaction flask through a syringe, and 1,4-bis(chloromethyl)benzene (4.2 g, 24 mmol) was added in portions in during stirring. The mixture was stirred at room temperature for 30 min, and then reacted at 60°C for 3 hours. The reaction solution was diluted with brine (75 mL), and extracted with ethyl acetate (30 mL) for 3 times. Organic phases were combined, washed with brine (30 mL) for 3 times, dried over anhydrous sodium sulfate, concentrated under reduced pressure, and purified by silica gel column chromatography (petroleum ether/ethyl acetate = 3:1) to obtain the title compound as a white solid (3.3 g, yield: 76%). LC-MS: m/z [M+H]⁺ = 537.

### Intermediate 35:

### 3-(6-(4-((4-(6-chloropyridin-2-yl)piperazin-1-yl)methyl)benzyl)-2-oxobenzo[cd]indol-1(2H)-yl)pipe ridin-2,6-dione

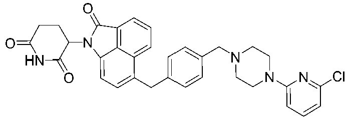

3-(2-oxo-6-(4-(piperazin-1-ylmethyl)benzyl)benzo[cd]indol-1(2H)-yl)piperidin-2,6-dione (660 mg, 1.31 mmol, hydrochloride, intermediate 3), 2,6-dichloropyridine (300 mg, 2.03 mmol), triethylamine (600 mg, 5.93 mmol), and cesium fluoride (250 mg, 1.65 mmol) were added into dimethyl sulfoxide (12 mL), and the mixture was subjected to a microwave reaction at 135°C for 1 hour. The reaction solution was cooled to room temperature, poured into water (50 mL), and extracted with ethyl acetate (30 mL) for 2 times. Organic phases were combined, washed with brine (20 mL) for 1 time, dried over anhydrous sodium sulfate, concentrated under reduced pressure, and purified by silica gel column chromatography (dichloromethane/ethyl acetate = 3:1) to obtain the title compound as a white solid (450 mg, yield: 59%). LC-MS: m/z [M+H]⁺ = 580.

### Intermediate 37: Tert-butyl 4-(azetidin-3-yl)piperazin-1-carboxylate

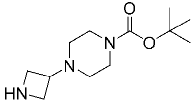

### Tert-butyl 4-(1-(benzyloxycarbonyl)azetidin-3-yl)piperazin-1-carboxylate

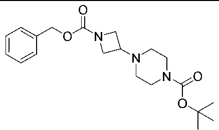

1-benzyloxycarbonylazetidin-3-one (0.93 g, 5 mmol, CAS: 105258-93-3) and 1-(tert-butoxycarbonyl)piperazine (1.03 g, 5 mmol, CAS: 57260-71-6) were placed in a reaction flask, nitrogen replacement was performed for 3 times, and dichloromethane (15 mL) and acetic acid (0.02 mL) were added into the reaction flask through a syringe. After the reaction system was stirred for a reaction at room temperature for 1 hour, sodium triacetoxyborohydride (1.6 g, 7.5 mmol, CAS: 56553-60-7) was added, and the reaction mixture was stirred at room temperature for 3 hours. Dichloromethane (50 mL) was added into the reaction solution, and the reaction solution was washed with brine (20 mL) for 2 times. Organic phases were dried over anhydrous sodium sulfate, concentrated under reduced pressure, and separated by silica gel column chromatography (dichloromethane/methanol = 10:1) to obtain the title compound as a white solid (1.5 g, yield: 80%). LC-MS: m/z [M+H]⁺ = 376.

### Tert-butyl 4-(azetidin-3-yl)piperazin-1-carboxylate

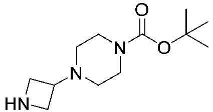

Tert-butyl 4-(1-(benzyloxycarbonyl)azetidin-3-yl)piperazin-1-carboxylate (3.0 g, 8 mmol) and 20% palladium hydroxide on carbon (0.56 g, about 50% of water content, CAS: 12135-22-7) were placed in a reaction flask, hydrogen replacement was performed for 3 times, and methanol (20 mL) and tetrahydrofuran (6 mL) were added into the reaction flask through a syringe. The reaction system was stirred at room temperature overnight. The reaction solution was filtered through celite. The filtrate was concentrated under reduced pressure to obtain the title compound as a white solid (1.8 g, yield: 93%). LC-MS: m/z [M+H]⁺ = 242.

Referring to the table below, except that raw materials listed in the "Raw material" column were used to replace the corresponding raw material, the following intermediates 50, 78, and 80 were prepared according to a preparation method of the intermediate 37.

| Number | Name | Structure | LC-MS (m/z [M+H]⁺) | Raw material |
|---|---|---|---|---|
| Intermediat e 50 | 4-(4-(azetidin-3-yl)piperazin-1-yl) -5-fluoro-2-(3-methyl-1H-1,2,4-triaz ol-1-yl)pyrimidine | 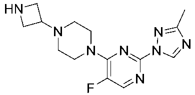 | 319 | Intermediate 49 |
| Intermediat e 78 | 4-(4-(azetidin-3-yl)piperazin-1-yl) -2-methyl-6-(3-methyl-1H-1,2,4-tria zol-1-yl)pyrimidine | 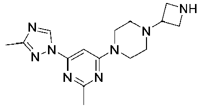 | 315 | Intermediate 72a |
| Intermediat e 80 | 4-(4-(azetidin-3-yl)piperazin-1-yl) -2-methyl-6-(4-(trifluoromethyl)-1H-imidazol-1-yl)pyrimidine | 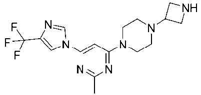 | 368 | Intermediate 73a |

### Intermediate 38:

### 4-((1-(2,6-dioxopiperidin-3-yl)-2-oxo-1,2-dihydrobenzo[cd]indol-6-yl)methyl)benzaldehyde

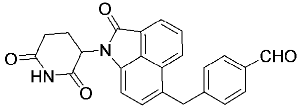

Intermediate 38 was synthesized according to a method provided in Patent Application No. WO2020210630A1.

### Intermediate 39:

### 3-(2-oxo-6-(4-((3-(piperazin-1-yl)azetidin-1-yl)methyl)benzyl)benzo[cd]indol-1(2H)-yl)piperidin-2, 6-dione

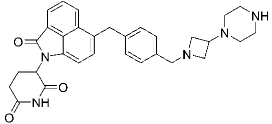

### Tert-butyl 4-(1-(4-((1-(2.6-dioxopiperidin-3-yl)-2-oxo-1,2-dihydrobenzo[cd]indol-6-yl)methyl)benzyl)azetidin-3-yl) piperazin-1-carboxylate

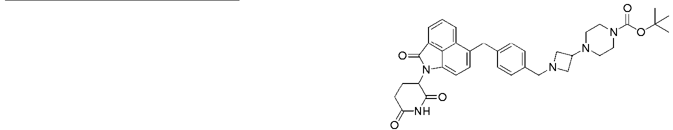

4-((1-(2,6-dioxopiperidin-3-yl)-2-oxo-1,2-dihydrobenzo[cd]indol-6-yl)methyl)benzaldehyde (0.31 g, 0.78 mmol, intermediate 38) and tert-butyl 4-(azetidin-3-yl)piperazin-1-carboxylate (0.26 g, 1.09 mmol, intermediate 37) were dissolved in dichloromethane (8 mL), into which acetic acid (0.09 mL) was slowly added dropwise. The mixture was stirred at room temperature for half an hour, then sodium triacetoxyborohydride (0.33 g, 1.56 mmol, CAS: 56553-60-7) was added in portions into the reaction system, and the reaction mixture was stirred at room temperature for 3 hours. Dichloromethane (50 mL) was added into the reaction solution, and the reaction solution was washed with brine (20 mL) for 2 times. Organic phases were dried over anhydrous sodium sulfate, concentrated under reduced pressure, and separated by silica gel column chromatography (dichloromethane/methanol = 10:1) to obtain the title compound as a white solid (0.35 g, yield: 72%) LC-MS: m/z [M+H]⁺ = 624.

### 3-(2-oxo-6-(4-((3-(piperazin-1-yl)azetidin-1-yl)methyl)benzyl)benzo[cd]indol-1(2H)-yl)piperidin-2, 6-dione

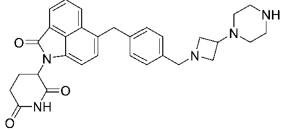

Tert-butyl 4-(1-(4-((1-(2,6-dioxopiperidin-3-yl)-2-oxo-1,2-dihydrobenzo[cd]indol-6-yl)methyl)benzyl)azetidin-3-yl)pip erazin-1-carboxylate (0.35 g, 0.56 mmol) was dissolved in dichloromethane (8.5 mL), into which trifluoroacetic acid (1.5 mL) was slowly added dropwise in an ice bath. The mixture was stirred for a reaction at room temperature for 3 hours. The reaction solution was directly concentrated under reduced pressure to obtain the title compound as a white solid (357 mg, yield: 100%, TFA salt). LC-MS: m/z [M+H]⁺ = 524.

Referring to the table below, except that a raw material listed in the "Raw material" column was used to replace the corresponding raw material, the following intermediate 55 was prepared according to a preparation method of the intermediate 39.

| Number | Name | Structure | LC-MS (m/z [M+H]⁺) | Raw material |
|---|---|---|---|---|
| Intermediat e 55 | 3-(2-oxo-6-(4-((3-(pipera zin-1-ylmethyl)azetidin-1 -yl)methyl)benzyl)benzo[ cd]indol-1(2H)-yl)piperidin-2,6-dione | 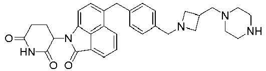 | 538 | Intermediate 54 |

### Intermediate 44: 2-(5-fluoro-4-(piperazin-1-yl)pyrimidin-2-yl)-5-methyl-1,3,4-thiadiazole

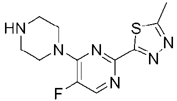

### Tert-butyl 4-(2-chloro-5-fluoropyrimidin-4-yl)piperazin-1-carboxylate

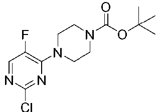

2,4-dichloro-5-fluoropyrimidine (1.7 g, 10 mmol, CAS: 2927-71-1) was dissolved in anhydrous N,N-dimethylformamide (20 mL), and triethylamine (2.1 mL, 15 mmol, d = 0.728 g/mL) was slowly added. The reaction solution was stirred at 0°C for 30 minutes, then 1-(tert-butoxycarbonyl)piperazine (2.0 g, 11 mmol, CAS: 57260-71-6) was slowly added, and the reaction mixture was stirred at room temperature for 4 hours. The reaction solution was diluted with water (200 mL), and extracted with dichloromethane (100 mL) for 3 times. Organic phases were combined, washed with brine (50 mL) for 2 times, dried and concentrated, and purified by silica gel column chromatography (petroleum ether/ethyl acetate = 5:1) to obtain the title compound as a white solid (2.82 g, yield: 89%). LC-MS: m/z [M+H]⁺ = 317.

### Tert-butyl 4-(2-cyano-5-fluoropyrimidin-4-yl)piperazin-1-carboxylate

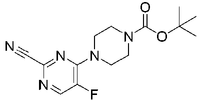

Tert-butyl 4-(2-chloro-5-fluoropyrimidin-4-yl)piperazin-1-carboxylate (1.6 g, 5 mmol), zinc cyanide (880 mg, 7.5 mmol), and tetrakis(triphenylphosphine)palladium (0.25 mmol, 289 mg, CAS: 14221-01-3) were added into a Schlenk tube, nitrogen replacement was performed for 3 times, and anhydrous N,N-dimethylformamide (5 mL) was added in a nitrogen atmosphere. Then, the reaction tube was stirred at 160°C for 2 hours. The reaction solution was cooled to room temperature, diluted with water (50 mL), and extracted with dichloromethane (50 mL) for 3 times. Organic phases were combined, washed with brine (20 mL) for 2 times, dried and concentrated under reduced pressure, and purified by silica gel column chromatography (petroleum ether/ethyl acetate = 5:1) to obtain the title compound as a white solid (1.2 g, yield: 71%). LC-MS: m/z [M+H]⁺ = 308.

### 4-(4-(tert-butoxycarbonyl)piperazin-1-yl)-5-fluoropyrimidin-2-carboxylic acid

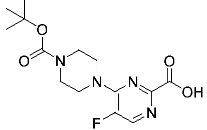

Tert-butyl 4-(2-cyano-5-fluoropyrimidin-4-yl)piperazin-1-carboxylate (1.2 g, 3.5 mmol) was added into a reaction flask, a 1 M sodium hydroxide solution (4.2 mL, 4.2 mmol) was slowly added dropwise at room temperature, and the mixture was stirred at room temperature overnight. 1 N hydrochloric acid was slowly added dropwise into the reaction solution in an ice bath to adjust a pH to 3, and the reaction solution was extracted with dichloromethane (50 mL) for 3 times. Organic phases were combined, washed with brine (20 mL) for 2 times, dried over anhydrous sodium sulfate, and concentrated under reduced pressure to obtain the title compound as a white solid (1.4 g, yield: 100%). LC-MS: m/z [M+H]⁺ = 327.

### Tert-butyl 4-(2-(2-acetylhydrazin-1-carbonyl)-5-fluoropyrimidin-4-yl)piperazin-1-carboxylate

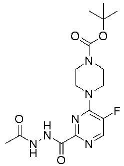

4-(4-(tert-butoxycarbonyl)piperazin-1-yl)-5-fluoropyrimidin-2-carboxylic acid (1.0 g, 3.0 mmol) and acetyl hydrazine (333 mg, 4.5 mmol, CAS: 1068-57-1) were dissolved in anhydrous acetonitrile (30 mL), diisopropylethylamine (0.63 mL, 3.6 mmol, d = 0.782 g/mL) was slowly added dropwise at room temperature, and the mixture was stirred at room temperature for half an hour. Then, benzotriazol-N,N,N',N'-tetramethyluronium hexafluorophosphate (1.4 g, 3.6 mmol, CAS: 94790-37-1, HBTU) was slowly added, and the reaction mixture was stirred at room temperature overnight. The reaction solution was diluted with water (30 mL), and extracted with dichloromethane (20 mL) for 3 times. Organic phases were combined, washed with brine (10 mL) for 2 times, dried over anhydrous sodium sulfate, and concentrated under reduced pressure to obtain the title compound as a white solid (830 mg, yield: 72%). LC-MS: m/z [M+H]⁺ = 383.

### Tert-butyl 4-(5-fluoro-2-(5-methyl-1,3,4-thiadiazol-2-yl)pyrimidin-4-yl)piperazin-1-carboxylate

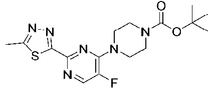

Tert-butyl 4-(2-(2-acetylhydrazin-1-carbonyl)-5-fluoropyrimidin-4-yl)piperazin-1-carboxylate (830 mg, 2.17 mmol) was added into toluene (20 mL), a Lawesson's reagent (1.8 g, 4.34 mmol, CAS: 19172-47-5) was slowly added at room temperature, and the mixture was stirred at 110°C for 2 hours. The reaction solution was concentrated under reduced pressure, and purified by silica gel column chromatography (petroleum ether/ethyl acetate = 5:1) to obtain the title compound as a white solid (726 mg, yield: 44%). LC-MS: m/z [M+H]⁺ = 381.

### 2-(5-fluoro-4-(piperazin-1-yl)pyrimidin-2-yl)-5-methyl-1,3,4-thiadiazole

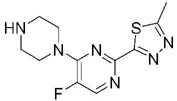

Tert-butyl 4-(5-fluoro-2-(5-methyl-1,3,4-thiadiazol-2-yl)pyrimidin-4-yl)piperazin-1-carboxylate (726 mg, 1.9 mmol) was added into dichloromethane (4 mL), trifluoroacetic acid (2 mL) was slowly added dropwise at 0°C, and the reaction solution was stirred at room temperature for 2 hours. The reaction solution was directly concentrated under reduced pressure to obtain the title compound as a colorless oil (740 mg, yield: 99%, TFA salt). LC-MS: m/z [M+H]⁺ = 281.

### Intermediate 47: 5-chloro-2-(3-methyl-1H-1,2,4-triazol-1-yl)-4-(piperazin-1-yl)pyrimidine

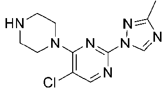

### Tert-butyl 4-(2,5-dichloropyrimidin-4-yl)piperazin-1-carboxylate

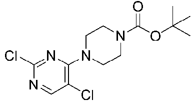

2,4,5-trichloropyrimidine (920 mg, 5 mmol, CAS: 5750-76-5) was dissolved in anhydrous N,N-dimethylformamide (10 mL), triethylamine (1.05 mL, 7.5 mmol, d = 0.728 g/mL) was slowly added, and the mixture was stirred at 0°C for 30 minutes. Then, 1-(tert-butoxycarbonyl)piperazine (1.02 g, 5.5 mmol, CAS: 57260-71-6) was slowly added, and the reaction mixture was stirred at room temperature for 4 hours. The reaction solution was diluted with water (100 mL), and extracted with dichloromethane (100 mL) for 3 times. Organic phases were combined, washed with brine (50 mL) for 2 times, dried and concentrated, and purified by silica gel column chromatography (petroleum ether/ethyl acetate = 5:1) to obtain the title compound as a white solid (1.43 g, yield: 86%). LC-MS: m/z [M+H]⁺ = 333.

### Tert-butyl 4-(5-chloro-2-(3-methyl-1H-1,2,4-triazol-1-yl)pyrimidin-4-yl)piperazin-1-carboxylate

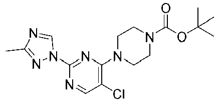

Tert-butyl 4-(2,5-dichloropyrimidin-4-yl)piperazin-1-carboxylate (332 mg, 1 mmol), 3-methyl-1H-1,2,4-triazole (92 mg, 1.1 mmol, CAS: 7170-01-6), cuprous iodide (40 mg, 0.2 mmol), and cesium carbonate (652 mg, 2 mmol) were added into a reaction flask, then anhydrous N,N-dimethylacetamide (5 mL) was added in a nitrogen atmosphere, and the mixture was stirred at 100°C for 3 hours. The reaction solution was cooled to room temperature, into which brine (50 mL) was added, and then extracted with ethyl acetate (20 mL) for 3 times. Organic phases were combined, dried over anhydrous sodium sulfate, concentrated under reduced pressure, and separated by silica gel column chromatography (petroleum ether/ethyl acetate = 3:1) to obtain the title compound as a white solid (231 mg, yield: 61%). LC-MS: m/z [M+H]⁺ = 380.

### 5-chloro-2-(3-methyl-1H-1,2,4-triazol-1-yl)-4-(piperazin-1-yl)pyrimidine

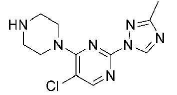

Tert-butyl 4-(5-chloro-2-(3-methyl-1H-1,2,4-triazol-1-yl)pyrimidin-4-yl)piperazin-1-carboxylate (231 mg, 0.61 mmol) was added into dichloromethane (2 mL), trifluoroacetic acid (1 mL) was slowly added dropwise at 0°C, and the reaction solution was stirred at room temperature for 2 hours. The reaction solution was directly concentrated under reduced pressure to obtain the title compound as a colorless oil (240 mg, yield: 100%, TFA salt). LC-MS: m/z [M+H]⁺ = 280.

Referring to the table below, except that raw materials listed in the "Raw material" column were used to replace the corresponding raw material, the following intermediates 49 and 70 were prepared according to a preparation method of the intermediate 47.

| Number | Name | Structure | LC-MS (m/z [M+H]⁺) | Raw material |
|---|---|---|---|---|
| Intermediat e 49 | 5-fluoro-2-(3-methyl-1H-1, 2,4-triazol-1-yl)-4-(piperazin-1-yl) pyrimidine | 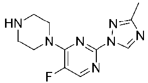 | 264 | 2,4-dichloro-5-fluoro pyrimidine (CAS: 2927-71-1) |
| Intermediat e 70 | 2-(3-methyl-1H-1,2,4-triazo 1-1-yl )-4-(piperazin-1-yl)-6-(trifluoromethyl)pyrimidine | 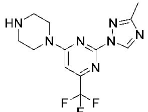 | 314 | 2,4-dichloro-6-trifluo ro methylpyrimidine (CAS: 16097-64-6) |

### Intermediate 53: 4-chloro-2-(3-methyl-1H-1,2.4-triazol-1-yl)-6-(trifluoromethyl)pyrimidine

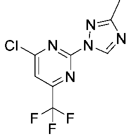

### 2-(3-methyl-1H-1,2,4-triazol-1-yl)-6-(trifluoromethyl)pyrimidin-4-ol

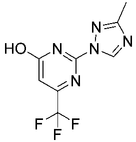

Cuprous iodide (0.67 g, 3.50 mmol) was added into a solution of 2-chloro-4-(methylthio)-6-(trifluoromethyl)pyrimidine (4 g, 17.50 mmol, intermediate 56), 3-methyl-1H-1,2,4-triazole (2.18 g, 26.24 mmol, CAS: 7170-01-6), and cesium carbonate (11.40 g, 34.99 mmol) in N,N-dimethylformamide (40 mL). Nitrogen replacement was performed for 3 times, and the mixture was stirred at 80°C under nitrogen atmosphere for 16 hours. The reaction solution was diluted with water (100 mL), and extracted with ethyl acetate (20 mL) for 3 times. Organic layers were combined, dried over anhydrous sodium sulfate, filtered and concentrated under reduced pressure, and purified by silica gel column chromatography (petroleum ether/ethyl acetate = 1:100) to obtain the title compound as a white solid (1400 mg, yield: 33%). LC-MS: m/z [M+H]⁺ = 246.

### 4-chloro-2-(3-methyl-1H-1,2,4-triazol-1-yl)-6-(trifluoromethyl)pyrimidine

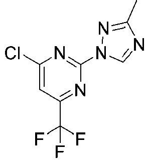

2-(3-methyl-1H-1,2,4-triazol-1-yl)-6-(trifluoromethyl)pyrimidin-4-ol (1.373 g, 5.60 mmol) was added into phosphorus oxychloride (5 mL), and the mixture was stirred at 100°C for 1 hour. The reaction solution was cooled to room temperature, concentrated under reduced pressure, and purified by silica gel column chromatography (petroleum ether/ethyl acetate = 1:10) to obtain the title compound as a light yellow solid (630 mg, yield: 43%). LC-MS: m/z [M+H]⁺ = 264.

### Intermediate 54: Tert-butyl 4-(azetidin-3-ylmethyl)piperazin-1-carboxylate

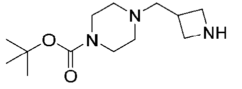

Intermediate 54 was synthesized according to a method provided in Patent Application No. WO2021155321A2.

### Intermediate 56: 2-chloro-4-(methylthio)-6-(trifluoromethyl)pyrimidine

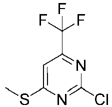

2,4-dichloro-6-trifluoromethylpyrimidine (3.472 g, 16 mmol, CAS: 16097-64-6) was added into tetrahydrofuran (20 mL), sodium thiomethoxide (6.16 g, 17.6 mmol, 20% aqueous solution, CAS: 5188-07-8) was slowly added dropwise at 0°C, and the mixture was stirred at room temperature overnight. A saturated ammonium chloride solution was added into the reaction solution to quench a reaction, and the reaction solution was extracted with ethyl acetate (50 mL) for 3 times. Organic phases were combined, dried over anhydrous sodium sulfate, and concentrated under reduced pressure to obtain the title compound as a white solid (3.66 g, yield: 100%). LC-MS: m/z [M+H]⁺ = 229.

### Intermediate 58: 1-(6-(piperazin-1-yl)pyridin-2-yl)-1H-imidazol-4-carboxamide

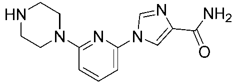

### Tert-butyl 4-(6-(4-(ethoxycarbonyl)-1H-imidazol-1-yl)pyridin-2-yl)piperazin-1-carboxylate

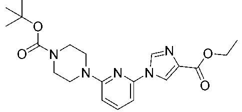

Tert-butyl 4-(6-bromopyridin-2-yl)piperazin-1-carboxylate (860.0 mg, 2.5 mmol, intermediate 23), ethyl imidazol-4-carboxylate (1.75 g, 12.5 mmol, CAS: 23785-21-9), a tris(dibenzylideneacetone)dipalladium-chloroform adduct (388.2 mg, 0.375 mmol, CAS: 52522-40-4), 2-di-tert-butylphosphino-2',4',6'-triisopropylbiphenyl (286.6 mg, 0.675 mmol, CAS: 564483-19-8), and potassium phosphate (1.06 g, 36 mmol, CAS: 7778-53-2) were added into a reaction flask, toluene (10 mL) was added into the reaction flask through a syringe in a nitrogen atmosphere, and the reaction mixture was stirred at 110°C for 12 hours. The reaction solution was cooled to room temperature, and filtered through celite. The filter cake was washed with ethyl acetate (15 mL). The filtrate was concentrated under reduced pressure, and purified by silica gel column chromatography (petroleum ether/ethyl acetate = 3:1) to obtain the title compound as a yellow solid (622.3 mg, yield: 62%). ¹H NMR (400 MHz, CDCl₃) δ 8.23 (s, 2H), 7.58 (t, J = 8.0 Hz, 1H), 6.67 (d, J = 7.6 Hz, 1H), 6.57 (d, J = 8.5 Hz, 1H), 4.37 (q, J = 7.1 Hz, 2H), 3.58 - 3.53 (m, 8H), 1.46 (s, 9H), 1.38 (t, J = 7.1 Hz, 3H).

### Tert-butyl 4-(6-(4-carbamoyl-1H-imidazol-1-yl)pyridin-2-yl)piperazin-1-carboxylate

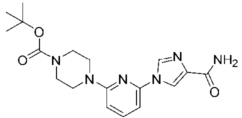

Tert-butyl 4-(6-(4-(ethoxycarbonyl)-1H-imidazol-1-yl)pyridin-2-yl)piperazin-1-carboxylate (250.0 mg, 0.67 mmol) was added into a reaction flask, methanol (6.8 mL) was added into the reaction flask through a syringe, and aqueous ammonia (2 mL) was slowly added dropwise into the reaction flask through a syringe in during stirring. The reaction mixture was stirred at 60°C for 12 hours. The reaction solution was concentrated under reduced pressure, and purified by silica gel column chromatography (dichloromethane/methanol = 30:1) to obtain the title compound as a white solid (135.8 mg, yield: 54%). ¹H NMR (400 MHz, CDCl₃) δ 8.25 (d, J = 5.5 Hz, 2H), 7.60 (t, J = 8.0 Hz, 1H), 6.68 (d, J = 7.6 Hz, 1H), 6.58 (d, J = 8.5 Hz, 1H), 3.59 - 3.54 (m, 8H), 1.47 (s, 9H).

### 1-(6-(piperazin-1-yl)pyridin-2-yl)-1H-imidazol-4-carboxamide

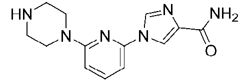

Tert-butyl 4-(6-(4-carbamoyl-1H-imidazol-1-yl)pyridin-2-yl)piperazin-1-carboxylate (130.0 mg, 0.35 mmol) was added into a reaction flask, ethyl acetate (1.5 mL) was added into the reaction flask through a syringe, and a 4 M solution of hydrogen chloride in ethyl acetate (8 mL) was added dropwise at 0°C. The reaction mixture was stirred at room temperature for 15 hours. The reaction mixture was filtered. The filter cake was washed with ethyl acetate (8 mL), collected, and dried to obtain the title compound as a yellow solid (113.5 mg, yield: 105%, hydrochloride). LC-MS: m/z [M+H]⁺ = 273.

### Intermediate 59: 5-(4-(methylsulfonyl)pyrimidin-2-yl)thiazole

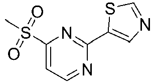

### 5-(4-(methylthio)pyrimidin-2-yl)thiazole

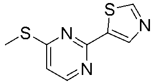

2-chloro-4-methylthiopyrimidine (1000 mg, 6.28 mmol, CAS: 49844-93-1), potassium carbonate (1811 mg, 12.56 mmol), tris(dibenzylideneacetone)dipalladium (56 mg, 0.06 mmol, CAS: 51364-51-3), 1,3,5,7-tetramethyl-6-phenyl-2,4,8-trioxa-6-phosphaadamantane (35 mg, 0.12 mmol, CAS: 97739-46-3), and thiazole-5-boronic acid pinacol ester (1524 mg, 6.9 mmol, CAS: 1086111-09-2) were added into a mixed solution of 1,4-dioxane (10 mL) and water (2 mL), nitrogen replacement was performed for 3 times, and the mixture was stirred at 100°C overnight. The reaction solution was cooled to room temperature, diluted with water (50 mL), and extracted with ethyl acetate (30 mL) for 3 times. Organic phases were combined, washed with brine (30 mL) for 2 times, dried over anhydrous sodium sulfate, concentrated under reduced pressure, and purified by silica gel column chromatography (petroleum ether/ethyl acetate = 5:1) to obtain the title compound as a white solid (100 mg, yield: 8%). LC-MS: m/z [M+H]⁺ = 210.

### 5-(4-(methylsulfonyl)pyrimidin-2-yl)thiazole

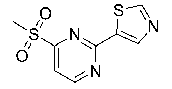

5-(4-(methylthio)pyrimidin-2-yl)thiazole (100 mg, 0.48 mmol) was dissolved in dichloromethane (5 mL), m-chloroperoxybenzoic acid (149 mg, 0.96 mmol) was added, and the mixture was stirred at 25°C for 2 hours. The reaction solution was filtered. The filter cake was washed with dichloromethane (5 mL). The filtrate was washed sequentially with a saturated sodium sulfite solution (20 mL) for 2 times and with brine (20 mL) for 1 time, dried over anhydrous sodium sulfate, concentrated under reduced pressure to dryness, and purified by silica gel column chromatography (petroleum ether/ethyl acetate = 1:1) to obtain the title compound as a white solid (50 mg, yield: 43%). LC-MS: m/z [M+H]⁺ = 242.

Referring to the table below, except that raw materials listed in the "Raw material" column were used to replace the corresponding raw material, the following intermediates 60, 61, 63, and 67 were prepared according to a preparation method of the intermediate 59.

| Number | Name | Structure | LC-MS (m/z [M+H]⁺) | Raw material |
|---|---|---|---|---|
| Intermediat e 60 | 5-(4-(methylsulfonyl)-6-(trifluoromethyl)pyrimidi n-2 -yl)thiazole | 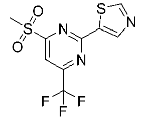 | 310 | Intermediate 56 |
| Intermediat e 61 | 5-(5-fluoro-4-(methylsulf onyl) pyrimidin-2-yl)thiazole | 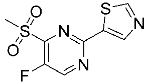 | 260 | 2-chloro-5-fluoro-4 (methylthio)pyrimi dine (CAS: 87789-51-3) |
| Intermediat e 63 | 2-(1-methyl-2-(trifluorom ethyl)-1H-imidazol-5-yl)-4-(methylsulfonyl)pyrimidi ne | 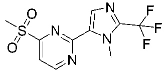 | 307 | Intermediate 62 |
| Intermediat e 67 | 3,5-dimethyl-4-(4-(methy 1 sulfonyl)pyrimidin-2-yl) isoxazole | 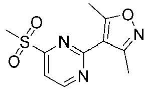 | 254 | 3,5-dimethylisoxaz ol-4-boronic acid (CAS: 16114-47-9) |

### Intermediate 62: (1-methyl-2-(trifluoromethyl)-1H-imidazol-5-yl)boronic acid

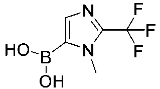

### 1-methyl-2-(trifluoromethyl)-1H-imidazole

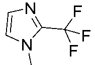

Iodomethane (0.69 mL, 11.02 mmol, d = 2.28 g/mL) was added into a solution of 2-(trifluoromethyl)-1H-imidazole (1 g, 7.35 mmol, CAS: 66675-22-7) and cesium carbonate (7.18 g, 22.05 mmol) in acetonitrile (10 mL), and the mixture was stirred at room temperature for 16 hours. The reaction solution was filtered. The filtrate was concentrated under reduced pressure, and purified by silica gel column chromatography (dichloromethane/methanol = 10:1) to obtain the title compound as a yellow oil (800 mg, yield: 72.5%). LC-MS: m/z [M+H]⁺ = 151.

### (1-methyl-2-(trifluoromethyl)-1H-imidazol-5-yl)boronic acid

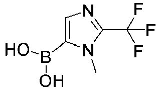

At -78°C and under nitrogen atmosphere, n-butyllithium (3.67 mL, 5.88 mmol, 1.6 M solution in hexane) was slowly added dropwise into a solution of 1-methyl-2-(trifluoromethyl)-1H-imidazole (735 mg, 4.90 mmol) in tetrahydrofuran (10 mL), and the mixture was stirred at -78°C for 10 minutes. Then, triisopropyl borate (1.36 mL, 5.88 mmol, d = 0.815 g/mL, CAS: 5419-55-6) was slowly added dropwise, and the reaction mixture was stirred at -78°C for 1 hour. A saturated ammonium chloride solution (5 mL) was slowly added dropwise into the reaction solution to quench a reaction, and the reaction solution was extracted with ethyl acetate (10 mL) for 3 times. Organic phases were dried over anhydrous sodium sulfate, filtered, concentrated under reduced pressure, and purified by prep-HPLC (13-43% (v/v) acetonitrile and water, and 0.1% formic acid) to obtain the title compound as a white solid (420 mg, yield: 44%). LC-MS: m/z [M+H]⁺ = 195.

### Intermediate 65: 2-(6-fluoropyridin-2-yl)-5-(trifluoromethyl)-1,3,4-thiadiazole

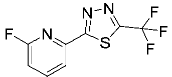

2-fluoropyridin-6-boronic acid (141 mg, 1 mmol, CAS: 916176-61-9), a [1,1'-bis(diphenylphosphino)ferrocene]dichloropalladium dichloromethane complex (82 mg, 0.1 mmol, CAS: 95464-05-4), and sodium carbonate (530 mg, 5 mmol) were added into a Schlenk tube, nitrogen replacement was performed for 3 times, and dioxane (4 mL), water (1 mL), and 2-bromo-5-trifluoromethyl-1,3,4-thiazole (280 mg, 1.2 mmol, CAS: 37461-61-3) were sequentially added in a nitrogen atmosphere. The reaction tube was moved to 100°C and stirred for 2 hours. The reaction solution was cooled to room temperature, and filtered through celite. The filtrate was concentrated under reduced pressure, and purified by silica gel column chromatography (petroleum ether/ethyl acetate = 5:1) to obtain the title compound as a white solid (110 mg, yield: 44%). LC-MS: m/z [M+H]⁺ = 250.

### Intermediate 66: 4-(methylsulfonyl)-2-(pyridin-3-yl)pyrimidine

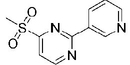

### 2-chloro-4-(methylsulfonyl)pyrimidine

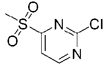

2-chloro-4-methylthiopyrimidine (1.6 g, 10 mmol, CAS: 49844-93-1) was dissolved in anhydrous dichloromethane (20 mL), m-chloroperbenzoic acid (5.2 g, 30 mmol) was slowly added under an ice bath, and the mixture was stirred at room temperature for 12 hours. A saturated sodium bicarbonate solution (20 mL) was slowly added into the reaction solution to quench a reaction, and the reaction solution was extracted with dichloromethane (100 mL) for 3 times. Organic phases were combined, washed with brine (50 mL) for 2 times, dried, concentrated under reduced pressure, and purified by silica gel column chromatography (petroleum ether/ethyl acetate = 5:1) to obtain the title compound as a white solid (1.6 g, yield: 84%). LC-MS: m/z [M+H]⁺ = 193.

### 4-(methylsulfonyl)-2-(pyridin-3-yl)pyrimidine

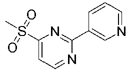

2-chloro-4-(methylsulfonyl)pyrimidine (1.6 g, 8.3 mmol), pyridin-3-boronic acid (2.1 g, 17 mmol, CAS: 1692-25-7), and a [1,1'-bis(diphenylphosphino)ferrocene]dichloropalladium dichloromethane complex (680 mg, 0.83 mmol, CAS: 95464-05-4) were added into a Schlenk tube, nitrogen replacement was performed for 3 times, and dioxane (8 mL) and water (2 mL) were sequentially added in a nitrogen atmosphere. The reaction tube was moved to 100°C and stirred for 2 hours. The reaction solution was cooled to room temperature, and filtered through celite. The filtrate was concentrated under reduced pressure, and purified by silica gel column chromatography (petroleum ether/ethyl acetate = 5:1) to obtain the title compound as a white solid (605 mg, yield: 31 %). LC-MS: m/z [M+H]⁺ = 236.

### Intermediate 68:

### 1-(4-(chloromethyl)benzyl)-4-(6-(3-methyl-1H-1,2,4-triazol-1-yl)pyridin-2-yl)piperazine

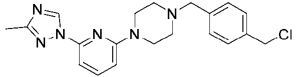

### 2-bromo-6-(3-methyl-1H-1,2,4-triazol-1-yl)pyridine

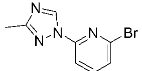

2,6-dibromopyridine (4.00 g, 17 mmol, CAS: 626-05-1), 3-methyl-1H-1,2,4-triazole (1.80 g, 22 mmol, CAS: 7170-01-6), cuprous iodide (1.30 g, 7 mmol), and cesium carbonate (11.00 g, 34 mmol) were added into N,N-dimethylformamide (40 mL), nitrogen replacement was performed for 3 times, and the mixture was stirred at 120°C for 4 hours. The reaction solution was cooled to room temperature, diluted with water (100 mL), and extracted with ethyl acetate (100 mL) for 2 times. Organic phases were combined, washed with brine (100 mL) for 1 time, dried over anhydrous sodium sulfate, concentrated under reduced pressure, and purified by silica gel column chromatography (ethyl acetate/petroleum ether = 4:1) to obtain the title compound as a white solid (2.16 g, yield: 53.5%). LC-MS: m/z [M+H]⁺ = 239/241.

### 1-(6-(3-methyl-1H-1,2,4-triazol-1-yl)pyridin-2-yl)piperazine

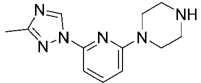

2-bromo-6-(3-methyl-1H-1,2,4-triazol-1-yl)pyridine (2.10 g, 9 mmol) was dissolved in N,N-dimethylformamide (20 mL), anhydrous piperazine (1.55 g, 18 mmol) and potassium carbonate (2.49 g, 18 mmol) were added, and the mixture was stirred at 120°C for 3.5 hours. The reaction solution was cooled to room temperature, diluted with water (100 mL), and extracted with a mixed solvent (dichloromethane/methanol = 20:1, 100 mL) for 3 times. Organic phases were combined, washed with brine (100 mL) for 1 time, dried over anhydrous sodium sulfate, concentrated under reduced pressure, and purified by silica gel column chromatography (dichloromethane/methanol = 20:1) to obtain the title compound as a white solid (1.22 g, yield: 57%). LC-MS: m/z [M+H]⁺ = 245.

### 1-(4-(chloromethyl)benzyl)-4-(6-(3-methyl-1H-1,2,4-triazol-1-yl)pyridin-2-yl)piperazine

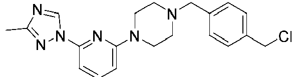

1-(6-(3-methyl-1H-1,2,4-triazol-1-yl)pyridin-2-yl)piperazine (0.26 g, 1 mmol) and 4-(chloromethyl)benzaldehyde (0.15 g, 1 mmol, CAS: 73291-09-5) were added into toluene (10 mL), and the mixture was stirred at room temperature for half an hour. Then, sodium triacetoxyborohydride (0.68 g, 3.2 mmol) was added in three portions at an interval of about 5 minutes each time, and the reaction mixture was stirred at room temperature overnight. The reaction solution was diluted with water (100 mL), and extracted with ethyl acetate (100 mL) for 2 times. Organic phases were combined, washed with brine (100 mL) for 1 time, dried over anhydrous sodium sulfate, concentrated under reduced pressure, and purified by silica gel column chromatography (dichloromethane/methanol = 20:1) to obtain the title compound as a white solid (0.4 g, yield: 98%). LC-MS: m/z [M+H]⁺ = 383.

Referring to the table below, except that raw materials listed in the "Raw material" column were used to replace the corresponding raw material, the following intermediates 72a, 72, 73a, 73, 79, 81, and 84 were prepared according to a preparation method of the intermediate 68.

| Number | Name | Structure | LC-MS (m/z [M+H]⁺) | Raw material |
|---|---|---|---|---|
| Intermediat e 72a | 2-methyl-4-(3-methyl-1H-1 ,2,4-triazol-1-yl)-6-(piperazin-1-yl) pyrimidine | 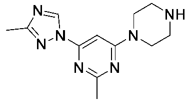 | 260 | 4,6-dichloro-2-methyl pyrimidine (CAS: 1780-26-3) |
| Intermediat e 72 | 4-(4-(4-(chloromethyl)benz yl) piperazin-1-yl)-2-methyl-6-(3-methyl-1H-1,2,4-triazol-1-y 1) pyrimidine | 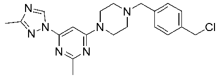 | 398 | Intermediate 72a |
| Intermediat e 73a | 2-methyl-4-(piperazin-1-yl) -6-(4-(trifluoromethyl)-1H-imida zol-1-yl)pyrimidine | 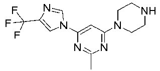 | 313 | 4,6-dichloro-2-methyl pyrimidine (CAS: 1780-26-3) and 4-(trifluoromethyl)-1H imidazole (CAS: 33468-69-8) |
| Intermediat e 73 | 4-(4-(4-(chloromethyl)benz yl) piperazin-1-yl)-2-methyl-6-(4-(trifluoromethyl)-1H-imida zol-1-yl)pyrimidine | 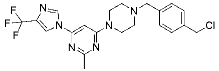 | 451 | Intermediate 73a |
| Intermediat e 79 | 4-(4-(1-(4-(chloromethyl)be nzyl) azetidin-3-yl)piperazin-1-yl )-2-methyl-6-(3-methyl-1H-1,2, 4-triazol-1-yl)pyrimidine | 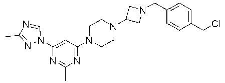 | 453 | Intermediate 78 |
| Intermediat e 81 | 4-(4-(1-(4-(chloromethyl)be nzyl) azetidin-3-yl)piperazin-1-yl )-2-methyl-6-(4-(trifluoromethy 1)-1H-imidazol-1-yl)pyrimi dine | 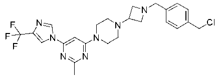 | 506 | Intermediate 80 |
| Intermediat e 84 | 4-(4-(1-(4-(chloromethyl)be nzyl) azetidin-3-yl)piperazin-1-yl )-5-fluoro-2-(3-methyl-1H-1,2, 4-triazol-1-yl)pyrimidine | 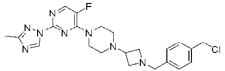 | 457 | Intermediate 50 |

### Intermediate 69: 5-(5-fluoro-4-(piperazin-1-yl)pyrimidin-2-yl)-2-methylthiazole

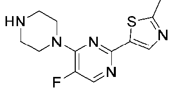

### Tert-butyl 4-(5-fluoro-2-(2-methylthiazol-5-yl)pyrimidin-4-yl)piperazin-1-carboxylate

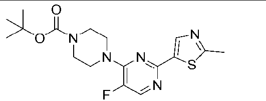

Tert-butyl 4-(2-chloro-5-fluoropyrimidin-4-yl)piperazin-1-carboxylate (634 mg, 2 mmol), 2-methylthiazol-5-boronic acid pinacol ester (675 mg, 3 mmol, CAS: 1218791-01-5), potassium phosphate (1.272 g, 6 mmol), and tetrakis(triphenylphosphine)palladium (115.6 mg, 0.1 mmol) were added into toluene (10 mL), nitrogen replacement was performed for 3 times, and the mixture was stirred at 130°C for 3 hours. The reaction solution was cooled to room temperature, and filtered through celite. The filtrate was concentrated under reduced pressure, and purified by silica gel column chromatography (petroleum ether/ethyl acetate = 1:1) to obtain the title compound as a white solid (493 mg, yield: 65%). LC-MS: m/z [M+H]⁺ = 380.

### 5-(5-fluoro-4-(piperazin-1-yl)pyrimidin-2-yl)-2-methylthiazole

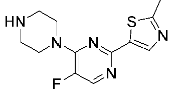

Tert-butyl 4-(5-fluoro-2-(2-methylthiazol-5-yl)pyrimidin-4-yl)piperazin-1-carboxylate (493 mg, 1.3 mmol) was added into dichloromethane (8.5 mL), trifluoroacetic acid (1.5 mL) was slowly added dropwise at 0°C, and the reaction solution was stirred at room temperature for 20 minutes. The reaction solution was directly concentrated under reduced pressure to obtain the title compound as a colorless oil (510 mg, yield: 100%, TFA salt). LC-MS: m/z [M+H]⁺ = 280.

### Intermediate 82: 2-chloro-6-(1-isopropyl-4,5-dihydro-1H-imidazol-2-yl)pyridine

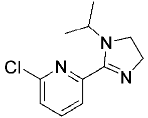

### 6-chloro-N-(2-(isopropylamino)ethyl)pyridinecarboxamide

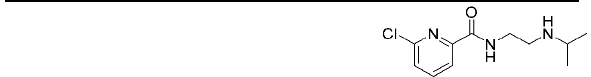

At room temperature, to a solution of methyl 6-chloropyridin-2-carboxylate (1000 mg, 5.83 mmol, CAS: 6636-55-1) and N-isopropylethylenediamine (0.88 mL, 6.99 mmol, d = 0.819 g/mL, CAS: 19522-67-9) in toluene (20 mL), trimethylaluminum (8.74 mL, 8.74 mmol, 1 M solution in hexane, CAS: 75-24-1) was slowly added, and the mixture was refluxed overnight under nitrogen atmosphere. Under ice bath conditions, water was added into the reaction solution to quench a reaction, and the reaction solution was extracted with ethyl acetate (50 mL) for 3 times. Organic phases were combined, dried over anhydrous sodium sulfate, filtered, and concentrated under reduced pressure to obtain the title compound as a light yellow oil (1400 mg, yield: 99%). LC-MS: m/z [M+H]⁺ = 242.

### 2-chloro-6-(1-isopropyl-4,5-dihydro-1H-imidazol-2-yl)pyridine

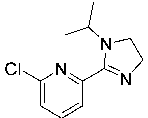

At room temperature, 6-chloro-N-(2-(isopropylamino)ethyl)pyridinecarboxamide (600 mg, 2.48 mmol) was dissolved in phosphorus oxychloride (10 mL), and the mixture was stirred at 110°C overnight under nitrogen atmosphere. The reaction solution was cooled to room temperature, concentrated under reduced pressure, and purified by flash liquid chromatography (C18 chromatographic column) to obtain the title compound as a white solid (130 mg, yield: 23%). LC-MS: m/z [M+H]⁺ = 224.

### Intermediate 85:

### 3-(2-oxo-6-(4-(piperazin-1-ylmethyl-d2)benzyl)benzo[cdlindol-1(2H)-yl)piperidin-2,6-dione

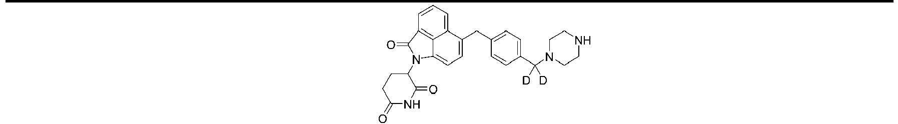

### Methyl 4-(((tert-butyldimethylsilyl)oxy)methyl)benzoate

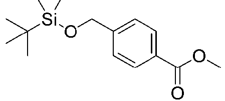

To a solution of methyl 4-(hydroxymethyl)benzoate (5850 mg, 32.46 mmol, CAS: 6908-41-4) and imidazole (4100 mg, 60.22 mmol) in N,N-dimethylformamide (100 mL), tert-butyldimethylsilyl chloride (6100 mg, 40.47 mmol, CAS: 18162-48-6) was added, and the mixture was stirred at room temperature for 3 hours. The reaction solution was slowly poured into a saturated ammonium chloride aqueous solution (400 mL), and extracted with ethyl acetate (200 mL) for 2 times. Organic phases were combined, washed with brine (200 mL) for 1 time, dried over anhydrous sodium sulfate, concentrated under reduced pressure, and purified by silica gel column chromatography (petroleum ether/dichloromethane = 4:1) to obtain the title compound as a light yellow liquid (9.1 g, yield: 95%). LC-MS: m/z [M+H]⁺ = 281.

### (4-(((tert-butyldimethylsilyl)oxy)methyl)ohenyl)methyl-d2-ol

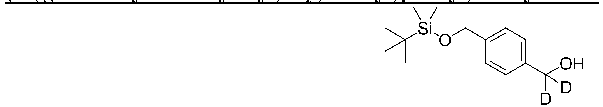

At 0°C, to a solution of lithium aluminum deuteride (2.1 g, 50.02 mmol, CAS: 14128-54-2) in tetrahydrofuran (100 mL), a solution of methyl 4-(((tert-butyldimethylsilyl)oxy)methyl)benzoate (5.1 g, 17.32 mmol) in tetrahydrofuran (30 mL) was slowly added dropwise, and the mixture was stirred at room temperature for 3 hours. The reaction solution was cooled to -10°C, sodium sulfate decahydrate (30 g) was added in portions, and then the mixture was heated to room temperature, and stirred for 30 minutes. The reaction solution was filtered. The filter cake was washed with dichloromethane (50 mL) for 1 time. The filtrate was concentrated under reduced pressure. The residue was dissolved in ethyl acetate (200 mL), and washed with brine (100 mL) for 1 time. The organic phase was dried, and concentrated under reduced pressure to obtain the title compound as a light yellow liquid (3.1 g, yield: 70%).

### (4-(((tert-butyldimethylsilyl)oxy)methyl)phenyl)methyl-d2 methanesulfonate

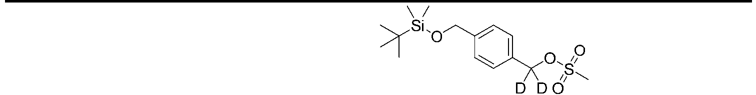

At 0°C, to a solution of (4-(((tert-butyldimethylsilyl)oxy)methyl)phenyl)methyl-d2-ol (1.5 g, 5.90 mmol) and triethylamine (1.85 mL, 13.34 mmol, d = 0.728 g/mL) in N,N-dimethylformamide (100 mL), methylsufonyl chloride (900 mg, 7.86 mmol, CAS: 124-63-0) was slowly added dropwise, and the mixture was stirred at room temperature for 2 hours. The reaction solution was concentrated under reduced pressure. The residue was dissolved in dichloromethane (100 mL), and washed with water (100 mL) for 1 time. The organic phase was dried, and concentrated under reduced pressure to obtain the title compound as a light yellow solid (1.65 g, yield: 85%).

### Tert-butyl 4-(4-((((tert-butyldimethylsilyl)oxy)methyl)phenyl)methyl-d2)piperazin-1-carboxylate

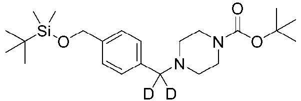

To a solution of 1-(tert-butoxycarbonyl)piperazine (1500 mg, 8.05 mmol, CAS: 57260-71-6) and potassium carbonate (3000 mg, 21.71 mmol) in N,N-dimethylformamide (30 mL), (4-(((tert-butyldimethylsilyl)oxy)methyl)phenyl)methyl-d2 methanesulfonate (1600 mg, 4.81 mmol) was added, and the mixture was stirred at 50°C for 2 hours. The reaction solution was slowly poured into water (150 mL), and extracted with ethyl acetate (50 mL) for 2 times. Organic phases were combined, washed with brine (50 mL) for 1 time, dried over anhydrous sodium sulfate, concentrated under reduced pressure, and purified by silica gel column chromatography (dichloromethane/ethyl acetate = 10:1) to obtain the title compound as a light yellow solid (2010 mg, yield: 99%). LC-MS: m/z [M+H]⁺ = 423.

### Tert-butyl 4-((4-(hydroxymethyl)phenyl)methyl-d2)piperazin-1-carboxylate

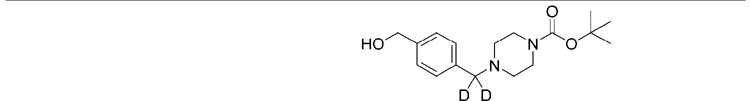

To a solution of tert-butyl 4-(4-((((tert-butyldimethylsilyl)oxy)methyl)phenyl)methyl-d2)piperazin-1-carboxylate (2100 mg, 4.97 mmol) in tetrahydrofuran (20 mL), a 1 N solution of tetrabutylammonium fluoride in tetrahydrofuran (10 mL, CAS: 429-41-4) was added, and the mixture was stirred at 20°C for 3 hours. The reaction solution was concentrated under reduced pressure. The residue was dissolved in ethyl acetate (25 mL), and washed with brine (20 mL) for 1 time. The organic phase was dried over anhydrous sodium sulfate, concentrated under reduced pressure, and purified by silica gel column chromatography (dichloromethane/methanol = 25:1) to obtain the title compound as a yellow liquid (1400 mg, yield: 91%). LC-MS: m/z [M+H]⁺ = 309.

### Tert-butyl 4-(4-(chloromethyl)phenyl)methyl-d2)piperazin-1-carboxylate

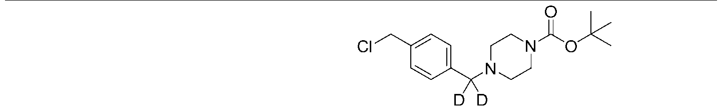

To a solution of tert-butyl 4-((4-(hydroxymethyl)phenyl)methyl-d2)piperazin-1-carboxylate (1500 mg, 4.86 mmol) in dichloromethane (20 mL), thionyl chloride (1500 mg, 12.61 mmol) was slowly added, and the mixture was stirred at 20°C for 2 hours. The reaction solution was concentrated under reduced pressure. The residue was dissolved in dichloromethane (25 mL), and washed with brine (20 mL) for 1 time. The organic phase was dried, and concentrated under reduced pressure to obtain the title compound as a yellow liquid (1300 mg, yield: 82%). LC-MS: m/z [M+H]⁺ = 327.

### Tert-butyl 4-((4-((2-oxo-1,2-dihydrobenzo[cd]indol-6-yl)methyl)phenyl)methyl-d2)piperazin-1-carboxylate

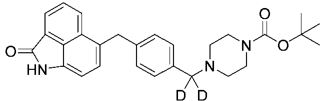

In a nitrogen atmosphere, tris(dibenzylideneacetone)dipalladium (60 mg, 0.07 mmol, CAS: 51364-51-3) and tris(2-methylphenyl)phosphine (100 mg, 0.33 mmol, CAS: 6163-58-2) were added into a mixed solution of tert-butyl 4-(4-(chloromethyl)phenyl)methyl-d2)piperazin-1-carboxylate (1200 mg, 3.67 mmol), 6-(4,4,5,5-tetramethyl-1,3,2-dioxaborolan-2-yl)benzo[cd]indol-2(1H)-one (1200 mg, 4.07 mmol, synthesized according to a method provided in Patent Application No. WO2020210630A1), and potassium phosphate (2500 mg, 11.78 mmol) in ethanol (8 mL) and toluene (20 mL), and the reaction mixture was stirred at 100°C for 10 hours. The reaction solution was cooled to room temperature, and filtered through celite. The filter cake was washed with ethyl acetate (20 mL) for 1 time. The filtrate was concentrated under reduced pressure, and purified by silica gel column chromatography (dichloromethane/ethyl acetate = 1:1) to obtain the title compound as a yellow solid (1150 mg, yield: 68%). LC-MS: m/z [M+H]⁺ = 460.

### Tert-butyl 4-((4-((1-(2,6-dioxopiperidin-3-yl)-2-oxo-1,2-dihydrobenzo[cd]indol-6-yl)methyl)phenyl)methyl-d2)pip erazin-1-carboxylate

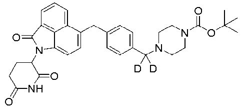

At 55°C, sodium hydride (2500 mg, 62.50 mmol, 60% stored in mineral oil) was added in portions into a solution of tert-butyl 4-((4-((2-oxo-1,2-dihydrobenzo[cd]indol-6-yl)methyl)phenyl)methyl-d2)piperazin-1-carboxylate (1150 mg, 2.50 mmol) in tetrahydrofuran (20 mL), and the mixture was stirred at 55°C for 30 minutes. Then, at the temperature, 3-bromopiperidin-2,6-dione (2100 mg, 10.94 mmol, CAS: 62595-74-8) was added in portions, and the reaction mixture was heated to 70°C for a reaction for 5 hours. The reaction solution was cooled to room temperature, slowly poured into a saturated ammonium chloride solution (150 mL), and extracted with ethyl acetate (50 mL) for 2 times. Organic phases were combined, washed with brine (50 mL) for 1 time, dried over anhydrous sodium sulfate, concentrated under reduced pressure, and purified by silica gel column chromatography (dichloromethane/ethyl acetate = 1:1) to obtain the title compound as a light yellow solid (800 mg, yield: 56%). LC-MS: m/z [M+H]⁺ = 571.

### 3-(2-oxo-6-(4-(piperazin-1-ylmethyl-d2)benzyl)benzo[cd]indol-1(2H)-yl)piperidin-2,6-dione

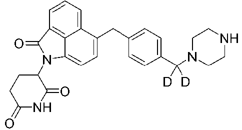

To a solution of tert-butyl 4-((4-((1-(2,6-dioxopiperidin-3-yl)-2-oxo-1,2-dihydrobenzo[cd]indol-6-yl)methyl)phenyl)methyl-d2)piperazi n-1-carboxylate (750 mg, 1.31 mmol) in dichloromethane (10 mL), trifluoroacetic acid (1.5 mL) was slowly added dropwise, and the mixture was stirred at 20°C for 2 hours. The reaction solution was concentrated under reduced pressure to obtain the title compound as a yellow solid (800 mg, yield: 104%, TFA salt). LC-MS: m/z [M+H]⁺ = 471.

### Example 1 (S)-3-(4-((4-((4-(2-(1H-imidazol-1-yl)pyrimidin-4-yl)piperazin-1-yl)methyl)benzyl)oxy)-1-oxoisoindolin -2-yl)piperidin-2,6-dione

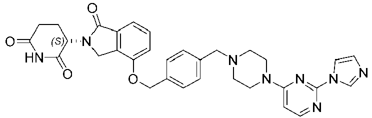

### Tert-butyl (S)-4-(4-((4-((4-(2-(1H-imidazol-1-yl)pyrimidin-4-yl)piperazin-1-yl)methyl)benzyl)oxy)-1-oxoisoindolin -2-yl)-5-amino-5-oxopentanoate

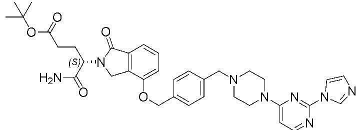

4-(4-(4-(chloromethyl)benzyl)piperazin-1-yl)-2-(1H-imidazol-1-yl)pyrimidine (2.0 g, 5.4 mmol, intermediate 2) was dissolved in anhydrous N,N-dimethylformamide (20 mL), and potassium carbonate (1.5 g, 10.8 mmol) was added at room temperature, and stirred for 30 minutes. Subsequently, a solution of tert-butyl (S)-5-amino-4-(4-hydroxy-1-oxoisoindolin-2-yl)-5-oxopentanoate (1.8 g, 5.4 mmol, intermediate 1) in N,N-dimethylformamide (5 mL) was slowly added dropwise into the above solution, and stirred at 45°C for 12 hours. When TLC (dichloromethane/methanol = 20:1) determined the completion, brine (100 mL) was added into the reaction solution, and the reaction solution was extracted with ethyl acetate (30 mL) for 3 times, and then purified by silica gel column chromatography (dichloromethane/methanol = 30:1) to obtain the title compound as a yellow oil (770 mg, yield: 21 %). LC-MS: m/z [M+H]⁺ = 667.

### (S)-3-(4-((4-((4-(2-(1H-imidazol-1-yl)pyrimidin-4-yl)piperazin-1-yl)methyl)benzyl)oxy)-1-oxoisoin dolin-2-yl)piperidin-2,6-dione

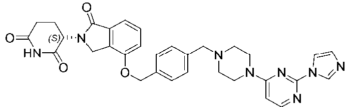

Tert-butyl (S)-4-(4-((4-((4-(2-(1H-imidazol-1-yl)pyrimidin-4-yl)piperazin-1-yl)methyl)benzyl)oxy)-1-oxoisoindolin-2-yl)-5-amino-5-oxopentanoate (770 mg, 1.15 mmol) and benzenesulfonic acid (760 mg, 4.8 mmol) were added into a reaction flask, anhydrous acetonitrile (50 mL) was added in a nitrogen atmosphere, and the reaction solution was stirred at 85°C for 1 hour. When TLC (dichloromethane/methanol = 20:1) determined the completion, the reaction was quenched with a saturated sodium bicarbonate solution (50 mL), and the reaction solution was extracted with ethyl acetate (30 mL) for 3 times. Organic phases were combined, dried over anhydrous sodium sulfate, concentrated under reduced pressure, and purified by silica gel column chromatography (dichloromethane/methanol = 15:1) to obtain the title compound as a white solid (43 mg, yield: 6%). ¹H NMR (500 MHz, CDCl₃) δ 8.97 (s, 1H), 8.51 (s, 1H), 8.11 (d, *J =* 6.2 Hz, 1H), 7.78 (s, 1H), 7.49 (d, *J =* 7.5 Hz, 1H), 7.42 (d, *J =* 7.8 Hz, 1H), 7.38 (m, 4 H), 7.09 (m, 2H), 6.34 (d, *J =* 6.2 Hz, 1H), 5.22 (dd, *J =* 13.3, 5.1 Hz, 1H), 5.13 (s, 2H), 4.45 (d, *J* = 16.5 Hz, 1H), 4.31 (d, *J* = 16.5 Hz, 1H), 3.70 (s, 4H), 3.57 (s, 2H), 2.85 (m, 2H), 2.54 (m, 4H), 2.32 (m, 1H), 2.19 (m, 1H). LC-MS: m/z [M+H]⁺ = 593.

### Example 2 3-(6-(4-((4-(6-(1H-imidazol-1-yl)pyridin-2-yl)piperazin-1-yl)methyl)benzyl)-2-oxobenzo[cd]indol-1(2H) -yl)piperidin-2,6-dione

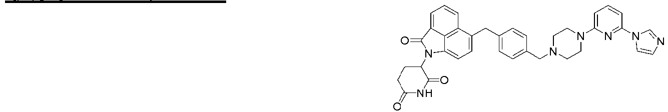

2-fluoro-6-(1H-imidazol-1-yl)pyridine (326 mg, 1.28 mmol, intermediate 4) and 3-(2-oxo-6-(4-(piperazin-1-ylmethyl)benzyl)benzo[cd]indol-1(2H)-yl)piperidin-2,6-dione (300 mg, 0.64 mmol, intermediate 3) were added into a 25 mL reaction flask, and then potassium carbonate (265 mg, 1.92 mmol) and dimethyl sulfoxide (5 mL) were added for a reaction at 90°C overnight. Brine (50 mL) was added into the reaction solution, and the reaction solution was extracted with ethyl acetate (20 mL) for 3 times. Organic phases were combined, dried over anhydrous sodium sulfate, concentrated under reduced pressure, and purified by silica gel column chromatography (dichloromethane/methanol = 20:1) to obtain the title compound as a yellow solid (45 mg, yield: 11.5%). ¹H NMR (500 MHz, CDCl₃) δ 8.27 (s, 1H), 8.23 (s, 1H), 8.11 (dd, J = 19.7, 7.6 Hz, 2H), 7.70 (dd, J = 8.3, 7.0 Hz, 1H), 7.59 - 7.50 (m, 2H), 7.24 (s, 2H), 7.17 (d, J = 7.8 Hz, 2H), 7.14 (s, 1H), 6.70 (d, J = 7.3 Hz, 1H), 6.59 (d, J = 7.6 Hz, 1H), 6.49 (d, J = 8.5 Hz, 1H), 5.33 (dd, J = 12.9, 5.4 Hz, 1H), 4.38 (s, 2H), 3.57 (t, J = 5.0 Hz, 4H), 3.52 (s, 2H), 2.95 (d, J = 4.6 Hz, 1H), 2.87 (ddd, J = 17.6, 13.3, 5.1 Hz, 1H), 2.73 (qd, J = 13.1, 4.7 Hz, 1H), 2.53 (t, J = 5.1 Hz, 4H), 2.29 (ddq, J = 10.5, 5.5, 2.5 Hz, 1H); LC-MS: m/z [M+H]⁺ =612.

### Example 3 3-(6-(4-((4-(2-(1H-imidazol-1-yl)pyrimidin-4-yl)piperazin-1-yl)methyl)benzyl)-2-oxobenzo[cd]indol-1(2 H)-yl)piperidin-2,6-dione

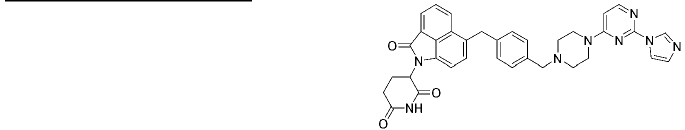

3-(2-oxo-6-(4-(piperazin-1-ylmethyl)benzyl)benzo[cd]indol-1(2H)-yl)piperidin-2,6-dione (234 mg, 0.5 mmol, intermediate 3), 2-(1H-imidazol-1-yl)-4-(methylsulfonyl)pyrimidine (168 mg, 0.75 mmol, intermediate 5), and N,N-diisopropylethylamine (387 mg, 3 mmol) were added into a 50 mL single-necked flask, then isopropanol (5 mL) was added, and the mixture was stirred at 100°C overnight. The reaction solution was directly concentrated under reduced pressure, and purified by silica gel column chromatography (dichloromethane/methanol = 20:1) to obtain the title compound as a yellow solid (45 mg, yield: 15%). ¹H NMR (500 MHz, CDCl₃) δ 8.50 (s, 1H), 8.32 (s, 1H), 8.13 - 8.08 (m, 3H), 7.78 (s, 1H), 7.72 - 7.68 (m, 1H), 7.23 (d, *J =* 7.9 Hz, 3H), 7.17 (d, *J =* 8.1 Hz, 2H), 7.09 (s, 1H), 6.70 (d, *J =* 7.3 Hz, 1H), 6.33 (d, *J =* 6.2 Hz, 1H), 5.33 (dd, *J =* 12.9, 5.4 Hz, 1H), 4.37 (s, 2H), 3.67 (s, 4H), 3.51 (s, 2H), 2.96 (dd, *J =* 13.9, 3.7 Hz, 1H), 2.90 - 2.83 (m, 1H), 2.73 (qd, *J =* 13.2, 4.7 Hz, 1H), 2.50 (t, *J =* 5.1 Hz, 4H), 2.31 - 2.26 (m, 1H); LC-MS: m/z [M+H]⁺ = 613.

### Example 4 3-(6-(4-((4-(6-(1H-imidazol-1-yl)pyridin-2-carbonyl)piperazin-1-yl)methyl)benzyl)-2-oxobenzo[cd]indo 1-1(2H)-yl)piperidin-2,6-dione

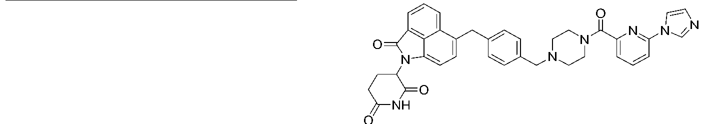

3-(2-oxo-6-(4-(piperazin-1-ylmethyl)benzyl)benzo[cd]indol-1(2H)-yl)piperidin-2,6-dione (0.46 g, 0.98 mmol, intermediate 3), 6-(1H-imidazol-1-yl)-2-picolinic acid (0.19 g, 0.98 mmol, intermediate 6), 1-ethyl-(3-dimethylaminopropyl)carbodiimide hydrochloride (0.28 g, 1.47 mmol, CAS: 25952-53-8), and 1-hydroxybenzotriazole (0.20 g, 1.47 mmol, CAS: 2592-95-2) were added into a 25 mL two-necked flask, then dichloromethane (10 mL) was added in a nitrogen atmosphere, and N-methylmorpholine (0.2 mL, 1.96 mmol, d = 0.92 g/mL) was added through a syringe. The mixture was stirred at room temperature overnight. The reaction solution was diluted with brine (30 mL), and extracted with dichloromethane (10 mL) for 3 times. Organic phases were combined, dried over anhydrous sodium sulfate, concentrated under reduced pressure, and purified by silica gel column chromatography (dichloromethane/methanol = 15:1) to obtain the title compound as a yellow solid (44 mg, yield: 7%). ¹H NMR (500 MHz, CDCl₃) δ8.75 (s, 1H), 8.35 (s, 1H), 8.13 (d, J = 8.13 Hz, 1H), 8.08 (d, J = 8.08 Hz, 1H), 7.93 (t, J = 7.93 Hz, 1H), 7.70 (dd, J = 8.3, 7.0 Hz, 1H), 7.60 - 7.58 (m, 2H), 7.39 (d, J = 8.2 Hz, 1H), 7.24 -7.21 (t, J = 7.3 Hz, 4H), 7.16 - 7.18 (m, 2H), 6.70 (d, J = 7.2 Hz 1H), 5.34 (dd, J = 12.9, 5.4 Hz, 1H), 4.37 (s, 2H), 3.83 - 3.82 (m, 2H), 3.62 - 3.60 (m, 2H), 3.52 (s, 2H), 2.98 - 2.93 (m, 1H), 2.90 - 2.83 (m, 1H), 2.77 - 2.69 (m, 1H), 2.57 (t, J = 5.1 Hz, 2H), 2.46 (t, J = 5.0 Hz, 2H), 2.31 - 2.25 (m, 1H); LC-MS: m/z [M+H]⁺=640.

### Example 5 3-(6-(4-((4-(2-(1H-imidazol-1-yl)pyrimidin-4-carbonyl)piperazin-1-yl)methyl)benzyl)-2-oxobenzo[cd]in dol-1(2H)-yl)piperidin-2,6-dione

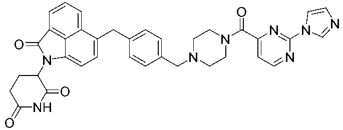

3-(2-oxo-6-(4-(piperazin-1-ylmethyl)benzyl)benzo[cd]indol-1(2H)-yl)piperidin-2,6-dione (500 mg, 1.0 mmol, intermediate 3), 2-(1H-imidazol-1-yl)pyrimidin-4-carboxylic acid (200 mg, 1.1 mmol, intermediate 7), 1-ethyl-(3-dimethylaminopropyl)carbodiimide hydrochloride (290 mg, 1.5 mmol, CAS: 25952-53-8), and 1-hydroxybenzotriazole (202 mg, 1.5 mmol, CAS: 2592-95-2) were added into a 25 mL two-necked flask, then dichloromethane (10 mL) was added in a nitrogen atmosphere, and N-methylmorpholine (0.22 mL, 2 mmol, d = 0.92 g/mL) was added through a syringe. The mixture was stirred at room temperature overnight. The reaction solution was diluted with brine (30 mL), and extracted with dichloromethane (10 mL) for 3 times. Organic phases were combined, dried over anhydrous sodium sulfate, concentrated under reduced pressure, and purified by silica gel column chromatography (dichloromethane/methanol = 20:1) to obtain the title compound as a yellow solid (53 mg, yield: 8%). ¹H NMR (500 MHz, CDCl₃) δ8.80 (d, *J =* 5.0 Hz, 1H), 8.57 (s, 1H), 8.38 (s, 1H), 8.12 (d, *J =* 8.5 Hz, 1H), 8.08 (d, *J =* 7.0 Hz, 1H), 7.84 (s, 1H), 7.69 (dd, *J =* 8.3, 7.0 Hz, 1H), 7.44 (d, *J =* 5.0 Hz, 1H), 7.21 (d, *J =* 7.8 Hz, 3H), 7.16 (m, 3H), 6.69 (d, *J =* 7.3 Hz, 1H), 5.32 (dd, *J =* 13.0, 5.4 Hz, 1H), 4.36 (s, 2H), 3.79 (m, 2H), 3.55 (m, 2H), 3.51 (s, 2H),2.95 (ddd, *J =* 17.4, 4.8, 2.6 Hz, 1H), 2.86 (ddd, *J =* 17.7, 13.5, 5.3 Hz, 1H), 2.72 (dd, *J =* 13.1, 4.7 Hz, 1H), 2.55 (m, 2H), 2.45 (m, 2H), 2.27 (m, 1H). LC-MS: m/z [M+H]⁺ =641.

### Example 6 N-(1-(4-((1-(2,6-dioxopiperidin-3-yl)-2-oxo-1,2-dihydrobenzo[cd]indol-6-yl)methyl)benzyl)piperidin-4-yl)-6-(1H-imidazol-1-yl)pyridin-2-carboxamide

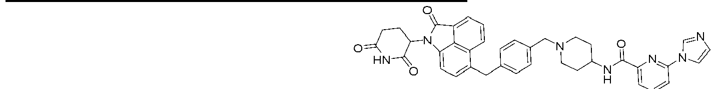

3-(6-(4-((4-aminopiperidin-1-yl)methyl)benzyl)-2-oxobenzo[cd]indol-1(2H)-yl)piperidin-2,6-dione (145 mg, 0.3 mmol, intermediate 11), 6-(1H-imidazol-1-yl)-2-picolinic acid (62 mg, 0.33 mmol, intermediate 6), benzotriazol-N,N,N',N'-tetramethyluronium hexafluorophosphate (61 mg, 0.45 mmol, CAS: 94790-37-1), and N,N-diisopropylethylamine (233 mg, 1.8 mmol) were added into dichloromethane (10 mL), and the mixture was stirred at room temperature for 2 hours. The reaction solution was diluted with brine (30 mL), and extracted with dichloromethane (10 mL) for 3 times. Organic phases were combined, dried over anhydrous sodium sulfate, concentrated under reduced pressure, and purified by silica gel column chromatography (dichloromethane/methanol = 20:1) to obtain the title compound as a yellow solid (41 mg, yield: 21%). ¹H NMR (500 MHz, Acetone-d6) δ9.98 (s, 1H), 8.57 (s, 1H), 8.40 (d, J = 10.0 Hz, 1H), 8.28 (d, J = 10 Hz, 1H), 8.14 (t, J = 10.0 Hz, 1H), 8.04 - 8.00 (m, 2H), 7.95 (s, 1H), 7.89 (d, J = 10.0 Hz, 1H), 7.76 (t, J = 7.5 Hz, 1H), 7.39 (d, J = 5.0 Hz, 1H), 7.34 - 7.30 (m, 4H), 7.10 - 7.07 (m, 2H), 5.59 (s, 2H), 5.44 (dd, J = 15.0, 5.0 Hz, 1H), 4.42 (s, 2H), 4.07- 4.05 (m, 1H) 3.95 (br, 2H), 3.04- 2.72 (m, 6H), 2.29- 2.24 (m, 1H), 2.06- 2.05 (m, 1H), 1.97- 1.89 (m, 2H); LC-MS: m/z [M+H]⁺ =654.

### Example 7 N-(1-(4-((1-(2,6-dioxopiperidin-3-yl)-2-oxo-1,2-dihydrobenzo[cd]indol-6-yl)methyl)benzyl)piperidin-4-yl)-2-(1H-imidazol-1-yl)pyrimidin-4-carboxamide

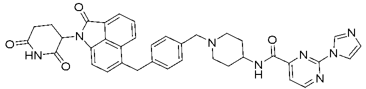

3-(6-(4-((4-aminopiperidin-1-yl)methyl)benzyl)-2-oxobenzo[cd]indol-1(2H)-yl)piperidin-2,6-dione (483 mg, 1.0 mmol, intermediate 11), 2-(1H-imidazol-1-yl)pyrimidin-4-carboxylic acid (200 mg, 1.1 mmol, intermediate 7), benzotriazol-N,N,N',N'-tetramethyluronium hexafluorophosphate (202 mg, 1.5 mmol, CAS: 94790-37-1), and N,N-diisopropylethylamine (774 mg, 6.0 mmol) were added into dichloromethane (10 mL), and the mixture was stirred at room temperature for 2 hours. The reaction solution was diluted with brine (30 mL), and extracted with dichloromethane (10 mL) for 3 times. Organic phases were combined, dried over anhydrous sodium sulfate, concentrated under reduced pressure, and purified by silica gel column chromatography (dichloromethane/methanol = 20:1) to obtain the title compound as a yellow solid (42 mg, yield: 6%). ¹H NMR (500 MHz, CDCl₃) δ8.87 (d, J = 5.0 Hz, 1H), 8.61 (s, 1H), 8.09 (d, J = 8.3 Hz, 1H), 8.04 (d, J = 7.0 Hz, 1H), 7.95 (d, J = 4.9 Hz, 1H), 7.86 (s, 1H), 7.72 (d, J = 8.4 Hz, 1H), 7.66 (t, J = 7.7 Hz, 1H), 7.22 (d, J = 7.8 Hz, 2H), 7.18 (d, J = 8.5 Hz, 2H), 7.14 (d, J = 7.9 Hz, 2H), 6.69 (d, J = 7.3 Hz, 1H), 5.31 (dd, J = 12.9, 5.4 Hz, 1H), 4.34 (s, 2H), 4.07 - 3.93 (m, 1H), 3.64 (p, J = 6.7 Hz, 1H), 3.51 (s, 2H), 3.06 (q, J = 7.4 Hz, 1H), 2.96 - 2.80 (m, 4H), 2.71 (qd, J = 13.0, 4.9 Hz, 1H), 2.22 (d, J = 12.5 Hz, 2H), 1.98 (d, J = 12.2 Hz, 2H), 1.71 (s, 2H); LC-MS: m/z [M+H]⁺ =655.

### Example 8 3-(6-(4-((4-((1-(6-(1H-imidazol-1-yl)pyridin-2-yl)piperidin-4-yl)methyl)piperazin-1-yl)methyl)benzyl)-2 -oxobenzo[cd]indol-1(2H)-yl)piperidin-2,6-dione

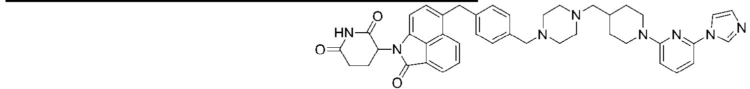

2-fluoro-6-(1H-imidazol-1-yl)pyridine (410 mg, 2.5 mmol, intermediate 4) and 3-(2-oxo-6-(4-((4-(piperidin-4-ylmethyl)piperazin-1-yl)methyl)benzyl)benzo[cd]indol-1 (2H)-yl)piperidin-2, 6-dione (900 mg, 1.3 mmol, intermediate 12) were dissolved in dimethyl sulfoxide (10 mL), then potassium carbonate (350 mg, 2.5 mmol) was added, and the mixture was stirred at room temperature for 6 hours. Brine (50 mL) was added into the reaction solution, and the reaction solution was extracted with ethyl acetate (20 mL) for 3 times. Organic phases were combined, dried over anhydrous sodium sulfate, concentrated under reduced pressure, and purified by silica gel column chromatography (dichloromethane/methanol = 20:1) to obtain the title compound as a yellow solid (48 mg, yield: 5%). ¹H NMR (500 MHz, CDCl₃)δ 8.95 (s, 1H), 8.29 (s, 1H), 8.12 (d, J = 8.3 Hz, 1H), 8.08 (d, J = 7.0 Hz, 1H), 7.69 (m, 1H), 7.54 (s, 1H), 7.50 (t, J = 8.0 Hz, 1H), 7.21 (dd, J = 7.5, 4.4 Hz, 3H), 7.14 (d, J = 8.0 Hz, 3H), 6.69 (d, J = 7.3 Hz, 1H), 6.52 (dd, J = 14.7, 8.0 Hz, 2H), 5.32 (dd, J = 12.9, 5.4 Hz, 1H), 4.35 (s, 2H), 4.30 (d, J = 16.2 Hz, 2H), 3.48 (s, 2H), 2.94 (m, 1H), 2.85 (m, 3H), 2.72 (q, J = 14.7, 13.9 Hz, 1H), 2.45 (s, 7H), 2.26 (m, 1H), 2.18(d, J = 7.0 Hz, 2H)1.82 (d, J = 15.1 Hz, 2H), 1.78-1.68 (m, 1H), 1.23-1.12 (m, 2H); LC-MS: m/z [M+H]⁺ =709.

### Example 9 3-(6-(4-((4-((2-(1H-imidazol-1-yl)pyrimidin-4-yl)amino)piperidin-1-yl)methyl)benzyl)-2-oxobenzo[cd]i ndol-1(2H)-yl)piperidin-2,6-dione

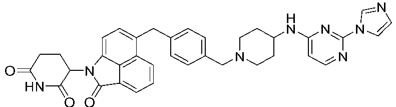

### 3-(6-(4-((4-(2-chloropyrimidin-4-yl)amino)piperidin-1-yl)methyl)benzyl)-2-oxobenzo[cd]indol-1(2 H)-yl)piperidin-2,6-dione

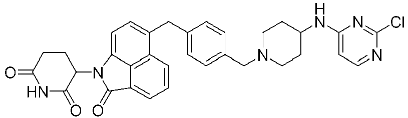

3-(6-(4-((4-aminopiperidin-1-yl)methyl)benzyl)-2-oxobenzo[cd]indol-1(2H)-yl)piperidin-2,6-dione (483 mg, 1.0 mmol, intermediate 11) and 2,4-dichloropyrimidine (298 mg, 2 mmol, CAS: 3934-20-1) were added into a reaction flask, then triethylamine (0.84 mL, 6 mmol, d = 0.728 g/mL) and anhydrous N,N-dimethylacetamide (10 mL) were added, and the mixture was stirred at room temperature for 3 hours. Brine (30 mL) was added into the reaction solution, and the reaction solution was extracted with ethyl acetate (15 mL) for 3 times. Organic phases were combined, dried over anhydrous sodium sulfate, concentrated under reduced pressure, and purified by silica gel column chromatography (dichloromethane/methanol = 20:1) to obtain the title compound as a yellow solid (417 mg, yield: 70%). LC-MS: m/z [M+H]⁺ = 595.

### 3-(6-(4-((4-((2-(1H-imidazol-1-yl)pyrimidin-4-yl)amino)piperidin-1-yl)methyl)benzyl)-2-oxobenzo[ cd]indol-1(2H)-yl)piperidin-2,6-dione

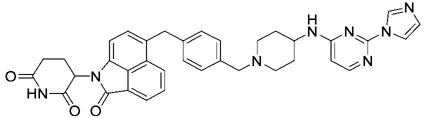

3-(6-(4-((4-(2-chloropyrimidin-4-yl)amino)piperidin-1-yl)methyl)benzyl)-2-oxobenzo[cd]indol-1(2H)-y 1)piperidin-2,6-dione (417 mg, 0.7 mmol), imidazole (110 mg, 1.4 mmol), cuprous iodide (27 mg, 0.14 mmol), and cesium carbonate (456 mg, 1.4 mmol) were added into a reaction flask, then anhydrous N,N-dimethylacetamide (5 mL) was added in a nitrogen atmosphere, and the mixture was stirred at 80°C overnight. Brine (30 mL) was added into the reaction solution, and the reaction solution was extracted with ethyl acetate (15 mL) for 3 times. Organic phases were combined, dried over anhydrous sodium sulfate, concentrated under reduced pressure, and separated by silica gel column chromatography (dichloromethane/methanol = 20:1) to obtain the title compound as a yellow solid (41 mg, yield: 9%). ¹H NMR (500 MHz, CDCl₃)δ 8.49 (s, 1H), 8.13 (d, J = 8.3 Hz, 1H), 8.08 (d, J = 7.1 Hz, 1H), 7.77 (s, 1H), 7.69 (dd, J = 8.3, 7.0 Hz, 1H), 7.21 (dd, J = 7.7, 6.2 Hz, 3H), 7.16 (d, J = 7.9 Hz, 2H), 7.09 (s, 1H), 6.69 (d, J = 7.2 Hz, 1H), 6.15 (d, J = 6.0 Hz, 1H), 5.32 (dd, J = 12.9, 5.4 Hz, 1H), 4.36 (d, J = 5.4 Hz, 2H), 3.50 (s, 2H), 2.98 - 2.87 (m, 1H), 2.90 - 2.80 (m, 3H), 2.72 (qd, J = 13.1, 4.7 Hz, 1H), 2.27 (dtd, J = 13.1, 5.2, 2.6 Hz, 1H), 2.16 (t, J = 11.3 Hz, 2H), 2.01 (d, J = 12.7 Hz, 2H), 1.81 (s, 2H), 1.56 (s, 4H); LC-MS: m/z [M+H]⁺ =627.

### Example 10 3-(6-(4-((4-(2-(1H-imidazol-1-yl)-6-methylpyrimidin-4-yl)piperazin-1-yl)methyl)benzyl)-2-oxobenzo[cd ]indol-1(2H)-yl)piperidin-2,6-dione

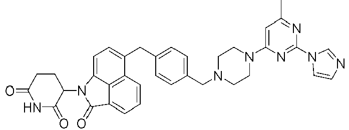

3-(2-oxo-6-(4-(piperazin-1-ylmethyl)benzyl)benzo[cd]indol-1(2H)-yl)piperidin-2,6-dione (234 mg, 0.5 mmol, intermediate 3), 2-(1H-imidazol-1-yl)-4-methyl-6-(methylsulfonyl)pyrimidine (179 mg, 0.75 mmol, intermediate 13), and N,N-diisopropylethylamine (387 mg, 3 mmol) were added into a 50 mL single-necked flask, then isopropanol (5 mL) was added, and the mixture was stirred at 100°C overnight. The reaction solution was directly concentrated under reduced pressure, and purified by silica gel column chromatography (dichloromethane/methanol = 20:1) to obtain the title compound as a yellow solid (41 mg, yield: 13%). ¹H NMR (500 MHz, CDCl₃) δ 8.50 (s, 1H), 8.43 (s, 1H), 8.10 (dd, J = 16.1, 7.6 Hz, 2H), 7.79 (s, 1H), 7.70 (dd, J = 8.3, 7.0 Hz, 1H), 7.23 (d, J = 8.1 Hz, 3H), 7.17 (d, J = 7.9 Hz, 2H), 7.07 (s, 1H), 6.70 (d, J = 7.3 Hz, 1H), 6.18 (s, 1H), 5.33 (dd, J = 13.0, 5.4 Hz, 1H), 4.37 (s, 2H), 3.65 (s, 4H), 3.51 (s, 2H), 3.00 - 2.92 (m, 1H), 2.92 - 2.81 (m, 1H), 2.73 (qd, J = 13.2, 4.8 Hz, 1H), 2.49 (t, J = 5.1 Hz, 4H), 2.34 (s, 3H), 2.28 (dt, J = 15.9, 5.2 Hz, 1H); LC-MS: m/z [M+H]⁺ = 627.

### Example 11 3-(6-(4-((4-(2-(1H-imidazol-1-yl)-6-methylpyrimidin-4-carbonyl)piperazin-1-yl)methyl)benzyl)-2-oxobe nzo[cd]indol-1(2H)-yl)piperidin-2,6-dione

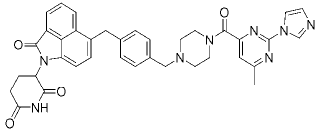

3-(2-oxo-6-(4-(piperazin-1-ylmethyl)benzyl)benzo[cd]indol-1(2H)-yl)piperidin-2,6-dione (0.46 g, 0.98 mmol, intermediate 3), 2-(1H-imidazol-1-yl)-6-methylpyrimidin-4-carboxylic acid (0.2 g, 0.98 mmol, intermediate 14), 1-ethyl-(3-dimethylaminopropyl)carbodiimide hydrochloride (0.28 g, 1.47 mmol, CAS: 25952-53-8), and 1-hydroxybenzotriazole (0.20 g, 1.47 mmol, CAS: 2592-95-2) were added into a 25 mL two-necked flask, then dichloromethane (10 mL) was added in a nitrogen atmosphere, N-methylmorpholine (0.2 mL, 1.96 mmol, d = 0.92 g/mL) was added through a syringe, and the mixture was stirred at room temperature overnight. The reaction solution was diluted with brine (30 mL), and extracted with dichloromethane (10 mL) for 3 times. Organic phases were combined, dried over anhydrous sodium sulfate, concentrated under reduced pressure, and purified by silica gel column chromatography (dichloromethane/methanol = 15:1) to obtain the title compound as a yellow solid (45 mg, yield: 7%). ¹H NMR (500 MHz, CDCl₃) δ8.73 (s, 1H), 8.58 (s, 1H), 8.12 (d, J = 8.3 Hz, 1H), 8.08 (d, J = 7.0 Hz, 1H), 7.84 (s, 1H), 7.69 (dd, J = 8.2, 7.1 Hz, 1H), 7.29 (s, 1H), 7.22-7.21 (m, 3H), 7.17-7.15 (m, 3H), 6.70 (d, J = 7.2 Hz, 1H), 5.33 (dd, J = 13.0, 5.4 Hz, 1H), 4.36 (s, 2H), 3.80 - 3.78 (m, 2H), 3.55 (s, 2H), 3.51 (s, 2H), 2.97-2.92 (m, 1H), 2.90-2.83 (m, 1H), 2.77-2.68 (m, 1H), 2.59 (s, 3H), 2.55 (t, J = 5.2 Hz, 2H), 2.45 (t, J = 4.8 Hz, 2H), 2.30-2.24 (m, 1H). LC-MS: m/z [M+H]⁺ =655.

### Example 12 N-(1-(4-((1-(2,6-dioxopiperidin-3-yl)-2-oxo-1,2-dihydrobenzo[cd]indol-6-yl)methyl)benzyl)piperidi n-4-yl)-2-(1H-imidazol-1-yl)-6-methylpyrimidin-4-carboxamide

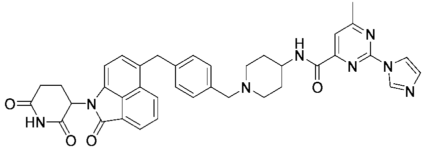

3-(6-(4-((4-aminopiperidin-1-yl)methyl)benzyl)-2-oxobenzo[cd]indol-1(2H)-yl)piperidin-2,6-dione (473 mg, 0.9 mmol, intermediate 11), 2-(1H-imidazol-1-yl)-6-methylpyrimidin-4-carboxylic acid (205 mg, 1.0 mmol, intermediate 14), benzotriazol-N,N,N',N'-tetramethyluronium hexafluorophosphate (204 mg, 1.5 mmol, CAS: 94790-37-1), and N,N-diisopropylethylamine (774 mg, 6.0 mmol) were added into dichloromethane (10 mL), and the mixture was stirred at room temperature for 2 hours. The reaction solution was diluted with brine (30 mL), and extracted with dichloromethane (10 mL) for 3 times. Organic phases were combined, dried over anhydrous sodium sulfate, concentrated under reduced pressure, and purified by silica gel column chromatography (dichloromethane/methanol = 20:1) to obtain the title compound as a yellow solid (50 mg, yield: 8%). ¹H NMR (500 MHz, CDCl₃) δ8.60 (s, 1H), 8.33 (s, 1H), 8.10 (dd, J = 19.4, 7.6 Hz, 2H), 7.87 (s, 1H), 7.83 (s, 1H), 7.72-7.63 (m, 2H), 7.25-7.20 (m, 3H), 7.17 (t, J = 6.4 Hz, 3H), 6.69 (d, J = 7.3 Hz, 1H), 5.36-5.29 (m, 1H), 4.37 (s, 2H), 4.00 (d, J = 8.4 Hz, 1H), 3.51 (s, 2H), 3.00-2.92 (m, 1H), 2.91-2.80 (m, 2H), 2.79-2.69 (m, 1H), 2.64 (s, 3H), 2.35-2.14 (m, 3H), 1.99 (d, J = 14.1 Hz, 2H), 1.68 (d, J = 10.9 Hz, 3H); LC-MS: m/z [M+H]⁺ =669.

### Example 13 3-(2-oxo-6-(4-((4-(2-(2-(trifluoromethyl)-1H-imidazol-1-yl)pyrimidin-4-yl)piperazin-1-yl)methyl)benzyl )benzo[cd]indol-1(2H)-yl)piperidin-26-dione

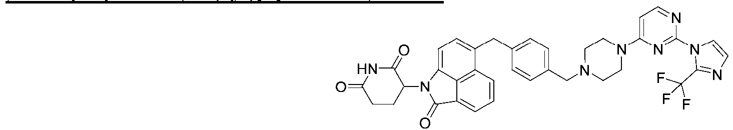

3-(2-oxo-6-(4-(piperazin-1-ylmethyl)benzyl)benzo[cd]indol-1(2H)-yl)piperidin-2,6-dione (234 mg, 0.5 mmol, intermediate 3), 4-(methylsulfonyl)-2-(2-(trifluoromethyl)-1H-imidazol-1-yl)pyrimidine (219 mg, 0.75 mmol, intermediate 15), and N,N-diisopropylethylamine (387 mg, 3 mmol) were added into a 50 mL single-necked flask, then isopropanol (5 mL) was added, and the mixture was stirred at 100°C overnight. The reaction solution was directly concentrated under reduced pressure, and purified by silica gel column chromatography (dichloromethane/methanol = 20:1) to obtain the title compound as a yellow solid (45 mg, yield: 13%). ¹H NMR (500 MHz, CDCl₃) δ8.21 (s, 1H), 8.14 (dd, *J* = 13.9, 7.2 Hz, 2H), 8.09 (d, *J* = 7.0 Hz, 1H), 7.90 (s, 1H), 7.70 (t, *J* = 7.7 Hz, 1H), 7.23 (d, *J* = 10.1 Hz, 3H), 7.17 (d, *J* = 7.8 Hz, 2H), 7.13 (s, 1H), 6.70 (d, *J* = 7.2 Hz, 1H), 6.40 (d, *J* = 6.2 Hz, 1H), 5.33 (dd, *J* = 12.9, 5.3 Hz, 1H), 4.37 (s, 2H), 3.68 (s, 4H), 3.50 (s, 2H), 2.96 (dt, *J* = 17.0, 3.9 Hz, 1H), 2.90 - 2.83 (m, 1H), 2.73 (qd, *J* = 13.2, 4.7 Hz, 1H), 2.49 (t, *J* = 5.1 Hz, 4H), 2.28 (dt, *J* = 16.1, 5.3 Hz, 1H); LC-MS: m/z [M+H]⁺ =681.

### Example 14 (S)-3-(4-((4-((4-(2-(1H-imidazol-1-yl)pyrimidin-4-carbonyl)piperazin-1-yl)methyl)benzyl)oxy)-1-oxoiso indolin-2-yl)piperidin-2,6-dione

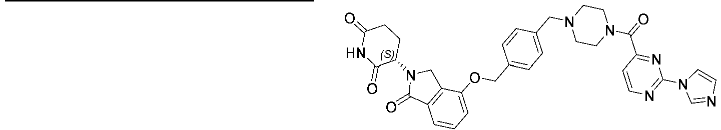

(S)-3-(4-((4-(chloromethyl)benzyl)oxy)-1-oxoisoindolin-2-yl)piperidin-2,6-dione (91.7 mg, 0.23 mmol, intermediate 16) and (2-(1H-imidazol-1-yl)pyrimidin-4-yl)(piperazin-1-yl)methanone hydrochloride (70.0 mg, 0.27 mmol, intermediate 17) were added into a reaction flask, acetonitrile (1 mL) was added into the reaction flask through a syringe, triethylamine (71.0 mg, 0.7 mmol) was added in during stirring, and the reaction solution was stirred at room temperature for 60 hours. The reaction solution was concentrated under reduced pressure, and purified by silica gel column chromatography (dichloromethane/methanol = 20:1) to obtain the title compound as a white solid (34.3 mg, yield: 24%). ¹H NMR (400 MHz, CDCl₃) δ 8.82 (d, J = 4.9 Hz, 1H), 8.58 (s, 2H), 7.86 (s, 1H), 7.48 (t, J = 6.9 Hz, 2H), 7.44 - 7.36 (m, 5H), 7.18 (s, 1H), 7.09 (d, J = 8.0 Hz, 1H), 5.22 (dd, J = 13.3, 5.0 Hz, 1H), 5.14 (s, 2H), 4.45 (d, J = 16.5 Hz, 1H), 4.31 (d, J = 16.6 Hz, 1H), 3.85 (s, 2H), 3.62 (s, 4H), 2.91 - 2.77 (m, 2H), 2.63 (s, 2H), 2.54 (s, 2H), 2.39 - 2.28 (m, 1H), 2.23 - 2.18 (m, 1H); LC-MS: m/z [M+H]⁺ =621.

### Example 15 N-((1R,4R)-4-(4-(4-(((2-((S)-2,6-dioxopiperidin-3-yl)-1-oxoisoindolin-4-yl)oxy)methyl)benzyl)piperazin -1-yl)cyclohexyl)-2-(1H-imidazol-1-yl)pyrimidin-4-carboxamide

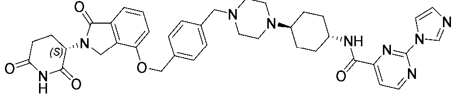

(S)-3-(4-((4-(chloromethyl)benzyl)oxy)-1-oxoisoindolin-2-yl)piperidin-2,6-dione (40 mg, 0.1 mmol, intermediate 16) and 2-(1H-imidazol-1-yl)-N-((1R,4R)-4-(piperazin-1-yl)cyclohexyl)pyrimidin-4-carboxamide hydrochloride (43 mg, 0.12 mmol, intermediate 19) were added into a reaction flask, acetonitrile (1 mL) was added into the reaction flask through a syringe, triethylamine (30 mg, 0.3 mmol) was added in during stirring, and the reaction solution was stirred at room temperature for 8 hours. The reaction solution was concentrated under reduced pressure, and purified by silica gel column chromatography (dichloromethane/methanol = 20:1) to obtain the title compound as a white solid (30.4 mg, yield: 42%). ¹H NMR (400 MHz, CDCl₃) δ 8.90 (d, J = 4.9 Hz, 1H), 8.72 (s, 1H), 8.67 (s, 1H), 7.99 (d, J = 4.9 Hz, 1H), 7.90 (s, 1H), 7.70 (s, 1H), 7.48 (d, J = 7.6 Hz, 1H), 7.41 (t, J = 7.7 Hz, 1H), 7.37 - 7.33 (m, 4H), 7.19 (s, 1H), 7.09 (d, J = 8.0 Hz, 1H), 5.21 (dd, J = 13.2, 5.0 Hz, 1H), 5.12 (s, 2H), 4.43 (d, J = 16.5 Hz, 1H), 4.32 (d, J = 16.7 Hz, 1H), 3.97 - 3.90 (m, 1H), 3.60 (s, 2H), 2.92 - 2.71 (m, 10H), 2.40 - 2.29 (m, 1H), 2.20 - 2.13 (m, 5H), 1.58 - 1.38 (m, 5H); LC-MS: m/z [M+H]⁺ =718.

### Example 16 N-((1R,4R)-4-(4-(4-(((2-((S)-2,6-dioxopiperidin-3-yl)-1-oxoisoindolin-4-yl)oxy)methyl)benzyl)piperazin -1-yl)cyclohexyl)-6-(1H-imidazol-1-yl)pyridin-2-carboxamide

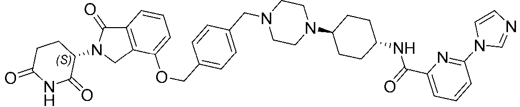

(S)-3-(4-((4-(chloromethyl)benzyl)oxy)-1-oxoisoindolin-2-yl)piperidin-2,6-dione (40 mg, 0.1 mmol, intermediate 16) and 6-(1H-imidazol-1-yl)-N-((1R,4R)-4-(piperazin-1-yl)cyclohexyl)pyridin-2-carboxamide hydrochloride (43 mg, 0.12 mmol, intermediate 20) were added into a reaction flask, acetonitrile (1 mL) was added into the reaction flask through a syringe, triethylamine (30 mg, 0.3 mmol) was added in during stirring, and the reaction solution was stirred at room temperature for 11 hours. The reaction solution was concentrated under reduced pressure, and purified by silica gel column chromatography (dichloromethane/methanol = 10:1) to obtain the title compound as a white solid (30.5 mg, yield: 42.5%). ¹H NMR (400 MHz, CDCl₃) δ 8.76 (s, 1H), 8.34 (s, 1H), 8.13 (d, J = 7.6 Hz, 1H), 7.99 (t, J = 7.9 Hz, 1H), 7.63 - 7.60 (m, 2H), 7.50 - 7.46 (m, 2H), 7.40 (t, J = 7.9 Hz, 1H), 7.36 - 7.31 (m, 4H), 7.22 (s, 1H), 7.08 (d, J = 8.0 Hz, 1H), 5.20 (dd, J = 13.3, 5.1 Hz, 1H), 5.11 (s, 2H), 4.42 (d, J = 16.6 Hz, 1H), 4.30 (d, J = 16.6 Hz, 1H), 3.96 - 3.86 (m, 1H), 3.58 (s, 2H), 2.92 - 2.68 (m, 10H), 2.39 - 2.28 (m, 2H), 2.20 - 2.10 (m, 5H), 1.56 - 1.34 (m, 4H); LC-MS: m/z [M+H]⁺ =717.

### Example 17 (S)-3-(4-((4-((4-((1-(6-(1H-imidazol-1-yl)pyridin-2-yl)piperidin-4-yl)methyl)piperazin-1-yl)methyl)benz yl)oxy)-1-oxoisoindolin-2-yl)pyridin-2,6-dione

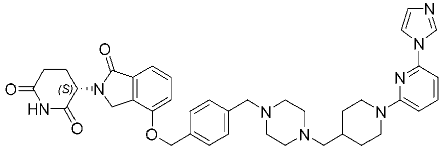

(S)-3-(4-((4-(chloromethyl)benzyl)oxy)-1-oxoisoindolin-2-yl)piperidin-2,6-dione (55 mg, 0.14 mmol, intermediate 16) and 1-((1-(6-(1H-imidazol-1-yl)pyridin-2-yl)piperidin-4-yl)methyl)piperazine hydrochloride (60 mg, 0.17 mmol, intermediate 21) were added into a reaction flask, acetonitrile (1 mL) was added into the reaction flask through a syringe, triethylamine (43 mg, 0.42 mmol) was added in during stirring, and the reaction solution was stirred at room temperature for 16 hours. The reaction solution was concentrated under reduced pressure, and purified by silica gel column chromatography (dichloromethane/methanol = 20:1) to obtain the title compound as a white solid (61 mg, yield: 63%). ¹H NMR (400 MHz, CDCl₃) δ 8.70 (d, J = 22.0 Hz, 1H), 8.32 (s, 1H), 7.58 (s, 1H), 7.51 - 7.41 (m, 3H), 7.36 - 7.32 (m, 4H), 7.14 (s, 1H), 7.11 (d, J = 8.0 Hz, 1H), 6.52 (d, J = 7.4 Hz, 1H), 6.22 (d, J = 8.3 Hz, 1H), 5.22 (dd, J = 13.4, 5.0 Hz, 1H), 5.12 (s, 2H), 4.42 (d, J = 16.6 Hz, 1H), 4.31 (d, J = 16.7 Hz, 1H), 3.74 - 3.69 (m, 1H), 3.64 - 3.56 (m, 3H), 3.43 - 3.36 (m, 1H), 3.08 - 3.03 (m, 1H), 2.92 - 2.77 (m, 2H), 2.51 - 2.17 (m, 14H), 1.72 - 1.62 (m, 3H); LC-MS: m/z [M+H]⁺ =689.

### Example 18 (S)-N-(1'-(4-(((2-(2,6-dioxopiperidin-3-yl)-1-oxoisoindolin-4-yl)oxy)methyl)benzyl)-[1,4'-bipiperidin]-4-yl)-2-(1H-imidazol-1-yl)pyrimidin-4-carboxamide

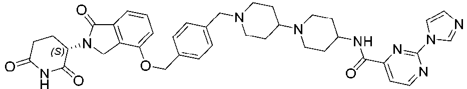

(S)-3-(4-((4-(chloromethyl)benzyl)oxy)-1-oxoisoindolin-2-yl)piperidin-2,6-dione (56 mg, 0.14 mmol, intermediate 16) and N-([1,4'-bipiperidin]-4-yl)-2-(1H-imidazol-1-yl)pyrimidin-4-carboxamide hydrochloride (60 mg, 0.17 mmol, intermediate 22) were added into a reaction flask, acetonitrile (1 mL) was added into the reaction flask through a syringe, triethylamine (43 mg, 0.42 mmol) was added in during stirring, and the reaction solution was stirred at 40°C for 10 hours. The reaction solution was concentrated under reduced pressure, and purified by silica gel column chromatography (dichloromethane/methanol = 10:1) to obtain the title compound as a white solid (13.2 mg, yield: 13%). ¹H NMR (400 MHz, CDCl₃) δ 8.90 (d, J = 4.9 Hz, 1H), 8.73 (brs, 1H), 8.64 (s, 1H), 7.98 (d, J = 4.9 Hz, 1H), 7.88 (s, 1H), 7.77 (d, J = 9.1 Hz, 1H), 7.48 (d, J = 7.5 Hz, 1H), 7.43 - 7.33 (m, 5H), 7.20 (s, 1H), 7.09 (d, J = 8.0 Hz, 1H), 5.21 (dd, J = 13.2, 5.2 Hz, 1H), 5.12 (s, 2H), 4.45 (d, J = 16.6 Hz, 1H), 4.31 (d, J = 16.5 Hz, 1H), 4.08 - 4.01 (m, 1H), 3.53 (s, 2H), 3.03 - 1.97 (m, 4H), 2.93 - 2.78 (m, 2H), 2.52 - 2.48 (m, 3H), 2.41 - 2.29 (m, 1H), 2.23 - 2.18 (m, 1H), 2.09 - 2.00 (m, 4H), 1.87 - 1.80 (m, 4H), 1.73 - 1.64 (m, 2H); LC-MS: m/z [M+H]⁺ =718.

### Example 19 (S)-3-(1-oxo-4-((4-((4-(6-(thiazol-5-yl)pyridin-2-yl)piperazin-1-yl)methyl)benzyl)oxy)isoindolin-2-yl)pip eridin-2,6-dione

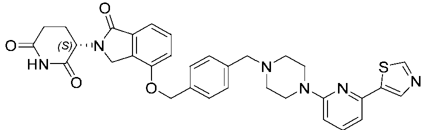

(S)-3-(4-((4-(chloromethyl)benzyl)oxy)-1-oxoisoindolin-2-yl)piperidin-2,6-dione (47 mg, 0.12 mmol, intermediate 16) and 5-(6-(piperazin-1-yl)pyridin-2-yl)thiazole hydrochloride (40 mg, 0.14 mmol, intermediate 24) were added into a reaction flask, acetonitrile (1 mL) was added into the reaction flask through a syringe, triethylamine (36 mg, 0.36 mmol) was added in during stirring, and the reaction solution was stirred at room temperature for 28 hours. The reaction solution was concentrated under reduced pressure, and purified by silica gel column chromatography (dichloromethane/methanol = 40:1) to obtain the title compound as a white solid (31.5 mg, yield: 43%). ¹H NMR (400 MHz, CDCl₃) δ 8.74 (s, 1H), 8.23 (s, 1H), 7.51 - 7.37 (m, 7H), 7.09 (d, J = 8.1 Hz, 1H), 7.00 (d, J = 8.2 Hz, 1H), 6.54 (d, J = 8.5 Hz, 1H), 5.17 (dd, J = 12.8, 5.7 Hz, 1H), 5.12 (s, 2H), 4.44 (d, J = 16.9 Hz, 1H), 4.31 (d, J = 16.5 Hz, 1H), 3.58 (s, 5H), 2.88 - 2.75 (m, 2H), 2.57 (s, 3H), 2.37 - 2.26 (m, 1H), 2.19 (s, 3H); LC-MS: m/z [M+H]⁺ =609.

### Example 20 3-(6-(4-((4-(2-(1-methyl-1H-imidazol-5-yl)pyrimidin-4-yl)piperazin-1-yl)methyl)benzyl)-2-oxobenzo[cd ]indol-1(2H)-yl)piperidin-2,6-dione

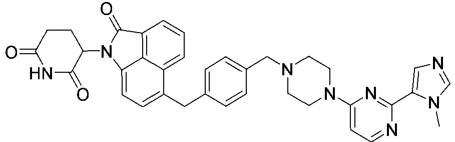

In a nitrogen atmosphere, 1,3,5,7-tetramethyl-6-phenyl-2,4,8-trioxa-6-phosphaadamantane (30 mg, 0.10 mmol, CAS: 97739-46-3) and tris(dibenzylideneacetone)dipalladium (30 mg, 0.03 mmol, CAS: 51364-51-3) were added into a mixed solution of 3-(6-(4-((4-(2-chloropyrimidin-4-yl)piperazin-1-yl)methyl)benzyl)-2-oxobenzo[cd]indol-1(2H)-yl)piperidin-2,6-dione (110 mg, 0.19 mmol, intermediate 25), 1-methylimidazol-5-boronic acid pinacol ester (61 mg, 0.29 mmol, CAS: 942070-72-6), cesium carbonate (185.30 mg, 0.57 mmol), and N,N-dimethylformamide (5 mL) for a reaction at 100°C for 3 hours. The reaction solution was purified by prep-HPLC (acetonitrile and water containing 0.1% of ammonium bicarbonate) to obtain the title compound as a yellow solid (21.6 mg, yield: 18%). ¹H NMR (400 MHz, DMSO-d6) δ11.13 (s, 1H), 8.34 (d, J = 8.3 Hz, 1H), 8.19 (d, J = 6.2 Hz, 1H), 8.08 (d, J = 6.9 Hz, 1H), 7.81 (t, J = 7.6 Hz, 1H), 7.71 (s, 1H), 7.60 (s, 1H), 7.42 (d, J = 7.2 Hz, 1H), 7.24 (dd, J = 16.1, 7.9 Hz, 4H), 7.11 (d, J = 7.3 Hz, 1H), 6.62 (d, J = 6.3 Hz, 1H), 5.45 (dd, J = 12.4, 4.9 Hz, 1H), 4.39 (s, 2H), 3.94 (s, 3H), 3.60 (s, 4H), 3.45 (s, 2H), 3.08 - 2.87 (m, 1H), 2.75 (m = 25.3, 12.2 Hz, 1H), 2.65 (m = 16.9 Hz, 1H), 2.40 (m, 4H), 2.17 - 2.00 (m, 1H); LC-MS: m/z [M+H]⁺ =627.

### Example 21 3-(6-(4-((4-(2-(2-methylthiazol-5-yl)-6-(trifluoromethyl)pyrimidin-4-yl)piperazin-1-yl)methyl)benzyl)-2 -oxobenzo[cd]indol-1(2H)-yl)piperidin-2,6-dione

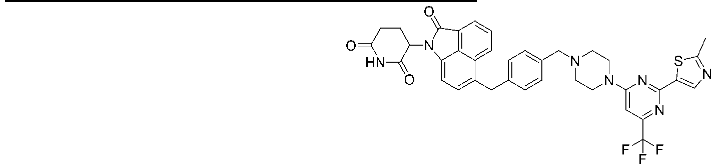

In a nitrogen atmosphere, [1,1'-bis(di-tert-butylphosphino)ferrocene]dichloropalladium (40.03 mg, 0.06 mmol, CAS: 95408-45-0) was added into a mixed solution of 3-(6-(4-((4-(2-chloro-6-(trifluoromethyl)pyrimidin-4-yl)piperazin-1-yl)methyl)benzyl)-2-oxobenzo[cd]indol-1(2H)-yl)piperidin-2,6-dione (200 mg, 0.31 mmol, intermediate 26), 2-methylthiazol-5-boronic acid pinacol ester (83.24 mg, 0.37 mmol, CAS: 1218791-01-5), cesium carbonate (424.22 mg, 1.30 mmol), and N,N-dimethylformamide (5 mL) for a reaction at 100°C for 3 hours. The reaction solution was purified by prep-HPLC (acetonitrile and water containing 0.1% of hydrochloric acid) to obtain the title compound as a yellow solid (15.6 mg, yield: 7%). ¹H NMR (400 MHz, DMSO-d6) δ11.13 (s, 1H), 10.36 (s, 1H), 8.45 - 8.31 (m, 2H), 8.14 - 8.04 (m, 1H), 7.87 - 7.77 (m, 1H), 7.50 - 7.37 (m, 5H), 7.25 (s, 1H), 7.16 - 7.08 (m, 1H), 5.54 - 5.39 (m, 1H), 4.50 - 4.39 (m, 2H), 4.35 - 4.22 (m, 2H), 3.47 - 3.44 (m, 4H), 3.17 - 3.03 (m, 2H), 2.74 - 2.65 (m, 5H), 2.33 (s, 2H); LC-MS: m/z [M+H]⁺ =712.

### Example 22 3-(6-(4-((4-(6-(5-methyl-1,3,4-thiadiazol-2-yl)pyridin-2-yl)piperazin-1-yl)methyl)benzyl)-2-oxobenzo[cd ]indol-1(2H)-yl)piperidin-2,6-dione

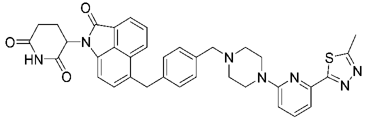

3-(2-oxo-6-(4-(piperazin-1-ylmethyl)benzyl)benzo[cd]indol-1(2H)-yl)piperidin-2,6-dione (250 mg, 0.5 mmol, hydrochloride, intermediate 3), 2-(6-chloropyridin-2-yl)-5-methyl-1,3,4-thiadiazole (209.57 mg, 0.99 mmol, intermediate 27), triethylamine (250.47 mg, 2.48 mmol), and cesium fluoride (112.79 mg, 0.74 mmol) were added into dimethyl sulfoxide (10 mL) for a microwave reaction at 140°C for 1 hour. The reaction solution was cooled to room temperature, poured into water (30 mL), and extracted with ethyl acetate (20 mL) for 3 times. Organic phases were combined, dried over anhydrous sodium sulfate, concentrated under reduced pressure, and purified by prep-HPLC (acetonitrile and water containing 0.1% of formic acid) to obtain the title compound as a yellow solid (98 mg, yield: 30%). ¹H NMR (400 MHz, DMSO-d6) δ 11.13 (s, 1H), 8.34 (d, J = 8.2 Hz, 1H), 8.08 (d, J = 7.0 Hz, 1H), 7.82 (t, J = 7.6 Hz, 1H), 7.70 (t, J = 7.9 Hz, 1H), 7.49 - 7.37 (m, 2H), 7.25 (q, J = 8.1 Hz, 4H), 7.12 (d, J = 7.3 Hz, 1H), 6.95 (d, J = 8.6 Hz, 1H), 5.45 (dd, J = 12.8, 5.0 Hz, 1H), 4.40 (s, 2H), 3.55 - 3.50 (m, 4H), 3.46 (s, 2H), 3.00 - 2.90 (m, 1H), 2.83 - 2.75 (m, 1H), 2.74 (s, 3H), 2.70 - 2.61 (m, 1H), 2.47 - 2.38 (m, 4H), 2.15 - 2.05 (m, 1H); LC-MS: m/z [M+H]⁺ = 644.

### Example 23 3-(6-(4-((4-(6-(3-methyl-1H-1,2,4-triazol-1-yl)pyridin-2-yl)piperazin-1-yl)methyl)benzyl)-2-oxobenzo[c d]indol-1(2H)-yl)piperidin-2,6-dione

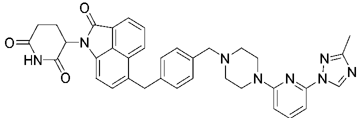

3-(2-oxo-6-(4-(piperazin-1-ylmethyl)benzyl)benzo[cd]indol-1(2H)-yl)piperidin-2,6-dione (250 mg, 0.5 mmol, hydrochloride, intermediate 3), 2-chloro-6-(3-methyl-1H-1,2,4-triazol-1-yl)pyridine (96.34 mg, 0.50 mmol, intermediate 28), triethylamine (250 mg, 2.47 mmol), and cesium fluoride (90 mg, 0.59 mmol) were added into dimethyl sulfoxide (2.5 mL) for a microwave reaction at 135°C for 1 hour. The reaction solution was cooled to room temperature, poured into water (10 mL) until a solid was precipitated, and then filtered. The filter cake was washed once with 5 mL of water. The filter cake was collected and dried to obtain a crude product. The crude product was purified by prep-HPLC (acetonitrile and water containing 0.1% of formic acid) to obtain the title compound as a yellow solid (48 mg, yield: 15%). ¹H NMR (400 MHz, DMSO-d6) δ 11.12 (s, 1H), 9.16 (s, 1H), 8.34 (d, J = 8.3 Hz, 1H), 8.08 (d, J = 7.0 Hz, 1H), 7.88 - 7.76 (m, 1H), 7.68 (t, J = 8.1 Hz, 1H), 7.41 (d, J = 7.4 Hz, 1H), 7.24 (q, J = 8.2 Hz, 4H), 7.11 (d, J = 7.3 Hz, 1H), 6.96 (d, J = 7.6 Hz, 1H), 6.76 (d, J = 8.5 Hz, 1H), 5.44 (dd, J = 12.8, 5.2 Hz, 1H), 4.39 (s, 2H), 3.54 (s, 4H), 3.45 (s, 2H), 3.03 - 2.89 (m, 1H), 2.76 (dt, J = 17.4, 10.9 Hz, 1H), 2.65 (d, J = 15.6 Hz, 1H), 2.46 - 2.38 (m, 4H), 2.34 (s, 3H), 2.12 - 2.01 (m, 1H); LC-MS: m/z [M+H]⁺ = 627.

### Example 24 3-(6-(4-((4-(6-(4-isopropyl-4H-1,2,4-triazol-3-yl)pyridin-2-yl)piperazin-1-yl)methyl)benzyl)-2-oxobenzo [cd]indol-1(2H)-yl)piperidin-2,6-dione

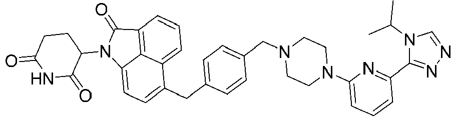

3-(2-oxo-6-(4-(piperazin-1-ylmethyl)benzyl)benzo[cd]indol-1(2H)-yl)piperidin-2,6-dione (468 mg, 1 mmol, intermediate 3) and 2-chloro-6-(4-isopropyl-4H-1,2,4-triazol-3-yl)pyridine (224 mg, 1 mmol, intermediate 29) were dissolved in dimethyl sulfoxide (5 mL), N,N-diisopropylethylamine (515 mg, 5 mmol) was added, and the reaction mixture was stirred at 130°C for 48 hours. The reaction solution was purified by prep-HPLC (acetonitrile and water containing 0.1% of formic acid) to obtain the title compound as a yellow solid (10 mg, yield: 1.4%, FA salt). ¹H NMR (400 MHz, DMSO-d6) δ 8.81 (s, 1H), 8.34 (d, J = 8.3 Hz, 1H), 8.23 (d, J = 1.7 Hz, 1H), 8.08 (d, J = 7.0 Hz, 1H), 7.81 (dd, J = 8.2, 7.0 Hz, 1H), 7.68 (dd, J = 8.6, 7.4 Hz, 1H), 7.42 (d, J = 7.3 Hz, 1H), 7.35 (d, J = 7.3 Hz, 1H), 7.24 (q, J = 8.1 Hz, 4H), 7.11 (d, J = 7.3 Hz, 1H), 6.92 (d, J = 8.6 Hz, 1H), 5.41 (ddd, J = 25.4, 13.1, 6.0 Hz, 2H), 4.39 (s, 2H), 3.47 (dd, J = 11.1, 6.0 Hz, 6H), 3.00 - 2.86 (m, 1H), 2.85 - 2.70 (m, 1H), 2.67 (d, J = 2.2 Hz, 1H), 2.44 (t, J = 5.0 Hz, 5H), 2.33 (p, J = 1.8 Hz, 1H), 1.42 (d, J = 6.7 Hz, 6H); LC-MS: m/z [M+H]⁺ = 655.

### Example 25 3-(2-oxo-6-(4-((4-(2-(4-(trifluoromethyl)-1H-imidazol-1-yl)pyrimidin-4-yl)piperazin-1-yl)methyl)benzyl )benzo[cd]indol-1(2H)-yl)piperidin-2,6-dione

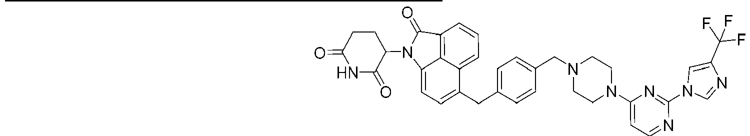

3-(2-oxo-6-(4-(piperazin-1-ylmethyl)benzyl)benzo[cd]indol-1(2H)-yl)piperidin-2,6-dione (234 mg, 0.5 mmol, intermediate 3), 4-(methylsulfonyl)-2-(4-(trifluoromethyl)-1H-imidazol-1-yl)pyrimidine (219 mg, 0.75 mmol, intermediate 30), and N,N-diisopropylethylamine (387 mg, 3 mmol) were added into a 50 mL single-necked flask, then isopropanol (5 mL) was added, and the mixture was stirred at 100°C overnight. The reaction solution was directly concentrated under reduced pressure, and purified by silica gel column chromatography (dichloromethane/methanol = 20:1) to obtain the title compound as a yellow solid (60 mg, yield: 18%). ¹H NMR (500 MHz, CDCl₃) δ 9.03 (s, 1H), 8.48 (s, 1H), 8.11 - 8.08 (m, 3H), 8.06 (d, *J* = 7.0 Hz, 1H), 7.67 (t, *J* = 7.6 Hz, 1H), 7.21 (d, *J* = 7.5 Hz, 3H), 7.16 (d, *J* = 7.8 Hz, 2H), 6.70 (d, *J* = 7.3 Hz, 1H), 6.37 (d, *J* = 6.2 Hz, 1H), 5.31 (dd, *J* = 12.9, 5.4 Hz, 1H), 4.35 (s, 2H), 3.66 (s, 4H), 3.50 (s, 2H), 2.95 - 2.80 (m, 2H), 2.70 (qd, *J* = 13.1, 4.9 Hz, 1H), 2.49 (t, *J* = 5.0 Hz, 4H), 2.28 - 2.20 (m, 1H); LC-MS: m/z [M+H]⁺ = 681.

### Example 26 3-(6-(4-((4-(2-(4-cyclopropyl-1H-imidazol-1-yl)pyrimidin-4-yl)piperazin-1-yl)methyl)benzyl)-2-oxoben zo[cd]indol-1(2H)-yl)piperidin-2,6-dione

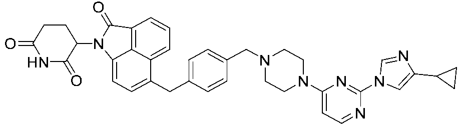

3-(2-oxo-6-(4-(piperazin-1-ylmethyl)benzyl)benzo[cd]indol-1(2H)-yl)piperidin-2,6-dione (230 mg, 0.5 mmol, intermediate 3), 2-(4-cyclopropyl-1H-imidazol-1-yl)-4-(methylsulfonyl)pyrimidine (200 mg, 0.75 mmol, intermediate 32), and N,N-diisopropylethylamine (320 mg, 2.5 mmol) were added into a 25 mL round-bottomed flask, then isopropanol (15 mL) was added, and the mixture was stirred at 100°C for 5 hours. The reaction solution was directly concentrated under reduced pressure, and purified by silica gel column chromatography (dichloromethane/methanol = 20:1) to obtain the title compound as a yellow solid (35 mg, yield: 11%). ¹H NMR (500 MHz, CDCl₃) δ 8.40 (s, 1H), 8.37 (s, 1H), 8.14 (d, J = 8.3 Hz, 1H), 8.10 (dd, J = 6.6, 3.3 Hz, 2H), 7.72 (dd, J = 8.3, 7.0 Hz, 1H), 7.47 (s, 1H), 7.25 (d, J = 6.4 Hz, 2H), 7.20 (t, J = 7.6 Hz, 3H), 6.72 (d, J = 7.3 Hz, 1H), 6.31 (d, J = 6.2 Hz, 1H), 5.37 - 5.33 (m, 1H), 4.39 (s, 2H), 3.69 (s, 4H), 3.53 (s, 2H), 3.0 - 2.96 (m, J = 19.2, 3.2 Hz, 1H), 2.92 - 2.88 (m, 1H), 2.79 - 2.70 (m, 1H), 2.52 (t, J = 5.0 Hz, 4H), 2.32 - 2.28 (m, 1H), 1.91 - 1.86 (m, 1H), 0.89 - 0.85 (m, 2H), 0.81 - 0.78 (m, 2H); LC-MS: m/z [M+H]⁺ = 653.

### Example 27 N-((1s,4s)-4-((1-(4-((1-(2,6-dioxopiperidin-3-yl)-2-oxo-1,2-dihydrobenzo[cd]indol-6-yl)methyl)benzyl)pi peridin-4-yl)oxy)cyclohexyl)-6-(1H-imidazol-1-yl)pyridin-2-carboxamide

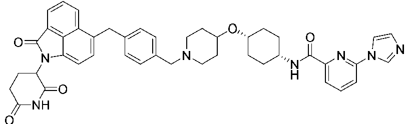

3-(6-(4-(4-((((1s,4s)-4-aminocyclohexyl)oxy)piperidin-1-yl)methyl)benzyl)-2-oxobenzo[cd]indol-1(2H) -yl)piperidin-2,6-dione (0.46 g, 0.98 mmol, intermediate 34), 6-(1H-imidazol-1-yl)-2-picolinic acid (0.19 g, 0.98 mmol, intermediate 6), 1-ethyl-(3-dimethylaminopropyl)carbodiimide hydrochloride (0.28 g, 1.47 mmol, CAS: 25952-53-8), and 1-hydroxybenzotriazole (0.20 g, 1.47 mmol, CAS: 2592-95-2) were added into a 25 mL two-necked flask, then dichloromethane (10 mL) was added in a nitrogen atmosphere, N-methylmorpholine (0.2 mL, 1.96 mmol, d = 0.92 g/mL) was added through a syringe, and the mixture was stirred at room temperature overnight. The reaction solution was diluted with brine (30 mL), and extracted with dichloromethane (10 mL) for 3 times. Organic phases were combined, dried over anhydrous sodium sulfate, concentrated under reduced pressure, and purified by silica gel column chromatography (dichloromethane/methanol = 10:1) to obtain the title compound as a yellow solid (40 mg, yield: 5%). ¹H NMR (500 MHz, DMSO-d6) δ11.11 (s, 1H), 8.93 (s, 1H), 8.62 (d, J = 8.4 Hz, 1H), 8.29 (d, J = 8.4 Hz, 1H), 8.23 (s, 1H), 8.10 (t, J = 7.9 Hz, 1H), 8.03 (d, J = 7.0 Hz, 1H), 7.94 (d, J = 8.2 Hz, 1H), 7.91 (d, J = 7.6 Hz, 1H), 7.77 (t, J = 7.6 Hz, 1H), 7.38 (d, J = 7.4 Hz, 1H), 7.25-7.23 (m, 4H), 7.11 (s, 1H), 7.07 (d, J = 7.3 Hz, 1H), 5.41 (dd, J = 12.9, 5.2 Hz, 1H), 4.34 (s, 2H), 3.85-3.79 (m, 1H), 3.57 (s, 1H), 3.33-3.31 (m, 3H), 2.95 - 2.88 (m, 1H), 2.82 - 2.67 (m, 3H), 2.64 - 2.59 (m, 1H), 2.05 (q, J = 8.8, 7.4 Hz, 1H), 1.76 (q, J = 12.2 Hz, 8H), 1.54 - 1.42 (m, 6H); LC-MS: m/z [M+H]⁺ =752.

### Example 28 3-(6-(4-((4-(6-(3-methyl-4H-1,2,4-triazol-4-yl)pyridin-2-yl)piperazin-1-yl)methyl)benzyl)-2-oxobenzo[c d]indol-1(2H)-yl)piperidin-2,6-dione

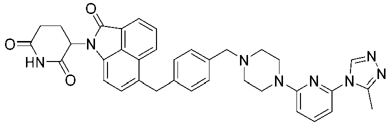

In a nitrogen atmosphere, cuprous oxide (100 mg, 0.69 mmol, CAS: 1317-39-1) and N1,N2-bis(furan-2-ylmethyl)oxalamide (120 mg, 0.48 mmol, CAS: 69010-90-8) were added into a mixed solution of 3-(6-(4-((4-(6-chloropyridin-2-yl)piperazin-1-yl)methyl)benzyl)-2-oxobenzo[cd]indol-1(2H)-yl)piperidin-2,6 -dione (450 mg, 0.78 mmol, intermediate 35), 3-methyl-1H-1,2,4-triazole (95 mg, 1.14 mmol, CAS: 7170-01-6), tripotassium phosphate (600 mg, 2.83 mmol), and dimethyl sulfoxide (10 mL), and the reaction solution was subjected to a microwave reaction at 150°C for 1 hour. The reaction solution was poured into water (40 mL) until a solid was precipitated, and then filtered. The filter cake was washed once with 10 mL of water, collected, and dried to obtain a crude product. The crude product was purified by prep-HPLC (acetonitrile and water containing 0.1% of formic acid) to obtain the title compound as a yellow solid (11.5 mg, yield: 2%). ¹H NMR (400 MHz, DMSO-d6) δ11.13 (s, 1H), 8.37 (d, J = 8.3 Hz, 1H), 8.09 (d, J = 7.0 Hz, 1H), 8.02 (s, 1H), 7.82 (t, J = 7.8 Hz, 2H), 7.48 (t, J = 8.2 Hz, 3H), 7.42 (d, J = 7.9 Hz, 2H), 7.13 (t, J = 7.2 Hz, 2H), 6.93 (d, J = 8.4 Hz, 1H), 5.45 (dd, J = 12.5, 5.5 Hz, 1H), 4.44 (s, 2H), 4.35 (d, J = 12.9 Hz, 2H), 4.31 - 4.24 (m, 2H), 3.40 - 3.28 (m, 4H), 3.09 - 3.00 (m, 2H), 2.98 - 2.89 (m, 1H), 2.82 - 2.73 (m, 1H), 2.71 (s, 3H), 2.68 - 2.60 (m, 1H), 2.12 - 2.06 (m, 1H); LC-MS: m/z [M+H]⁺ =627.

### Example 29 3-(6-(4-((4-(2-methyl-6-(4-(trifluoromethyl)-1H-imidazol-1-yl)pyrimidin-4-yl)piperazin-1-yl)methyl)b enzyl)-2-oxobenzo[cd]indol-1(2H)-yl)piperidin-2,6-dione

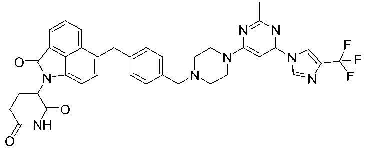

3-(2-oxo-6-(4-(piperazin-1-ylmethyl)benzyl)benzo[cd]indol-1(2H)-yl)piperidin-2,6-dione (150 mg, 0.32 mmol, intermediate 3), 2-methyl-4-(methylsulfonyl)-6-(4-(trifluoromethyl)-1H-imidazol-1-yl)pyrimidine (147 mg, 0.48 mmol, intermediate 36), and N,N-diisopropylethylamine (0.265 mL, 1.6 mmol, d = 0.782 g/mL) were added into a 50 mL single-necked flask, then dimethyl sulfoxide (3 mL) was added, and the mixture was stirred at 120°C for 5 hours. The reaction solution was purified by prep-HPLC (acetonitrile and water containing 0.1% of formic acid) to obtain the title compound as a yellow solid (95 mg, yield: 43%). ¹H NMR (400 MHz, DMSO-d6) δ 11.15 (s, 1H), 8.71 (s, 1H), 8.63 (t, J = 1.5 Hz, 1H), 8.33 (d, J = 8.3 Hz, 1H), 8.08 (d, J = 7.0 Hz, 1H), 7.81 (dd, J = 8.3, 7.0 Hz, 1H), 7.42 (d, J = 7.4 Hz, 1H), 7.25 (q, J = 8.0 Hz, 4H), 7.12 (d, J = 7.3 Hz, 1H), 7.04 (s, 1H), 5.45 (dd, J = 13.0, 5.4 Hz, 1H), 4.39 (s, 2H), 3.70 (s, 4H), 3.46 (s, 2H), 3.02 - 2.89 (m, 1H), 2.85 - 2.60 (m, 2H), 2.41 (m, 7H), 2.13 - 2.06 (m, 1H); LC-MS: m/z [M+H]⁺ = 695.

### Example 30 (S)-3-(6-(4-((4-(2-methyl-6-(4-(trifluoromethyl)-1H-imidazol-1-yl)pyrimidin-4-yl)piperazin-1-yl)methyl )benzyl)-2-oxobenzo[cd]indol-1(2H)-yl)piperidin-2,6-dione

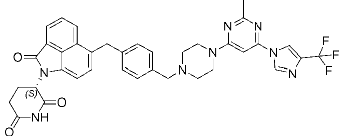

### Example 31 (R)-3-(6-(4-((4-(2-methyl-6-(4-(trifluoromethyl)-1H-imidazol-1-yl)pyrimidin-4-yl)piperazin-1-yl)methyl )benzyl)-2-oxobenzo[cd]indol-1(2H)-yl)piperidin-2,6-dione

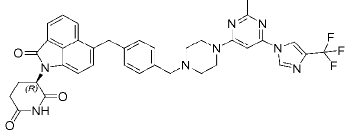

Chiral separation: 27 g of 3-(6-(4-((4-(2-methyl-6-(4-(trifluoromethyl)-1H-imidazol-1-yl)pyrimidin-4-yl)piperazin-1-yl)methyl)benzyl) -2-oxobenzo[cd]indol-1(2H)-yl)piperidin-2,6-dione (Example 29) was separated into enantiomers by a chiral normal-phase preparative high performance liquid chromatography (HPLC) method. First, preparative fractions were evaporated separately under reduced pressure to obtain solid substances. Then, the solid substances were suspended in a mixture of acetonitrile and water (2:3), and maintained in a dry ice/acetone bath until the acetonitrile-water mixture was solidified. Then, the frozen mixture was freeze-dried in a freeze dryer for 20 hours to obtain (S)-3-(6-(4-((4-(2-methyl-6-(4-(trifluoromethyl)-1H-imidazol-1-yl)pyrimidin-4-yl)piperazin-1-yl)methyl)ben zyl)-2-oxobenzo[cd]indol-1(2H)-yl)piperidin-2,6-dione in Example 30 (first elution peak, RT = 1.659 minutes, temporarily designated as "S" ABS) (11.5 g, 99.37%ee) and (R)-3-(6-(4-((4-(2-methyl-6-(4-(trifluoromethyl)-1H-imidazol-1-yl)pyrimidin-4-yl)piperazin-1-yl)methyl)be nzyl)-2-oxobenzo[cd]indol-1(2H)-yl)piperidin-2,6-dione in Example 31 (second elution peak, RT = 2.133 minutes, temporarily designated as 'R' ABS) (12.5 g, 100%ee).

### Chiral analysis method (analytical separation method):

instrument: Shimadzu LC-20AB with PDA detector;
chromatographic column: Chiralpak IC-3 100×4.6 mm I.D., 3 µm;
mobile phase: A: hexane (0.1% DEA), B: IPA:MeCN=2:1;
isocratic elution: B: 60%;
flow rate: 1.0 mL/min;
chromatographic column temperature: 35°C;
wavelength: 254 nm.

### Chiral preparation method (preparative separation method):

instrument: Shimadzu LC-A HPLC;
chromatographic column: Chiralpak IC, 250×30 mm, 10 µm;
mobile phase: A: DCM, B: IPA;
isocratic elution: B: 50%;
flow rate: 150 mL/min;
chromatographic column temperature: R.T.;
wavelength: 220 nm, 254 nm;
sample preparation: dissolving a sample in about 2,000 mL of DCM/IPA;
injection amount: 200 mL;
cycle time: 35 min.

Example 30: ¹H NMR (400 MHz, DMSO-d6) δ 11.13 (s, 1H), 8.71 (s, 1H), 8.62 (s, 1H), 8.33 (d, J = 8.4 Hz, 1H), 8.08 (d, J = 6.8 Hz, 1H), 7.81 (dd, J = 8.4, 7.2 Hz, 1H), 7.42 (d, J = 7.6 Hz, 1H), 7.26 (d, J = 8.2 Hz, 2H), 7.22 (d, J = 8.2 Hz, 2H), 7.11 (d, J = 7.2 Hz, 1H), 7.03 (s, 1H), 5.45 (dd, J = 12.8, 5.2 Hz, 1H), 4.39 (s, 2H), 3.69 (s, 4H), 3.46 (s, 2H), 3.02 - 2.89 (m, 1H), 2.81 - 2.70 (m, 1H), 2.67 - 2.63 (m, 1H), 2.43 - 2.38 (m, 7H), 2.15 - 2.04 (m, 1H); LC-MS: m/z [M+H]⁺ = 695.

Example 31: ¹H NMR (400 MHz, DMSO-d6) δ 11.12 (s, 1H), 8.70 (s, 1H), 8.62 (s, 1H), 8.32 (d, J = 8.4 Hz, 1H), 8.07 (d, J = 6.8 Hz, 1H), 7.81 (dd, J = 8.4, 7.2 Hz, 1H), 7.42 (d, J = 7.6 Hz, 1H), 7.26 (d, J = 8.2 Hz, 2H), 7.22 (d, J = 8.2 Hz, 2H), 7.11 (d, J = 7.2 Hz, 1H), 7.03 (s, 1H), 5.45 (dd, J = 12.8, 5.2 Hz, 1H), 4.39 (s, 2H), 3.69 (s, 4H), 3.45 (s, 2H), 3.02 - 2.89 (m, 1H), 2.83 - 2.69 (m, 1H), 2.69 - 2.61 (m, 1H), 2.43 - 2.38 (m, 7H), 2.13 - 2.04 (m, 1H); LC-MS: m/z [M+H]⁺ = 695.

### Example 32 3-(6-(4-(3-(4-(6-(1H-imidazol-1-yl)pyridin-2-yl)piperazin-1-yl)azetidin-1-yl)methyl)benzyl)-2-oxobenzo [cd]indol-1(2H)-yl)piperidin-2,6-dione

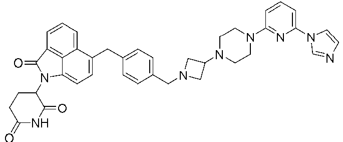

3-(2-oxo-6-(4-((3-(piperazin-1-yl)azetidin-1-yl)methyl)benzyl)benzo[cd]indol-1(2H)-yl)piperidin-2,6-di one (357 mg, 0.56 mmol, intermediate 39), 2-fluoro-6-(1H-imidazol-1-yl)pyridine (180 mg, 1.12 mmol, intermediate 4), and potassium carbonate (230 mg, 1.68 mmol) were added into dimethyl sulfoxide (5 mL), and the mixture was stirred at 100°C for 2 hours. The reaction solution was cooled to room temperature, diluted with water (50 mL), and extracted with ethyl acetate (20 mL) for 3 times. Organic phases were combined, washed with water (20 mL) for 2 times and with brine (10 mL) for 1 time, dried over anhydrous sodium sulfate, concentrated under reduced pressure, and separated by silica gel column chromatography (dichloromethane/methanol = 20:1) to obtain the title compound as a yellow solid (0.33 g, yield: 89%). ¹H NMR (500 MHz, CDCl₃) δ 9.20 (s, 1H), 8.28 (s, 1H), 8.08 (t, J = 7.7 Hz, 2H), 7.68 (dd, J = 8.3, 7.0 Hz, 1H), 7.56 - 7.53 (m, 2H), 7.22 - 7.19 (m, 3H), 7.16 - 7.14 (m, 3H), 6.68 (d, J = 7.3 Hz, 1H), 6.61 (d, J = 7.6 Hz, 1H), 6.50 (d, J = 8.4 Hz, 1H), 5.33 (dd, J = 13.0, 5.4 Hz, 1H), 4.35 (s, 2H), 3.68 (s, 2H), 3.60 - 3.58 (m, 6H), 3.05 (s, 3H), 2.96 - 2.82 (m, 2H), 2.76 - 2.67 (m, 1H), 2.39 (t, J = 5.0 Hz, 4H), 2.29 - 2.24 (m, 1H); LC-MS: m/z [M+H]⁺ = 667.

### Example 33 (S)-3-(2-oxo-6-(4-((4-(2-(4-(trifluoromethyl)-1H-imidazol-1-yl)pyrimidin-4-yl)piperazin-1-yl)methyl)be nzyl)benzo[cd]indol-1(2H)-yl)piperidin-2,6-dione

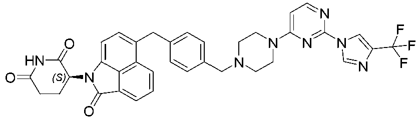

### Example 34 (R)-3-(2-oxo-6-(4-((4-(2-(4-(trifluoromethyl)-1H-imidazol-1-yl)pyrimidin-4-yl)piperazin-1-yl)methyl)be nzyl)benzo[cd]indol-1(2H)-yl)piperidin-2,6-dione

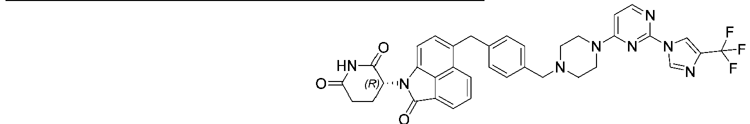

Chiral separation: 480 mg of 3-(2-oxo-6-(4-((4-(2-(4-(trifluoromethyl)-1H-imidazol-1-yl)pyrimidin-4-yl)piperazin-1-yl)methyl)benzyl)be nzo[cd]indol-1(2H)-yl)piperidin-2,6-dione (Example 25) was separated into enantiomers by a chiral normal-phase preparative HPLC method. First, preparative fractions were evaporated separately under reduced pressure to obtain solid substances. Then, the solid substances were suspended in a mixture of acetonitrile and water (2:3), and maintained in a dry ice/acetone bath until the acetonitrile-water mixture was solidified. Then, the frozen mixture was freeze-dried in a freeze dryer for 20 hours to obtain (S)-3-(2-oxo-6-(4-((4-(2-(4-(trifluoromethyl)-1H-imidazol-1-yl)pyrimidin-4-yl)piperazin-1-yl)methyl)benzyl )benzo[cd]indol-1(2H)-yl)piperidin-2,6-dione in Example 33 (first elution peak, RT = 3.360 minutes, temporarily designated as "S" ABS) (194.4 mg, 99.58%ee, yellow solid) and (R)-3-(2-oxo-6-(4-((4-(2-(4-(trifluoromethyl)-1H-imidazol-1-yl)pyrimidin-4-yl)piperazin-1-yl)methyl)benzyl )benzo[cd]indol-1(2H)-yl)piperidin-2,6-dione in Example 34 (second elution peak, RT = 3.997 minutes, temporarily designated as 'R' ABS) (190.1 mg, 98.99%ee, yellow solid).

Except that the mobile phase was adjusted to "A: MeCN, B: MeOH (0.05% DEA)" and the isocratic elution was performed with 50% B, a chiral analysis method was the same as that in Examples 30 and 31. Except for the following adjustments, a chiral preparation method was the same as that in Examples 30 and 31:
mobile phase: A: MeCN, B: MeOH;
isocratic elution: B: 65%;
flow rate: 120 mL/min;
sample preparation: dissolving a sample in about 30 mL of DCM/MeOH;
injection amount: 10 mL.

Example 33: ¹H NMR (400 MHz, DMSO-d6) δ 11.15 (s, 1H), 8.68 (s, 1H), 8.46 (s, 1H), 8.34 (d, J = 8.4 Hz, 1H), 8.21 (d, J = 6.4 Hz, 1H), 8.08 (d, J = 6.8 Hz, 1H), 7.81 (t, J = 7.6 Hz, 1H), 7.42 (d, J = 7.2 Hz, 1H), 7.27 (d, J = 8.0 Hz, 2H), 7.23 (d, J = 8.0 Hz, 2H), 7.12 (d, J = 7.2 Hz, 1H), 6.81 (d, J = 6.4 Hz, 1H), 5.46 (dd, J = 13.2, 5.6 Hz, 1H), 4.39 (s, 2H), 3.70 (t, J = 5.0 Hz, 4H), 3.46 (s, 2H), 3.04 - 2.90 (m, 1H), 2.83 - 2.60 (m, 2H), 2.40 (t, J = 5.0 Hz, 4H), 2.16 - 2.03 (m, 1H); LC-MS: m/z [M+H]⁺ = 681.

Example 34: ¹H NMR (400 MHz, DMSO-d6) δ 11.15 (s, 1H), 8.68 (s, 1H), 8.46 (s, 1H), 8.34 (d, J = 8.3 Hz, 1H), 8.21 (d, J = 6.2 Hz, 1H), 8.08 (d, J = 7.0 Hz, 1H), 7.81 (dd, J = 8.3, 7.0 Hz, 1H), 7.42 (d, J = 7.3 Hz, 1H), 7.27 (d, J = 7.9 Hz, 2H), 7.23 (d, J = 8.0 Hz, 2H), 7.12 (d, J = 7.3 Hz, 1H), 6.81 (d, J = 6.3 Hz, 1H), 5.46 (dd, J = 13.0, 5.3 Hz, 1H), 4.39 (s, 2H), 3.92 - 3.55 (m, 4H), 3.46 (s, 2H), 3.04 - 2.90 (m, 1H), 2.83 - 2.60 (m, 2H), 2.40 (t, J = 5.0 Hz, 4H), 2.16 - 2.03 (m, 1H); LC-MS: m/z [M+H]⁺ = 681.

### Example 35 (S)-3-(6-(4-((4-(6-(5-methyl-1,3,4-thiadiazol-2-yl)pyridin-2-yl)piperazin-1-yl)methyl)benzyl)-2-oxobenz o[cd]indol-1(2H)-yl)piperidin-2,6-dione

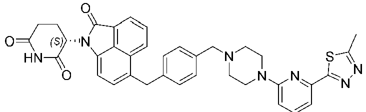

### Example 36 (R)-3-(6-(4-((4-(6-(5-methyl-1,3,4-thiadiazol-2-yl)pyridin-2-yl)piperazin-1-yl)methyl)benzyl)-2-oxobenz o[cd]indol-1(2H)-yl)piperidin-2,6-dione

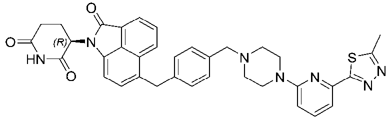

Chiral separation: 550 mg of 3-(6-(4-((4-(6-(5-methyl-1,3,4-thiadiazol-2-yl)pyridin-2-yl)piperazin-1-yl)methyl)benzyl)-2-oxobenzo[cd]in dol-1(2H)-yl)piperidin-2,6-dione (Example 22) was separated into enantiomers by a chiral normal-phase preparative HPLC method. First, preparative fractions were evaporated separately under reduced pressure to obtain solid substances. Then, the solid substances were suspended in a mixture of acetonitrile and water (2:3), and maintained in a dry ice/acetone bath until the acetonitrile-water mixture was solidified. Then, the frozen mixture was freeze-dried in a freeze dryer for 20 hours to obtain (S)-3-(6-(4-((4-(6-(5-methyl-1,3,4-thiadiazol-2-yl)pyridin-2-yl)piperazin-1-yl)methyl)benzyl)-2-oxobenzo[c d]indol-1(2H)-yl)piperidin-2,6-dione in Example 35 (first elution peak, RT = 5.265 minutes, temporarily designated as "S" ABS) (101 mg, 99.02%ee, yellow solid) and (R)-3-(6-(4-((4-(6-(5-methyl-1,3,4-thiadiazol-2-yl)pyridin-2-yl)piperazin-1-yl)methyl)benzyl)-2-oxobenzo[c d]indol-1(2H)-yl)piperidin-2,6-dione in Example 36 (second elution peak, RT = 6.465 minutes, temporarily designated as 'R' ABS) (95 mg, 99.19%ee, yellow solid).

Except that the detection wavelength was adjusted to 220 nm, a chiral analysis method was the same as that in Examples 33 and 34. Except that a sample was dissolved in about 250 mL of DCM/MeOH and the injection volume was adjusted to 15 mL, a chiral preparation method was the same as that in Examples 33 and 34.

Example 35: ¹H NMR (400 MHz, DMSO-d6) δ 11.13 (s, 1H), 8.34 (d, J = 8.4 Hz, 1H), 8.08 (d, J = 7.0 Hz, 1H), 7.81 (t, J = 8.0 Hz, 1H), 7.69 (t, J = 8.0 Hz, 1H), 7.43 (dd, J = 10.4, 8.4 Hz, 2H), 7.26 (d, J = 8.0 Hz, 2H), 7.23 (d, J = 8.4 Hz, 2H), 7.11 (d, J = 7.3 Hz, 1H), 6.94 (d, J = 8.8 Hz, 1H), 5.45 (dd, J = 12.8, 5.4 Hz, 1H), 4.39 (s, 2H), 3.52 (t, J = 4.4 Hz, 4H), 3.45 (s, 2H), 3.02 - 2.90 (m, 1H), 2.81 - 2.76 (m, 1H), 2.73 (s, 3H), 2.69 - 2.61 (m, 1H), 2.43 (t, J = 4.4 Hz, 4H), 2.16 - 2.03 (m, 1H); LC-MS: m/z [M+H]⁺ = 644.

Example 36: ¹H NMR (400 MHz, DMSO-d6) δ 11.13 (s, 1H), 8.34 (d, J = 8.4 Hz, 1H), 8.08 (d, J = 7.0 Hz, 1H), 7.81 (t, J = 8.0 Hz, 1H), 7.69 (t, J = 8.0 Hz, 1H), 7.43 (dd, J = 10.4, 8.4 Hz, 2H), 7.26 (d, J = 8.0 Hz, 2H), 7.23 (d, J = 8.4 Hz, 2H), 7.11 (d, J = 7.3 Hz, 1H), 6.94 (d, J = 8.8 Hz, 1H), 5.45 (dd, J = 12.8, 5.4 Hz, 1H), 4.39 (s, 2H), 3.52 (t, J = 4.4 Hz, 4H), 3.45 (s, 2H), 3.02 - 2.90 (m, 1H), 2.81 - 2.76 (m, 1H), 2.73 (s, 3H), 2.69 - 2.61 (m, 1H), 2.43 (t, J = 4.4 Hz, 4H), 2.16 - 2.03 (m, 1H); LC-MS: m/z [M+H]⁺ = 644.

### Example 37 (S)-3-(6-(4-((4-(6-(3-methyl-1H-1,2,4-triazol-1-yl)pyridin-2-yl)piperazin-1-yl)methyl)benzyl)-2-oxobenz o[cd]indol-1(2H)-yl)piperidin-2,6-dione

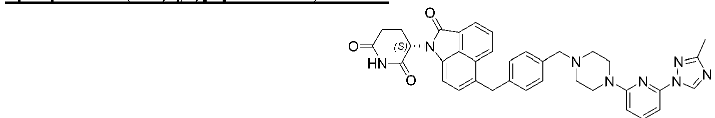

### Example 38 (R)-3-(6-(4-((4-(6-(3-methyl-1H-1,2,4-triazol-1-yl)pyridin-2-yl)piperazin-1-yl)methyl)benzyl)-2-oxoben zo[cd]indol-1(2H)-yl)piperidin-2,6-dione

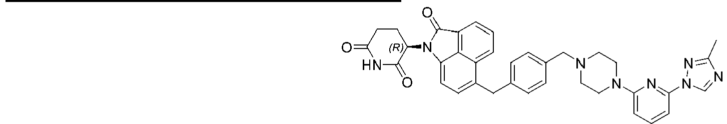

Chiral separation: 500 mg of 3-(6-(4-((4-(6-(3-methyl-1H-1,2,4-triazol-1-yl)pyridin-2-yl)piperazin-1-yl)methyl)benzyl)-2-oxobenzo[cd]in dol-1(2H)-yl)piperidin-2,6-dione (Example 23) was separated into enantiomers by a chiral normal-phase preparative HPLC method. First, preparative fractions were evaporated separately under reduced pressure to obtain solid substances. Then, the solids were suspended in a mixture of acetonitrile and water (2:3), and maintained in a dry ice/acetone bath until the acetonitrile-water mixture was solidified. Then, the frozen mixture was freeze-dried in a freeze dryer for 20 hours to obtain (S)-3-(6-(4-((4-(6-(3-methyl-1H-1,2,4-triazol-1-yl)pyridin-2-yl)piperazin-1-yl)methyl)benzyl)-2-oxobenzo[c d]indol-1(2H)-yl)piperidin-2,6-dione in Example 37 (first elution peak, RT = 4.269 minutes, temporarily designated as "S" ABS) (185 mg, 98.37%ee, yellow solid) and (R)-3-(6-(4-((4-(6-(3-methyl-1H-1,2,4-triazol-1-yl)pyridin-2-yl)piperazin-1-yl)methyl)benzyl)-2-oxobenzo[c d]indol-1(2H)-yl)piperidin-2,6-dione in Example 38 (second elution peak, RT = 5.211 minutes, temporarily designated as 'R' ABS) (195 mg, 98.90%ee, yellow solid).

A chiral analysis method and a chiral preparation method were the same as those in Examples 33 and 34.

Example 37: ¹H NMR (400 MHz, DMSO-d6) δ 11.14 (s, 1H), 9.17 (s, 1H), 8.34 (d, J = 8.0 Hz, 1H), 8.08 (d, J = 7.2 Hz, 1H), 7.82 (t, J = 8.0 Hz, 1H), 7.68 (t, J = 8.0 Hz, 1H), 7.42 (d, J = 7.4 Hz, 1H), 7.27 (d, J = 8.0 Hz, 2H), 7.23 (d, J = 8.0 Hz, 2H), 7.12 (d, J = 7.2 Hz, 1H), 6.96 (d, J = 7.6 Hz, 1H), 6.77 (d, J = 8.4 Hz, 1H), 5.45 (dd, J = 12.8, 5.2 Hz, 1H), 4.39 (s, 2H), 3.54 (t, J = 4.4 Hz, 4H), 3.45 (s, 2H), 3.01 - 2.89 (m, 1H), 2.84 - 2.61 (m, 2H), 2.42 (t, J = 4.4 Hz, 4H), 2.35 (s, 3H), 2.15 - 2.05 (m, 1H); LC-MS: m/z [M+H]⁺ = 627.

Example 38: ¹H NMR (400 MHz, DMSO-d6) δ 11.14 (s, 1H), 9.17 (s, 1H), 8.34 (d, J = 8.0 Hz, 1H), 8.08 (d, J = 7.2 Hz, 1H), 7.82 (t, J = 8.0 Hz, 1H), 7.68 (t, J = 8.0 Hz, 1H), 7.42 (d, J = 7.4 Hz, 1H), 7.27 (d, J = 8.0 Hz, 2H), 7.23 (d, J = 8.0 Hz, 2H), 7.12 (d, J = 7.2 Hz, 1H), 6.96 (d, J = 7.6 Hz, 1H), 6.77 (d, J = 8.4 Hz, 1H), 5.45 (dd, J = 12.8, 5.2 Hz, 1H), 4.39 (s, 2H), 3.54 (t, J = 4.4 Hz, 4H), 3.45 (s, 2H), 3.01 - 2.89 (m, 1H), 2.84 - 2.61 (m, 2H), 2.42 (t, J = 4.4 Hz, 4H), 2.35 (s, 3H), 2.15 - 2.05 (m, 1H); LC-MS: m/z [M+H]⁺ = 627.

### Example 39 3-(2-oxo-6-(4-((4-(2-(3-(trifluoromethyl)-1H-1,2,4-triazol-1-yl)pyrimidin-4-yl)piperazin-1-yl)methyl)be nzyl)benzo[cd]indol-1(2H)-yl)piperidin-2,6-dione

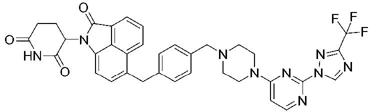

The title compound was synthesized by replacing 4-(methylsulfonyl)-2-(2-(trifluoromethyl)-1H-imidazol-1-yl)pyrimidine (intermediate 15) with 4-(methylsulfonyl)-2-(3-(trifluoromethyl)-1H-1,2,4-triazol-1-yl)pyrimidine (intermediate 40) using a method similar to that in Example 13. ¹H NMR (500 MHz, CDCl₃) δ 9.12 (s, 1H), 8.66 (s, 1H), 8.21 (d, J = 6.2 Hz, 1H), 8.10 (d, J = 8.3 Hz, 1H), 8.07 (d, J = 7.0 Hz, 1H), 7.68 (t, J = 7.6 Hz, 1H), 7.22 (t, 3H), 7.17 (d, J = 7.8 Hz, 2H), 6.70 (d, J = 7.3 Hz, 1H), 6.47 (d, J = 6.2 Hz, 1H), 5.32 (dd, J = 12.9, 5.4 Hz, 1H), 4.35 (s, 2H), 3.76 (s, 4H), 3.53 (s, 2H), 2.95 - 2.81 (m, 2H), 2.71 (qd, 1H), 2.53 (s, 4H), 2.28 - 2.23 (m, 1H); LC-MS: m/z [M+H]⁺ = 682.

### Example 40 3-(2-oxo-6-(4-((4-(1-(2-(4-(trifluoromethyl)-1H-imidazol-1-yl)pyrimidin-4-yl)piperidin-4-yl)piperazin-1 -yl)methyl)benzyl)benzo[cd]indol-1(2H)-yl)piperidin-2,6-dione

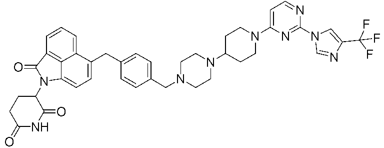

The title compound was synthesized by replacing 3-(2-oxo-6-(4-(piperazin-1-ylmethyl)benzyl)benzo[cd]indol-1(2H)-yl)piperidin-2,6-dione (intermediate 3) with 3-(2-oxo-6-(4-((4-(piperidin-4-yl)piperazin-1-yl)methyl)benzyl)benzo[cd]indol-1(2H)-yl)piperidin-2,6-dione (intermediate 42) using a method similar to that in Example 25. ¹H NMR (500 MHz, CDCl₃) δ 8.80 (s, 1H), 8.52 (s, 1H), 8.13 - 8.10 (m, 2H), 8.06 (d, J = 4.8 Hz, 2H), 7.68 - 7.65 (m, 1H), 7.22 (d, J = 7.8 Hz, 2H), 7.15 (dd, J = 16.5, 7.6 Hz, 3H), 6.67 (d, J = 7.2 Hz, 1H), 6.41 (d, J = 6.3 Hz, 1H), 5.32 - 5.28 (m, 1H), 4.32 (s, 2H), 3.73 (s, 2H), 2.96 - 2.71 (m, 16H), 2.27 (dd, J = 7.9, 5.2 Hz, 1H), 1.99 (d, J = 11.8 Hz, 2H), 1.55 - 1.48 (m, 2H); LC-MS: m/z [M+H]⁺ = 764.

### Example 41 3-(6-(4-((4-(2-(3-methyl-1H-1,2,4-triazol-1-yl)pyrimidin-4-yl)piperazin-1-yl)methyl)benzyl)-2-oxobenzo [cd]indol-1(2H)-yl)piperidin-2,6-dione

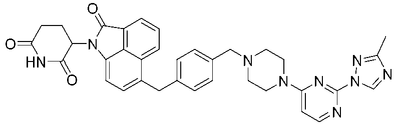

The title compound was synthesized by replacing 2-chloro-6-(4-isopropyl-4H-1,2,4-triazol-3-yl)pyridine (intermediate 29) with 2-(3-methyl-1H-1,2,4-triazol-1-yl)-4-(methylsulfonyl)pyrimidine (intermediate 43) using a method similar to that in Example 24. ¹H NMR (400 MHz, DMSO-d₆) δ 11.14 (s, 1H), 9.20 (s, 1H), 8.33 (d, J = 8.2 Hz, 1H), 8.20 (d, J = 6.2 Hz, 1H), 8.08 (d, J = 7.0 Hz, 1H), 7.81 (t, J = 7.6 Hz, 1H), 7.42 (d, J = 7.4 Hz, 1H), 7.24 (dd, J = 17.0, 8.1 Hz, 4H), 7.11 (d, J = 7.3 Hz, 1H), 6.78 (d, J = 6.3 Hz, 1H), 5.45 (dd, J = 13.0, 5.2 Hz, 1H), 4.39 (s, 2H), 3.67 (m, 4H), 3.46 (s, 2H), 3.10 - 2.89 (m, 1H), 2.87 - 2.71 (m, 1H), 2.71 - 2.65 (m, 1H), 2.41 - 2.37 (m, 4H), 2.34 (s, 3H), 2.14 - 1.95 (m, 1H); LC-MS: m/z [M+H]⁺ = 628.

### Example 42 3-(6-(4-((4-(5-fluoro-2-(5-methyl-1,3,4-thiadiazol-2-yl)pyrimidin-4-yl)piperazin-1-yl)methyl)benzyl)-2-oxobenzo[cd]indol-1(2H)-yl)piperidin-2,6-dione

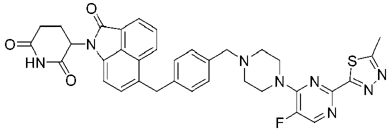

2-(5-fluoro-4-(piperazin-1-yl)pyrimidin-2-yl)-5-methyl-1,3,4-thiadiazole (740 mg, 1.9 mmol, TFA salt, intermediate 44) and 4-((1-(2,6-dioxopiperidin-3-yl)-2-oxo-1,2-dihydrobenzo[cd]indol-6-yl)methyl)benzaldehyde (797 mg, 2 mmol, intermediate 38) were added into 1,2-dichloroethane (5 mL) and acetic acid (230 mg, 3.8 mmol), and the mixture was stirred at room temperature for 20 minutes. Then, sodium triacetoxyborohydride (805 mg, 3.8 mmol, CAS: 56553-60-7) was added, and the reaction mixture was stirred at room temperature for 3 hours. A saturated sodium bicarbonate solution (50 mL) was added into the reaction solution to quench a reaction, and the reaction solution was extracted with dichloromethane (30 mL) for 3 times. Combined organic phases were dried over anhydrous sodium sulfate, concentrated under reduced pressure, and separated by silica gel column chromatography (dichloromethane/methanol = 20:1) to obtain the title compound as a yellow solid (81 mg, yield: 7%). ¹H NMR (500 MHz, CDCl₃) δ 8.31 (s, 1H), 8.12 (t, J = 7.6 Hz, 2H), 8.08 (d, J = 7.0 Hz, 1H), 7.74-7.64 (m, 1H), 7.22 (d, J = 7.8 Hz, 3H), 7.16 (d, J = 8.0 Hz, 2H), 6.70 (d, J = 7.3 Hz, 1H), 5.33 (dd, J = 12.9, 5.3 Hz, 1H), 4.37 (s, 2H), 3.87 (s, 4H), 3.50 (s, 2H), 2.94 (s, 1H), 2.91-2.81 (m, 1H), 2.79 (s, 3H), 2.78-2.67 (m, 1H), 2.53 (s, 4H), 2.34-2.21 (m, 1H); LC-MS: m/z [M+H]⁺ = 663.

### Example 43 3-(6-(4-((4-(2-(4-chloro-1H-imidazol-1-yl)pyrimidin-4-yl)piperazin-1-yl)methyl)benzyl)-2-oxobenzo[cd] indol-1(2H)-yl)piperidin-2,6-dione

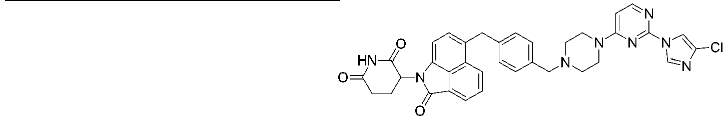

The title compound was synthesized by replacing 2-chloro-6-(4-isopropyl-4H-1,2,4-triazol-3-yl)pyridine (intermediate 29) with 2-(4-chloro-1H-imidazol-1-yl)-4-(methylsulfonyl)pyrimidine (intermediate 45) using a method similar to that in Example 24. ¹H NMR (400 MHz, DMSO-d₆) δ 11.14 (s, 1H), 8.48 (d, J = 1.4 Hz, 1H), 8.33 (d, J = 8.3 Hz, 1H), 8.17 (d, J = 6.3 Hz, 1H), 8.08 (d, J = 7.0 Hz, 1H), 7.94 (d, J = 1.4 Hz, 1H), 7.81 (dd, J = 8.0, 7.2 Hz, 1H), 7.42 (d, J = 7.4 Hz, 1H), 7.24 (dd, J = 17.9, 8.1 Hz, 4H), 7.11 (d, J = 7.3 Hz, 1H), 6.76 (d, J = 6.3 Hz, 1H), 5.45 (dd, J = 12.8, 5.3 Hz, 1H), 4.39 (s, 2H), 3.67 (s, 4H), 3.45 (s, 2H), 3.02 - 2.88 (m, 1H), 2.83 - 2.71 (m, 1H), 2.69 - 2.59 (m, 1H), 2.44 - 2.34 (m, 4H), 2.15 - 2.04 (m, 1H); LC-MS: m/z [M+H]⁺ = 647.

### Example 44 3-(6-(4-((4-(2-(4-cyano-1H-imidazol-1-yl)pyrimidin-4-yl)piperazin-1-yl)methyl)benzyl)-2-oxobenzo[cd]i ndol-1(2H)-yl)piperidin-2,6-dione

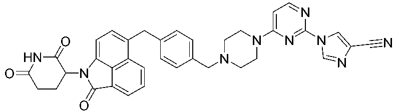

The title compound was synthesized by replacing 2-chloro-6-(4-isopropyl-4H-1,2,4-triazol-3-yl)pyridine (intermediate 29) with 2-(4-cyano-1H-imidazol-1-yl)-4-(methylsulfonyl)pyrimidine (intermediate 46) using a method similar to that in Example 24. ¹H NMR (400 MHz, DMSO-d₆) δ 11.15 (s, 1H), 8.86 (d, J = 1.2 Hz, 1H), 8.71 (d, J = 1.3 Hz, 1H), 8.34 (d, J = 8.3 Hz, 1H), 8.21 (d, J = 6.3 Hz, 1H), 8.08 (d, J = 7.0 Hz, 1H), 7.81 (dd, J = 8.2, 7.0 Hz, 1H), 7.42 (d, J = 7.4 Hz, 1H), 7.30 - 7.17 (m, 4H), 7.12 (d, J = 7.3 Hz, 1H), 6.83 (d, J = 6.3 Hz, 1H), 5.45 (dd, J = 12.8, 5.2 Hz, 1H), 4.39 (s, 2H), 3.73 (s, 4H), 3.46 (s, 2H), 3.01 - 2.90 (m, 1H), 2.82 - 2.71 (m, 1H), 2.63 (s, 1H), 2.39 (t, J = 5.0 Hz, 4H), 2.14 - 2.06 (m, 1H); LC-MS: m/z [M+H]⁺ = 638.

### Example 45 3-(6-(4-((4-(5-chloro-2-(3-methyl-1H-1,2,4-triazol-1-yl)pyrimidin-4-yl)piperazin-1-yl)methyl)benzyl)-2-oxobenzo[cd]indol-1(2H)-yl)piperidin-2,6-dione

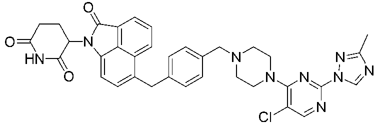

The title compound was synthesized by replacing 2-(5-fluoro-4-(piperazin-1-yl)pyrimidin-2-yl)-5-methyl-1,3,4-thiadiazole (intermediate 44) with 5-chloro-2-(3-methyl-1H-1,2,4-triazol-1-yl)-4-(piperazin-1-yl)pyrimidine (intermediate 47) using a method similar to that in Example 42. ¹H NMR (500 MHz, CDCl₃) δ 8.90 (s, 1H), 8.36 (s, 1H), 8.24 (s, 1H), 8.10 (dd, J = 16.6, 7.6 Hz, 2H), 7.74-7.65 (m, 1H), 7.23 (d, J = 7.1 Hz, 3H), 7.17 (d, J = 8.1 Hz, 2H), 6.69 (d, J = 7.3 Hz, 1H), 5.33 (dd, J = 13.0, 5.4 Hz, 1H), 4.37 (s, 2H), 3.85 (s, 4H), 3.51 (s, 2H), 2.94 (s, 1H), 2.92-2.80 (m, 1H), 2.73 (dd, J = 13.2, 4.6 Hz, 1H), 2.55 (s, 4H), 2.49 (s, 3H), 2.34-2.23 (m, 1H); LC-MS: m/z [M+H]⁺ = 662.

### Example 46 (S)-3-(6-(4-((4-(2-methyl-6-(3-methyl-1H-1,2,4-triazol-1-yl)pyrimidin-4-yl)piperazin-1-yl)methyl)benzy l)-2-oxobenzo[cd]indol-1(2H)-yl)piperidin-2,6-dione

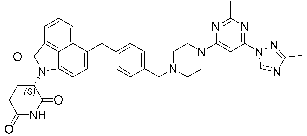

### Example 47 (R)-3-(6-(4-((4-(2-methyl-6-(3-methyl-1H-1,2,4-triazol-1-yl)pyrimidin-4-yl)piperazin-1-yl)methyl)benzy l)-2-oxobenzo[cd]indol-1(2H)-yl)piperidin-2,6-dione

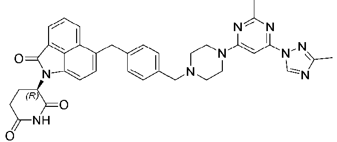

### 3-(6-(4-((4-(2-methyl-6-(3-methyl-1H-1,2,4-triazol-1-yl)pyrimidin-4-yl)piperazin-1-yl)methyl)benz yl)-2-oxobenzo[cd]indol-1(2H)-yl)piperidin-2,6-dione

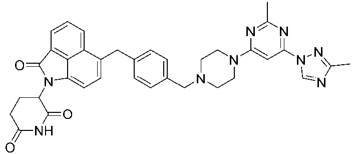

The title compound was synthesized by replacing 2-chloro-6-(4-isopropyl-4H-1,2,4-triazol-3-yl)pyridine (intermediate 29) with 2-methyl-4-(3-methyl-1H-1,2,4-triazol-1-yl)-6-(methylsulfonyl)pyrimidine (intermediate 48) using a method similar to that in Example 24. Chiral separation: 150 mg of 3-(6-(4-((4-(2-methyl-6-(3-methyl-1H-1,2,4-triazol-1-yl)pyrimidin-4-yl)piperazin-1-yl)methyl)benzyl)-2-oxo benzo[cd]indol-1(2H)-yl)piperidin-2,6-dione was separated into enantiomers by a chiral normal-phase preparative HPLC method. First, preparative fractions were evaporated separately under reduced pressure to obtain solid substances. Then, the solids were suspended in a mixture of acetonitrile and water (2:3), and maintained in a dry ice/acetone bath until the acetonitrile-water mixture was solidified. Then, the frozen mixture was freeze-dried in a freeze dryer for 20 hours to obtain (S)-3-(6-(4-((4-(2-methyl-6-(3-methyl-1H-1,2,4-triazol-1-yl)pyrimidin-4-yl)piperazin-1-yl)methyl)benzyl)-2 -oxobenzo[cd]indol-1(2H)-yl)piperidin-2,6-dione in Example 46 (first elution peak, RT = 2.254 minutes, temporarily designated as "S" ABS) (24 mg, 97.56%ee, yellow solid) and (R)-3-(6-(4-((4-(2-methyl-6-(3-methyl-1H-1,2,4-triazol-1-yl)pyrimidin-4-yl)piperazin-1-yl)methyl)benzyl)-2 -oxobenzo[cd]indol-1(2H)-yl)piperidin-2,6-dione in Example 47 (second elution peak, RT = 2.920 minutes, temporarily designated as 'R' ABS) (17 mg, 97.52%ee, yellow solid).

Except that the isocratic elution was performed with 80% of B, a chiral analysis method was the same as that in Examples 30 and 31. Except for the following adjustments, a chiral preparation method was the same as that in Examples 30 and 31:
mobile phase: A: hexane, B: IPA:MeCN=2:1 (0.1% NH₃·H₂O);
isocratic elution: B: 80%;
flow rate: 80 mL/min;
sample preparation: dissolving a sample in about 30 mL of DCM/IPA;
injection amount: 3 mL.

Example 46: ¹H NMR (400 MHz, DMSO-d6) δ 11.15 (s, 1H), 9.14 (s, 1H), 8.33 (d, J = 8.4 Hz, 1H), 8.08 (d, J = 6.8 Hz, 1H), 7.81 (t, J = 7.8 Hz, 1H), 7.42 (d, J = 7.2 Hz, 1H), 7.26 (d, J = 7.8 Hz, 2H), 7.22 (d, J = 7.8 Hz, 2H), 7.11 (d, J = 7.6 Hz, 1H), 6.78 (s, 1H), 5.52 - 5.39 (m, 1H), 4.39 (s, 2H), 3.64 (s, 4H), 3.43 (s, 2H), 2.99 - 2.91 (m, 1H), 2.84 - 2.63 (m, 2H), 2.41 - 2.33 (m, 10H), 1.36 - 1.24 (m, 1H); LC-MS: m/z [M+H]⁺ = 642.

Example 47: ¹H NMR (400 MHz, DMSO-d6) δ 11.15 (s, 1H), 9.14 (s, 1H), 8.33 (d, J = 8.4 Hz, 1H), 8.08 (d, J = 6.8 Hz, 1H), 7.81 (t, J = 7.8 Hz, 1H), 7.42 (d, J = 7.2 Hz, 1H), 7.26 (d, J = 7.8 Hz, 2H), 7.22 (d, J = 7.8 Hz, 2H), 7.11 (d, J = 7.6 Hz, 1H), 6.78 (s, 1H), 5.52 - 5.39 (m, 1H), 4.39 (s, 2H), 3.64 (s, 4H), 3.43 (s, 2H), 2.99 - 2.91 (m, 1H), 2.84 - 2.63 (m, 2H), 2.41 - 2.33 (m, 10H), 1.36 - 1.24 (m, 1H); LC-MS: m/z [M+H]⁺ = 642.

### Example 48 3-(6-(4-((3-(4-(2-methyl-6-(3-methyl-1H-1,2,4-triazol-1-yl)pyrimidin-4-yl)piperazin-1-yl)azetidin-1-yl) methyl)benzyl)-2-oxobenzo[cd]indol-1(2H)-yl)piperidin-2,6-dione

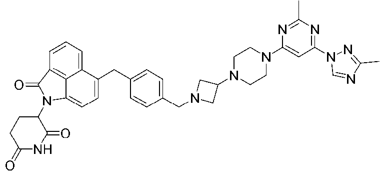

The title compound was synthesized by replacing 3-(2-oxo-6-(4-(piperazin-1-ylmethyl)benzyl)benzo[cd]indol-1(2H)-yl)piperidin-2,6-dione (intermediate 3) with 3-(2-oxo-6-(4-((3-(piperazin-1-yl)azetidin-1-yl)methyl)benzyl)benzo[cd]indol-1(2H)-yl)piperidin-2,6-dione (intermediate 39) and replacing 2-methyl-4-(methylsulfonyl)-6-(4-(trifluoromethyl)-1H-imidazol-1-yl)pyrimidine (intermediate 36) with 2-methyl-4-(3-methyl-1H-1,2,4-triazol-1-yl)-6-(methylsulfonyl)pyrimidine (intermediate 48) using a method similar to that in Example 29. ¹H NMR (400 MHz, DMSO-d6) δ 11.11 (s, 1H), 9.13 (s, 1H), 8.31 (d, J = 8.2 Hz, 1H), 8.07 (d, J = 6.9 Hz, 1H), 7.80 (t, J = 7.5 Hz, 1H), 7.40 (d, J = 7.2 Hz, 1H), 7.21 (d, J = 7.6 Hz, 2H), 7.16 (d, J = 7.8 Hz, 2H), 7.10 (d, J = 7.2 Hz, 1H), 6.81 (s, 1H), 5.44 (dd, J = 12.8, 5.1 Hz, 1H), 4.37 (s, 2H), 3.64 (s, 5H), 3.49 (s, 2H), 3.01 - 2.65 (m, 7H), 2.41 (s, 3H), 2.37 (s, 3H), 2.28 (s, 4H), 2.10 (d, J = 5.4 Hz, 1H); LC-MS: m/z [M+H]⁺ = 697.

### Example 49 3-(6-(4-((3-(4-(5-fluoro-2-(3-methyl-1H-1,2,4-triazol-1-yl)pyrimidin-4-yl)piperazin-1-yl)azetidin-1-yl)m ethyl)benzyl)-2-oxobenzo[cd]indol-1(2H)-yl)piperidin-2,6-dione

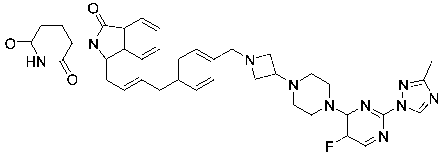

The title compound was synthesized by replacing 2-(5-fluoro-4-(piperazin-1-yl)pyrimidin-2-yl)-5-methyl-1,3,4-thiadiazole (intermediate 44) with 4-(4-(azetidin-3-yl)piperazin-1-yl)-5-fluoro-2-(3-methyl-1H-1,2,4-triazol-1-yl)pyrimidine (intermediate 50) using a method similar to that in Example 42. ¹H NMR (500 MHz, CDCl₃) δ 9.03 (s, 1H), 8.86 (s, 1H), 8.06 (q, 3H), 7.66 (t, 1H), 7.19 (t, 3H), 7.14 (d, J = 7.9 Hz, 2H), 6.67 (d, J = 7.3 Hz, 1H), 5.31 (dd, J = 13.0, 5.4 Hz, 1H), 4.33 (s, 2H), 3.86 (t, J = 4.9 Hz, 4H), 3.67 (s, 2H), 3.55 (s, 2H), 3.05 (s, 3H), 2.94 - 2.83 (m, 2H), 2.74 - 2.65 (m, 1H), 2.47 (s, 3H), 2.40 (t, J = 5.0 Hz, 4H), 2.31 - 2.21 (m, 1H); LC-MS: m/z [M+H]⁺ =701.

### Example 50 3-(6-(4-((4-(6-(-4-chloro-1H-imidazol-1-yl)-2-methylpyrimidin-4-yl)piperazin-1-yl)methyl)benzyl)-2-ox obenzo[cd]indol-1(2H)-yl)piperidin-2,6-dione

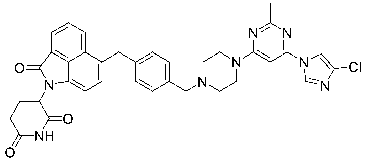

The title compound was synthesized by replacing 2-methyl-4-(methylsulfonyl)-6-(4-(trifluoromethyl)-1H-imidazol-1-yl)pyrimidine (intermediate 36) with 4-(4-chloro-1H-imidazol-1-yl)-2-methyl-6-(methylsulfonyl)pyrimidine (intermediate 51) using a method similar to that in Example 29. ¹H NMR (400 MHz, DMSO-d6) δ 11.15 (s, 1H), 8.51 (d, J = 1.5 Hz, 1H), 8.33 (d, J = 8.3 Hz, 1H), 8.12 - 8.05 (m, 2H), 7.81 (t, J = 7.6 Hz, 1H), 7.42 (d, J = 7.4 Hz, 1H), 7.24 (q, J = 8.0 Hz, 4H), 7.11 (d, J = 7.2 Hz, 1H), 6.90 (s, 1H), 5.45 (dd, J = 12.9, 5.4 Hz, 1H), 4.39 (s, 2H), 3.67 (s, 4H), 3.45 (s, 2H), 2.96 (ddd, J = 16.9, 13.1, 5.3 Hz, 1H), 2.82 - 2.70 (m, 1H), 2.69 - 2.61 (m, 1H), 2.39 (d, J = 4.5 Hz, 7H), 2.13 - 2.05 (m, 1H); LC-MS: m/z [M+H]⁺ =661.

### Example 51 3-(6-(4-((4-(6-(-4-cyano-1H-imidazol-1-yl)-2-methylpyrimidin-4-yl)piperazin-1-yl)methyl)benzyl)-2-oxo benzo[cd]indol-1(2H)-yl)piperidin-2,6-dione

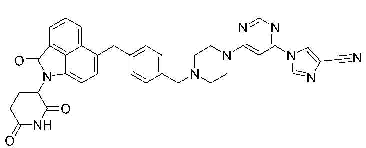

The title compound was synthesized by replacing 2-methyl-4-(methylsulfonyl)-6-(4-(trifluoromethyl)-1H-imidazol-1-yl)pyrimidine (intermediate 36) with 4-(4-cyano-1H-imidazol-1-yl)-2-methyl-6-(methylsulfonyl)pyrimidine (intermediate 52) using a method similar to that in Example 29. ¹H NMR (400 MHz, DMSO-d6) δ 11.11 (s, 1H), 8.88 (d, J = 1.3 Hz, 1H), 8.73 (d, J = 1.3 Hz, 1H), 8.32 (d, J = 8.3 Hz, 1H), 8.07 (d, J = 7.0 Hz, 1H), 7.81 (dd, J = 8.3, 7.0 Hz, 1H), 7.41 (d, J = 7.4 Hz, 1H), 7.24 (q, J = 8.0 Hz, 4H), 7.11 (d, J = 7.3 Hz, 1H), 7.01 (s, 1H), 5.44 (dd, J = 12.9, 5.4 Hz, 1H), 4.39 (s, 2H), 3.69 (s, 4H), 3.46 (s, 2H), 3.01 - 2.90 (m, 1H), 2.83 - 2.62 (m, 2H), 2.41 (d, J = 5.8 Hz, 7H), 2.14 - 2.06 (m, 1H); LC-MS: m/z [M+H]⁺ =652.

### Example 52 3-(6-(4-((3-(4-(2-(3-methyl-1H-1,2,4-triazol-1-yl)-6-(trifluoromethyl)pyrimidin-4-yl)piperazin-1-yl)azeti din-1-yl)methyl)benzyl)-2-oxobenzo[cd]indol-1(2H)-yl)piperidin-2,6-dione

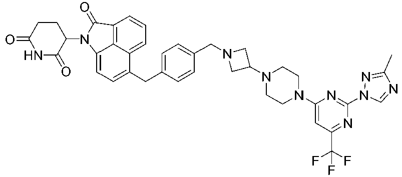

The title compound was synthesized by replacing 2-methyl-4-(methylsulfonyl)-6-(4-(trifluoromethyl)-1H-imidazol-1-yl)pyrimidine (intermediate 36) with 4-chloro-2-(3-methyl-1H-1,2,4-triazol-1-yl)-6-(trifluoromethyl)pyrimidine (intermediate 53) and replacing 3-(2-oxo-6-(4-(piperazin-1-ylmethyl)benzyl)benzo[cd]indol-1(2H)-yl)piperidin-2,6-dione (intermediate 3) with 3-(2-oxo-6-(4-((3-(piperazin-1-yl)azetidin-1-yl)methyl)benzyl)benzo[cd]indol-1(2H)-yl)piperidin-2,6-dione (intermediate 39) using a method similar to that in Example 29. ¹H NMR (400 MHz, DMSO-d6) δ 11.14 (s, 1H), 9.31 (s, 1H), 8.32 (d, J = 8.2 Hz, 1H), 8.07 (d, J = 7.0 Hz, 1H), 7.84 - 7.78 (m, 1H), 7.41 (d, J = 7.4 Hz, 1H), 7.29 (s, 1H), 7.22 (d, J = 8.1 Hz, 2H), 7.16 (d, J = 8.1 Hz, 2H), 7.11 (d, J = 7.2 Hz, 1H), 5.50 - 5.39 (m, 1H), 4.37 (s, 2H), 3.92 (s, 2H), 3.69 (s, 2H), 3.49 (s, 2H), 3.30 (d, J = 6.0 Hz, 2H), 3.00 - 2.91 (m, 1H), 2.90 - 2.71 (m, 4H), 2.66 (d, J = 10.6, 8.8 Hz, 1H), 2.36 (s, 3H), 2.33 - 2.28 (m, 4H), 2.13 - 2.04 (m, 1H); LC-MS: m/z [M+H]⁺ =751.

### Example 53 3-(6-(4-((3-(4-(6-(3-methyl-1H-1,2,4-triazol-1-yl)pyridin-2-yl)piperazin-1-yl)azetidin-1-yl)methyl)benzy l)-2-oxobenzo[cd]indol-1(2H)-yl)piperidin-2,6-dione

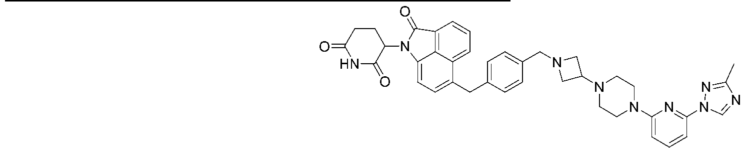

The title compound was synthesized by replacing 2-methyl-4-(methylsulfonyl)-6-(4-(trifluoromethyl)-1H-imidazol-1-yl)pyrimidine (intermediate 36) with 2-chloro-6-(3-methyl-1H-1,2,4-triazol-1-yl)pyridine (intermediate 28) and replacing 3-(2-oxo-6-(4-(piperazin-1-ylmethyl)benzyl)benzo[cd]indol-1(2H)-yl)piperidin-2,6-dione (intermediate 3) with 3-(2-oxo-6-(4-((3-(piperazin-1-yl)azetidin-1-yl)methyl)benzyl)benzo[cd]indol-1(2H)-yl)piperidin-2,6-dione (intermediate 39) using a method similar to that in Example 29. ¹H NMR (400 MHz, DMSO-d6) δ 11.11 (s, 1H), 9.17 (s, 1H), 8.32 (d, J = 8.2 Hz, 1H), 8.07 (d, J = 7.0 Hz, 1H), 7.81 (t, J = 7.7 Hz, 1H), 7.70 (t, J = 8.1 Hz, 1H), 7.41 (d, J = 7.2 Hz, 1H), 7.23 (d, J = 8.2 Hz, 2H), 7.18 (d, J = 8.0 Hz, 2H), 7.10 (d, J = 7.3 Hz, 1H), 6.97 (d, J = 7.5 Hz, 1H), 6.80 (d, J = 8.7 Hz, 1H), 5.51 - 5.36 (m, 1H), 4.37 (s, 2H), 3.55 (s, 6H), 3.37 (s, 2H), 2.97 (d, J = 12.2 Hz, 1H), 2.91 (s, 3H), 2.79 - 2.71 (m, 1H), 2.67 (s, 1H), 2.35 (s, 3H), 2.31 (s, 4H), 2.15 - 2.03 (m, 1H); LC-MS: m/z [M+H]⁺ =682.

### Example 54 3-(6-(4-((3-(4-(2-methyl-6-(4-(trifluoromethyl)-1H-imidazol-1-yl)pyrimidin-4-yl)piperazin-1-yl)azetidin -1-yl)methyl)benzyl)-2-oxobenzo[cd]indol-1(2H)-yl)piperidin-2,6-dione

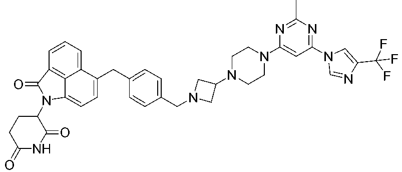

The title compound was synthesized by replacing 3-(2-oxo-6-(4-(piperazin-1-ylmethyl)benzyl)benzo[cd]indol-1(2H)-yl)piperidin-2,6-dione (intermediate 3) with 3-(2-oxo-6-(4-((3-(piperazin-1-yl)azetidin-1-yl)methyl)benzyl)benzo[cd]indol-1(2H)-yl)piperidin-2,6-dione (intermediate 39) using a method similar to that in Example 29. ¹H NMR (400 MHz, DMSO-d6) δ 11.11 (s, 1H), 8.71 (s, 1H), 8.63 (s, 1H), 8.32 (d, J = 8.2 Hz, 1H), 8.07 (d, J = 7.0 Hz, 1H), 7.81 (t, 1H), 7.41 (d, J = 7.4 Hz, 1H), 7.22 (d, J = 8.0 Hz, 2H), 7.16 (d, J = 8.0 Hz, 2H), 7.10 (d, J = 7.3 Hz, 1H), 7.05 (s, 1H), 5.44 (dd, J = 13.0, 5.5 Hz, 1H), 4.37 (s, 2H), 3.70 (s, 4H), 3.51 (s, 2H), 3.40 (s, 1H), 3.31 (s, 1H), 3.01 - 2.92 (m, 1H), 2.90 - 2.82 (m, 3H), 2.81 - 2.73 (m, 1H), 2.67 (d, J = 1.3 Hz, 1H), 2.41 (s, 3H), 2.29 (s, 4H), 2.13 - 2.05 (m, 1H); LC-MS: m/z [M+H]⁺ =750.

### Example 55 3-(6-(4-((3-((4-(6-(1H-imidazol-1-yl)pyridin-2-yl)piperazin-1-yl)methyl)azetidin-1-yl)methyl)benzyl)-2-oxobenzo[cd]indol-1(2H)-yl)piperidin-2,6-dione

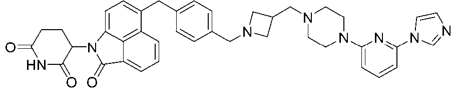

The title compound was synthesized by replacing 3-(2-oxo-6-(4-((4-(piperidin-4-ylmethyl)piperazin-1-yl)methyl)benzyl)benzo[cd]indol-1 (2H)-yl)piperidin-2, 6-dione (intermediate 12) with 3-(2-oxo-6-(4-((3-(piperazin-1-ylmethyl)azetidin-1-yl)methyl)benzyl)benzo[cd]indol-1 (2H)-yl)piperidin-2,6-dione (intermediate 55) using a method similar to that in Example 8. ¹H NMR (500 MHz, CDCl₃) δ 8.31 (s, 1H), 8.11 (d, J = 8.3 Hz, 1H), 8.07 (d, J = 7.0 Hz, 1H), 7.68 (m, 1H), 7.53 (t, J = 8.0 Hz, 2H), 7.17 (m, 7H), 6.68 (d, J = 7.3 Hz, 1H), 6.58 (d, J = 7.6 Hz, 1H), 6.47 (d, J = 8.5 Hz, 1H), 5.32 (dd, J = 12.7, 5.2 Hz, 1H), 4.35 (t, J = 11.9 Hz, 2H), 3.54 (m, 8H), 2.89 (m, 4H), 2.75 (m, 2H), 2.58 (d, J = 6.7 Hz, 2H), 2.46 (m, 4H), 2.25 (m, 1H); LC-MS: m/z [M+H]⁺ =681.

### Example 56 3-(2-oxo-6-(4-((4-(6-(trifluoromethyl)-2-(4-(trifluoromethyl)-1H-imidazol-1-yl)pyrimidin-4-yl)piperazi n-1-yl)methyl)benzyl)benzo[cd]indol-1(2H)-yl)piperidin-2,6-dione

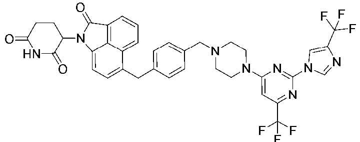

The title compound was synthesized by replacing 4-(methylsulfonyl)-2-(4-(trifluoromethyl)-1H-imidazol-1-yl)pyrimidine (intermediate 30) with 4-(methylsulfonyl)-6-(trifluoromethyl)-2-(4-(trifluoromethyl)-1H-imidazol-1-yl)pyrimidine (intermediate 57) using a method similar to that in Example 25. ¹H NMR (500 MHz, CDCl₃) δ 8.86 (s, 1H), 8.49 (s, 1H), 8.11 (d, J = 1.4 Hz, 1H), 8.09 (d, J = 8.3 Hz, 1H), 8.06 (d, J = 7.0 Hz, 1H), 7.70 - 7.64 (m, 1H), 7.22 - 7.20 (m, 3H), 7.17 (d, J = 7.9 Hz, 2H), 6.70 (d, J = 8.5 Hz, 2H), 5.33 (dd, J = 13.0, 5.4 Hz, 1H), 4.35 (s, 2H), 3.93 (s, 2H), 3.52 (s, 4H), 2.95 - 2.83 (m, 2H), 2.76 - 2.66 (m, 1H), 2.53 (t, J = 5.1 Hz, 4H), 2.28 - 2.21 (m, 1H); LC-MS: m/z [M+H]⁺ =749.

### Example 57 (S)-1-(6-(4-(4-(((2-(2,6-dioxopiperidin-3-yl)-1-oxoisoindolin-4-yl)oxy)methyl)benzyl)piperazin-1-yl)pyri din-2-yl)-1H-imidazol-4-carboxamide

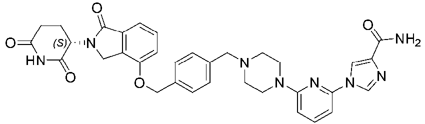

The title compound was synthesized by replacing 5-(6-(piperazin-1-yl)pyridin-2-yl)thiazole hydrochloride (intermediate 24) with 1-(6-(piperazin-1-yl)pyridin-2-yl)-1H-imidazol-4-carboxamide (intermediate 58) using a method similar to that in Example 19. ¹H NMR (400 MHz, DMSO-d6) δ 10.97 (s, 1H), 8.51 (s, 1H), 8.31 (s, 1H), 7.67 (t, J = 8.1 Hz, 1H), 7.48 - 7.43 (m, 4H), 7.36 - 7.30 (m, 4H), 7.23 (s, 1H), 7.06 (d, J = 7.6 Hz, 1H), 6.76 (d, J = 8.7 Hz, 1H), 5.21 (s, 2H), 5.09 (dd, J = 13.6, 4.9 Hz, 1H), 4.40 (d, J = 17.7 Hz, 1H), 4.23 (d, J = 17.5 Hz, 1H), 3.54 (s, 5H), 2.93 - 2.84 (m, 1H), 2.57 - 2.37 (m, 7H), 1.98 - 1.92 (m, 1H); LC-MS: m/z [M+H]⁺ =635.

### Example 58 3-(2-oxo-6-(4-((4-(2-(thiazol-5-yl)pyrimidin-4-yl)piperazin-1-yl)methyl)benzyl)benzo[cd]indol-1(2H)-yl )piperidin-2,6-dione

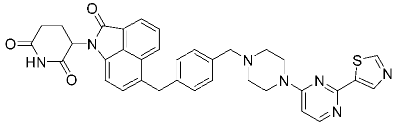

The title compound was synthesized by replacing 2-methyl-4-(methylsulfonyl)-6-(4-(trifluoromethyl)-1H-imidazol-1-yl)pyrimidine (intermediate 36) with 5-(4-(methylsulfonyl)pyrimidin-2-yl)thiazole (intermediate 59) using a method similar to that in Example 29. ¹H NMR (400 MHz, DMSO-d6) δ 11.12 (s, 1H), 9.14 (s, 1H), 8.50 (s, 1H), 8.34 (d, J = 8.4 Hz, 1H), 8.20 (d, J = 6.0 Hz, 1H), 8.08 (d, J = 6.4 Hz, 1H), 7.84 - 7.78 (m, 1H), 7.42 (d, J = 6.4 Hz, 1H), 7.26 (d, J = 8.0 Hz, 2H), 7.23 (d, J = 8.0 Hz, 2H), 7.11 (d, J = 7.6 Hz, 1H), 6.71 (d, J = 6.4 Hz, 1H), 5.45 (dd, J = 13.0, 5.6 Hz, 1H), 4.39 (s, 2H), 3.65 (s, 4H), 3.46 (s, 2H), 3.00 - 2.91 (m, 1H), 2.81 - 2.66 (m, 2H), 2.40 (t, J = 5.1 Hz, 4H), 2.13 - 2.06 (m, 1H); LC-MS: m/z [M+H]⁺ =630.

### Example 59 3-(2-oxo-6-(4-((4-(2-(thiazol-5-yl)-6-(trifluoromethyl)pyrimidin-4-yl)piperazin-1-yl)methyl)benzyl)benz o[cd]indol-1(2H)-yl)piperidin-2,6-dione

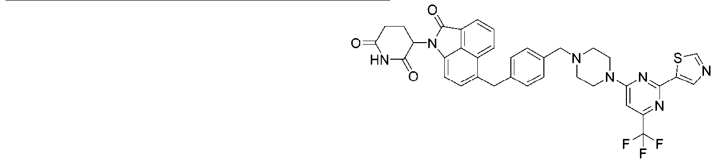

The title compound was synthesized by replacing 2-methyl-4-(methylsulfonyl)-6-(4-(trifluoromethyl)-1H-imidazol-1-yl)pyrimidine (intermediate 36) with 5-(4-(methylsulfonyl)-6-(trifluoromethyl)pyrimidin-2-yl)thiazole (intermediate 60) using a method similar to that in Example 29. ¹H NMR (400 MHz, DMSO-d6) δ 11.12 (s, 1H), 9.14 (s, 1H), 8.50 (s, 1H), 8.34 (d, J = 8.4 Hz, 1H), 8.20 (d, J = 6.0 Hz, 1H), 8.08 (d, J = 6.4 Hz, 1H), 7.84 - 7.78 (m, 1H), 7.42 (d, J = 6.4 Hz, 1H), 7.26 (d, J = 8.0 Hz, 2H), 7.23 (d, J = 8.0 Hz, 2H), 7.11 (d, J = 7.6 Hz, 1H), 6.71 (d, J = 6.4 Hz, 1H), 5.45 (dd, J = 13.0, 5.6 Hz, 1H), 4.39 (s, 2H), 3.65 (s, 4H), 3.46 (s, 2H), 3.00 - 2.91 (m, 1H), 2.81 - 2.66 (m, 2H), 2.40 (t, J = 5.1 Hz, 4H), 2.13 - 2.06 (m, 1H); LC-MS: m/z [M+H]⁺ =630.

### Example 60 3-(6-(4-((4-(5-fluoro-2-(thiazol-5-yl)pyrimidin-4-yl)piperazin-1-yl)methyl)benzyl)-2-oxobenzo[cd]indol-1(2H)-yl)piperidin-2,6-dione

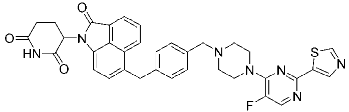

The title compound was synthesized by replacing 2-methyl-4-(methylsulfonyl)-6-(4-(trifluoromethyl)-1H-imidazol-1-yl)pyrimidine (intermediate 36) with 5-(5-fluoro-4-(methylsulfonyl)pyrimidin-2-yl)thiazole (intermediate 61) using a method similar to that in Example 29. ¹H NMR (400 MHz, DMSO-d6) δ 11.12 (s, 1H), 9.14 (s, 1H), 8.50 (s, 1H), 8.34 (d, J = 8.4 Hz, 1H), 8.20 (d, J = 6.0 Hz, 1H), 8.08 (d, J = 6.4 Hz, 1H), 7.84 - 7.78 (m, 1H), 7.42 (d, J = 6.4 Hz, 1H), 7.26 (d, J = 8.0 Hz, 2H), 7.23 (d, J = 8.0 Hz, 2H), 7.11 (d, J = 7.6 Hz, 1H), 6.71 (d, J = 6.4 Hz, 1H), 5.45 (dd, J = 13.0, 5.6 Hz, 1H), 4.39 (s, 2H), 3.65 (s, 4H), 3.46 (s, 2H), 3.00 - 2.91 (m, 1H), 2.81 - 2.66 (m, 2H), 2.40 (t, J = 5.1 Hz, 4H), 2.13 - 2.06 (m, 1H); LC-MS: m/z [M+H]⁺ =630.

### Example 61 3-(6-(4-((4-(2-(1-methyl-2-(trifluoromethyl)-1H-imidazol-5-yl)pyrimidin-4-yl)piperazin-1-yl)methyl)be nzyl)-2-oxobenzo[cd]indol-1(2H)-yl)piperidin-2,6-dione

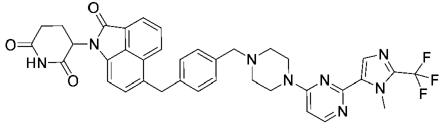

The title compound was synthesized by replacing 2-methyl-4-(methylsulfonyl)-6-(4-(trifluoromethyl)-1H-imidazol-1-yl)pyrimidine (intermediate 36) with 2-(1-methyl-2-(trifluoromethyl)-1H-imidazol-5-yl)-4-(methylsulfonyl)pyrimidine (intermediate 63) using a method similar to that in Example 29. ¹H NMR (400 MHz, DMSO-d6) δ 11.14 (s, 1H), 8.34 (d, J = 8.3 Hz, 1H), 8.26 (d, J = 6.2 Hz, 1H), 8.08 (d, J = 7.0 Hz, 1H), 7.81 (dd, J = 8.2, 7.0 Hz, 1H), 7.71 (s, 1H), 7.42 (d, J = 7.4 Hz, 1H), 7.25 (q, J = 8.0 Hz, 4H), 7.11 (d, J = 7.3 Hz, 1H), 6.75 (d, J = 6.3 Hz, 1H), 5.45 (dd, J = 12.8, 5.4 Hz, 1H), 4.39 (s, 2H), 4.12 (s, 3H), 3.63 (s, 4H), 3.45 (s, 2H), 3.01 - 2.90 (m, 1H), 2.83 - 2.70 (m, 1H), 2.69 - 2.61 (m, 1H), 2.40 (t, J = 5.0 Hz, 4H), 2.14 - 2.05 (m, 1H); LC-MS: m/z [M+H]⁺ =695.

### Example 62 3-(2-oxo-6-(4-((4-(6-(3-(trifluoromethyl)-1H-1,2,4-triazol-1-yl)pyridin-2-yl)piperazin-1-yl)methyl)benzy l)benzo[cd]indol-1(2H)-yl)piperidin-2,6-dione

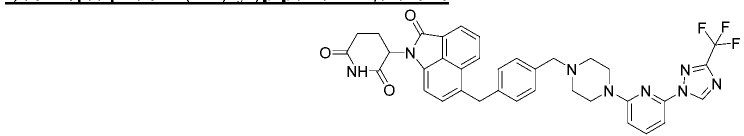

The title compound was synthesized by replacing 2-fluoro-6-(1H-imidazol-1-yl)pyridine (intermediate 4) with 2-fluoro-6-(3-(trifluoromethyl)-1H-1,2,4-triazol-1-yl)pyridine (intermediate 64) using a method similar to that in Example 2. ¹H NMR (500 MHz, CDCl₃) δ 9.04 (s, 1H), 8.76 (s, 1H), 8.10 (d, J = 8.3 Hz, 1H), 8.07 (d, J = 7.0 Hz, 1H), 7.67 (t, J = 7.6 Hz, 1H), 7.60 (t, J = 8.0 Hz, 1H), 7.24 (d, J = 8.0 Hz, 2H), 7.21 (d, J = 7.3 Hz, 1H), 7.17 (d, J = 7.8 Hz, 2H), 7.13 (d, J = 7.6 Hz, 1H), 6.70 (d, J = 7.3 Hz, 1H), 6.60 (d, J = 8.5 Hz, 1H), 5.33 (dd, J = 12.9, 5.4 Hz, 1H), 4.35 (s, 2H), 3.58 (t, J = 5.1 Hz, 4H), 3.52 (s, 2H), 2.91 - 2.84 (m, 2H), 2.71 (qd, J = 13.1, 4.9 Hz, 1H), 2.55 (q, J = 5.2, 4.4 Hz, 4H), 2.27 - 2.23 (m, 1H); LC-MS: m/z [M+H]⁺ =681.

### Example 63 3-(2-oxo-6-(4-((4-(6-(5-(trifluoromethyl)-1,3,4-thiadiazol-2-yl)pyridin-2-yl)piperazin-1-yl)methyl)benzy l)benzo[cd]indol-1(2H)-yl)piperidin-2,6-dione

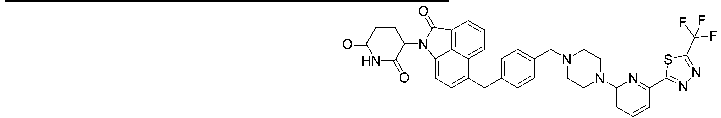

The title compound was synthesized by replacing 2-fluoro-6-(1H-imidazol-1-yl)pyridine (intermediate 4) with 2-(6-fluoropyridin-2-yl)-5-(trifluoromethyl)-1,3,4-thiadiazole (intermediate 65) using a method similar to that in Example 2. ¹H NMR (500 MHz, CDCl₃) δ 8.42 (s, 1H), 8.12 (d, J = 8.3 Hz, 1H), 8.08 (d, J = 7.0 Hz, 1H), 7.73-7.67 (m, 1H), 7.64 (d, J = 7.2 Hz, 1H), 7.60 (t, J = 7.8 Hz, 1H), 7.26-7.21 (m, 3H), 7.17 (d, J = 7.9 Hz, 2H), 6.75 (d, J = 8.3 Hz, 1H), 6.70 (d, J = 7.2 Hz, 1H), 5.33 (dd, J = 12.9, 5.3 Hz, 1H), 4.37 (s, 2H), 3.64-3.54 (m, 4H), 3.52 (s, 2H), 3.03 - 2.80 (m, 2H), 2.79 - 2.63 (m, 1H), 2.59 - 2.49 (m, 4H), 2.34 - 2.21 (m, 1H); LC-MS: m/z [M+H]⁺ =698.

### Example 64 3-(2-oxo-6-(4-((4-(2-(pyridin-3-yl)pyrimidin-4-yl)piperazin-1-yl)methyl)benzyl)benzo[cd]indol-1(2H)-yl )piperidin-2,6-dione

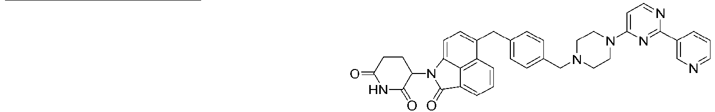

The title compound was synthesized by replacing 4-(methylsulfonyl)-2-(4-(trifluoromethyl)-1H-imidazol-1-yl)pyrimidine (intermediate 30) with 4-(methylsulfonyl)-2-(pyridin-3-yl)pyrimidine (intermediate 66) using a method similar to that in Example 25. ¹H NMR (500 MHz, CDCl₃) δ 9.53 (s, 1H), 8.66 (s, 1H), 8.59 (d, J = 8.1 Hz, 1H), 8.29 (d, J = 6.2 Hz, 2H), 8.11 (dd, J = 18.3, 7.6 Hz, 2H), 7.76-7.66 (m, 1H), 7.39-7.33 (m, 1H), 7.24-7.13 (m, 5H), 6.70 (d, J = 7.1 Hz, 1H), 6.42 (d, J = 6.2 Hz, 1H), 5.33 (dd, J = 13.0, 5.5 Hz, 1H), 4.38 (s, 2H), 3.73 (s, 4H), 3.53 (s, 2H), 3.02-2.93 (m, 1H), 2.92 - 2.83 (m, 1H), 2.81-2.66 (m, 1H), 2.53 (d, J = 7.3 Hz, 4H), 2.36-2.21 (m, 1H); LC-MS: m/z [M+H]⁺ =624.

### Example 65 3-(6-(4-((4-(2-(3,5-dimethylisoxazol-4-yl)pyrimidin-4-yl)piperazin-1-yl)methyl)benzyl)-2-oxobenzo[cd]i ndol-1(2H)-yl)piperidin-2,6-dione

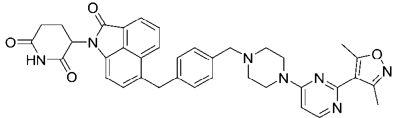

The title compound was synthesized by replacing 4-(methylsulfonyl)-2-(4-(trifluoromethyl)-1H-imidazol-1-yl)pyrimidine (intermediate 30) with 3,5-dimethyl-4-(4-(methylsulfonyl)pyrimidin-2-yl)isoxazole (intermediate 67) using a method similar to that in Example 25. ¹H NMR (500 MHz, CDCl₃) δ 8.48 (s, 1H), 8.23 (d, J = 6.2 Hz, 1H), 8.09 (dd, J = 12.6, 6.9 Hz, 2H), 7.73 - 7.70 (m, 1H), 7.24 (d, J = 6.9 Hz, 3H), 7.18 (d, J = 8.0 Hz, 2H), 6.71 (d, J = 7.3 Hz, 1H), 6.39 (d, J = 6.7 Hz, 1H), 6.33 (d, J = 6.2 Hz, 1H), 5.34 (dd, J = 12.9, 5.4 Hz, 1H), 4.38 (s, 2H), 3.65 (s, 3H), 3.52 (s, 2H), 2.99-2.92 (m, 1H), 2.91 - 2.84 (m, 1H), 2.75 (s, 1H), 2.72 (s, 3H), 2.53 (s, 3H), 2.50 (s, 4H), 2.32-2.27(m, 1H); LC-MS: m/z [M+H]⁺ =642.

### Example 66 (S)-3-(4-((4-((4-(6-(3-methyl-1H-1,2,4-triazol-1-yl)pyridin-2-yl)piperazin-1-yl)methyl)benzyl)oxy)-1-ox oisoindolin-2-yl)piperidin-2,6-dione

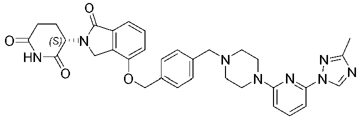

The title compound was synthesized by replacing 4-(4-(4-(chloromethyl)benzyl)piperazin-1-yl)-2-(1H-imidazol-1-yl)pyrimidine (intermediate 2) with 1-(4-(chloromethyl)benzyl)-4-(6-(3-methyl-1H-1,2,4-triazol-1-yl)pyridin-2-yl)piperazine (intermediate 68) using a method similar to that in Example 1. ¹H NMR (400 MHz, DMSO-d6) δ 10.98 (s, 1H), 9.29 (s, 1H), 7.82 (dd, J = 8.5, 7.6 Hz, 1H), 7.62 (d, J = 8.0 Hz, 2H), 7.56 (d, J = 8.1 Hz, 2H), 7.50 (t, J = 7.8 Hz, 1H), 7.35 (dd, J = 7.8, 6.0 Hz, 2H), 7.09 (d, J = 7.7 Hz, 1H), 6.91 (d, J = 8.5 Hz, 1H), 5.33 (s, 2H), 5.13 (dd, J = 13.3, 5.1 Hz, 1H), 4.56 (d, J = 13.7 Hz, 2H), 4.48 - 4.37 (m, 3H), 4.29 (d, J = 17.4 Hz, 1H), 3.41 (d, J = 11.6 Hz, 2H), 3.22 (d, J = 13.7 Hz, 2H), 3.13 (s, 2H), 2.93 (ddd, J = 17.4, 13.6, 5.4 Hz, 1H), 2.64 - 2.55 (m, 1H), 2.44 (dd, J = 13.2, 4.5 Hz, 1H), 2.37 (s, 3H), 2.05 - 1.97 (m, 1H); LC-MS: m/z [M+H]⁺ =607.

### Example 67 3-(6-(4-((4-(5-fluoro-2-(2-methylthiazol-5-yl)pyrimidin-4-yl)piperazin-1-yl)methyl)benzyl)-2-oxobenzo[ cdlindol-1(2H)-yl)piperidin-2,6-dione

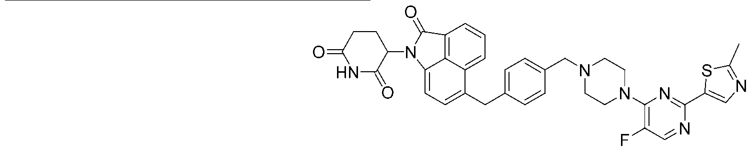

The title compound was synthesized by replacing 2-(5-fluoro-4-(piperazin-1-yl)pyrimidin-2-yl)-5-methyl-1,3,4-thiadiazole (intermediate 44) with 5-(5-fluoro-4-(piperazin-1-yl)pyrimidin-2-yl)-2-methylthiazole (intermediate 69) using a method similar to that in Example 42. ¹H NMR (500 MHz, CDCl₃) δ 8.40 (s, 1H), 8.19 (s, H), 8.12 (d, J = 8.3 Hz, 1H), 8.09 (d, J = 7.0 Hz, 1H), 8.00 (d, J = 6.6 Hz, 1H), 7.70 (t, 1H), 7.23 (t, 3H), 7.17 (d, J = 7.9 Hz, 2H), 6.70 (d, J = 7.3 Hz, 1H), 5.33 (dd, J = 13.0, 5.4 Hz, 1H), 4.37 (s, 2H), 3.82 (s, 4H), 3.50 (s, 2H), 2.98 - 2.93 (m, 1H), 2.86 (ddd, J = 17.5, 13.3, 5.0 Hz, 1H), 2.77 - 2.72 (m, 1H), 2.70 (s, 3H), 2.52 (s, 4H), 2.30 - 2.25 (m, 1H); LC-MS: m/z [M+H]⁺ = 662.

### Example 68 3-(6-(4-((4-(5-fluoro-2-(3-methyl-1H-1,2,4-triazol-1-yl)pyrimidin-4-yl)piperazin-1-yl)methyl)benzyl)-2-oxobenzo[cd]indol-1(2H)-yl)piperidin-2,6-dione

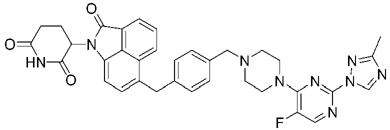

The title compound was synthesized by replacing 2-(5-fluoro-4-(piperazin-1-yl)pyrimidin-2-yl)-5-methyl-1,3,4-thiadiazole (intermediate 44) with 5-fluoro-2-(3-methyl-1H-1,2,4-triazol-1-yl)-4-(piperazin-1-yl)pyrimidine (intermediate 49) using a method similar to that in Example 42. ¹H NMR (500 MHz, CDCl₃) δ 8.85 (s, 1H), 8.25 (s, 1H), 8.10 (dd, J = 15.4, 7.6 Hz, 2H), 8.05 (d, J = 6.1 Hz, 1H), 7.70 (t, 1H), 7.22 (d, J = 8.8 Hz, 3H), 7.17 (d, J = 7.9 Hz, 2H), 6.69 (d, J = 7.2 Hz, 1H), 5.33 (dd, J = 12.9, 5.4 Hz, 1H), 4.37 (s, 2H), 3.86 (t, 4H), 3.50 (s, 2H), 2.99 - 2.94 (m, 1H), 2.90 - 2.83 (m, 1H), 2.77 - 2.69 (m, 1H), 2.53 (t, J = 4.9 Hz, 4H), 2.48 (s, 3H), 2.31 - 2.26 (m, 1H); LC-MS: m/z [M+H]⁺ = 646.

### Example 69 3-(6-(4-((4-(2-(3-methyl-1H-1,2,4-triazol-1-yl)-6-(trifluoromethyl)pyrimidin-4-yl)piperazin-1-yl)methyl )benzyl)-2-oxobenzo[cd]indol-1(2H)-yl)piperidin-2,6-dione

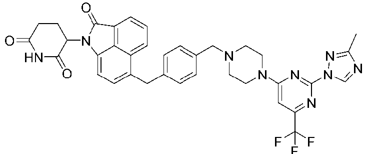

The title compound was synthesized by replacing 2-(5-fluoro-4-(piperazin-1-yl)pyrimidin-2-yl)-5-methyl-1,3,4-thiadiazole (intermediate 44) with 2-(3-methyl-1H-1,2,4-triazol-1-yl)-4-(piperazin-1-yl)-6-(trifluoromethyl)pyrimidine (intermediate 70) using a method similar to that in Example 42. ¹H NMR (500 MHz, CDCl₃) δ 9.02 (s, 1H), 8.18 (s, 1H), 8.10 (dd, *J* = 13.1, 7.7 Hz, 2H), 7.76 - 7.65 (m, 1H), 7.24 - 7.20 (m, 3H), 7.18 (d, *J* = 7.9 Hz, 2H), 6.70 (m, 2H) 5.33 (dd, *J* = 13.0, 5.4 Hz, 1H), 4.38 (s, 2H), 3.97 (s, 2H), 3.52 (s, 4H), 3.04 - 2.92 (m, 1H), 2.88 (d, *J* = 5.2 Hz, 1H), 2.79 - 2.66 (m, 1H), 2.57 - 2.45 (m, 7H), 2.32 - 2.21 (m, 1H); LC-MS: m/z [M+H]⁺ = 696.

### Example 70 3-(6-(4-((4-(2-methyl-6-(3-(trifluoromethyl)-1H-1,2,4-triazol-1-yl)pyrimidin-4-yl)piperazin-1-yl)methyl )benzyl)-2-oxobenzo[cd]indol-1(2H)-yl)piperidin-2,6-dione

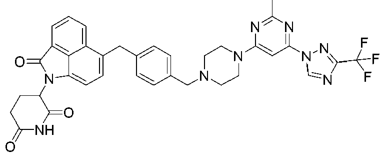

The title compound was synthesized by replacing 2-methyl-4-(methylsulfonyl)-6-(4-(trifluoromethyl)-1H-imidazol-1-yl)pyrimidine (intermediate 36) with 2-methyl-4-(methylsulfonyl)-6-(3-(trifluoromethyl)-1H-1,2,4-triazol-1-yl)pyrimidine (intermediate 71) using a method similar to that in Example 29. ¹H NMR (400 MHz, DMSO-d6) δ 11.15 (s, 1H), 9.60 (s, 1H), 8.34 (d, J = 8.3 Hz, 1H), 8.08 (d, J = 7.0 Hz, 1H), 7.81 (dd, J = 8.3, 7.0 Hz, 1H), 7.42 (d, J = 7.4 Hz, 1H), 7.25 (q, J = 7.9 Hz, 4H), 7.12 (d, J = 7.3 Hz, 1H), 6.92 (s, 1H), 5.45 (dd, J = 13.1, 5.3 Hz, 1H), 4.39 (s, 2H), 3.68 (s, 4H), 3.45 (s, 2H), 3.02 - 2.90 (m, 1H), 2.82 - 2.73 (m, 1H), 2.71 - 2.63 (m, 1H), 2.45 (s, 3H), 2.41 (d, J = 6.0 Hz, 4H), 2.15 - 2.05 (m, 1H); LC-MS: m/z [M+H]⁺ =696.

### Example 71 (S)-3-(4-((4-((4-(2-methyl-6-(3-methyl-1H-1,2,4-triazol-1-yl)pyrimidin-4-yl)piperazin-1-yl)methyl)benz yl)oxy)-1-oxoisoindolin-2-yl)piperidin-2,6-dione

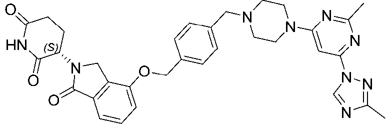

The title compound was synthesized by replacing 4-(4-(4-(chloromethyl)benzyl)piperazin-1-yl)-2-(1H-imidazol-1-yl)pyrimidine (intermediate 2) with 4-(4-(4-(chloromethyl)benzyl)piperazin-1-yl)-2-methyl-6-(3-methyl-1H-1,2,4-triazol-1-yl)pyrimidine (intermediate 72) using a method similar to that in Example 1. ¹H NMR (400 MHz, DMSO-d6) δ 10.98 (s, 1H), 9.15 (s, 1H), 7.53 - 7.42 (m, 3H), 7.40 - 7.29 (m, 4H), 6.82 (s, 1H), 5.25 (s, 2H), 5.12 (dd, J = 13.2, 5.1 Hz, 1H), 4.43 (d, J = 17.5 Hz, 1H), 4.27 (d, J = 17.5 Hz, 1H), 3.68 (s, 4H), 3.54 (s, 2H), 2.92 (ddd, J = 17.3, 13.6, 5.4 Hz, 1H), 2.63 - 2.54 (m, 1H), 2.51 - 2.37 (s, 11H), 2.03 - 1.93 (m, 1H); LC-MS: m/z [M+H]⁺ =622.

### Example 72 (S)-3-(4-((4-((4-(2-methyl-6-(4-(trifluoromethyl)-1H-imidazol-1-yl)pyrimidin-4-yl)piperazin-1-yl)methy l)benzyl)oxy)-1-oxoisoindolin-2-yl)piperidin-2,6-dione

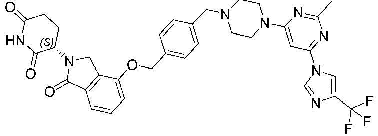

The title compound was synthesized by replacing 4-(4-(4-(chloromethyl)benzyl)piperazin-1-yl)-2-(1H-imidazol-1-yl)pyrimidine (intermediate 2) with 4-(4-(4-(chloromethyl)benzyl)piperazin-1-yl)-2-methyl-6-(4-(trifluoromethyl)-1H-imidazol-1-yl)pyrimidine (intermediate 73) using a method similar to that in Example 1. ¹H NMR (400 MHz, DMSO-d6) δ 10.97 (s, 1H), 8.72 (s, 1H), 8.64 -8.64 (m, 1H), 7.51 - 7.46 (m, 3H),7.38 -7.33 (m, 4H), 7.06 (s,1H), 5.25 (s, 2H), 5.14 - 5.10 (m, 1H), 4.45 - 4.41 (d, J = 17.5 Hz, 1H), 4.29 - 4.25 (d, J = 17.5 Hz, 1H), 3.74 (s, 4H), 3.55 (s, 2H), 2.91 - 2.89 (m, 1H), 2.60 - 2.56 (m, 1H), 2.51 - 2.50 (m, 4H), 2.47 - 2.42 (m, 4H), 2.01 - 1.98 (m, 1H); LC-MS: m/z [M+H]⁺ =675.

### Example 73 3-(6-(4-((4-(2-methyl-6-(4-(trifluoromethyl)-1H-1,2,3-triazol-1-yl)pyrimidin-4-yl)piperazin-1-yl)methyl )benzyl)-2-oxobenzo[cd]indol-1(2H)-yl)piperidin-2,6-dione

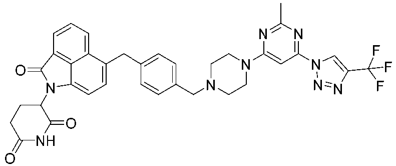

The title compound was synthesized by replacing 2-methyl-4-(methylsulfonyl)-6-(4-(trifluoromethyl)-1H-imidazol-1-yl)pyrimidine (intermediate 36) with 2-methyl-4-(methylsulfonyl)-6-(4-(trifluoromethyl)-1H-1,2,3-triazol-1-yl)pyrimidine (intermediate 74) using a method similar to that in Example 29. ¹H NMR (400 MHz, DMSO-d6) δ 11.12 (s, 1H), 8.81 (s, 1H), 8.33 (d, J = 8.4 Hz, 1H), 8.07 (d, J = 7.0 Hz, 1H), 7.81 (dd, J = 8.3, 7.0 Hz, 1H), 7.42 (d, J = 7.3 Hz, 1H), 7.30 - 7.19 (m, 5H), 7.11 (d, J = 7.3 Hz, 1H), 5.44 (dd, J = 12.9, 5.4 Hz, 1H), 4.39 (s, 2H), 3.71 (s, 4H), 3.46 (s, 2H), 2.95 (ddd, J = 17.0, 13.2, 5.2 Hz, 1H), 2.78 (td, J = 12.8, 4.2 Hz, 1H), 2.72 - 2.61 (m, 1H), 2.48 - 2.37 (m, 7H), 2.14 - 2.06 (m, 1H); LC-MS: m/z [M+H]⁺ =696.

### Example 74 3-(6-(4-((4-(2-(4-chloro-1H-imidazol-1-yl)-6-(trifluoromethyl)pyrimidin-4-yl)piperazin-1-yl)methyl)ben zyl)-2-oxobenzo[cd]indol-1(2H)-yl)piperidin-2,6-dione

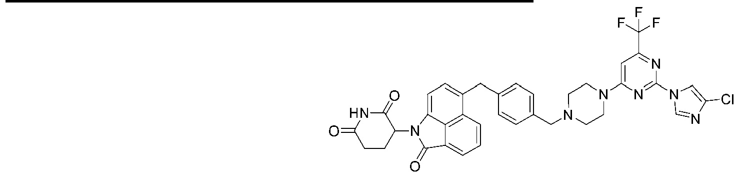

The title compound was synthesized by replacing 2-methyl-4-(methylsulfonyl)-6-(4-(trifluoromethyl)-1H-imidazol-1-yl)pyrimidine (intermediate 36) with 2-(4-chloro-1H-imidazol-1-yl)-4-(methylsulfonyl)-6-(trifluoromethyl)pyrimidine (intermediate 75) using a method similar to that in Example 29. ¹H NMR (400 MHz, DMSO-d6) δ 11.14 (s, 1H), 8.51 (d, J = 1.3 Hz, 1H), 8.34 (s, 1H), 8.08 (d, J = 7.0 Hz, 1H), 7.98 (d, J = 1.4 Hz, 1H), 7.86 - 7.72 (m, 1H), 7.42 (d, J = 7.3 Hz, 1H), 7.27 (d, J = 8.0 Hz, 2H), 7.22 (d, J = 7.8 Hz, 3H), 7.11 (d, J = 7.3 Hz, 1H), 5.45 (dd, J = 12.2, 5.1 Hz, 1H), 4.39 (s, 2H), 3.93 (s, 2H), 3.68 (s, 2H), 3.47 (s, 2H), 3.03 - 2.89 (m, 1H), 2.82 - 2.72 (m, 1H), 2.70 - 2.60 (m, 1H), 2.41 (s, 4H), 2.16 - 2.02 (m, 1H); LC-MS: m/z [M+H]⁺ =715.

### Example 75 3-(6-(4-((4-(2-(4-cyano-1H-imidazol-1-yl)-6-(trifluoromethyl)pyrimidin-4-yl)piperazin-1-yl)methyl)ben zyl)-2-oxobenzo[cd]indol-1(2H)-yl)piperidin-2,6-dione

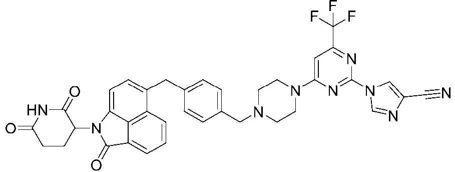

The title compound was synthesized by replacing 2-methyl-4-(methylsulfonyl)-6-(4-(trifluoromethyl)-1H-imidazol-1-yl)pyrimidine (intermediate 36) with 2-(4-cyano-1H-imidazol-1-yl)-4-(methylsulfonyl)-6-(trifluoromethyl)pyrimidine (intermediate 76) using a method similar to that in Example 29. ¹H NMR (400 MHz, DMSO-d6) δ 11.14 (s, 1H), 8.88 (s, 1H), 8.73 (s, 1H), 8.33 (d, J = 8.3 Hz, 1H), 8.08 (d, J = 7.0 Hz, 1H), 7.87 - 7.75 (m, 1H), 7.42 (d, J = 7.3 Hz, 1H), 7.33 - 7.17 (m, 5H), 7.11 (d, J = 7.3 Hz, 1H), 5.45 (dd, J = 12.6, 5.0 Hz, 1H), 4.39 (s, 2H), 3.95 (s, 2H), 3.69 (s, 2H), 3.48 (s, 2H), 3.03 - 2.89 (m, 1H), 2.82 - 2.72 (m, 1H), 2.70 - 2.60 (m, 1H), 2.42 (s, 4H), 2.16 - 2.03 (m, 1H); LC-MS: m/z [M+H]⁺ =706.

### Example 76 3-(6-(4-((4-(2-methyl-6-(4-(trifluoromethyl)-2H-1,2,3-triazol-2-yl)pyrimidin-4-yl)piperazin-1-yl)methyl )benzyl)-2-oxobenzo[cd]indol-1(2H)-yl)piperidin-2,6-dione

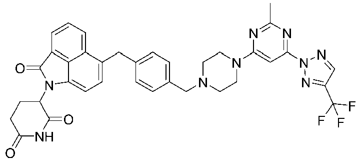

The title compound was synthesized by replacing 2-methyl-4-(methylsulfonyl)-6-(4-(trifluoromethyl)-1H-imidazol-1-yl)pyrimidine (intermediate 36) with 2-methyl-4-(methylsulfonyl)-6-(4-(trifluoromethyl)-2H-1,2,3-triazol-2-yl)pyrimidine (intermediate 77) using a method similar to that in Example 29. ¹H NMR (400 MHz, DMSO-d6) δ 11.13 (s, 1H), 9.14 (s, 1H), 8.46 (s, 1H), 8.34 (d, J = 8.0 Hz, 1H), 8.27 (d, J = 6.8 Hz, 1H), 8.08 (d, J = 6.8 Hz, 1H), 7.81 (dd, J = 8.4, 7.2 Hz, 1H), 7.42 (d, J = 7.2 Hz, 1H), 7.27 (d, J = 8.0 Hz, 2H), 7.22 (d, J = 8.0 Hz, 2H), 7.11 (d, J = 7.2 Hz, 1H), 5.45 (dd, J = 12.8, 5.2 Hz, 1H), 4.39 (s, 2H), 3.75 (t, J = 4.8 Hz, 4H), 3.45 (s, 2H), 3.01 - 2.89 (m, 1H), 2.82 - 2.63 (m, 2H), 2.46 (t, J = 4.8 Hz, 4H), 2.14 - 2.04 (m, 1H); LC-MS: m/z [M+H]⁺ =648.

### Example 77 (S)-3-(4-((4-((3-(4-(2-methyl-6-(3-methyl-1H-1,2,4-triazol-1-yl)pyrimidin-4-yl)piperazin-1-yl)azetidin-1 -yl)methyl)benzyl)oxy)-1-oxoisoindolin-2-yl)piperidin-2,6-dione

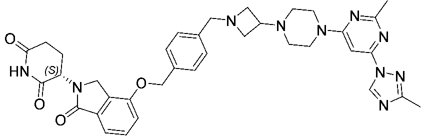

The title compound was synthesized by replacing 4-(4-(4-(chloromethyl)benzyl)piperazin-1-yl)-2-(1H-imidazol-1-yl)pyrimidine (intermediate 2) with 4-(4-(1-(4-(chloromethyl)benzyl)azetidin-3-yl)piperazin-1-yl)-2-methyl-6-(3-methyl-1H-1,2,4-triazol-1-yl)p yrimidine (intermediate 79) using a method similar to that in Example 1. ¹H NMR (400 MHz, DMSO-d6) δ 10.99 (s, 1H), 7.58 (d, J = 8.2 Hz, 2H), 7.53 (d, J = 7.8 Hz, 2H), 7.49 (s, 1H), 7.38 - 7.29 (m, 3H), 6.93 (d, J = 32.2 Hz, 1H), 5.30 (s, 2H), 5.13 (dd, J = 13.3, 5.2 Hz, 1H), 4.46 (s, 2H), 4.42 (d, J = 4.6 Hz, 3H), 4.28 (d, J = 17.4 Hz, 2H), 4.20 - 3.99 (m, 6H), 3.73 (s, 4H), 2.97 - 2.85 (m, 1H), 2.78 (s, 2H), 2.60 (d, J = 17.8 Hz, 1H), 2.45 (d, J = 7.2 Hz, 6H); LC-MS: m/z [M+H]⁺ =677.

### Example 78 (S)-3-(4-((4-((3-(4-(2-methyl-6-(4-(trifluoromethyl)-1H-imidazol-1-yl)pyrimidin-4-yl)piperazin-1-yl)aze tidin-1-yl)methyl)benzyl)oxy)-1-oxoisoindolin-2-yl)piperidin-2,6-dione

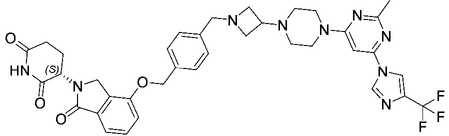

The title compound was synthesized by replacing 4-(4-(4-(chloromethyl)benzyl)piperazin-1-yl)-2-(1H-imidazol-1-yl)pyrimidine (intermediate 2) with 4-(4-(1-(4-(chloromethyl)benzyl)azetidin-3-yl)piperazin-1-yl)-2-methyl-6-(4-(trifluoromethyl)-1H-imidazol-1-yl)pyrimidine (intermediate 81) using a method similar to that in Example 1. ¹H NMR (400 MHz, DMSO-d6) δ 10.99 (s, 1 H), 8.74 (s, 1 H), 8.66 (s, 1 H), 7.71 - 7.43 (m, 5 H), 7.40 - 7.25 (m, 2 H), 7.11 (s, 1 H), 5.30 (s, 2 H), 5.13 (dd, J = 5.1, 13.2 Hz, 1 H), 4.52 - 4.35 (m, 3 H), 4.28 (d, J = 17.6 Hz, 1 H), 4.17 - 4.05 (m, 5 H), 3.76 (br. s., 4 H), 3.31 (d, J = 6.8 Hz, 1 H), 3.03 - 2.85 (m, 1 H), 2.59 (d, J = 16.9 Hz, 1 H), 2.50 - 2.38 (m, 7 H), 2.08 - 1.94 (m, 1 H); LC-MS: m/z [M+H]⁺ =730.

### Example 79 3-(6-(4-((4-(6-(1-isopropyl-4,5-dihydro-1H-imidazol-2-yl)pyridin-2-yl)piperazin-1-yl)methyl)benzyl)-2-oxobenzo[cd]indol-1(2H)-yl)piperidin-2,6-dione

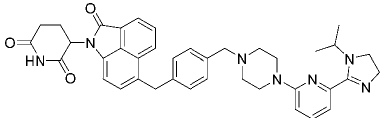

The title compound was synthesized by replacing 2-(6-chloropyridin-2-yl)-5-methyl-1,3,4-thiadiazole (intermediate 27) with 2-chloro-6-(1-isopropyl-4,5-dihydro-1H-imidazol-2-yl)pyridine (intermediate 82) using a method similar to that in Example 22. ¹H NMR (400 MHz, DMSO-d6) δ 11.13 (s, 1H), 10.43 (s, 1H), 10.23 (s, 1H), 8.35 (d, J = 8.3 Hz, 1H), 8.09 (d, J = 7.0 Hz, 1H), 7.93 - 7.85 (m, 1H), 7.82 (dd, J = 8.3, 7.0 Hz, 1H), 7.50 - 7.34 (m, 5H), 7.26 (d, J = 8.6 Hz, 1H), 7.20 - 7.09 (m, 2H), 5.45 (dd, J = 13.0, 5.4 Hz, 1H), 4.60 (s, 1H), 4.43 (s, 2H), 4.31 (br. s., 4H), 4.05 (dd, J = 12.1, 8.5 Hz, 2H), 3.93 (dd, J = 12.5, 8.8 Hz, 2H), 3.40 (br. s., 2H), 3.21 (br. s., 2H), 3.05 (s, 2H), 3.03 - 2.89 (m, 1H), 2.83 - 2.71 (m, 1H), 2.67 - 2.63 (m, 1H), 2.13 - 2.06 (m, 1H), 1.22 (d, J = 6.7 Hz, 6H); LC-MS: m/z [M+H]⁺ =656.

### Example 80 (S)-3-(1-oxo-4-((4-((4-(2-(thiazol-5-yl)pyrimidin-4-yl)piperazin-1-yl)methyl)benzyl)oxy)isoindolin-2-yl) piperidin-2,6-dione

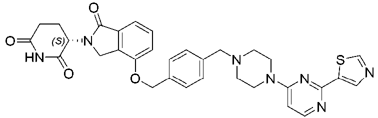

The title compound was synthesized by replacing 5-(6-(piperazin-1-yl)pyridin-2-yl)thiazole hydrochloride (intermediate 24) with 5-(4-(piperazin-1-yl)pyrimidin-2-yl)thiazole hydrochloride (intermediate 83) using a method similar to that in Example 19. ¹H NMR (400 MHz, CDCl₃) δ 8.82 (s, 1H), 8.59 (s, 1H), 8.32 (s, 1H), 8.21 (d, J = 6.2 Hz, 1H), 7.51 (d, J = 7.5 Hz, 1H), 7.41 (d, J = 19.0 Hz, 5H), 7.10 (d, J = 8.0 Hz, 1H), 6.37 (d, J = 6.3 Hz, 1H), 5.23 (dd, J = 13.2, 5.1 Hz, 1H), 5.15 (s, 2H), 4.47 (d, J = 16.5 Hz, 1H), 4.33 (d, J = 16.5 Hz, 1H), 3.72 (s, 4H), 3.59 (s, 2H), 2.93 - 2.78 (m, 2H), 2.56 (s, 4H), 2.35 (qd, J = 13.0, 5.1 Hz, 1H), 2.23 - 2.19 (m, 1H); LC-MS: m/z [M+H]⁺ =610.

### Example 81 (S)-3-(4-((4-((3-(4-(5-fluoro-2-(3-methyl-1H-1,2,4-triazol-1-yl)pyrimidin-4-yl)piperazin-1-yl)azetidin-1-yl)methyl)benzyl)oxy)-1-oxoisoindolin-2-yl)piperidin-2,6-dione

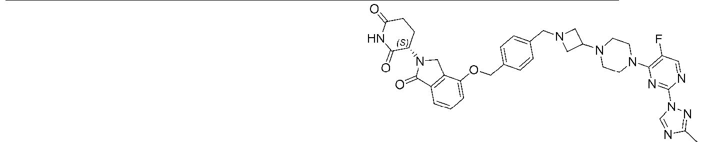

The title compound was synthesized by replacing 4-(4-(4-(chloromethyl)benzyl)piperazin-1-yl)-2-(1H-imidazol-1-yl)pyrimidine (intermediate 2) with 4-(4-(1-(4-(chloromethyl)benzyl)azetidin-3-yl)piperazin-1-yl)-5-fluoro-2-(3-methyl-1H-1,2,4-triazol-1-yl)py rimidine (intermediate 84) using a method similar to that in Example 1. ¹H NMR (400 MHz, DMSO-d6) δ 11.15 - 10.87 (m, 1H), 9.27 - 9.08 (m, 1H), 8.36 (t, J = 8.3 Hz, 1H), 7.84 - 7.45 (m, 2H), 7.45 - 7.26 (m, 3H), 7.26 - 7.08 (m, 1H), 6.98 (s, 1H), 5.11 (d, J = 13.8 Hz, 2H), 4.42 - 4.17 (m, 3H), 4.06 (d, J = 13.0 Hz, 2H), 3.94 (d, J = 17.4 Hz, 4H), 3.82 (s, 9H), 3.09 (s, 2H), 2.94 (d, J = 12.4 Hz, 1H), 2.40 - 2.21 (m, 3H), 2.08 - 1.90 (m, 1H); LC-MS: m/z [M+H]⁺ =681.

### Example 82 3-(6-(4-((4-(2-methyl-6-(4-(trifluoromethyl)-1H-imidazol-1-yl)pyrimidin-4-yl)piperazin-1-yl)methyl-d2 )benzyl)-2-oxobenzo[cd]indol-1(2H)-yl)piperidin-2,6-dione

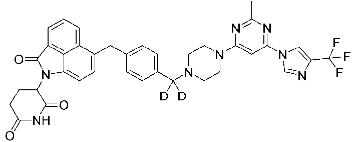

The title compound was synthesized by replacing 3-(2-oxo-6-(4-(piperazin-1-ylmethyl)benzyl)benzo[cd]indol-1(2H)-yl)piperidin-2,6-dione (intermediate 3) with 3-(2-oxo-6-(4-(piperazin-1-ylmethyl-d2)benzyl)benzo[cd]indol-1(2H)-yl)piperidin-2,6-dione (intermediate 85) using a method similar to that in Example 29. ¹H NMR (400 MHz, DMSO-d6) δ 11.15 (s, 1H), 8.71 (s, 1H), 8.63 (s, 1H), 8.32 (d, J = 8.2 Hz, 1H), 8.08 (d, J = 7.0 Hz, 1H), 7.81 (dd, J = 8.2, 7.2 Hz, 1H), 7.42 (d, J = 7.2 Hz, 1H), 7.24 (q, J = 8.2 Hz, 4H), 7.11 (d, J = 7.2 Hz, 1H), 7.03 (s, 1H), 5.45 (dd, J = 12.8, 5.4 Hz, 1H), 4.39 (s, 2H), 3.69 (s, 4H), 3.01 - 2.90 (m, 1H), 2.76 (tt, J = 17.2, 8.6 Hz, 1H), 2.65 (d, J = 16.8 Hz, 1H), 2.44 - 2.36 (m, 7H), 2.12 - 2.06 (m, 1H); LC-MS: m/z [M+H]⁺ =697.

### Biological activity test examples

### Test Example 1. Cell Titer-GLO (CTG) assay for proliferation of NCI-H929 cells

1. Experimental objective: To determine inhibitory activities of compounds on NCI-H929 multiple myeloma cells.
2. Experimental method:
   NCI-H929 cells, purchased from Nanjing Cobioer Biotechnology Co., Ltd, were cultured in RPMI1640 (Viva; C3010-0500) medium containing 10% fetal bovine serum (Viva, C04002-500) and 1% of penicillin-streptomycin at 37°C with 5% of CO₂ and saturated humidity conditions.

The cells in logarithmic growth phase were plated at 5000 cells per well of a 96-well plate and after overnight incubation, compounds at 0.00512 nM, 0.0256 nM, 0.128 nM, 0.64 nM, 3.2 nM, 16 nM, 80 nM, and 400 nM were dosed into the plate with each dose in 2 duplicate wells. Cells were incubated at 37°C, 5% CO₂ for 72 hours. After the incubation, the plate was placed at room temperature for half an hour, and then 50 µL of Cell Titer-Glo^{®} (Promega, G7573) was added. The plate was placed on a shaker for uniform mixing for 2 minutes and was placed in the dark at room temperature for 10 minutes. The luminance signal was measured using SpectraMax Paradigm (Molecular Devices).

Cell activity inhibition rate (%) = 100-(RLU_{compound}-RLU_{blank})/(RLU_{control}-RLU_{blank})×100%, where control refers to the 0.1% DMSO treatment group, and blank refers to the culture medium control. IC50 values and maximum inhibition rates of the compounds were calculated using a nonlinear regression function "Dose-Response-Inhibition" in Graphpad Prism 7.0.

**Table 1: In vitro IC50 of compounds on NCI-H929 multiple myeloma cells**

| **Example No.** | **H929 IC50 (nM)** | **Example No.** | **H929 IC50 (nM)** | **Example No.** | **H929 IC50 (nM)** |
|---|---|---|---|---|---|
| **1** | 0.03 | **29** | 0.02 | **55** | 0.2 |
| **2** | 0.02 | **30** | 0.01 | **56** | 0.1 |
| **3** | 0.003 | **31** | 0.01 | **57** | 0.1 |
| **4** | 0.03 | **32** | <0.005 | **58** | 0.03 |
| **5** | 0.06 | **33** | 0.02 | **59** | 0.2 |
| **6** | 0.07 | **34** | 0.03 | **60** | 0.03 |
| **7** | 0.05 | **35** | 0.01 | **61** | 0.02 |
| **8** | 0.04 | **36** | 0.02 | **62** | 0.07 |
| **9** | 0.07 | **37** | 0.01 | **63** | 0.2 |
| **10** | 0.03 | **38** | 0.03 | **64** | 0.02 |
| **11** | 0.05 | 39 | 0.01 | **65** | 0.03 |
| **12** | 0.07 | 40 | 0.01 | 66 | 0.1 |
| **13** | 0.07 | **41** | 0.006 | 67 | 0.07 |
| **15** | 0.1 | **42** | 0.01 | **68** | 0.02 |
| **16** | 0.05 | **43** | 0.02 | **69** | 0.04 |
| **17** | 0.1 | **44** | 0.01 | **70** | 0.1 |
| **19** | 0.3 | **45** | 0.01 | **71** | 0.1 |
| **20** | 0.01 | **46** | 0.01 | **72** | 0.1 |
| **21** | 0.2 | **47** | 0.02 | **73** | 0.03 |
| **22** | 0.02 | **48** | 0.002 | **74** | 0.1 |
| **23** | 0.01 | **49** | 0.003 | **75** | 0.02 |
| **24** | 0.1 | **50** | 0.01 | **76** | 0.1 |
| **25** | 0.04 | **51** | 0.01 | **77** | 0.1 |
| **26** | 0.06 | **52** | 0.01 | **78** | 0.1 |
| **27** | 0.1 | **53** | 0.003 | **79** | 1.6 |
| **28** | 0.03 | **54** | 0.004 | **80** | 0.4 |
| | | | | **82** | 0.02 |

| | | | | | |
|---|---|---|---|---|---|
| Conclusion: The compounds in the examples of the present application exhibit good cell proliferation inhibitory activities against the NCI-H929 cells. | | | | | |

### Test Example 2. Protein degradation assay (In-cell Western)

NCI-H929 cells were inoculated into a 96-well plate at 100,000 cells per well and cultured overnight. Compounds at concentrations of 1600 nM, 400 nM, 100 nM, 25 nM, 6.25 nM, 1.56 nM, 0.39 nM, and 0.1 nM were dosed with duplicate wells, and the cells were incubated in an incubator at 37°C for 24 hours. After the incubation, the supernatant was removed by centrifugation, and 150 µL of 4% paraformaldehyde was added. The cells were incubated at room temperature for 20 minutes, then, 200 µL of a washing solution (0.1% TritonX-100, Beyotime; ST1722) was added and incubated at room temperature on a shaker. After repeating the washing four times, 150 µL of a Licor INTERCEPT blocking solution (Lico, Cat No. 927-70001) was added, and the cells were incubated on a shaker at room temperature for 1.5 hours. Antibodies IKZF1 (Cell Signaling; Cat No. 14859S), IKZF3 (Cell Signaling; Cat No. 15103S), GSPT1 (Proteintech, Cat No. 10763-1-AP), CK1α (Thermo; 7117067), and SALL4 (Abcam, ab22675) were diluted with an antibody dilution solution (Intercept Blocking Buffer, LI-COR, 211114) at 1:100 and 50 µL of each diluted primary antibody was added into the 96-well plate. The cells were incubated overnight on a shaker at 4°C. After the primary antibodies were removed and repeated washing was performed with PBST for 4 times, 50 µL of a diluted secondary antibody (Licor, Cat No. 926-68070) was added, and IRDye 800CW and IRDye 680CW were added at 0.5 µL simultaneously. The secondary antibody was sucked away, PBST was added, repeated washing was performed on a shaker at room temperature for 4 times, and a signal intensity was detected using an infrared laser imaging system (Lico, Odyssey-CLx).

**Table 2: Protein degradation activities in several examples**

| Example No. | In-cell Western IKZF 1 DC50(nM) | In-cell Western IKZF1 Dmax(%) | In-cell Western IKZF3 DC50(nM) | In-cell Western IKZF3 Dmax(%) |
|---|---|---|---|---|
| 1 | 0.31 | 75.8 | 0.12 | 71.4 |
| 3 | 0.081 | 73.6 | 0.050 | 76.6 |
| 4 | 0.38 | 73.6 | 0.19 | 70.3 |
| 5 | 0.23 | 68.4 | 0.12 | 73.8 |
| 21 | 8.86 | 57.1 | 0.22 | 74.1 |
| 22 | 0.10 | 65.3 | 0.056 | 76.4 |
| 23 | 0.077 | 65.8 | 0.036 | 77.1 |
| 25 | 0.98 | 60.0 | 0.075 | 76.2 |
| 30 | 0.37 | 74.1 | 0.093 | 73.2 |
| 31 | 0.34 | 77.0 | 0.16 | 75.0 |

| | | | | |
|---|---|---|---|---|
| Conclusion: The compounds in the examples of the present application exhibit good degradation effects on the target proteins IKZF1/3 and no obvious degradation effects on other non-target proteins such as GSPT1, CK1α, and SALL4 (for example, DC50 values determined for these proteins were greater than 10,000 nM) with good selectivity. | | | | |

### Test Example 3. IKZF1 protein degradation assay (HiBiT)

Degradation activities of compounds against a target protein were detected using a constructed K562-HiBiT-IKZF1 cell line (a HiBiT tag was added to the endogenous target protein IKZF1) (the cell line was constructed by WuXi AppTec). K562-HiBiT-IKZF1 cells were cultured in IMDM (GIBCO, 12440053) culture medium containing 10% of FBS. The cells in a logarithmic growth phase were plated at 10000 cells per well into a 96-well and cultured at 37°C, 5% CO₂ with 100% relative humidity. The compounds at 10 µM, 3.33 µM, 1.11 µM, 0.37 µM, 0.12 µM, 0.04 µM, 0.0137 µM, 0.0046 µM, and 0.0015 µM were dosed with 0.2% DMSO as the final concentration. The cells were cultured in an incubator for 24 h, and the detection was performed according to the instruction of a Promega Nano-Glo ^{®} HiBiT Lytic Detection System assay kit (Promega, Cat No. N3050). In Specific, the plate was placed at room temperature for 30 min. After removing the supernatant, 25 µL of PBS and 25 µL of a 2x detection reagent (1 mL of a lysis buffer + 20 µL of a Nano-Glo^{®} HiBiT lytic substrate + 10 µL of an LgBiT protein) were added into each well, then the plate was shaken on an orbital shaker at 300 rpm for 3 minutes. After standing 10 minutes, the HiBiT luminescence signal was detected on a 2104 EnVision plate reader (PerkinElmer). Target protein degradation rate (DR): DR (%) = (1-(RLU_{compound}-RLU_{blank control})/(RLU_{vehicle control}-RLU_{blank control}))×100%.

Conclusion: The compounds in the examples of the present application exhibit good degradation activities against the target protein IKZF1, and the compounds in some examples can achieve a degradation activity of up to 10 times that of compound CC-92480.

### Test Example 4. CRBN binding assay (HTRF)

Bonding activity between CRBN and compounds were detected by the HTRF competition assay using a CRBN binding kit (Cisbio, 64BDCRBNPEG). First, a reagent in the kit was equilibrated to room temperature and diluted to 1 fold with double-distilled water, and both the reagent and samples were uniformly mixed by thorough vortexing for later use. An europium-labeled specific GST antibody and an XL665-labeled thalidomide-red reagent (Cisbio, Cat No. 64BDCRBNPEG) were mixed in equal volumes in advance and then added into a 384-well white microplate at 10 µL per well. 5 µL of a GST-labeled CRBN WT protein (Cisbio, Cat No. 64BDCRBNPEG) was added to each well for uniform mixing, and then 5 µL of the compounds to be tested, a standard substance (provided in the kit), a positive control CC-92480, and a negative control (diluent) were respectively added, where concentrations of the compounds to be tested were respectively 10 µM, 3.33 µM, 1.11 µM, 0.37 µM, 0.12 µM, 0.04 µM, 0.013 µM, and 0.0044 µM, and concentrations of the standard substance were respectively 200 µM, 40 µM, 8 µM, 1.6 µM, 0.32 µM, 0.064 µM, and 0.0128 µM. After uniform mixing, contents in the 384-well plate were sealed and incubated in the dark at room temperature for 3 hours. The 384-well white microplate was placed in a microplate reader to detect fluorescence (ex: 320 nm, em: 620 nm/665 nm). HTRF ratio = (signal at 665 nm/signal at 620 nm)×10000, which represents the binding strength of each compound.

**Table 3: The IC50 of CRBN binding activity of several examples**

| **Example No.** | **HTRF CRBN IC50(nM)** |
|---|---|
| **CC-92480** | 108 |
| **21** | 34 |
| **22** | 20 |
| **23** | 19 |
| **25** | 38 |
| **30** | 27 |
| **37** | 8 |

| | |
|---|---|
| Conclusion: Compared with CC-92480, the compounds in the examples of the present application exhibit a good binding activities to CRBN. | |

### Test Example 5. Inhibitory activities of compounds on JJN3, RPMI-8226, TMD8, and Mino cells

JJN3, RPMI-8226, TMD8, and Mino cells were purchased from Nanjing Cobioer Biotechnology Co., Ltd. The RPMI-8226, TMD8, and Mino cells were cultured in RPMI1640 medium (Viva, Cat No. C3010-0500). The JJN3 cells were cultured in IMDM medium (Procell, Cat No. PM150510). All the media contained 10% of fetal bovine serum (Viva, Cat No. C04002-500) and 1% of penicillin-streptomycin. The cells were cultured in an incubator at 37°C, 5% CO₂ with saturated humidity conditions. The cells in logarithmic growth phase were were inoculated into a 96-well plate at 5000 cells per well and cultured overnight. Compounds at concentrations of 0.00512 nM, 0.0256 nM, 0.128 nM, 0.64 nM, 3.2 nM, 16 nM, 80 nM, and 400 nM were dosed with 0.2% DMSO as the final concentration. The cells were cultured in an incubator at 37°C, 5% CO₂ for 72 hours. After the incubation, the plate was placed at room temperature for half an hour, then 50 µL of Cell Titer-Glo^{®} (Promega, Cat No. G7573) was added, and the plate was placed on a shaker for uniform mixing for 2 minutes. Then, incubation was performed in the dark at room temperature for 10 minutes, and a luminance signal was measured using SpectraMax Paradigm (Molecular Devices). Cell activity inhibition rate (%) = 100-(RLU_{compound}-RLU_{blank})/(RLU_{control}-RLU_{blank})×100%, where control refers to the 0.2% DMSO treatment group, and blank refers to the culture medium control. IC50 values and maximum inhibition rates of the compounds were calculated using a nonlinear regression function "Dose-Response-Inhibition" in Graphpad Prism 7.0.

Conclusion: The compounds in the examples of the present application exhibit good cell proliferation inhibitory activities against the JJN3, RPMI-8226, TMD8, and Mino cells etc.

### Test Example 6. Liver microsome stability test

A phase I metabolic stability test was carried out by using an in vitro liver microsome incubation system to simulate physiological conditions of liver microsomes and adding a redox coenzyme NADPH for relevant reactions. First, 445 µL of a mouse or human liver microsome working solution (mouse: Cat No. M1000, Xenotech; human: Cat No. 452117, Corning) at a concentration of 0.56 mg/mL was separately added into 2 96-well plates, and named a T60 incubation plate (with addition of NADPH) and a non co-factor (NCF) 60 incubation plate (without addition of NADPH), respectively.

The above incubation plates were preincubated in a water bath at 37°C for approximately 10 minutes, and then 5 µL of test compounds or control compounds (testosterone, diclofenac, and propafenone) at a concentration of 100 µM were added. 50 µL of a potassium phosphate buffer was added into each well of the NCF60 incubation plate to initiate a reaction. One 96-well T0 termination plate was prepared, and 180 µL of a termination solution (acetonitrile solution containing 200 ng/mL tolbutamide and 200 ng/mL labetalol) and 6 µL of an NADPH regeneration system working solution (NADPH powder was dissolved in a 10 mM MgCl₂ solution to prepare a 10 mM NADPH solution) were added. 54 µL of a sample was taken out from the T60 incubation plate, and added into the T0 termination plate (to generate a T0 sample). 44 µL of an NADPH regeneration system working solution was added into each well of the T60 incubation plate to initiate a reaction. Only 54 µL of a microsome working solution, 6 µL of an NADPH regeneration system working solution, and 180 µL of a termination solution were added into a blank plate. Thus, in samples of the test compounds or control compounds, final reaction concentrations of the compounds, the testosterone, the diclofenac, and the propafenone were 1 µM, the concentration of the liver microsomes was 0.5 mg/mL, and final concentrations of DMSO and acetonitrile in the reaction system were 0.01% (v/v) and 0.99% (v/v), respectively. After incubation for an appropriate time (e.g., 5, 15, 30, 45, and 60 minutes), 180 µL of a termination solution (acetonitrile solution containing 200 ng/mL tolbutamide and 200 ng/mL labetalol) was added into each sample well of the termination plate, and then 60 µL of a sample was taken out from the T60 incubation plate to terminate the reaction. All the sample plates were uniformly mixed, and centrifuged at 3,220 g for 20 minutes. Then, 80 µL of a supernatant was taken from each well, and diluted with 240 µL of pure water for liquid chromatography-tandem mass spectrometry analysis.

Conclusion: The compounds in the examples of the present application have good liver microsome stability.

### Test Example 7. CYP450 enzyme inhibition test

This experiment was performed to determine the potential of compounds to inhibit isoenzymes (CYP1A2, CYP2C9, CYP2C19, CYP2D6, and CYP3A4) of human liver microsome cytochrome P450. First, human liver microsomes stored in a refrigerator at below -60°C were thawed on ice (Corning, Cat No. 452117). After complete dissolution, the human liver microsomes were diluted with a potassium phosphate buffer (PB) to prepare a working solution at a certain concentration (0.253 mg/mL). 20.0 µL of a substrate mixture (10 µM Phenacetin, 5 µM Diclofenac, 30 µM S-mephenytoin, 5 µM Dextromethorphan, and 2 µM Midazolam) was added into a reaction plate, then 158 µL of the human liver microsome working solution was added into the reaction plate, the reaction plate was placed on ice, and 2.00 µL of test compounds and specific inhibitors at various concentrations (5.00, 1.50, 0.500, 0.150, 0.0500, 0.0150, and 0.00500 mM) were added. After preincubated in a water bath at 37°C for 10 minutes, 20.0 µL of a coenzyme factor (10 mM NADPH) solution was added into the reaction plate, and the reaction plate was placed in a water bath for incubation at 37°C for 10 minutes. Then, 400 µL of a pre-cooled acetonitrile solution (an internal standard containing 200 ng/mL tolbutamide and labetalol) was added to terminate the reaction. The reaction plate was placed on a shaker and shaken for uniform mixing for 10 minutes. Then, centrifugation was performed at 4°C and 4,000 rpm for 20 minutes. 200 µL of a supernatant was taken and added into 100 µL of water for sample dilution. Finally, the plate was sealed and shaken for uniform mixing for 10 minutes, followed by LC/MS/MS detection.

Conclusion: The compounds in the examples of the present application do not have obvious inhibitory effects on isoenzymes of the human liver microsome cytochrome P450.

### Test Example 8. hERG potassium ion channel action test

An HEK293 cell line stably expressing an hERG ion channel (Item No. K1236) used in this experiment was purchased from Invitrogen. The cells were cultured in DMEM medium (Thermofisher, Cat No. 10569044) containing 10% of fetal bovine serum (Shanghai Bohan, Cat No. BS-0005-500). A small slide containing HEK293 cells was placed in a perfusion tank of a micromanipulation table, a tip end of a glass electrode was determined and placed at the center of the field of view, then the electrode was adjusted to approach the cells, and meanwhile, a micromanipulator was finely tuned to enable the electrode to gradually approach surfaces of the cells. In a voltage clamp mode, a transient capacitive current (C_{fast}) was compensated, and then a brief negative pressure was repeatedly applied to rupture a membrane to ultimately form a whole-cell recording mode. Under the condition that a membrane potential was clamped at -60 mV, a slow capacitive current (C_{slow}), a cell membrane capacitance (Cₘ), and an input membrane resistance (Ra) were compensated for, respectively. After the cells were stable, the clamp voltage was changed to -90 mV, a sampling frequency was set at 20 kHz, and a filter frequency was 10 kHz. A detection condition for a leak current was that the clamp voltage was changed to -80 mV for a duration of 500 ms. hERG current test method: a depolarization command voltage was applied for 4.8 seconds to depolarize the membrane potential from -80 mV to +30 mV, and then a repolarization voltage was immediately applied for 5.2 seconds to decrease the membrane potential to -50 mV to remove channel inactivation so as to observe an hERG tail current. A peak value of the tail current was the magnitude of an hERG current. The hERG current for detecting test compounds was continuously recorded for 120 seconds before administration to evaluate the stability of the hERG current generated by the test cells. Only the stable cells within an acceptable evaluation criteria range were allowed to enter subsequent compound testing. Test for inhibitory effects of the test compounds on the hERG current: first, the hERG current measured in an extracellular solution containing 0.1% DMSO was used as a detection baseline. After the hERG current remained stable for at least 5 minutes, solutions containing the test compounds were sequentially perfused around the cells from a low concentration to a high concentration. After each perfusion was completed, waiting was performed for approximately 5 minutes to enable the compounds to fully act on the cells, and the hERG current was simultaneously recorded. After the recorded current was stable, last 5 hERG current values were recorded, and their average value was used as a final current value at a specific concentration. After the compounds were tested, 450 nM dofetilide was added to a same cell to completely inhibit its current to serve as a positive control of the cell. Meanwhile, the positive compound dofetilide was synchronously tested using a same patch clamp system before and after a drug test assay to ensure reliability and sensitivity of an entire detection system. The above test steps were repeated on two separate test cells (n = 2).

Conclusion: The compounds in the examples of the present application do not have obvious inhibitory effects on the hERG potassium ion channel current.

### Test Example 9. In vitro proliferation inhibition test on PBMCs

PBMCs cells were purchased from Milecell Biotechnology Co., Ltd. The PBMCs cells were resuspended in RPMI-1640 (Solarbio, 11965) medium (containing 10% of FBS: Solarbio, S903)and cultured in an incubator for overnight at 37°C, 5% CO₂.After overnight incubation, the cells were stained with AOPI, and counted by a cell counter, in which a proportion of viable cells was required to be above 80%. the cells above were inoculated into a 96-well plate at 20,000 cells per well, and compounds at concentrations of 10,000 nM, 2,500 nM, 625 nM, 156.25 nM, 39 nM, 9.8 nM, 2.4 nM, 0.6 nM, and 0.15 nM were dosed with 0.2% DMSO as the final concentration. Each compound was tested in duplicate wells. After the 96-well plate was placed in an incubator for incubation at 37°C, 5% CO₂ for 72 hours, 50 µL of Cell Titer-Glo^{®} (Promega, Cat No. G7573) was added and uniformly mixed, then incubation was performed in the dark for 10 minutes, and a luminance signal value was read on SpectraMax Paradigm (Molecular Devices).

Conclusion: The compounds in the examples of the present application have no obvious inhibitory effects on the PBMCs.

### Test Example 10. Pharmacokinetic test on mice

Clear solutions of test compounds were administered into ICR mice (fasted overnight) by tail intravenous injection, or administered into ICR mice (fed) by gavage. Intravenous and oral administration doses were 1 mg/kg and 1 mg/kg, respectively. For the administration by intravenous injection, at 0 h (before the administration) and at 0.083, 0.25, 0.5, 1, 2, 4, 6, 8, and 24 h after the administration, 50 µL of blood was collected by cheek puncture and placed in anticoagulant tubes containing heparin sodium, and mixtures were mixed by thorough vortex at 4°C and centrifuged at 6,000 rpm for 3 minutes. For the administration by oral gavage, at 0 h (before the administration) and at 0.083, 0.25, 1, 2, 4, 6, 8, and 24 h after the administration, blood was collected by cheek puncture and placed in anticoagulant tubes containing heparin sodium, and mixtures were mixed by thorough vortex and centrifuged at 6,000 rpm for 3 minutes. Plasma drug concentrations were determined by an LC-MS/MS method, and relevant pharmacokinetic parameters were calculated using Phoenix WinNonlin 8.2.0 pharmacokinetic software by a linear log trapezoidal method with a non-compartmental model. An intravenous administration vehicle was 15% of DMA + 50% of PEG400 + 35% of D5W, or 10% of DMSO + 15% of Solutol + 75% of Saline. An oral (gavage) administration vehicle was 0.25% of Tween 80 + 99.75% (0.5% CMC aqueous solution) or 10% of DMSO + 15% of Solutol + 75% of Saline.

Conclusion: The compounds in the examples of the present application have good oral absorption in the mice.

### Test Example 11. Pharmacokinetic test on Cynomolgus macaques

Washed-out Cynomolgus macaques (non-naive) used in this experiment were provided by Shanghai ChemPartner. The animals were at least 3 years old and were male with a body weight of 3-5 kg, and the body weights of all the animals used in the experiment were within ±20% of an average body weight of their respective gender. All the Cynomolgus macaques were fasted overnight before administration. Intravenous and oral administration doses were 0.1 mg/kg and 1 mg/kg, respectively. At 0.083, 0.25, 0.5, 1, 2, 4, 8, and 24 h after the administration, approximately 300 µL of blood samples were collected by puncture from cephalic veins of forelimbs or saphenous veins of hind limbs into anticoagulant centrifuge tubes containing K2-EDTA, and the samples were centrifuged (at 3,000 g and 4°C for 5 minutes) within 15 minutes after collection to obtain plasma. Concentrations of compounds in the plasma of the Cynomolgus macaques were determined by an LC-MS/MS internal standard method, data analysis was performed using WinNonlin 8.2 software, and pharmacokinetic parameters, such as a peak concentration (Cₘₐₓ), a time to peak (Tₘₐₓ), a half-life (t_{1/2}), a clearance rate (Cl), a steady-state apparent volume of distribution (Vss), an area under a concentration-time curve (AUC), a mean residence time (MRT), and a bioavailability (F), were calculated using a non-compartmental model. An intravenous administration vehicle was 10% of DMSO + 15% of Solutol + 75% of Saline. An oral (gavage) administration vehicle was 10% of DMSO + 15% of Solutol + 75% of Saline.

Conclusion: The compounds in the examples of the present application have good oral absorption in the Cynomolgus macaques.

### Test Example 12. In vivo efficacy experiment on mice

NOD/SCID mice, used in this experiment were provided by Beijing Vital River Laboratory Animal Technology Co., Ltd., were female and were 6-8 weeks old with a body weight of 18-22 g. After arrival, the animals were quarantined and acclimated in an experimental environment for 7 days before starting the experiment. NCI-H929 cells in logarithmic growth phase (added with matrigel, Corning, BD356230, at a volume ratio of 1:1) were subcutaneously inoculated into right backs of the mice. When an average tumor volume reached approximately 100-150 mm³, grouping and administration were performed. Oral QD administration was performed continuously for 21 days, and tumor measurement and weighing were performed for 3 times per week, where the tumor volume was measured using a vernier caliper. A formula is TV = 0.5×a×b², where a is a longest diameter of a tumor, and b is a shortest diameter of the tumor. The tumor weight was measured at the end of the experiment. Antitumor efficacy of the compounds was evaluated by TGI (%). TGI (%) reflects a tumor growth inhibition rate, where TGI (%) = [(1-(mean tumor volume at end of administration in a treatment group-mean tumor volume at start of administration in the treatment group)/(mean tumor volume at end of treatment in a vehicle control group-mean tumor volume at start of treatment in the vehicle control group))]×100%.

Conclusion: The compounds in the examples of the present application have good inhibitory effects on growth of an NCI-H929 transplantation tumor model of the mice.

The above examples are merely illustrative of the embodiments of the present application. However, the protection scope of the present application is not limited to the above embodiments and examples. Any modifications, equivalent substitutions, improvements, and the like made within the spirit and scope of the present application should be included within the protection scope of the present application.

## Claims

1. A compound of formula (I), a pharmaceutically acceptable salt thereof, a deuterated compound thereof, a stereoisomer thereof, a tautomer thereof, or a mixture thereof: 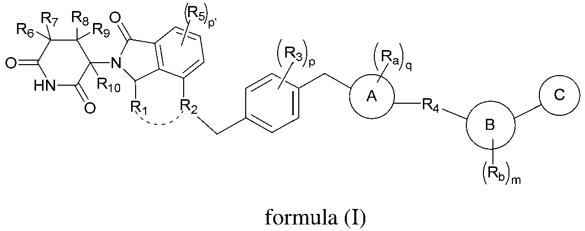 wherein,
ring A is selected from 3-10 membered monocyclic heterocyclyl, 6-13 membered bridged heterocyclyl, or 6-13 membered fused heterocyclyl;
ring B is selected from 5-6 membered heteroaryl or 5-6 membered heterocyclyl;
ring C is selected from optionally substituted 5-10 membered heteroaryl or optionally substituted 5-10 membered heterocyclyl;
a dashed line between R₁ and R₂ represents connection or disconnection;
when R₁ and R₂ are disconnected, R₁ is selected from H or D, and R₂ is selected from O, NH, or S; when R₁ and R₂ are connected, R₁ and R₂, together with the carbon atoms to which they are attached, form an optionally substituted 5-6 membered aromatic ring;
R₃ is selected from H, D, or halogen;
Rₐ is each independently selected from H, C₁-C₆ alkyl, cyano, C₁-C₆ haloalkyl, or C₃-C₁₀ cycloalkyl, wherein the C₁-C₆ alkyl and C₃-C₁₀ cycloalkyl are each optionally substituted with one or more of H, halogen, hydroxy, or cyano; or two Rₐ attached to the same carbon atom, together with the carbon atom to which they are attached, form C₃-C₆ cycloalkyl or 4-6 membered heterocyclyl;
R₄ is selected from a single bond, -C(O)-, -NR₁₁C(O)-, -NR₁₁-, -CR₁₁R₁₂, -CR₁₁R₁₂-(4-6 membered heterocyclyl)-, 4-6 membered heterocyclyl, -(C₃-C₆ cycloalkyl)-NR₁₁C(O)-, or -O-(C₅-C₆ cycloalkyl)-NR₁₁C(O)-, wherein the heterocyclyl and cycloalkyl are each optionally substituted with one or more selected from H, halogen, C₁-C₆ alkyl, or C₁-C₆ haloalkyl; or R₄ and any one Rₐ, together with the atoms to which they are attached, form C₃-C₈ cycloalkyl or 4-6 membered heterocyclyl;
R₅ is selected from H, C₁-C₆ alkyl, C₁-C₆ haloalkyl, or halogen;
R₆, R₇, R₈, R₉, and R₁₀ are each independently selected from H or D;
R₁₁ and R₁₂ are each independently selected from H or C₁-C₆ alkyl; or R₁₁ and R₁₂, together with the carbon atoms to which they are attached, form C₃-C₈ cycloalkyl or 4-6 membered heterocyclyl;
R_{b} is selected from H, halogen, C₁-C₆ alkyl, C₁-C₃ haloalkyl, C₃-C₆ cycloalkyl, C₁-C₆ alkoxy, optionally substituted amino, or cyano; or two adjacent R_{b}, together with the carbon atoms to which they are attached respectively, form C₃-C₆ cycloalkyl, 4-6 membered heterocyclyl, 5-6 membered heteroaryl, or 5-6 membered aryl;
m is selected from 0, 1, 2, or 3;
p is selected from 0, 1, 2, 3, or 4;
p' is selected from 0, 1, 2, or 3; and
q is selected from 0, 1, 2, 3, or 4.

2. The compound, the pharmaceutically acceptable salt thereof, the deuterated compound thereof, the stereoisomer thereof, the tautomer thereof, or the mixture thereof according to claim 1, wherein the ring A is selected from 4-6 membered monocyclic heterocyclyl or 6-8 membered bridged heterocyclyl;
preferably, the ring A is selected from 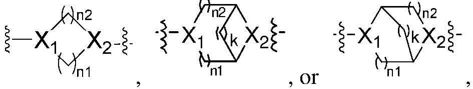
wherein
at least one of X₁ and X₂ is N, and the other one is optionally C or N; and
n1, n2, and k are each independently selected from 1 or 2; and
further preferably, the ring A is selected from

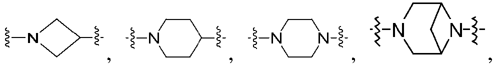

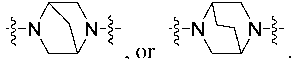

3. The compound, the pharmaceutically acceptable salt thereof, the deuterated compound thereof, the stereoisomer thereof, the tautomer thereof, or the mixture thereof according to claim 1 or 2, wherein Rₐ is each independently selected from H, C₁-C₆ alkyl, C₁-C₆ haloalkyl, or C₃-C₆ cycloalkyl, wherein the C₁-C₆ alkyl is optionally substituted with one or more of H, halogen, hydroxy, or cyano; or two Rₐ attached to the same carbon atom, together with the carbon atom to which they are attached, form C₃-C₆ cycloalkyl;
preferably, Rₐ is each independently selected from H, C₁-C₃ alkyl, or C₁-C₃ haloalkyl, wherein the C₁-C₃ alkyl or C₁-C₃ haloalkyl is each optionally substituted with one or more of H, hydroxy, or cyano; Rₐ is further preferably selected from H, -CH₂OH, -CH₂CH₂OH, -CN, or -CH₂CN; and
preferably, two Rₐ attached to the same carbon atom, together with the atom to which they are attached, form C₃-C₆ cycloalkyl, and further preferably form cyclopropyl.

4. The compound, the pharmaceutically acceptable salt thereof, the deuterated compound thereof, the stereoisomer thereof, the tautomer thereof, or the mixture thereof according to any one of claims 1-3, wherein the ring B is selected from 6-membered heteroaryl;
preferably, the ring B is selected from 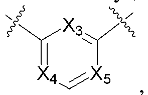
wherein X₃, X₄, and X₅ are each independently selected from C or N; and at least one of X₃, X₄, and X₅ is N; and
further preferably, the ring B is selected from

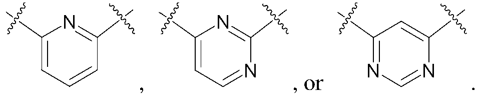

5. The compound, the pharmaceutically acceptable salt thereof, the deuterated compound thereof, the stereoisomer thereof, the tautomer thereof, or the mixture thereof according to any one of claims 1-4, wherein R_{b} is each independently selected from H, halogen, C₁-C₆ alkyl, C₁-C₃ haloalkyl, C₃-C₆ cycloalkyl, C₁-C₆ alkoxy, optionally substituted amino, or cyano; and m is selected from 0, 1, 2, or 3; and
preferably, R_{b} is each independently selected from H, F, Cl, methyl, ethyl, isopropyl, cyclopropyl, or trifluoromethyl; and m is selected from 0 or 1.

6. The compound, the pharmaceutically acceptable salt thereof, the deuterated compound thereof, the stereoisomer thereof, the tautomer thereof, or the mixture thereof according to any one of claims 1-5, wherein the ring C is selected from optionally substituted 5-6 membered heteroaryl or optionally substituted 5-6 membered heterocyclyl;
preferably, the ring C is selected from 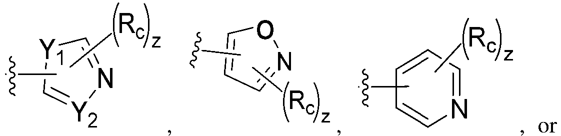 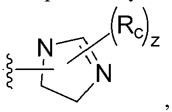 wherein
R_{c} is independently selected from H, C₁-C₆ alkyl, C₃-C₆ cycloalkyl, C₁-C₃ haloalkyl, halogen, -C(O)R₁₃, or cyano; z is selected from 0, 1, 2, 3, or 4; Y₁ is selected from S or N; Y₂ is selected from C or N; and R₁₃ is selected from H, NH₂, C₁-C₃ alkyl, C₃-C₆ cycloalkyl, or C₁-C₃ haloalkyl;
further preferably, the ring C is selected from 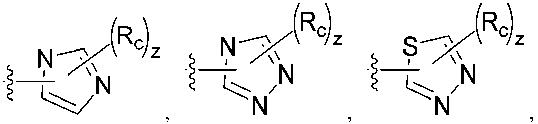 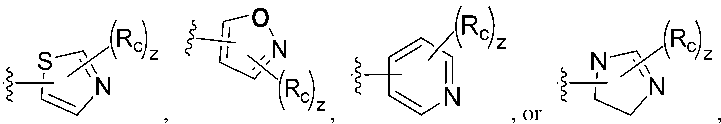 wherein
R_{c} is independently selected from H, methyl, ethyl, isopropyl, cyclopropyl, trifluoromethyl, F, Cl, -C(O)NH₂, or cyano; and z is selected from 0, 1, or 2; and
furthermore preferably, the ring C is selected from 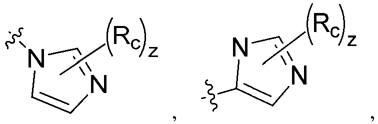 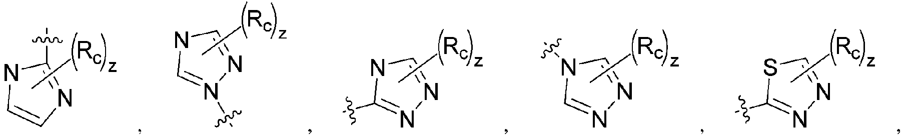 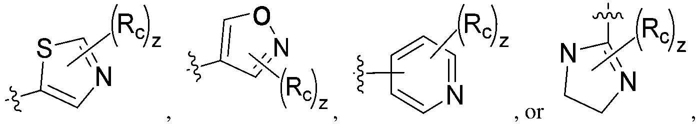 wherein
R_{c} is independently selected from H, methyl, ethyl, isopropyl, cyclopropyl, trifluoromethyl, F, Cl, -C(O)NH₂, or cyano; and z is selected from 0, 1, or 2.

7. The compound, the pharmaceutically acceptable salt thereof, the deuterated compound thereof, the stereoisomer thereof, the tautomer thereof, or the mixture thereof according to any one of claims 1-6, wherein R₄ is selected from a single bond, -C(O)-, -NHC(O)-, -NH-, -CH₂-(5-6 membered heterocyclyl)-, 5-6 membered heterocyclyl, -(C₅-C₆ cycloalkyl)-NHC(O)-, or -O-(C₅-C₆ cycloalkyl)-NHC(O)-;
preferably, R₄ is selected from a single bond, -C(O)-, -NHC(O)-, -NH-, 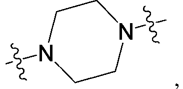 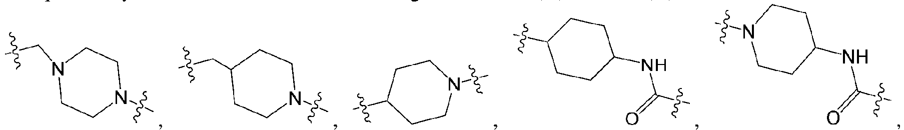 or 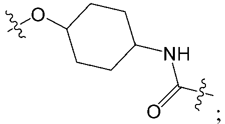 and
further preferably, R₄ is selected from a single bond.

8. The compound, the pharmaceutically acceptable salt thereof, the deuterated compound thereof, the stereoisomer thereof, the tautomer thereof, or the mixture thereof according to any one of claims 1-7, wherein the compound of formula (I) is a compound of the following general formula:

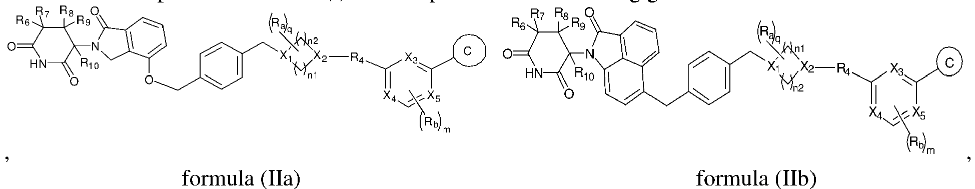

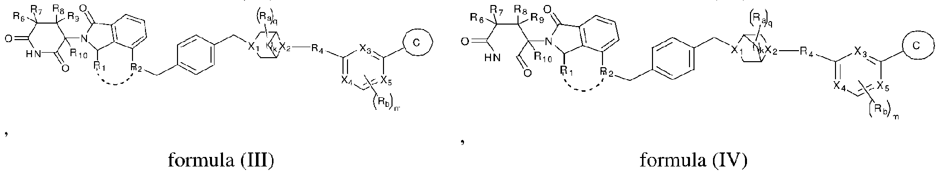

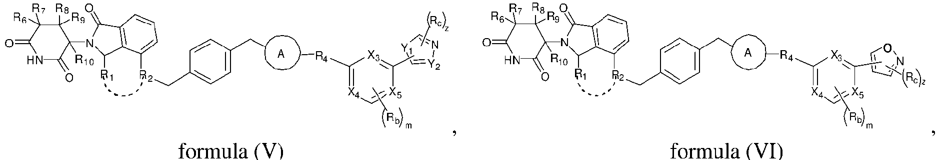

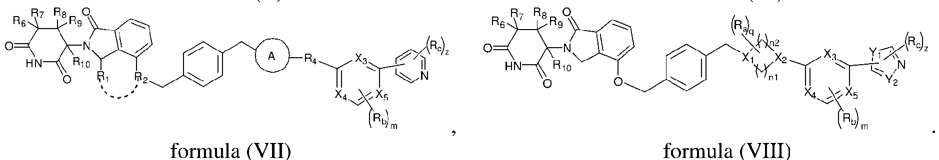

9. The compound, the pharmaceutically acceptable salt thereof, the deuterated compound thereof, the stereoisomer thereof, the tautomer thereof, or the mixture thereof according to claim 1, wherein the ring A is selected from 4-8 membered monocyclic heterocycloalkyl or 6-10 membered bridged heterocycloalkyl containing 1-3 heteroatoms selected from N, O, or S;
the ring B is selected from 5-6 membered heteroaryl containing 1-3 heteroatoms selected from N, O, or S;
the ring C is selected from 5-6 membered heteroaryl or 5-6 membered heterocyclyl containing 1-3 heteroatoms selected from N, O, or S, wherein the 5-6 membered heteroaryl or 5-6 membered heterocyclyl is optionally substituted with one or more selected from H, C₁-C₆ alkyl, C₃-C₆ cycloalkyl, C₁-C₃ haloalkyl, halogen, -C(O)R₁₃, or cyano, wherein R₁₃ is selected from H, NH₂, C₁-C₃ alkyl, C₃-C₆ cycloalkyl, or C₁-C₃ haloalkyl;
the dashed line between R₁ and R₂ represents connection or disconnection;
when R₁ and R₂ are disconnected, R₁ is selected from H or D, and R₂ is selected from O or S;
when R₁ and R₂ are connected, R₁ and R₂, together with the carbon atoms to which they are attached, form a benzene ring;
R₃ is selected from H or D;
Rₐ is each independently selected from H, C₁-C₆ alkyl, C₁-C₆ haloalkyl, or C₃-C₆ cycloalkyl, wherein the C₁-C₆ alkyl is optionally substituted with one or more of H, halogen, hydroxy, or cyano; or two Rₐ attached to the same carbon atom, together with the carbon atom to which they are attached, form C₃-C₆ cycloalkyl;
R₄ is selected from a single bond, -C(O)-, -NR₁₁C(O)-, -NR₁₁-, -CR₁₁R₁₂, -CR₁₁R₁₂-(4-6 membered heterocycloalkyl)-, 4-6 membered heterocycloalkyl, -(C₃-C₆ cycloalkyl)-NR₁₁C(O)-, or -O-(C₅-C₆ cycloalkyl)-NR₁₁C(O)-, wherein the heterocycloalkyl or cycloalkyl is optionally substituted with one or more selected from H or C₁-C₃ alkyl; the heterocycloalkyl comprises 1-3 heteroatoms selected from N, O, or S; and R₁₁ and R₁₂ are each independently selected from H or C₁-C₃ alkyl;
R₅ is H;
R₆, R₇, R₈, R₉, and R₁₀ are each independently H;
R_{b} is each independently selected from H, halogen, C₁-C₆ alkyl, C₁-C₃ haloalkyl, C₃-C₆ cycloalkyl, amino, or cyano;
m is selected from 0, 1, or 2;
p is selected from 0, 1, or 2;
p' is selected from 0, 1, or 2; and
q is selected from 0, 1, or 2.

10. The compound, the pharmaceutically acceptable salt thereof, the deuterated compound thereof, the stereoisomer thereof, the tautomer thereof, or the mixture thereof according to claim 1, wherein the ring A is selected from 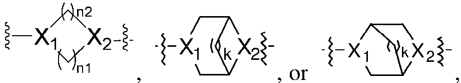 wherein at least one of X₁ and X₂ is N, and the other one is optionally C or N; and n1, n2, and k are each independently selected from 1 or 2;
the ring B is selected from 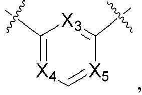
wherein X₃, X₄, and X₅ are each independently selected from C or N; and at least one of X₃, X₄, and X₅ is N;
the ring C is selected from 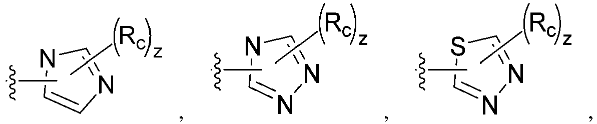 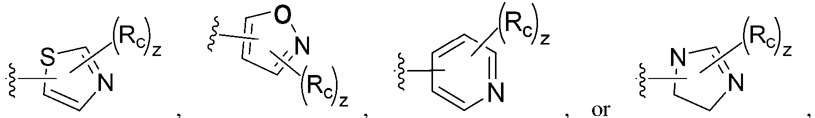 wherein R_{c} is independently selected from H, C₁-C₆ alkyl, C₃-C₆ cycloalkyl, C₁-C₃ haloalkyl, halogen, -C(O)R₁₃, or cyano; z is selected from 0, 1, 2, 3, or 4; and R₁₃ is selected from H, NH₂, C₁-C₃ alkyl, C₃-C₆ cycloalkyl, or C₁-C₃ haloalkyl;
the dashed line between R₁ and R₂ represents connection or disconnection;
when R₁ and R₂ are disconnected, R₁ is selected from H or D, and R₂ is selected from O or S;
when R₁ and R₂ are connected, R₁ and R₂, together with the carbon atoms to which they are attached, form a benzene ring;
R₃ is selected from H or D;
Rₐ is each independently selected from H or C₁-C₆ alkyl, wherein the C₁-C₆ alkyl is optionally substituted with one or more of hydroxy or cyano; or two Rₐ attached to the same carbon atom, together with the carbon atom to which they are attached, form C₃-C₆ cycloalkyl;
R₄ is selected from a single bond, -C(O)-, -NHC(O)-, -NH-, -CH₂-(5-6 membered heterocycloalkyl)-, 5-6 membered heterocycloalkyl, -(C₅-C₆ cycloalkyl)-NHC(O)-, or -O-(C₅-C₆ cycloalkyl)-NR₁₁C(O)-, wherein the heterocycloalkyl comprises 1-3 heteroatoms selected from N, O, or S, and R₁₁ is selected from H or C₁-C₃ alkyl;
R₅ is H;
R₆, R₇, R₈, R₉, and R₁₀ are each independently H;
R_{b} is each independently selected from H, F, Cl, Br, methyl, ethyl, n-propyl, isopropyl, cyclopropyl, fluoromethyl, fluoroethyl, fluoropropyl, chloromethyl, chloroethyl, chloropropyl, bromomethyl, bromoethyl, or bromopropyl;
m is selected from 0 or 1;
p is selected from 0, 1, or 2;
p' is selected from 0, 1, or 2; and
q is selected from 0, 1, or 2.

11. The compound, the pharmaceutically acceptable salt thereof, the deuterated compound thereof, the stereoisomer thereof, the tautomer thereof, or the mixture thereof according to any one of claims 1-10, wherein the compound is:

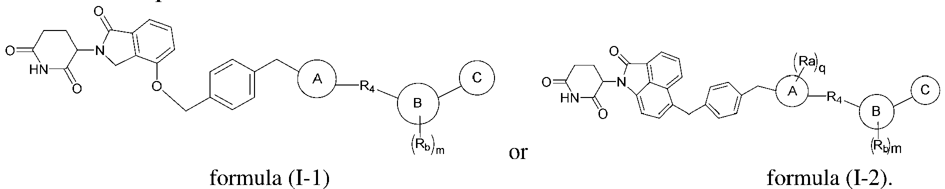

12. The compound, the pharmaceutically acceptable salt thereof, the deuterated compound thereof, the stereoisomer thereof, the tautomer thereof, or the mixture thereof according to any one of claims 1-11, wherein the compound is selected from:

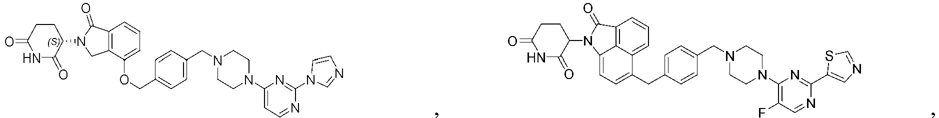

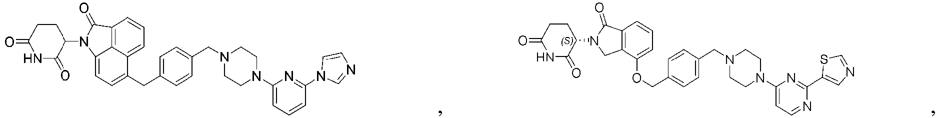

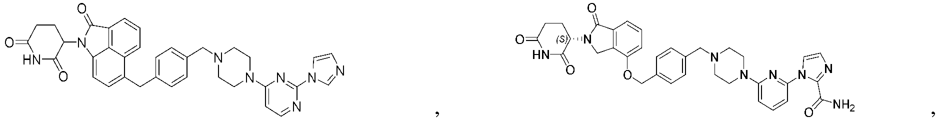

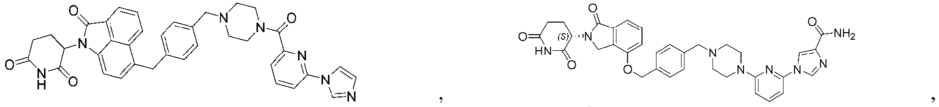

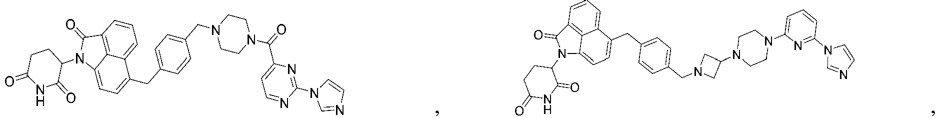

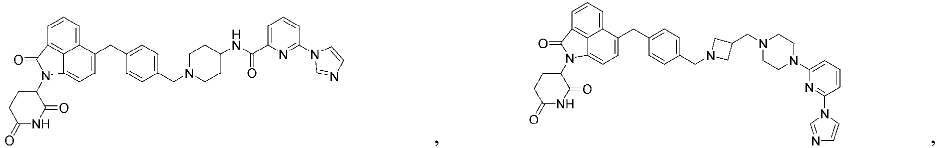

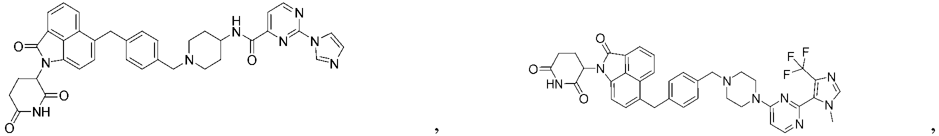

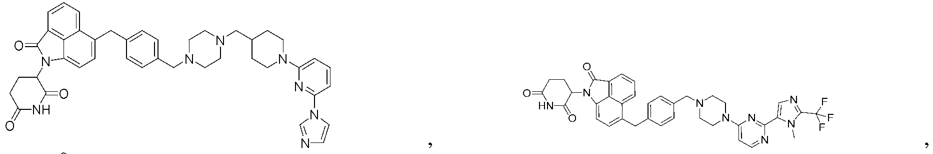

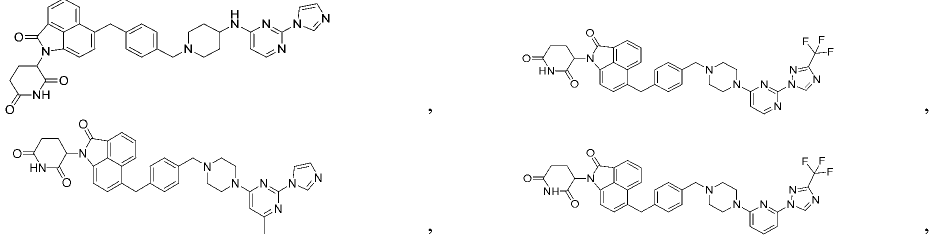

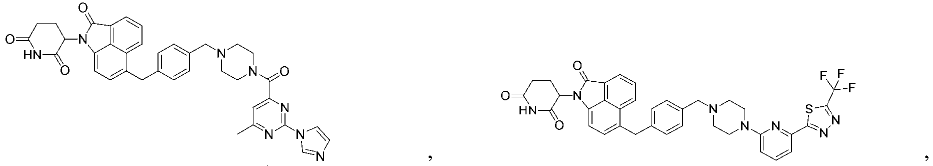

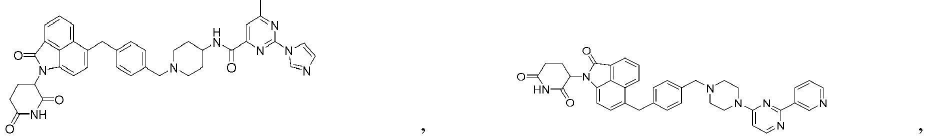

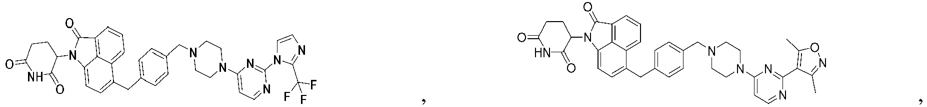

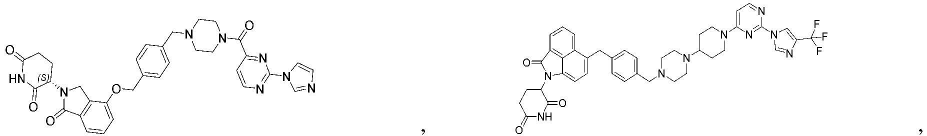

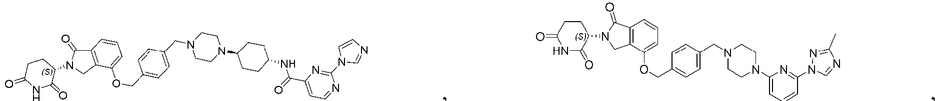

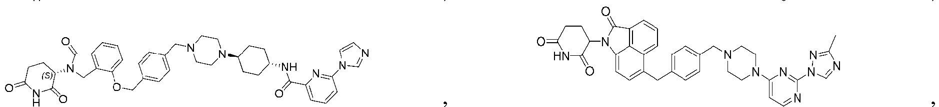

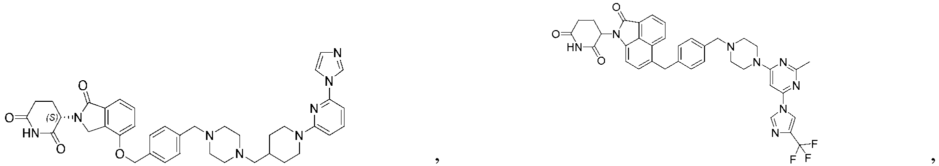

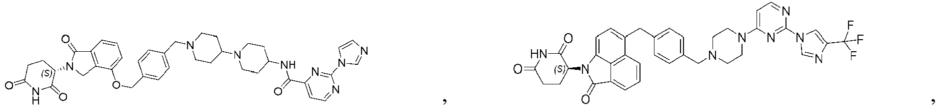

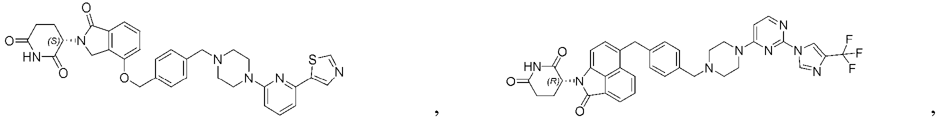

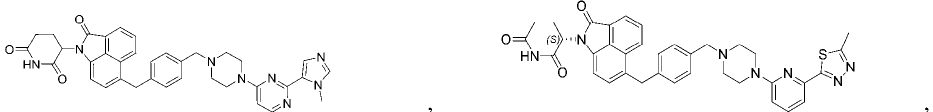

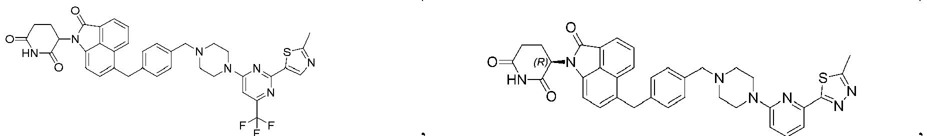

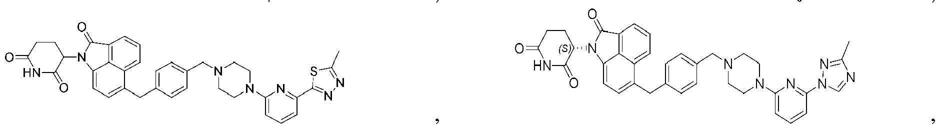

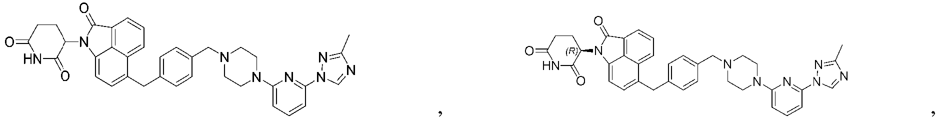

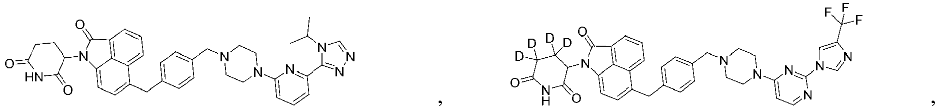

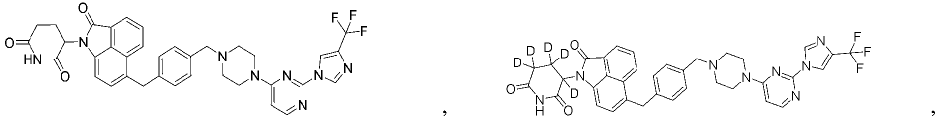

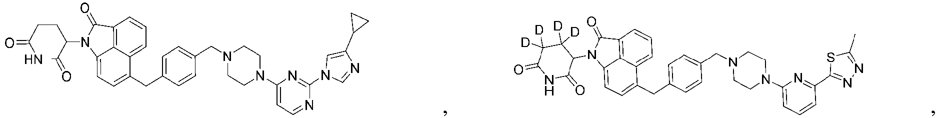

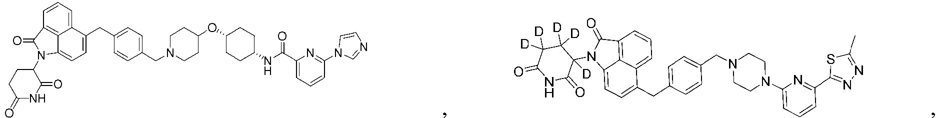

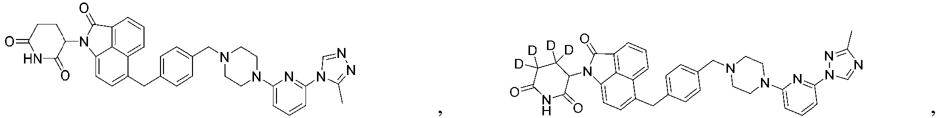

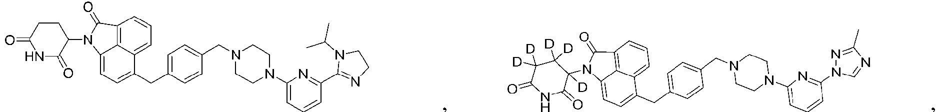

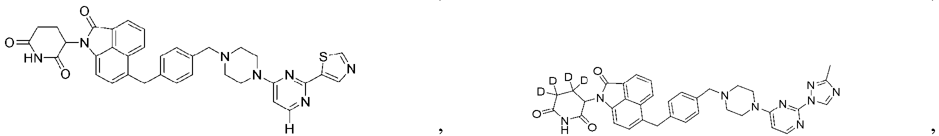

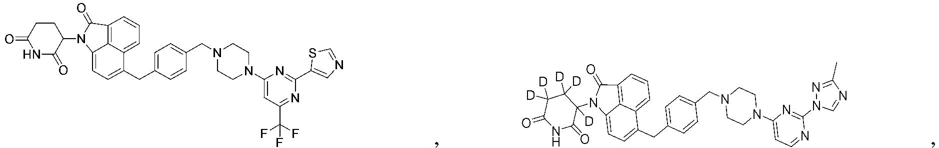

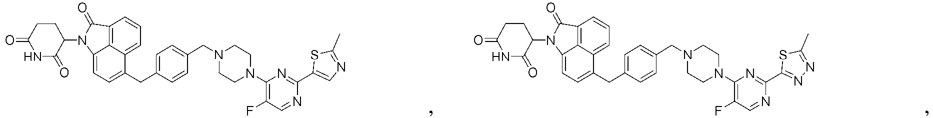

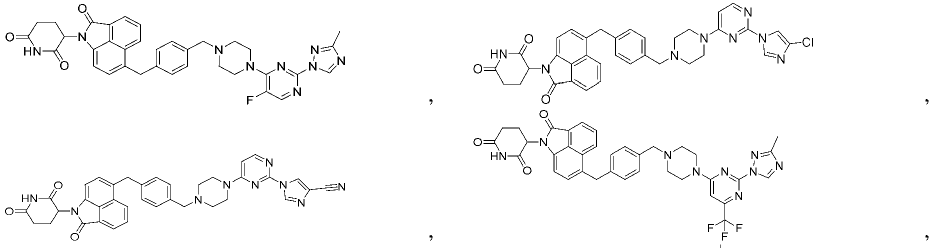

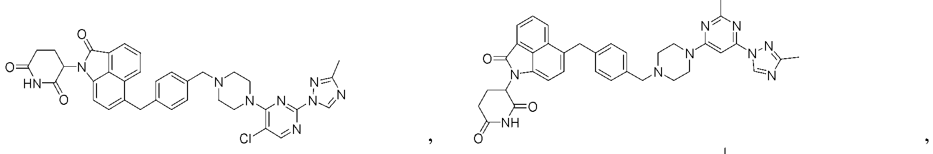

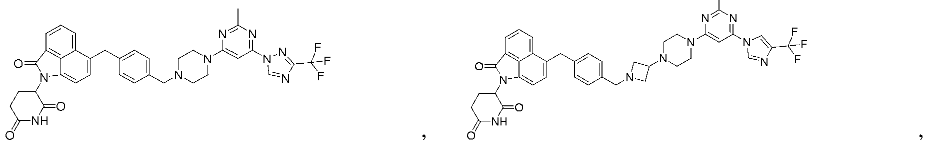

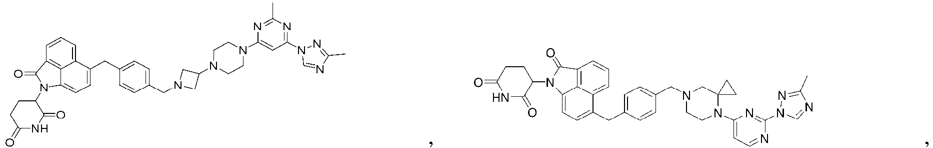

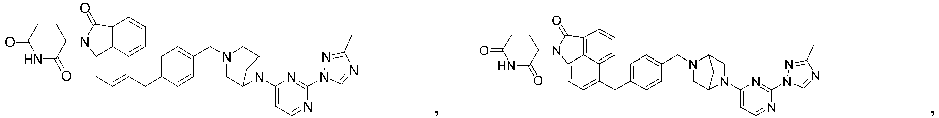

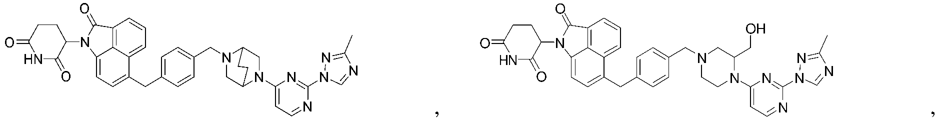

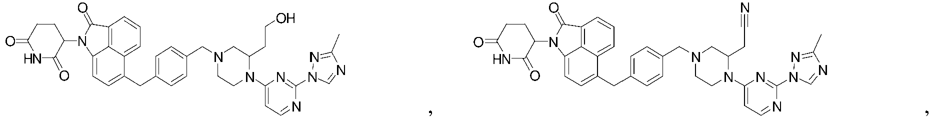

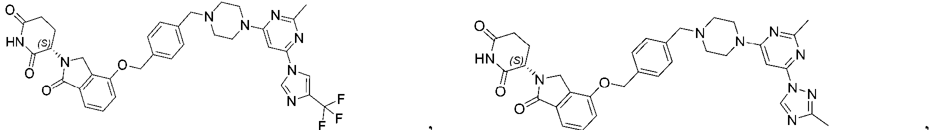

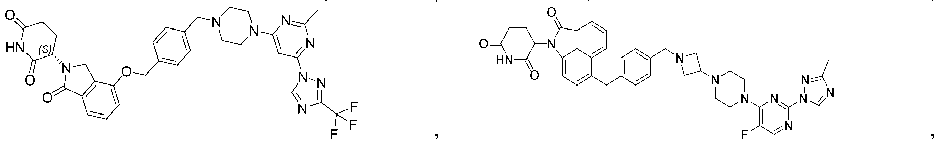

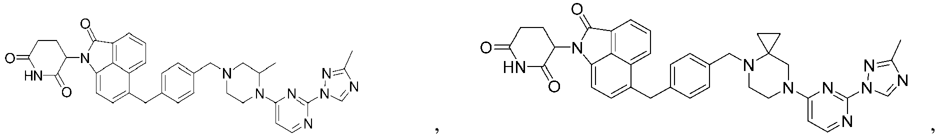

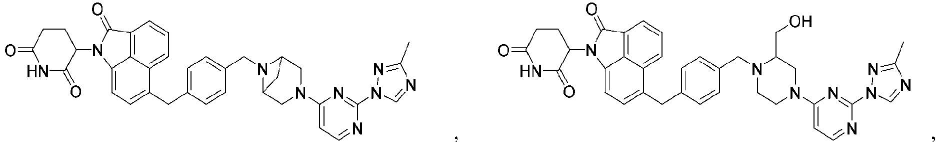

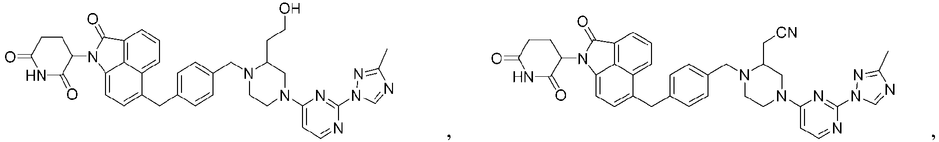

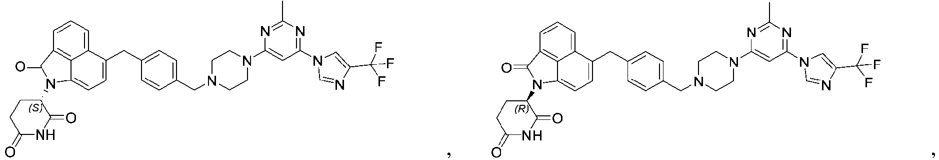

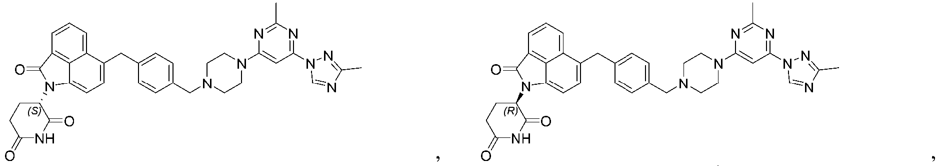

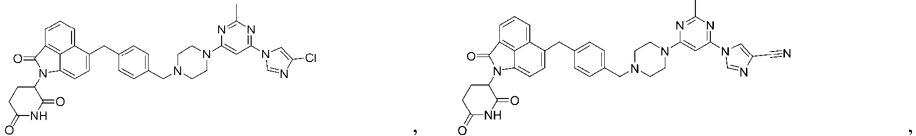

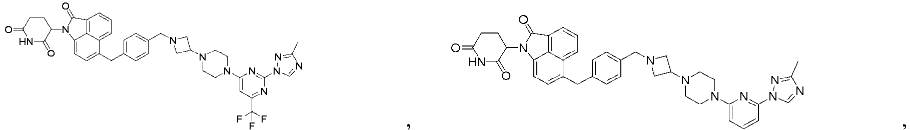

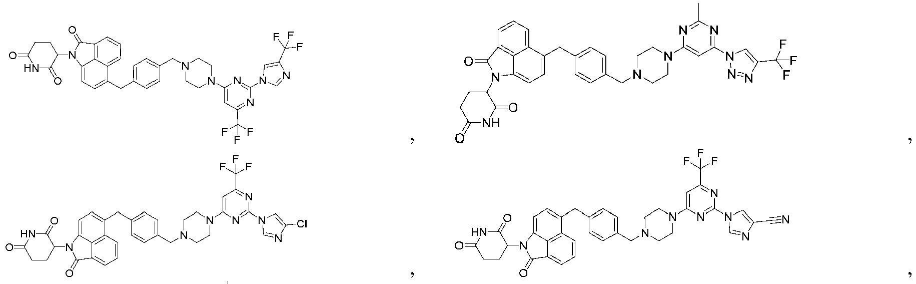

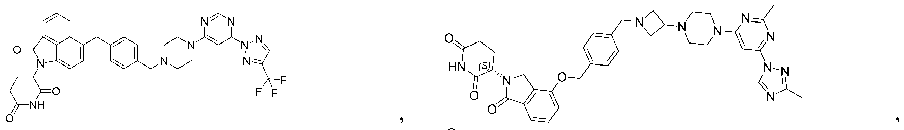

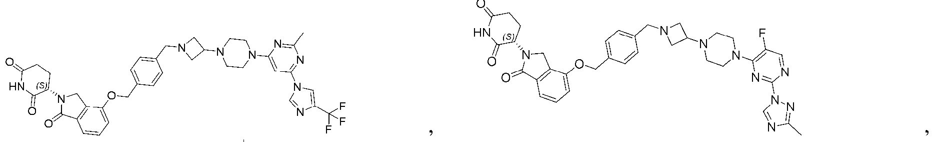

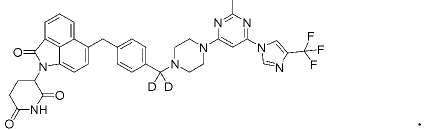

13. The compound, the pharmaceutically acceptable salt thereof, the deuterated compound thereof, the stereoisomer thereof, the tautomer thereof, or the mixture thereof according to any one of claims 1-12 for use in preventing and/or treating a cereblon-mediated disease, wherein
preferably, the disease is cancer, a tumor, an immune disease, or an inflammatory disease;
preferably, the immune disease is an autoimmune disease;
preferably, the disease is a hematological malignancy; and
preferably, the disease is multiple myeloma, leukemia, lymphocytic leukemia, chronic lymphocytic leukemia, Hodgkin's lymphoma, or non-Hodgkin's lymphoma.

14. The compound, the deuterated compound thereof, the stereoisomer thereof, the tautomer thereof, or the mixture thereof for use according to claim 13, wherein the disease is mediated by IKZF1 and/or IKZF3.

15. The compound, the pharmaceutically acceptable salt thereof, the deuterated compound thereof, the stereoisomer thereof, the tautomer thereof, or the mixture thereof according to any one of claims 1-12 for use in the immunomodulation of dual targets of IKZF1 and IKZF3.
